(19) Europäisches Patentamt
European Patent Office
Office européen des brevets

(11) **EP 4 059 505 B1**

(12) **EUROPEAN PATENT SPECIFICATION**

(45) Date of publication and mention of the grant of the patent:
**21.02.2024 Bulletin 2024/08**

(21) Application number: **20886910.7**

(22) Date of filing: **13.11.2020**

(51) International Patent Classification (IPC):
***A61K 31/7088*** *(2006.01)* ***A61K 9/51*** *(2006.01)*
***A61K 9/00*** *(2006.01)* ***A61K 48/00*** *(2006.01)*
***C12N 15/113*** *(2010.01)* ***C12N 15/88*** *(2006.01)*
***C12N 15/11*** *(2006.01)*

(52) Cooperative Patent Classification (CPC):
**A61K 9/5123; A61K 9/0019; A61K 31/7088; A61K 48/0041; C12N 15/88;** C12N 15/111; C12N 15/113; C12N 2320/32

(86) International application number:
**PCT/JP2020/042513**

(87) International publication number:
**WO 2021/095876 (20.05.2021 Gazette 2021/20)**

(54) **LIPID COMPOSITION**

FETTZUSAMMENSETZUNG

COMPOSITION LIPIDIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **15.11.2019 JP 2019207118**

(43) Date of publication of application:
**21.09.2022 Bulletin 2022/38**

(60) Divisional application:
**24150973.6**

(73) Proprietor: **FUJIFILM Corporation**
**Tokyo 106-8620 (JP)**

(72) Inventors:
- **SEKIGUCHI, Takahiro**
**Ashigarakami-gun, Kanagawa 258-8577 (JP)**
- **ENDO, Taisuke**
**Ashigarakami-gun, Kanagawa 258-8577 (JP)**
- **KANEUMI, Shun**
**Ashigarakami-gun, Kanagawa 258-8577 (JP)**
- **NORO, Masaki**
**Ashigarakami-gun, Kanagawa 258-8577 (JP)**
- **TANABE, Shintaro**
**Ashigarakami-gun, Kanagawa 258-8577 (JP)**
- **YAMAMOTO, Masahiko**
**Ashigarakami-gun, Kanagawa 258-8577 (JP)**

(74) Representative: **Klunker IP Patentanwälte PartG mbB**
**Destouchesstraße 68**
**80796 München (DE)**

(56) References cited:
**WO-A1-2015/095340**
**WO-A1-2015/095346**
**WO-A1-2018/087753**
**WO-A1-2019/235635**
**WO-A1-2020/246581**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 059 505 B1

**Description**

Field of the Invention

The present invention relates to a lipid composition containing lipids and a nucleic acid.

Description of the Related Art

With the development of techniques enabling the delivery of nucleic acids to cells, nucleic acid drugs have been actively developed. As one of the nucleic acid delivery techniques, a method of administering nucleic acid-containing particles consisting of particles (liposomes or lipid particles) encapsulating nucleic acids is known. In this technique, the nucleic acid-containing particles are prepared using lipids that have an amino group or the like and turn into cations at a low pH, and appropriate charge is applied to the particles for the delivery of nucleic acids. For example, as a compound to be incorporated into the lipid particles, Patent Document 1 discloses a compound having an ester group, an acetal group, or the like as a linking group that links an aliphatic group to an amino group. Patent Document 2 discloses a compound having a vinyloxy group, an amide group, an oxime group, or the like as a linking group that links an aliphatic group to an amino group. In the present specification, the aforementioned lipids that have an amino group or the like and turn into a cation at a low pH are called a cationic lipid in some cases.

In addition, Patent Document 3 describes cationic lipids for delivering biologically active agents to cells and tissues. Patent Document 4 describes lipid nanoparticles containing a compound called DLin-MC3-DMA as a cationic lipid.

There are studies on changing type and compositional ratio of lipid compounds used for manufacturing nucleic acid-containing particles. Patent Document 5 describes nucleic acid-liquid particles containing (a) a nucleic acid; (b) a cationic lipid that constitutes about 50 mol% to about 85 mol% of the total lipids present in the particles; (c) a non-cationic lipid that constitutes about 13 mol% to about 49.5 mol% of the total lipids present in the particles; and (d) a composite lipid that constitutes about 0.5 mol% to about 2 mol% of the total lipids present in the particles and inhibits the aggregation of the particles. Patent Document 6 describes a lipid preparation containing 40% to 65% of a cationic lipid having a specific structure, 5% to 10% of a neutral lipid, 25% to 40% of sterol, and 0.5% to 10% of PEG or a PEG-modified lipid.

Prior Art Documents

Patent Documents

Patent Document 1: WO2010/054401A
Patent Document 2: WO2010/054405A
Patent Document 3: WO2015/095340A
Patent Document 4: US2013-0245107A
Patent Document 5: WO2009/127060A
Patent Document 6: WO2010/144740A

**SUMMARY OF THE INVENTION**

As described above, there are reports of lipid compositions containing lipids and nucleic acids, but lipid composition capable of delivering a wide variety of nucleic acids is desired. In addition, because the lipids having an amino group are known to have toxicity, there is a demand for a technique enabling more efficient delivery of nucleic acids.

The present invention has been made in consideration of the above circumstances, and an object thereof is to provide a lipid composition capable of achieving excellent nucleic acid delivery for a wide variety of nucleic acids.

In order to achieve the above object, the inventors of the present invention conducted intensive studies. As a result, the inventors have accomplished the present invention by finding that excellent nucleic acid delivery can be achieved by using a lipid composition which contains first lipid that is lipid represented by Formula (1) or a salt thereof, sterols, and a nucleic acid, and in which a ratio of the number of moles of the first lipid in the lipid composition to the number of moles of sterols in the lipid composition is 0.300 or more and less than 1.299. According to the present invention, the following compositions are provided.

<1> A lipid composition containing a first lipid which is a lipid represented by Formula (1) or a salt thereof, sterols, and nucleic acid, in which a ratio of the number of moles of the first lipid in the lipid composition to the number of moles of sterols in the lipid composition is 0.300 or more and less than 1.299.

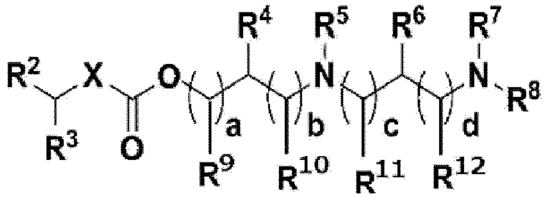

(1)

In the formula, X represents $-NR^1-$ or -O-,

$R^1$ represents a hydrogen atom, a hydrocarbon group having 6 to 24 carbon atoms, or a group represented by $R^{21}-L^1-R^{22}-$, where $R^{21}$ represents a hydrocarbon group having 1 to 24 carbon atoms, $L^1$ represents -O(CO)O-, -O(CO)-, -(CO)O-, -O-, or a group represented by the following formula,

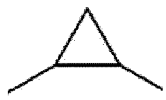

, and $R^{22}$ represents a divalent hydrocarbon linking group having 1 to 18 carbon atoms,
$R^2$ and $R^3$ each independently represent a hydrogen atom, a hydrocarbon group having 3 to 24 carbon atoms, or a group represented by $R^{31}-L^2-R^{32}-$, where $R^{31}$ represents a hydrocarbon group having 1 to 24 carbon atoms, $L^2$ represents -O(CO)O-, -O(CO)-, -(CO)O-, -O-, or a group represented by the following formula,

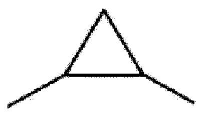

, and $R^{32}$ represents a divalent hydrocarbon linking group having 1 to 18 carbon atoms,
$R^4$, $R^5$, $R^6$, $R^7$, $R^8$, $R^9$, $R^{10}$, $R^{11}$, and $R^{12}$ each independently represent a hydrogen atom or an alkyl group having 1 to 18 carbon atoms which may be substituted,
groups in any one or more pairs among $R^4$ and $R^5$, $R^{10}$ and $R^5$, $R^5$ and $R^{12}$, $R^4$ and $R^6$, $R^5$ and $R^6$, $R^6$ and $R^7$, $R^6$ and $R^{10}$, $R^{12}$ and $R^7$, and $R^7$ and $R^8$ may be linked to each other to form a 4- to 7-membered ring which may contain an O atom,
a substituent on the alkyl group having 1 to 18 carbon atoms which may be substituted is a hydroxyl group, a carboxyl group, an amino group represented by $-NR^{45}R^{46}$, a substituted or unsubstituted aryl group, a substituted or unsubstituted heteroaryl group, or a group represented by $-O(CO)O-R^{41}$, $-O(CO)-R^{42}$, $-(CO)O-R^{43}$, or $-O-R^{44}$, where $R^{41}$, $R^{42}$, $R^{43}$, $R^{44}$, $R^{45}$, and $R^{46}$ each independently represent a hydrocarbon group having 1 to 18 carbon atoms,
a substituent on the substituted or unsubstituted aryl group and on the substituted or unsubstituted heteroaryl group is an alkyl group having 1 to 18 carbon atoms, a hydroxyl group, a carboxyl group, an amino group represented by $-NR^{45}R^{46}$, or a group represented by $-O(CO)O-R^{41}$, $-O(CO)-R^{42}$, $-(CO)O-R^{43}$, or $-O-R^{44}$, where $R^{41}$, $R^{42}$, $R^{43}$, $R^{44}$, $R^{45}$, and $R^{46}$ each independently represent a hydrocarbon group having 1 to 18 carbon atoms, and
a, b, c, and d each independently represent an integer of 0 to 3, a + b is 1 or more, and c + d is 1 or more.

<2> The lipid composition described in <1>, in which the sterols are cholesterol or a derivative thereof.
<3> The lipid composition described in <1> or <2>, further containing a lipid having a nonionic hydrophilic polymer structure.
<4> The lipid composition described in <3>, in which the lipid having a nonionic hydrophilic polymer structure is a lipid having a polyethylene glycol structure.
<5> The lipid composition described in <4>, in which the lipid having a polyethylene glycol structure is a lipid having a diacylglycerol structure and a polyethylene glycol structure.
<6> The lipid composition described in any one of <3> to <5>, in which a content of the lipid having a nonionic hydrophilic polymer structure with respect to the total lipids is 0.2 to 10 mol%.
<7> The lipid composition described in any one of <1> to <6>, in which a content of the first lipid with respect to the total lipids is 20 to 55 mol%.

<8> The lipid composition described in any one of <1> to <7>, in which a content of the sterols with respect to the total lipids is 20 to 70 mol%.

<9> The lipid composition described in any one of <1> to <8>, in which a content of the nucleic acid with respect to the total lipids is 1% to 25% by mass.

<10> The lipid composition described in any one of <1> to <9>, in which the compound represented by Formula (1) is a compound represented by Formula (2).

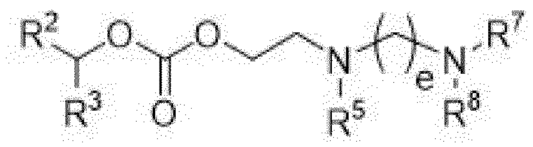

(2)

In the formula, $R^2$ and $R^3$ each independently represent a hydrogen atom, a hydrocarbon group having 3 to 24 carbon atoms, or a group represented by $R^{31}$-$L^2$-$R^{32}$-,

where $R^{31}$ represents a hydrocarbon group having 1 to 24 carbon atoms,
$L^2$ represents -O(CO)O-, -O(CO)-, -(CO)O-, -O-, or a group represented by the following formula,

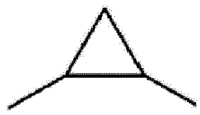

, and $R^{32}$ represents a divalent hydrocarbon linking group having 1 to 18 carbon atoms,
$R^5$ represents a hydrogen atom or an alkyl group having 1 to 18 carbon atoms which may be substituted,
$R^7$ and $R^8$ each independently represent a hydrogen atom or an alkyl group having 1 to 18 carbon atoms which may be substituted,
a substituent on the alkyl group having 1 to 18 carbon atoms which may be substituted is a hydroxyl group, a carboxyl group, an amino group represented by -$NR^{45}R^{46}$, a substituted or unsubstituted aryl group, a substituted or unsubstituted heteroaryl group, or a group represented by -O(CO)O-$R^{41}$, -O(CO)-$R^{42}$, -(CO)O-$R^{43}$, or -O-$R^{44}$, where $R^{41}$, $R^{42}$, $R^{43}$, $R^{44}$, $R^{45}$, and $R^{46}$ each independently represent a hydrocarbon group having 1 to 18 carbon atoms,
a substituent on the substituted or unsubstituted aryl group and on the substituted or unsubstituted heteroaryl group is an alkyl group having 1 to 18 carbon atoms, a hydroxyl group, a carboxyl group, an amino group represented by -$NR^{45}R^{46}$, or a group represented by -O(CO)O-$R^{41}$, -O(CO)-$R^{42}$, -(CO)O-$R^{43}$, or -O-$R^{44}$, where $R^{41}$, $R^{42}$, $R^{43}$, $R^{44}$, $R^{45}$, and $R^{46}$ each independently represent a hydrocarbon group having 1 to 18 carbon atoms, and
e represents 2 or 3.

<11> The lipid composition described in < 10>, in which in Formula (2),

at least one of $R^2$ or $R^3$ represents a hydrocarbon group with 3 to 24 carbon atoms containing one or more unsaturated bonds, $R^2$ and $R^3$ each independently represent a group represented by $R^{31}$-$L^2$-$R^{32}$-, or one of $R^2$ and $R^3$ represents a group represented by $R^{31}$-$L^2$-$R^{32}$- and the other represents a hydrocarbon group having 3 to 24 carbon atoms;
$R^5$ represents an unsubstituted alkyl group having 1 to 18 carbon atoms or an alkyl group having 1 to 18 carbon atoms substituted with -O(CO)-$R^{42}$ or -(CO)O-$R^{43}$;
$R^7$ and $R^8$ each independently represent an alkyl group having 1 to 4 carbon atoms; and
$R^{31}$, $L^2$, $R^{32}$, $R^{42}$ and $R^{43}$ have the same definitions as $R^{31}$, $L^2$, $R^{32}$, $R^{42}$, and $R^{43}$ in <10>.

<12> The lipid composition described in any one of <1> to <11>, further containing a pharmaceutically acceptable carrier.

<13> The lipid composition described in any one of <1> to <12>, in which the lipid composition is a composition for introducing nucleic acids into cells.

<14> The lipid composition described in any one of <1> to <12>, which is a composition for in vivo delivery of nucleic acids.

The lipid composition according to the embodiment of the present invention can achieve excellent nucleic acid delivery.

## DESCRIPTION OF THE PREFERRED EMBODIMENTS

Hereinafter, the present invention will be specifically described.

In the present specification, "to" shows a range including numerical values described before and after "to" as a minimum value and a maximum value respectively.

The lipid composition of the present invention is a lipid composition containing a first lipid which is a lipid represented by the formula (1) or a salt thereof, sterols, and nucleic acid, in which a ratio of the number of moles of the first lipid in the lipid composition to the number of moles of sterols in the lipid composition is 0.300 or more and less than 1.299.

<Ratio of number of moles of first lipid in lipid composition to number of moles of sterols in lipid composition>

In the lipid composition according to the embodiment of the present invention, the ratio of the number of moles of the first lipid in the lipid composition to the number of moles of sterols in the lipid composition is 0.300 or more and less than 1.299, whereby the lipid composition according to the embodiment of the present invention can achieve excellent nucleic acid delivery.

The lower limit of the ratio of the number of moles of the first lipid in the lipid composition to the number of moles of sterols in the lipid composition is preferably 0.350 or more, and may be 0.400 or more, 0.500 or more, or 0.600 or more.

The upper limit of the ratio of the number of moles of the first lipid in the lipid composition to the number of moles of sterols in the lipid composition is preferably 1.250 or less, and may be 1.200 or less.

<Lipid represented by Formula (1) or salt thereof>

The lipid composition according to an embodiment of the present invention contains a lipid represented by Formula (1) or a salt thereof.

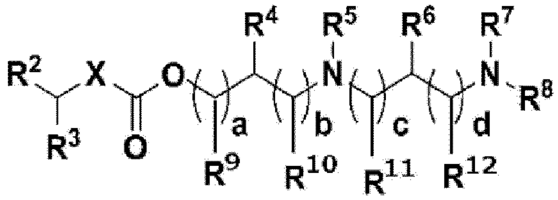

(1)

In the formula, X represents $-NR^1-$ or -O-,

$R^1$ represents a hydrogen atom, a hydrocarbon group having 6 to 24 carbon atoms, or a group represented by $R^{21}-L^1-R^{22}-$, where $R^{21}$ represents a hydrocarbon group having 1 to 24 carbon atoms, $L^1$ represents -O(CO)O-, -O(CO)-, -(CO)O-, -O-, or a group represented by the following formula,

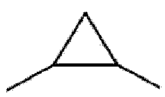

, and $R^{22}$ represents a divalent hydrocarbon linking group having 1 to 18 carbon atoms,

$R^2$ and $R^3$ each independently represent a hydrogen atom, a hydrocarbon group having 3 to 24 carbon atoms, or a group represented by $R^{31}-L^2-R^{32}-$, where $R^{31}$ represents a hydrocarbon group having 1 to 24 carbon atoms, $L^2$ represents -O(CO)O-, -O(CO)-, -(CO)O-, -O-, or a group represented by the following formula,

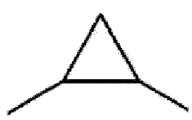

, and $R^{32}$ represents a divalent hydrocarbon linking group having 1 to 18 carbon atoms,

$R^4$, $R^5$, $R^6$, $R^7$, $R^8$, $R^9$, $R^{10}$, $R^{11}$, and $R^{12}$ each independently represent a hydrogen atom or an alkyl group having 1 to 18 carbon atoms which may be substituted,

groups in any one or more pairs among $R^4$ and $R^5$, $R^{10}$ and $R^5$, $R^5$ and $R^{12}$, $R^4$ and $R^6$, $R^5$ and $R^6$, $R^6$ and $R^7$, $R^6$ and $R^{10}$, $R^{12}$ and $R^7$, and $R^7$ and $R^8$ may be linked to each other to form a 4- to 7-membered ring which may contain an O atom,

a substituent on the alkyl group having 1 to 18 carbon atoms which may be substituted is a hydroxyl group, a carboxyl group, an amino group represented by $-NR^{45}R^{46}$, a substituted or unsubstituted aryl group, a substituted or unsubstituted heteroaryl group, or a group represented by $-O(CO)O-R^{41}$, $-O(CO)-R^{42}$, $-(CO)O-R^{43}$, or $-O-R^{44}$, where $R^{41}$, $R^{42}$, $R^{43}$, $R^{44}$, $R^{45}$, and $R^{46}$ each independently represent a hydrocarbon group having 1 to 18 carbon atoms,

the substituent on the substituted or unsubstituted aryl group and on the substituted or unsubstituted heteroaryl group is an alkyl group having 1 to 18 carbon atoms, a hydroxyl group, a carboxyl group, an amino group represented by $-NR^{45}R^{46}$, or a group represented by $-O(CO)O-R^{41}$, $-O(CO)-R^{42}$, $-(CO)O-R^{43}$, or $-O-R^{44}$, where $R^{41}$, $R^{42}$, $R^{43}$, $R^{44}$, $R^{45}$, and $R^{46}$ each independently represent a hydrocarbon group having 1 to 18 carbon atoms, and

a, b, c, and d each independently represent an integer of 0 to 3, a + b is 1 or more, and c + d is 1 or more.

As the hydrocarbon group having 6 to 24 carbon atoms that is represented by $R^1$ and the hydrocarbon group having 3 to 24 carbon atoms that is represented by $R^2$ and $R^3$, an alkyl group, an alkenyl group, or an alkynyl group is preferable, and an alkyl group or an alkenyl group is more preferable. The alkyl group having 6 to 24 carbon atoms and the alkyl group having 3 to 24 carbon atoms may be linear or branched or may be chainlike or cyclic. The alkyl group having 6 to 24 carbon atoms is preferably an alkyl group having 6 to 20 carbon atoms, and the alkyl group having 3 to 24 carbon atoms is more preferably an alkyl group having 6 to 20 carbon atoms. Specifically, examples thereof include a hexyl group, a heptyl group, an octyl group, a nonyl group, a decyl group, an undecyl group, a dodecyl group, a tridecyl group, a trimethyldodecyl group (preferably a 3,7,11-trimethyldodecyl group), a tetradecyl group, a pentadecyl group, a hexadecyl group, a tetramethylhexadecyl group (preferably a 3,7,11,15-tetramethylhexadecyl group), a heptadecyl group, an octadecyl group, a nonadecyl group, an icosyl group, and the like. The alkenyl group having 6 to 24 carbon atoms and the alkenyl group having 3 to 24 carbon atoms may be linear or branched or may be chainlike or cyclic. The alkenyl group having 6 to 24 carbon atoms is preferably an alkenyl group having 6 to 20 carbon atoms, and the alkenyl group having 3 to 24 carbon atoms is more preferably an alkenyl group having 6 to 20 carbon atoms. Specifically, examples thereof include a hexenyl group, a heptenyl group, an octenyl group, a nonenyl group, a decenyl group, an undecenyl group, a dodecenyl group, a dodecadienyl group, a tridecenyl group, a tetradecenyl group, a pentadecenyl group, a hexadecenyl group (preferably a (Z)-hexadec-9-enyl group), a hexadecadienyl group, a heptadecenyl group (preferably a (Z)-heptadec-8-enyl group), a heptadecadienyl group (preferably a (8Z,11Z)-heptadeca-8,11-dienyl group), an octadecenyl group (preferably a (Z)-octadec-9-enyl group), an octadecadienyl group (preferably a (9Z,12Z)-octadeca-9,12-dienyl group), a nonadecenyl group, an icosenyl group (preferably a (Z)-icos-11-enyl group), an icosadienyl group (preferably a (11Z,14Z)-icosa-11,14-dienyl group), and the like. The alkynyl group having 6 to 24 carbon atoms is preferably an alkynyl group having 6 to 20 carbon atoms, and the alkynyl group having 3 to 24 carbon atoms is more preferably an alkynyl group having 6 to 20 carbon atoms. Specifically, examples thereof include a hexynyl group, a heptynyl group, an octynyl group, a nonynyl group, a decynyl group, an undecynyl group, a dodecynyl group, a tetradecynyl group, a pentadecynyl group, a hexadecynyl group, a heptadecynyl group, an octadecynyl group, and the like. All of the above alkenyl groups preferably have one double bond or two double bonds. All of the above alkynyl groups preferably have one triple bond or two triple bonds.

The hydrocarbon group having 1 to 24 carbon atoms that is represented by $R^{21}$ and $R^{31}$ is preferably an alkyl group having 10 to 24 carbon atoms, an alkenyl group having 10 to 24 carbon atoms, or an alkynyl group having 10 to 24 carbon atoms. The alkyl group having 10 to 24 carbon atoms may be linear or branched or may be chainlike or cyclic. The alkyl group having 10 to 24 carbon atoms is preferably an alkyl group having 12 to 24 carbon atoms. Specifically, examples thereof include a decyl group, an undecyl group, a dodecyl group, a tridecyl group, a trimethyldodecyl group (preferably a 3,7,11-trimethyldodecyl group), a tetradecyl group, a pentadecyl group, a hexadecyl group, a tetramethylhexadecyl group (preferably a 3,7,11,15-tetramethylhexadecyl group), a heptadecyl group, an octadecyl group, a 2-butylhexyl group, a 2-butyloctyl group, a 1-pentylhexyl group, a 2-pentylheptyl group, a 3-pentyloctyl group, a 1-hexylheptyl group, a 1-hexylnonyl group, a 2-hexyloctyl group, a 2-hexyldecyl group, a 3-hexylnonyl group, a 1-heptyloctyl group, a 2-heptylnonyl group, a 2-heptylundecyl group, a 3-heptyldecyl group, a 1-octylnonyl group, a 2-octyldecyl group, a 2-octyldodecyl group, a 3-octylundecyl group, a 2-nonylundecyl group, a 3-nonyldodecyl group, a 2-decyldodecyl group, a 2-decyltetradecyl group, a 3-decyltridecyl group, a 2-(4,4-dimethylpentan-2-yl)-5,7,7-trimethyloctyl group, and the like. The alkenyl group having 10 to 24 carbon atoms may be linear or branched or may be chainlike or cyclic. Specifically, examples thereof include a decenyl group, an undecenyl group, a dodecenyl group, a dodecadienyl group, tridecenyl group (preferably a (Z)-tridec-8-enyl group), a tetradecenyl group (preferably a tetradec-9-enyl group), a pentadecenyl group (preferably a (Z)-pentadec-8-enyl group), a hexadecenyl group (preferably a (Z)-hexadec-9-enyl group), a hexadecadienyl group, a heptadecenyl group (preferably a (Z)-heptadec-8-enyl group), a heptadecadienyl group (preferably a (8Z,11Z)-heptadeca-8,11-dienyl group), an octadecenyl group (preferably a (Z)-octadec-9-enyl group), an octadecadienyl group (preferably a (9Z,12Z)-octadeca-9,12-dienyl group), and the like. The alkynyl group

having 10 to 24 carbon atoms may be linear or branched or may be chainlike or cyclic. Specifically, examples thereof include a decynyl group, an undecynyl group, a dodecynyl group, a tetradecynyl group, a pentadecynyl group, a hexadecynyl group, a heptadecynyl group, an octadecynyl group, and the like. All of the above alkenyl groups preferably have one double bond or two double bonds. All of the above alkynyl groups preferably have one triple bond or two triple bonds.

The divalent hydrocarbon linking group having 1 to 18 carbon atoms that is represented by $R^{22}$ and $R^{32}$ is preferably an alkylene group having 1 to 18 carbon atoms or an alkenylene group having 2 to 18 carbon atoms. The alkylene group having 1 to 18 carbon atoms may be linear or branched or may be chainlike or cyclic. The number of carbon atoms in the alkylene group is preferably 1 to 12, more preferably 1 to 10, and still more preferably 2 to 10. Specifically, examples thereof include a methylene group, an ethylene group, a trimethylene group, a tetramethylene group, a pentamethylene group, a hexamethylene group, a heptamethylene group, an octamethylene group, a nonamethylene group, a decamethylene group, an undecamethylene group, a dodecamethylene group, and the like. The alkenylene group having 2 to 18 carbon atoms may be linear or branched or may be chainlike or cyclic. The number of carbon atoms in the alkenylene group is preferably 1 to 12, and more preferably 2 to 10.

-O(CO)O-, -O(CO)-, and -(CO)O- are in a preferred range of $L^1$, and -O(CO)- and -(CO)O- are in a more preferred range of $L^1$.

-O(CO)O-, -O(CO)-, and -(CO)O- are in a preferred range of $L^2$, and -O(CO)- and -(CO)O- are in a more preferred range of $L^2$.

The alkyl group having 1 to 18 carbon atoms which may be substituted and which represented by $R^4$, $R^6$, $R^9$, $R^{10}$, $R^{11}$, and $R^{12}$ may be linear or branched or may be chainlike or cyclic. The number of carbon atoms in the alkyl group is preferably 1 to 12. Specifically, examples thereof include a methyl group, an ethyl group, a propyl group, an isopropyl group, a cyclopropyl group, a butyl group, an isobutyl group, a tert-butyl group, a cyclobutyl group, a pentyl group, a cyclopentyl group, a hexyl group, a cyclohexyl group, a heptyl group, an octyl group, a nonyl group, a decyl group, an undecyl group, a dodecyl group, and the like. In a case where the alkyl group has a substituent, as the substituent, a hydroxyl group, a carboxyl group, or a group represented by -O(CO)O-$R^{41}$, -O(CO)-$R^{42}$, -(CO)O-$R^{43}$, or -O-$R^{44}$ is preferable, and a group represented by -O(CO)-$R^{42}$ or -(CO)O-$R^{43}$ is more preferable.

The alkyl group having 1 to 18 carbon atoms which may be substituted and which represented by $R^5$, $R^7$, and $R^8$ may be linear or branched or may be chainlike or cyclic. The number of carbon atoms in the alkyl group is preferably 1 to 12, and more preferably 1 to 8. Specifically, examples thereof include a methyl group, an ethyl group, a propyl group, an isopropyl group, a cyclopropyl group, a butyl group, an isobutyl group, a tert-butyl group, a cyclobutyl group, a pentyl group, a cyclopentyl group, a hexyl group, a cyclohexyl group, a heptyl group, an octyl group, a nonyl group, a decyl group, an undecyl group, a dodecyl group, and the like. In a case where the alkyl group has a substituent, as the substituent, a hydroxyl group, a carboxyl group, or a group represented by -O(CO)O-$R^{41}$, -O(CO)-$R^{42}$, -(CO)O-$R^{43}$, or -O-$R^{44}$ is preferable, and a group represented by -O(CO)-$R^{42}$, -(CO)O-$R^{43}$, or -O-$R^{44}$ is more preferable.

Examples of the 4- to 7-membered ring which may contain an O atom include an azetidine ring, a pyrrolidine ring, a piperidine ring, a morpholine ring, and an azepane ring. The 4- to 7-membered ring is preferably a 6-membered ring and is preferably a piperidine ring or a morpholine ring.

In a case where the alkyl group having 1 to 18 carbon atoms which is represented by $R^4$, $R^5$, $R^6$, $R^7$, $R^8$, $R^9$, $R^{10}$, $R^{11}$, and $R^{12}$ and which may be substituted has a substituted or unsubstituted aryl group as a substituent, the number of carbon atoms in the aryl group is preferably 6 to 22, more preferably 6 to 18, and still more preferably 6 to 10. Specifically, examples of the aryl group include a phenyl group, a naphthyl group, an anthracenyl group, a phenanthrenyl group, and the like. As the substituent on the aryl group, an alkyl group having 1 to 18 carbon atoms, a hydroxyl group, a carboxyl group, an amino group represented by -$NR^{45}R^{46}$, or a group represented by -O(CO)O-$R^{41}$, -O(CO)-$R^{42}$, -(CO)O-$R^{43}$, or -O-$R^{44}$ is preferable, and a hydroxyl group or a carboxyl group is more preferable. Specifically, examples of the substituted aryl group include a hydroxyphenyl group, a carboxyphenyl group, and the like.

In a case where the alkyl group having 1 to 18 carbon atoms which is represented by $R^4$, $R^5$, $R^6$, $R^7$, $R^8$, $R^9$, $R^{10}$, $R^{11}$, and $R^{12}$ and which may be substituted has a substituted or unsubstituted heteroaryl group as a substituent, the number of carbon atoms in the heteroaryl group is preferably 1 to 12, and more preferably 1 to 6. Specifically, examples of the heteroaryl group include a pyridyl group, a pyrazolyl group, an imidazolyl group, a benzimidazolyl group, a thiazolyl group, an oxazolyl group, and the like. As the substituent on the heteroaryl group, an alkyl group having 1 to 18 carbon atoms, a hydroxyl group, a carboxyl group, an amino group represented by -$NR^{45}R^{46}$, or a group represented by -O(CO)O-$R^{41}$, -O(CO)-$R^{42}$, -(CO)O-$R^{43}$, or -O-$R^{44}$ is preferable, and a hydroxyl group or a carboxyl group is more preferable. Specifically, examples of the substituted or unsubstituted heteroaryl group include a hydroxypyridyl group, a carboxypyridyl group, a pyridonyl group, and the like.

As hydrocarbon group having 1 to 18 carbon atoms that is represented by $R^{41}$, $R^{42}$, $R^{43}$, $R^{44}$, $R^{45}$, and $R^{46}$, an alkyl group having 1 to 18 carbon atoms, an alkenyl group having 2 to 18 carbon atoms, or an alkynyl group having 2 to 18 carbon atoms is preferable, and an alkyl group having 1 to 18 carbon atoms or an alkenyl group having 2 to 18 carbon atoms is more preferable. The alkyl group having 1 to 18 carbon atoms may be linear or branched or may be

chainlike or cyclic. The number of carbon atoms in the alkyl group is preferably 3 to 18, and more preferably 5 to 18. Specifically, examples thereof include a propyl group, an isopropyl group, a cyclopropyl group, a butyl group, an isobutyl group, a tert-butyl group, a cyclobutyl group, a pentyl group, a cyclopentyl group, a hexyl group, a cyclohexyl group, a heptyl group, an octyl group, a nonyl group, a decyl group, an undecyl group, a dodecyl group, a tridecyl group, a trimethyldodecyl group (preferably a 3,7,11-trimethyldodecyl group), a tetradecyl group, a pentadecyl group, a hexadecyl group, a heptadecyl group, an octadecyl group, and the like. The alkenyl group having 2 to 18 carbon atoms may be linear or branched or may be chainlike or cyclic. The number of carbon atoms in the alkenyl group is preferably 3 to 18, and more preferably 5 to 18. Specifically, examples thereof include an allyl group, a prenyl group, a pentenyl group, a hexenyl group, a heptenyl group, an octenyl group, a nonenyl group (preferably a (Z)-2-nonenyl group or an (E)-2-nonenyl group), a decenyl group, an undecenyl group, a dodecenyl group, a dodecadienyl group, a tridecenyl group (preferably a (Z)-tridec-8-enyl group), a tetradecenyl group (preferably a tetradec-9-enyl group), a pentadecenyl group (preferably a (Z)-pentadec-8-enyl group), a hexadecenyl group (preferably a (Z)-hexadec-9-enyl group), a hexadecadienyl group, a heptadecenyl group (preferably a (Z)-heptadec-8-enyl group), a heptadecadienyl group (preferably a (8Z,11Z)-heptadeca-8,11-dienyl group), an octadecenyl group (preferably a (Z)-octadec-9-enyl group), an octadecadienyl group (preferably a (9Z,12Z)-octadeca-9,12-dienyl group), and the like. The alkynyl group having 2 to 18 carbon atoms may be linear or branched or may be chainlike or cyclic. The number of carbon atoms in the alkynyl group is preferably 3 to 18, and more preferably 5 to 18. Specifically, examples thereof include a propargyl group, a butynyl group, a pentynyl group, a hexynyl group, a heptynyl group, an octynyl group, a nonynyl group, a decynyl group, an undecynyl group, a dodecynyl group, a tetradecynyl group, a pentadecynyl group, a hexadecynyl group, a heptadecynyl group, an octadecynyl group, and the like.

In a case where X represents $-NR^1-$, $R^1$ preferably represents a hydrocarbon group having 6 to 24 carbon atoms or a group represented by $R^{21}-L^1-R^{22}-$. In this case, it is preferable that one of $R^2$ and $R^3$ represent a hydrogen atom and the other represent a hydrocarbon group having 6 to 24 carbon atoms or a group represented by $R^{31}-L^2-R^{32}-$.

In a case where X represents -O-, it is preferable that $R^2$ and $R^3$ each independently represent a hydrocarbon group having 6 to 24 carbon atoms or a group represented by $R^{31}-L^2-R^{32}-$.

It is preferable that $R^4$, $R^6$, $R^9$, $R^{10}$, $R^{11}$, and $R^{12}$ each represent a hydrogen atom.

$R^5$ is preferably a hydrogen atom, an alkyl group having 1 to 18 carbon atoms, an alkyl group having 1 to 18 carbon atoms which may be substituted with $-O(CO)-R^{42}$ or $-(CO)O-R^{43}$, an alkyl group having 1 to 18 carbon atoms which may be substituted with an aryl group, or an alkyl group having 1 to 18 carbon atoms which may be substituted with a hydroxyl group. In a case where $R^5$ is an alkyl group, $R^5$ may be linked to $R^4$, $R^6$, $R^{10}$, and $R^{12}$ to form a ring which may contain an O atom. Particularly, $R^5$ is preferably an alkyl group having 1 to 18 carbon atoms, an alkyl group having 1 to 18 carbon atoms which may be substituted with $-O(CO)-R^{42}$ or $-(CO)O-R^{43}$, an alkyl group having 1 to 12 carbon atoms which may be substituted with an aryl group, or an alkyl group having 1 to 8 carbon atoms which may be substituted with a hydroxyl group, and more preferably an alkyl group having 1 to 18 carbon atoms or an alkyl group having 1 to 18 carbon atoms which may be substituted with $-O(CO)-R^{42}$ or $-(CO)O-R^{43}$.

$R^7$ and $R^8$ preferably each independently represent a hydrogen atom, a hydrocarbon group having 1 to 18 carbon atoms, an alkyl group having 1 to 18 carbon atoms which may be substituted with $-O(CO)-R^{42}$ or $-(CO)O-R^{43}$, an alkyl group having 1 to 8 carbon atoms which may be substituted with an aryl group, or an alkyl group having 1 to 8 carbon atoms which may be substituted with a hydroxyl group. Alternatively, it is preferable that $R^7$ and $R^8$ be linked to each other to form a 4- to 7-membered ring which may contain an O atom.

$R^5$ is not linked to $R^7$ or $R^8$ and does not form a ring with $R^7$ or $R^8$.

a + b is preferably 1 or 2, and more preferably 1. c + d is preferably 1 or 2, and more preferably 1.

The compound represented by Formula (1) is preferably a compound represented by Formula (1-1).

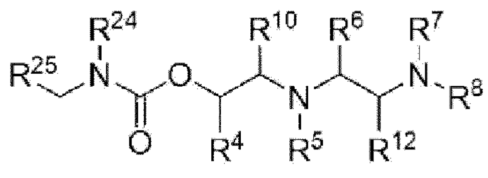

(1-1)

$R^{24}$ represents a hydrogen atom, a hydrocarbon group having 6 to 24 carbon atoms, or a group represented by $R^{21}-L^1-R^{22}-$, $R^{21}$ represents a hydrocarbon group having 1 to 24 carbon atoms, $L^1$ represents -O(CO)O-, -O(CO)-, -(CO)O-, -O-, or a group represented by the following formula,

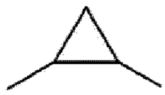

$R^{22}$ represents a divalent hydrocarbon linking group having 1 to 18 carbon atoms.

$R^{25}$ represents a hydrogen atom, a hydrocarbon group having 3 to 24 carbon atoms, or a group represented by $R^{31}$-$L^2$-$R^{32}$-, $R^{31}$ represents a hydrocarbon group having 1 to 24 carbon atoms, $L^2$ represents -O(CO)O-, -O(CO)-, -(CO)O-, -O-, or a group represented by the following formula,

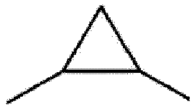

$R^{32}$ represents a divalent hydrocarbon linking group having 1 to 18 carbon atoms.

$R^4$, $R^5$, $R^6$, $R^7$, $R^8$, $R^{10}$, and $R^{12}$ each independently represent a hydrogen atom or an alkyl group having 1 to 18 carbon atoms which may be substituted, and

groups in any one or more pairs among $R^4$ and $R^5$, $R^{10}$ and $R^5$, $R^5$ and $R^{12}$, $R^4$ and $R^6$, $R^5$ and $R^6$, $R^6$ and $R^7$, $R^6$ and $R^{10}$, $R^{12}$ and $R^7$, and $R^7$ and $R^8$ may be linked to each other to form a 4- to 7-membered ring which may contain an O atom. However, it is preferable that $R^5$ be not linked to $R^7$ or $R^8$ and do not form a ring with $R^7$ or $R^8$.

a substituent on the alkyl group having 1 to 18 carbon atoms which may be substituted is a hydroxyl group, a carboxyl group, an amino group represented by -$NR^{45}R^{46}$, a substituted or unsubstituted aryl group, a substituted or unsubstituted heteroaryl group, or a group represented by -O(CO)O-$R^{41}$, -O(CO)-$R^{42}$, -(CO)O-$R^{43}$, or -O-$R^{44}$, where $R^{41}$, $R^{42}$, $R^{43}$, $R^{44}$, $R^{45}$, and $R^{46}$ each independently represent a hydrocarbon group having 1 to 18 carbon atoms,

The substituent on the substituted or unsubstituted aryl group and on the substituted or unsubstituted heteroaryl group is an alkyl group having 1 to 18 carbon atoms, a hydroxyl group, a carboxyl group, an amino group represented by -$NR^{45}R^{46}$, or a group represented by -O(CO)O-$R^{41}$, -O(CO)-$R^{42}$, -(CO)O-$R^{43}$, or -O-$R^{44}$, and $R^{41}$, $R^{42}$, $R^{43}$, $R^{44}$, $R^{45}$, and $R^{46}$ each independently represent a hydrocarbon group having 1 to 18 carbon atoms.

The definitions and preferred ranges of $R^4$, $R^5$, $R^6$, $R^7$, $R^8$, $R^{10}$, and $R^{12}$ in Formula (1-1) are the same as those of $R^4$, $R^5$, $R^6$, $R^7$, $R^8$, $R^{10}$, and $R^{12}$ in Formula (1).

$R^{24}$ in Formula (1-1) is preferably an alkyl group having 6 to 24 carbon atoms or an alkenyl group having 6 to 24 carbon atoms. The alkyl group having 6 to 24 carbon atoms may be linear or branched or may be chainlike or cyclic. The alkyl group having 6 to 24 carbon atoms is preferably an alkyl group having 8 to 20 carbon atoms. Specifically, examples thereof include an octyl group, a nonyl group, a decyl group, an undecyl group, a dodecyl group, a tridecyl group, a trimethyldodecyl group (preferably a 3,7,11-trimethyldodecyl group), a tetradecyl group, a pentadecyl group, a hexadecyl group, a tetramethylhexadecyl group (preferably a 3,7,11,15-tetramethylhexadecyl group), a heptadecyl group, an octadecyl group, a nonadecyl group, an icosyl group, and the like. The alkenyl group having 6 to 24 carbon atoms may be linear or branched or may be chainlike or cyclic. The alkenyl group having 6 to 24 carbon atoms is preferably an alkenyl group having 8 to 20 carbon atoms. Specifically, examples thereof include an octenyl group, a nonenyl group, a decenyl group, an undecenyl group, a dodecenyl group, a dodecadienyl group, a tridecenyl group, a tetradecenyl group, a pentadecenyl group, a hexadecenyl group (preferably a (Z)-hexadec-9-enyl group), a hexadecadienyl group, a heptadecenyl group (preferably a (Z)-heptadec-8-enyl group), a heptadecadienyl group (preferably a (8Z,11Z)-heptadeca-8,11-dienyl group), an octadecenyl group (preferably a (Z)-octadec-9-enyl group), an octadecadienyl group (preferably a (9Z,12Z)-octadeca-9,12-dienyl group), a nonadecenyl group, an icosenyl group (preferably a (Z)-icos-11-enyl group), an icosadienyl group (preferably a (11Z,14Z)-icosa-11,14-dienyl group), and the like.

It is preferable that all of the above alkenyl groups have one double bond or two double bonds.

$R^{25}$ in Formula (1-1) is preferably an alkyl group having 6 to 24 carbon atoms or an alkenyl group having 6 to 24 carbon atoms. The alkyl group having 6 to 24 carbon atoms may be linear or branched or may be chainlike or cyclic. The alkyl group having 6 to 24 carbon atoms is preferably an alkyl group having 7 to 20 carbon atoms. Specifically, examples thereof include a hexyl group, a heptyl group, an octyl group, a nonyl group, a decyl group, an undecyl group, a dodecyl group, a tridecyl group, a trimethyldodecyl group (preferably a 3,7,11-trimethyldodecyl group), a tetradecyl group, a pentadecyl group, a hexadecyl group, a tetramethylhexadecyl group (preferably a 3,7,11,15-tetramethylhexadecyl group), a heptadecyl group, an octadecyl group, and the like. The alkenyl group having 6 to 24 carbon atoms may be linear or branched or may be chainlike or cyclic. The alkenyl group having 6 to 24 carbon atoms is preferably an alkenyl group having 8 to 20 carbon atoms. Specifically, examples thereof include an octenyl group, a nonenyl group, a decenyl group, an undecenyl group, a dodecenyl group, a dodecadienyl group, a tridecenyl group, a tetradecenyl

group, a pentadecenyl group, a hexadecenyl group (preferably a (Z)-hexadec-9-enyl group), a hexadecadienyl group, a heptadecenyl group (preferably a (Z)-heptadec-8-enyl group), a heptadecadienyl group (preferably a (8Z,11Z)-heptadeca-8,11-dienyl group), an octadecenyl group (preferably a (Z)-octadec-9-enyl group), an octadecadienyl group (preferably a (9Z,12Z)-octadeca-9,12-dienyl group), a nonadecenyl group, an icosenyl group (preferably a (Z)-icos-11-enyl group), an icosadienyl group (preferably a (11Z,14Z)-icosa-11,14-dienyl group), and the like.

It is preferable that all of the above alkenyl groups have one double bond or two double bonds.

In a preferred embodiment,

X represents -O-;
$R^2$, $R^3$, $R^{31}$, $L^2$, and $R^{32}$ have the same definitions as $R^2$, $R^3$, $R^{31}$, $L^2$, and $R^{32}$ in Formula (1),
$R^4$, $R^5$, $R^6$, $R^7$, $R^8$, $R^9$, $R^{10}$, $R^{11}$, and $R^{12}$ each independently represent a hydrogen atom or an alkyl group having 1 to 18 carbon atoms which may be substituted,
the substituent on the alkyl group having 1 to 18 carbon atoms which may be substituted and the substituent on the substituted or unsubstituted aryl group and on the substituted or unsubstituted heteroaryl group have the same definitions as those in Formula (1),
a + b is 1, and c + d is 1 or 2.

In a more preferred embodiment, the compound represented by Formula (1) is a compound represented by Formula (2).

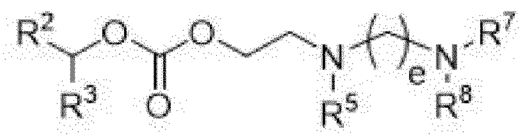

(2)

In the formula, $R^2$ and $R^3$ each independently represent a hydrogen atom, a hydrocarbon group having 3 to 24 carbon atoms, or a group represented by $R^{31}$-$L^2$-$R^{32}$-,

where $R^{31}$ represents a hydrocarbon group having 1 to 24 carbon atoms,
$L^2$ represents -O(CO)O-, -O(CO)-, -(CO)O-, -O-, or a group represented by the following formula,

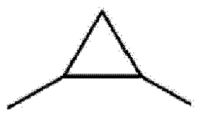

, and $R^{32}$ represents a divalent hydrocarbon linking group having 1 to 18 carbon atoms,
$R^5$ represents a hydrogen atom or an alkyl group having 1 to 18 carbon atoms which may be substituted,
$R^7$ and $R^8$ each independently represent a hydrogen atom or an alkyl group having 1 to 18 carbon atoms which may be substituted,
a substituent on the alkyl group having 1 to 18 carbon atoms which may be substituted is a hydroxyl group, a carboxyl group, an amino group represented by $-NR^{45}R^{46}$, a substituted or unsubstituted aryl group, a substituted or unsubstituted heteroaryl group, or a group represented by -O(CO)O-$R^{41}$, -O(CO)-$R^{42}$, -(CO)O-$R^{43}$, or -O-$R^{44}$, where $R^{41}$, $R^{42}$, $R^{43}$, $R^{44}$, $R^{45}$, and $R^{46}$ each independently represent a hydrocarbon group having 1 to 18 carbon atoms,
a substituent on the substituted or unsubstituted aryl group and on the substituted or unsubstituted heteroaryl group is an alkyl group having 1 to 18 carbon atoms, a hydroxyl group, a carboxyl group, an amino group represented by $-NR^{45}R^{46}$, or a group represented by -O(CO)O-$R^{41}$, -O(CO)-$R^{42}$, -(CO)O-$R^{43}$, or -O-$R^{44}$, where $R^{41}$, $R^{42}$, $R^{43}$, $R^{44}$, $R^{45}$, and $R^{46}$ each independently represent a hydrocarbon group having 1 to 18 carbon atoms, and
e represents 2 or 3.
$R^2$, $R^3$, $R^5$, $R^7$, and $R^8$ have the same definitions as $R^2$, $R^3$, $R^5$, $R^7$, and $R^8$ in Formula (1).

Formula (2) preferably represents a compound in which $R^7$ and $R^8$ each independently represent a hydrogen atom or an alkyl group having 1 to 18 carbon atoms, the substituent on the alkyl group having 1 to 18 carbon atoms which is represented by $R^5$ and which may be substituted is a hydroxyl group, a substituted or unsubstituted aryl group, or a group represented by -O(CO)O-$R^{41}$, -O(CO)-$R^{42}$, -(CO)O-$R^{43}$, or -O-$R^{44}$, $R^{41}$, $R^{42}$, $R^{43}$, $R^{44}$, $R^{45}$, and $R^{46}$ each independently represent a hydrocarbon group having 1 to 18 carbon atoms, the substituent on the substituted or unsubstituted aryl group is an alkyl group having 1 to 18 carbon atoms, a hydroxyl group, a carboxyl group, an amino group

represented by $-NR^{45}R^{46}$, or a group represented by $-O(CO)O-R^{41}$, $-O(CO)-R^{42}$, $-(CO)O-R^{43}$, or $-O-R^{44}$, and $R^{41}$, $R^{42}$, $R^{43}$, $R^{44}$, $R^{45}$, and $R^{46}$ each independently represent a hydrocarbon group having 1 to 18 carbon atoms.

Formula (2) more preferably represents a compound in which $R^2$ and $R^3$ each independently represent a hydrocarbon group having 3 to 24 carbon atoms or a group represented by $R^{31}-L^2-R^{32}-$, $L^2$ represents -O(CO)- or -(CO)O-, $R^7$ and $R^8$ each independently represent a hydrogen atom or an alkyl group having 1 to 18 carbon atoms, the substituent on the alkyl group having 1 to 18 carbon atoms which may be substituted is an unsubstituted aryl group, $-O(CO)-R^{42}$, or $-(CO)O-R^{43}$, and $R^{42}$ and $R^{43}$ each independently represent a hydrocarbon group having 1 to 18 carbon atoms.

Formula (2) still more preferably represents a compound in which $R^2$ and $R^3$ each independently represent a hydrogen atom or a hydrocarbon group having 3 to 24 carbon atoms, $R^7$ and $R^8$ each independently represent a hydrogen atom or an alkyl group having 1 to 18 carbon atoms, the substituent on the alkyl group having 1 to 18 carbon atoms which may be substituted is an unsubstituted aryl group or a group represented by $-O(CO)-R^{42}$ or $-(CO)O-R^{43}$, and $R^{42}$ and $R^{43}$ each independently represent a hydrocarbon group having 1 to 18 carbon atoms.

Formula (2) preferably represents a compound in which at least one of $R^2$ or $R^3$ represents a group represented by $R^{31}-L^2-R^{32}-$, $L^2$ represents -O(CO)- or -(CO)O-, $R^7$ and $R^8$ each independently represent a hydrogen atom or an alkyl group having 1 to 18 carbon atoms, the substituent on the alkyl group having 1 to 18 carbon atoms which may be substituted is an unsubstituted aryl group or a group represented by $-O(CO)-R^{42}$ or $-(CO)O-R^{43}$, and $R^{42}$ and $R^{43}$ each independently represent a hydrocarbon group having 1 to 18 carbon atoms.

Formula (2) more preferably represents a compound in which $R^2$ and $R^3$ each independently represent a group represented by $R^{31}-L^2-R^{32}-$, $L^2$ represents -O(CO)- or -(CO)O-, $R^7$ and $R^8$ each independently represent a hydrogen atom or an alkyl group having 1 to 18 carbon atoms, the substituent on the alkyl group having 1 to 18 carbon atoms which may be substituted is an unsubstituted aryl group or a group represented by $-O(CO)-R^{42}$ or $-(CO)O-R^{43}$, and $R^{42}$ and $R^{43}$ each independently represent a hydrocarbon group having 1 to 18 carbon atoms.

Formula (2) preferably represents a compound in which one of $R^2$ and $R^3$ represents a group represented by $R^{31}-L^2-R^{32}-$ and the other represents a hydrocarbon group having 3 to 24 carbon atoms, $L^2$ represents -O(CO)- or -(CO)O-, $R^7$ and $R^8$ each independently represent a hydrogen atom or an alkyl group having 1 to 18 carbon atoms, the substituent on the alkyl group having 1 to 18 carbon atoms which may be substituted is an unsubstituted aryl group or a group represented by $-O(CO)-R^{42}$ or $-(CO)O-R^{43}$, and $R^{42}$ and $R^{43}$ each independently represent a hydrocarbon group having 1 to 18 carbon atoms.

Formula (2) more preferably represents a compound in which one of $R^2$ and $R^3$ represents a group represented by $R^{31}-L^2-R^{32}-$ and the other represents a hydrocarbon group having 6 carbon atoms, $L^2$ represents -O(CO)- or -(CO)O-, $R^7$ and $R^8$ each independently represent a hydrogen atom or an alkyl group having 1 to 18 carbon atoms, the substituent on the alkyl group having 1 to 18 carbon atoms which may be substituted is a group represented by $-O(CO)-R^{42}$ or $-(CO)O-R^{43}$, and $R^{42}$ and $R^{43}$ each independently represent a hydrocarbon group having 1 to 18 carbon atoms.

Formula (2) still more preferably represents a compound in which one of $R^2$ and $R^3$ represents a group represented by $R^{31}-L^2-R^{32}-$ and the other represents a hydrocarbon group having 6 carbon atoms, $L^2$ represents -O(CO)- or -(CO)O-, $R^5$ represents a hydrogen atom or an alkyl group having 1 to 18 carbon atoms, and $R^7$ and $R^8$ each independently represent a hydrogen atom or an alkyl group having 1 to 18 carbon atoms.

Formula (2) even still more preferably represents a compound in which one of $R^2$ and $R^3$ represents a group represented by $R^{31}-L^2-R^{32}-$ and the other represents a hydrocarbon group having 6 carbon atoms, $L^2$ represents -O(CO)- or -(CO)O-, $R^5$ represents a hydrogen atom or an alkyl group having 1 to 18 carbon atoms, $R^7$ and $R^8$ each independently represent a hydrogen atom or an alkyl group having 1 to 18 carbon atoms, and e represents 2.

Formula (2) even still more preferably represents a compound in which one of $R^2$ and $R^3$ represents a group represented by $R^{31}-L^2-R^{32}-$ and the other represents a hydrocarbon group having 3 to 5 carbon atoms, $L^2$ represents -O(CO)- or -(CO)O-, $R^5$ represents a hydrogen atom or an alkyl group having 1 to 18 carbon atoms, and $R^7$ and $R^8$ each independently represent a hydrogen atom or an alkyl group having 1 to 18 carbon atoms.

Formula (2) even still more preferably represents a compound in which one of $R^2$ and $R^3$ represents a group represented by $R^{31}-L^2-R^{32}-$ and the other represents a hydrocarbon group having 3 to 5 carbon atoms, $L^2$ represents -O(CO)- or -(CO)O-, $R^5$ represents a hydrogen atom or an alkyl group having 1 to 18 carbon atoms, $R^7$ and $R^8$ each independently represent a hydrogen atom or an alkyl group having 1 to 18 carbon atoms, and e represents 2.

Formula (2) even still more preferably represents a compound in which one of $R^2$ and $R^3$ represents a group represented by $R^{31}-L^2-R^{32}-$ and the other represents a hydrocarbon group having 6 carbon atoms, $L^2$ represents -O(CO)- or -(CO)O-, $R^5$ represents a hydrogen atom or a substituted alkyl group having 1 to 18 carbon atoms, $R^7$ and $R^8$ each independently represent a hydrogen atom or an alkyl group having 1 to 18 carbon atoms, the substituent on the substituted alkyl group having 1 to 18 carbon atoms is a group represented by $-O(CO)-R^{42}$ or $-(CO)O-R^{43}$, and $R^{42}$ and $R^{43}$ each independently represent a hydrocarbon group having 1 to 18 carbon atoms.

Formula (2) even still more preferably represents a compound in which one of $R^2$ and $R^3$ represents a group represented by $R^{31}-L^2-R^{32}-$ and the other represents a hydrocarbon group having 6 carbon atoms, $L^2$ represents -O(CO)-

or -(CO)O-, $R^5$ represents a hydrogen atom or a substituted alkyl group having 1 to 18 carbon atoms, $R^7$ and $R^8$ each independently represent a hydrogen atom or an alkyl group having 1 to 18 carbon atoms, the substituent on the substituted alkyl group having 1 to 18 carbon atoms is a group represented by -O(CO)-$R^{42}$ or -(CO)O-$R^{43}$, $R^{42}$ and $R^{43}$ each independently represent a hydrocarbon group having 1 to 18 carbon atoms, and e represents 2.

In a preferred embodiment, Formula (2) represents a compound in which

at least one of $R^2$ or $R^3$ represents a hydrocarbon group with 3 to 24 carbon atoms containing one or more unsaturated bonds, $R^2$ and $R^3$ each independently represent a group represented by $R^{31}$-$L^2$-$R^{32}$-, or one of $R^2$ and $R^3$ represents a group represented by $R^{31}$-$L^2$-$R^{32}$- and the other represents a hydrocarbon group having 3 to 24 carbon atoms;
$R^5$ represents an unsubstituted alkyl group having 1 to 18 carbon atoms or an alkyl group having 1 to 18 carbon atoms substituted with -O(CO)-$R^{42}$ or -(CO)O-$R^{43}$; and
$R^7$ and $R^8$ each independently represent an alkyl group having 1 to 4 carbon atoms
($R^{31}$, $L^2$, $R^{32}$, $R^{42}$, and $R^{43}$ have the same definitions as $R^{31}$, $L^2$, $R^{32}$, $R^{42}$, and $R^{43}$ in Formula (2)).

The compound represented by Formula (1) may form a salt.
Examples of the salt in a basic group include salts with mineral acids such as hydrochloric acid, hydrobromic acid, nitric acid, and sulfuric acid; salts with organic carboxylic acids such as formic acid, acetic acid, citric acid, oxalic acid, fumaric acid, maleic acid, succinic acid, malic acid, tartaric acid, aspartic acid, trichloroacetic acid, and trifluoroacetic acid; and salts with sulfonic acids such as methanesulfonic acid, benzene sulfonic acid, p-toluenesulfonic acid, mesitylenesulfonic acid, and naphthalenesulfonic acid.
Examples of the salt in an acidic group include salts with alkali metals such as sodium and potassium; salts with alkaline earth metals such as calcium and magnesium; ammonium salts; salts with nitrogen-containing organic bases such as trimethylamine, triethylamine, tributylamine, pyridine, N,N-dimethylaniline, N-methylpiperidine, N-methylmorpholine, diethylamine, dicyclohexylamine, procaine, dibenzylamine, N-benzyl-β-phenethylamine, 1-ephenamine, and N,N'-dibenzylethylenediamine, and the like.
Among the above salts, for example, pharmacologically acceptable salts are preferable.
Specifically, preferred examples of the compound represented by Formula (1) include the compounds described in Examples 1 to 135 which will be described later. However, the present invention is not limited thereto.
The compounds described in Examples 1 to 135 are called Compounds 1 to 135 respectively.
Among the above, Compounds 24, 30, 31, 50, 56, 69, 88, 89, 91, 93, 103, 112, 118, 119, 134, and 135 are particularly preferable.
The method for manufacturing the compound represented by Formula (1) will be described.
The compound represented by Formula (1) can be manufactured using known methods in combination. For example, the compound can be manufactured by the following manufacturing method.

[Manufacturing method 1]

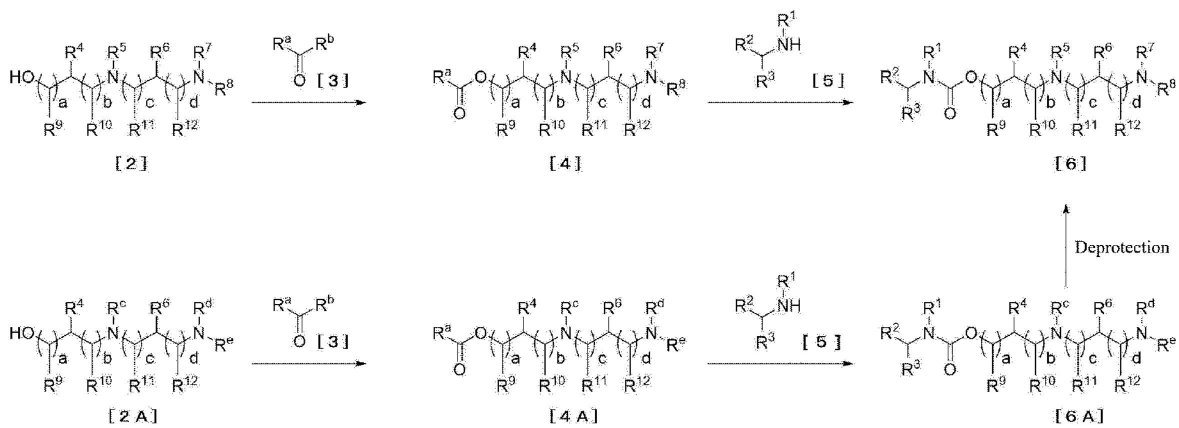

[2] [4] [6]
[2A] [4A] [6A]

"In the formula, $R^a$ and $R^b$ each represent a leaving group; $R^c$, $R^d$, and $R^e$ each represent an amino protecting group or an imino protecting group; and $R^1$, $R^2$, $R^3$, $R^4$, $R^5$, $R^6$, $R^7$, $R^8$, $R^9$, $R^{10}$, $R^{11}$, and $R^{12}$ have the same definitions as $R^1$, $R^2$, $R^3$, $R^4$, $R^5$, $R^6$, $R^7$, $R^8$, $R^9$, $R^{10}$, $R^{11}$, and $R^{12}$ described above." Examples of the leaving group include a chloro group, a fluoro group, a bromo group, a trichloromethoxy group, a 4-nitro-phenoxy group, a 2,4-dinitrophenoxy

group, a 2,4,6-trichlorophenoxy group, a pentafluorophenoxy group, a 2,3,5,6-tetrafluorophenoxy group, an imidazolyl group, a triazolyl group, a 3,5-dioxo-4-methyl-1,2,4-oxadiazolidyl group, a N-hydroxysuccinimidyl group, and the like. Examples of the amino protecting group and the imino protecting group include a tert-butoxycarbonyl group, a benzyloxycarbonyl group, a 2-nitrobenzenesulfonyl group, a benzyl group, and the like.

(1-1)

As the compound represented by Formula [3], for example, 4-nitrophenyl chloroformate, 1,1'-carbonyldiimidazole, triphosgene, phosgene, and the like are known.

The compound represented by Formula [4] can be manufactured by reacting the compound represented by Formula [2] with the compound represented by Formula [3] in the presence of a base.

The solvent used in this reaction is not particularly limited as long as the solvent does not affect the reaction. Examples of the solvent include halogenated hydrocarbons, ethers, esters, amides, nitriles, sulfoxides, and aromatic hydrocarbons. These solvents may be used by being mixed together.

As the solvent, for example, ethers are preferable, and tetrahydrofuran is more preferable.

The used amount of the solvent is not particularly limited, but is only required to be 1 to 500 times (v/w) the amount of the compound represented by Formula [2].

Examples of the base used in this reaction include an inorganic base and an organic base. As the base, an organic base is preferable. Specifically, examples thereof include triethylamine, N,N-diisopropylethylamine, 4-methylmorpholine, pyridine, N,N-dimethylaminopyridine, and the like.

The used amount of the base is only required to be 1 to 50 times and preferably 1 to 10 times the molar amount of the compound represented by Formula [2].

The used amount of the compound represented by Formula [3] is not particularly limited, but is only required to be 0.3 to 10 times (v/w) the amount of the compound represented by Formula [2].

This reaction is required only to carry out at -30°C to 150°C, preferably at 0°C to 100°C for 5 minutes to 48 hours.

(1-2)

As the compound represented by Formula [5], for example, di((9Z,12Z)-octadeca-9,12-dien-1-yl)amine, dihexadecylamine, and the like are known.

The compound represented by Formula [6] can be manufactured by reacting the compound represented by Formula [4] with the compound represented by Formula [5] in the presence of a base.

The solvent used in this reaction is not particularly limited as long as the solvent does not affect the reaction. Examples of the solvent include halogenated hydrocarbons, ethers, esters, amides, nitriles, sulfoxides, and aromatic hydrocarbons. These solvents may be used by being mixed together.

As the solvent, for example, ethers are preferable, and tetrahydrofuran is more preferable.

The used amount of the solvent is not particularly limited, but is only required to be 1 to 500 times (v/w) the amount of the compound represented by Formula [4].

Examples of the base used in this reaction include an inorganic base and an organic base. As the base, an organic base is preferable. Specifically, examples thereof include triethylamine, N,N-diisopropylethylamine, 4-methylmorpholine, pyridine, N,N-dimethylaminopyridine, and the like.

The used amount of the base is only required to be 1 to 50 times and preferably 1 to 10 times the molar amount of the compound represented by Formula [4].

The used amount of the compound represented by Formula [5] is not particularly limited, but is only required to be 1 to 10 times (v/w) the amount of the compound represented by Formula [4].

This reaction is required only to carry out at -30°C to 150°C, preferably at 0°C to 100°C for 5 minutes to 48 hours.

(1-3)

As the compound represented by Formula [2A], for example, tert-butyl(2-((tert-butoxycarbonyl)amino)ethyl)(2-hydroxyethyl)carbamate, tert-butyl(2-((2-hydroxyethyl)(methyl)amino)ethyl)carbamate, and the like are known.

The compound represented by Formula [6A] can be manufactured by reacting the compound represented by Formula [2A] with the compound represented by Formula [3] in the presence of a base, and then reacting the compound represented by Formula [4A] with the compound represented by Formula [5] in the presence of a base.

This reaction may be performed according to the manufacturing methods (1-1) and (1-2).

(1-4)

The compound represented by Formula [6] can be manufactured by deprotecting the compound represented by Formula [6A].

This reaction may be performed, for example, according to the method described in T. W. Greene et al., Protective Groups in Organic Synthesis, 4th Edition, pp. 696-926, 2007, John Wiley & Sons, INC.

[Manufacturing method 2]

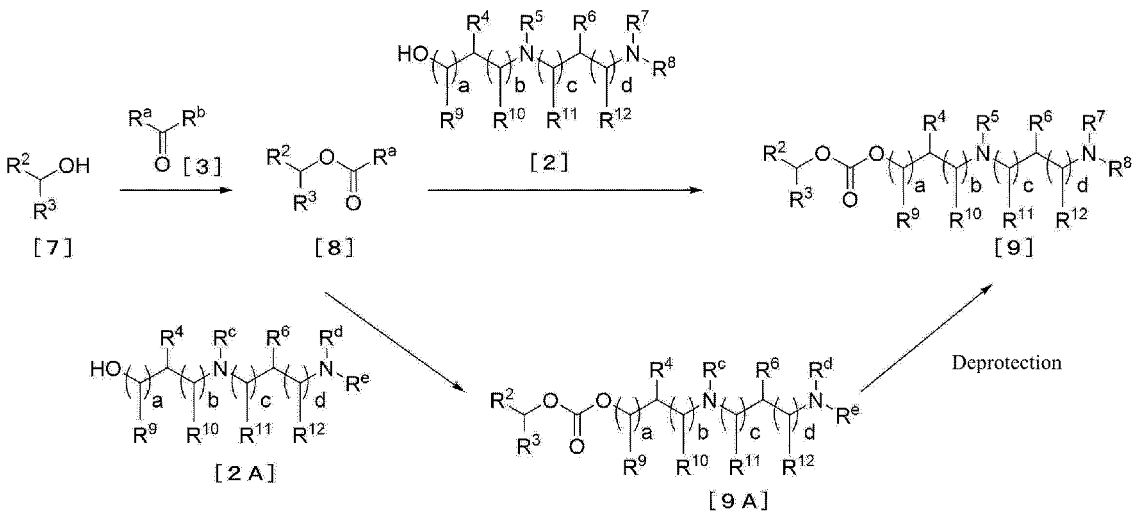


"In the formula, $R^a$ and $R^b$ each represent a leaving group; $R^c$, $R^d$, and $R^e$ each represent an amino protecting group or an imino protecting group; and $R^1$, $R^2$, $R^3$, $R^4$, $R^5$, $R^6$, $R^7$, $R^8$, $R^9$, $R^{10}$, $R^{11}$, and $R^{12}$ have the same definitions as $R^1$, $R^2$, $R^3$, $R^4$, $R^5$, $R^6$, $R^7$, $R^8$, $R^9$, $R^{10}$, $R^{11}$, and $R^{12}$ described above." Examples of the leaving group include a chloro group, a fluoro group, a bromo group, a trichloromethoxy group, a 4-nitro-phenoxy group, a 2,4-dinitrophenoxy group, a 2,4,6-trichlorophenoxy group, a pentafluorophenoxy group, a 2,3,5,6-tetrafluorophenoxy group, an imidazolyl group, a triazolyl group, a 3,5-dioxo-4-methyl-1,2,4-oxadiazolidyl group, a N-hydroxysuccinimidyl group, and the like. Examples of the amino protecting group and the imino protecting group include a tert-butoxycarbonyl group, a benzyloxycarbonyl group, a 2-nitrobenzenesulfonyl group, a benzyl group, and the like.

(2-1)

As the compound represented by Formula [3], for example, 4-nitrophenyl chloroformate, 1,1'-carbonyldiimidazole, triphosgene, phosgene, and the like are known.

The compound represented by Formula [8] can be manufactured by reacting the compound represented by Formula [7] with the compound represented by Formula [3] in the presence of a base.

This reaction may be performed according to the manufacturing method (1-1).

(2-2)

The compound represented by Formula [9] can be manufactured by reacting the compound represented by Formula [8] with the compound represented by Formula [2] in the presence of a base.

The solvent used in this reaction is not particularly limited as long as the solvent does not affect the reaction. Examples of the solvent include halogenated hydrocarbons, ethers, esters, amides, nitriles, sulfoxides, and aromatic hydrocarbons. These solvents may be used by being mixed together.

As the solvent, for example, ethers are preferable, and tetrahydrofuran is more preferable.

The used amount of the solvent is not particularly limited, but is only required to be 1 to 500 times (v/w) the amount of the compound represented by Formula [8].

Examples of the base used in this reaction include an inorganic base and an organic base. As the base, an organic base is preferable. Specifically, examples thereof include triethylamine, N,N-diisopropylethylamine, 4-methylmorpholine, pyridine, N,N-dimethylaminopyridine, and the like.

The used amount of the base is only required to be 1 to 50 times and preferably 1 to 10 times the molar amount of the compound represented by Formula [8].

The used amount of the compound represented by Formula [2] is not particularly limited, but is only required to be 1 to 10 times (v/w) the amount of the compound represented by Formula [8].

This reaction is required only to carry out at -30°C to 150°C, preferably at 0°C to 100°C for 5 minutes to 48 hours.

(2-3)

As the compound represented by Formula [2A], for example, tert-butyl(2-((tert-butoxycarbonyl)amino)ethyl)(2-hydroxyethyl)carbamate, tert-butyl(2-((2-hydroxyethyl)(methyl)amino)ethyl)carbamate, and the like are known.

The compound represented by Formula [9] can be manufactured by reacting the compound represented by Formula [8] with the compound represented by Formula [2A] in the presence of a base, and then deprotecting the compound represented by Formula [9A] in the presence of a base.

This reaction may be performed according to the manufacturing methods (2-2) and (1-4).

[Manufacturing method 3]

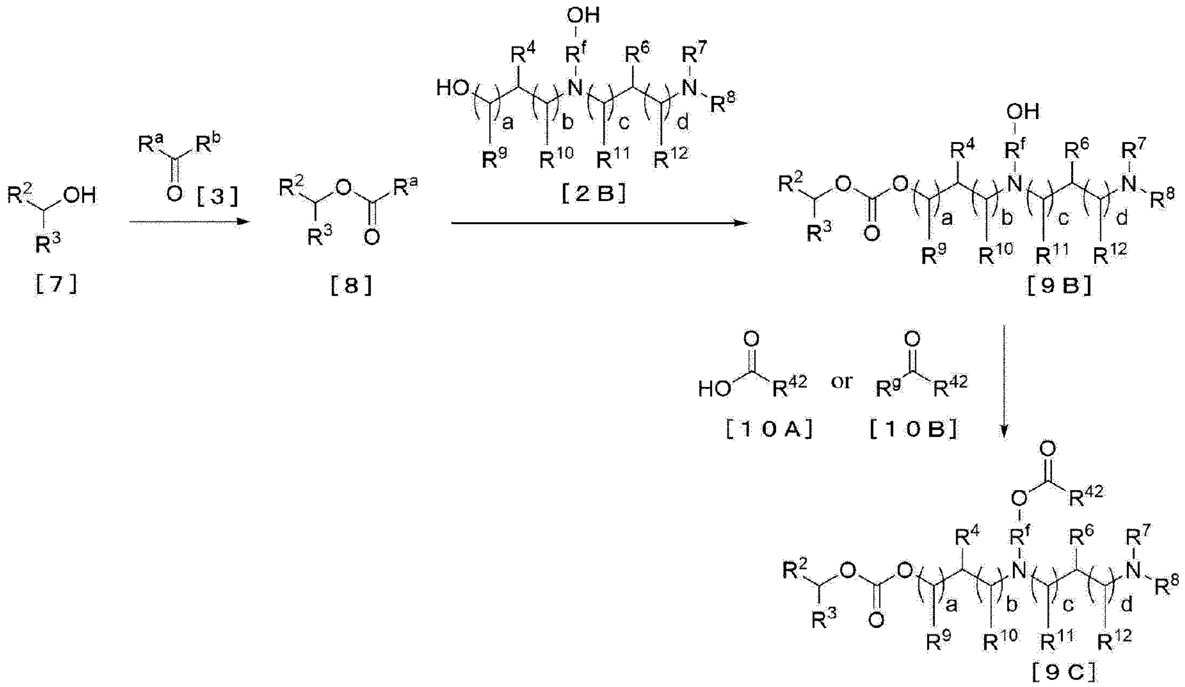

[7] [3] [8] [2B] [9B]

[10A] or [10B]

[9C]

"In the formula, $R^a$, $R^b$, and $R^g$ each represent a leaving group; $R^f$ represents an alkyl group having 1 to 18 carbon atoms; and $R^1$, $R^2$, $R^3$, $R^4$, $R^5$, $R^6$, $R^7$, $R^8$, $R^9$, $R^{10}$, $R^{11}$, $R^{12}$, and $R^{42}$ have the same definitions as $R^1$, $R^2$, $R^3$, $R^4$, $R^5$, $R^6$, $R^7$, $R^8$, $R^9$, $R^{10}$, $R^{11}$, $R^{12}$, and $R^{42}$ described above." Examples of the leaving group include a chloro group, a fluoro group, a bromo group, a trichloromethoxy group, a 4-nitro-phenoxy group, a 2,4-dinitrophenoxy group, a 2,4,6-trichlorophenoxy group, a pentafluorophenoxy group, a 2,3,5,6-tetrafluorophenoxy group, an imidazolyl group, a triazolyl group, a 3,5-dioxo-4-methyl-1,2,4-oxadiazolidyl group, a N-hydroxysuccinimidyl group, and the like.

(3-1)

As the compound represented by Formula [3], for example, 4-nitrophenyl chloroformate, 1,1'-carbonyldiimidazole, triphosgene, phosgene, and the like are known.

The compound represented by Formula [8] can be manufactured by reacting the compound represented by Formula [7] with the compound represented by Formula [3] in the presence of a base.

This reaction may be performed according to the manufacturing method (1-1).

(3-2)

As the compound represented by Formula [2B], for example, 2,2'-((2-(diethylamino)ethyl)azanediyl)bis(ethan-1-ol), 2,2'-((3-(diethylamino)propyl)azanediyl)bis(ethan-1-ol), and the like are known.

The compound represented by Formula [9B] can be manufactured by reacting the compound represented by Formula [8] with the compound represented by Formula [2B] in the presence of a base.

This reaction may be performed according to the manufacturing method (2-2).

(3-3)

As the compound represented by Formula [10A], for example, dodecanoic acid, decanoic acid, nonanoic acid, octanoic acid, and the like are known.

The compound represented by Formula [9C] can be manufactured by reacting the compound represented by Formula [9B] with the compound represented by Formula [10A] in the presence of a condensing agent or an acid halide and in the presence of a base.

The solvent used in this reaction is not particularly limited as long as the solvent does not affect the reaction. Examples of the solvent include halogenated hydrocarbons, ethers, esters, amides, nitriles, sulfoxides, and aromatic hydrocarbons. These solvents may be used by being mixed together.

As the solvent, for example, ethers are preferable, and tetrahydrofuran is more preferable.

The used amount of the solvent is not particularly limited, but is required to be 1 to 500 times (v/w) the amount of the compound represented by Formula [9B].

Examples of the base used in this reaction include an inorganic base and an organic base. As the base, an

organic base is preferable. Specifically, examples thereof include triethylamine, N,N-diisopropylethylamine, 4-methylmorpholine, pyridine, N,N-dimethylaminopyridine, and the like.

The used amount of the base is only required to be 1 to 50 times and preferably 1 to 10 times the molar amount of the compound represented by Formula [9B].

Examples of the condensing agent used in this reaction include carbodiimides such as N,N'-dicyclohexylcarbodiimide and 1-ethyl-3-(3-dimethylaminopropyl)carbodiimide; carbonyls such as carbonyldiimidazole; acid azides such as diphenylphosphoryl azide; acid cyanides such as diethylphosphoryl cyanide; 2-ethoxy-1-ethoxycarbonyl-1,2-dihydroquinoline; O-benzotriazol-1 -yl-1,1,3,3-tetramethyluronium=hexafluorophosphate; O-(7-azabenzotriazol-1-yl)-1,1,3,3-tetramethyluronium=hexafluorophosphate; and the like.

Examples of the acid halide used in this reaction include carboxylic acid halides such as acetyl chloride and trifluoroacetyl chloride; sulfonic acid halides such as methanesulfonyl chloride and tosyl chloride; chloroformic acid esters such as ethyl chloroformate and isobutyl chloroformate; and the like.

The used amount of the compound represented by Formula [10A] is not particularly limited, but is only required to be 1 to 10 times (v/w) the amount of the compound represented by Formula [9B].

This reaction is required only to carry out at -30°C to 150°C, preferably at 0°C to 100°C for 5 minutes to 48 hours.

(3-4)

As the compound represented by Formula [10B], for example, dodecanoic acid chloride, decanoic acid chloride, nonanoic acid chloride, octanoic acid chloride, and the like are known.

The compound represented by Formula [9C] can be manufactured by reacting the compound represented by Formula [9B] with the compound represented by Formula [10B] in the presence of a base.

The compound represented by Formula [10B] can be manufactured by reacting the compound represented by Formula [10A] with thionyl chloride, oxalyl chloride, or the like.

The solvent used in this reaction is not particularly limited as long as the solvent does not affect the reaction. Examples of the solvent include halogenated hydrocarbons, ethers, esters, amides, nitriles, sulfoxides, and aromatic hydrocarbons. These solvents may be used by being mixed together.

As the solvent, for example, ethers are preferable, and tetrahydrofuran is more preferable.

The used amount of the solvent is not particularly limited, but is required to be 1 to 500 times (v/w) the amount of the compound represented by Formula [9B].

Examples of the base used in this reaction include an inorganic base and an organic base.

The used amount of the base is only required to be 1 to 50 times and preferably 1 to 10 times the molar amount of the compound represented by Formula [9B].

The used amount of the compound represented by Formula [10B] is not particularly limited, but is only required to be 1 to 10 times (v/w) the amount of the compound represented by Formula [2B].

This reaction is required only to carry out at -30°C to 150°C, preferably at 0°C to 100°C for 5 minutes to 48 hours.

Next, the synthesis of the compound represented by Formula [2], which is a raw material for manufacturing the compound according to the embodiment of the present invention, will be described.

[Manufacturing method 4]

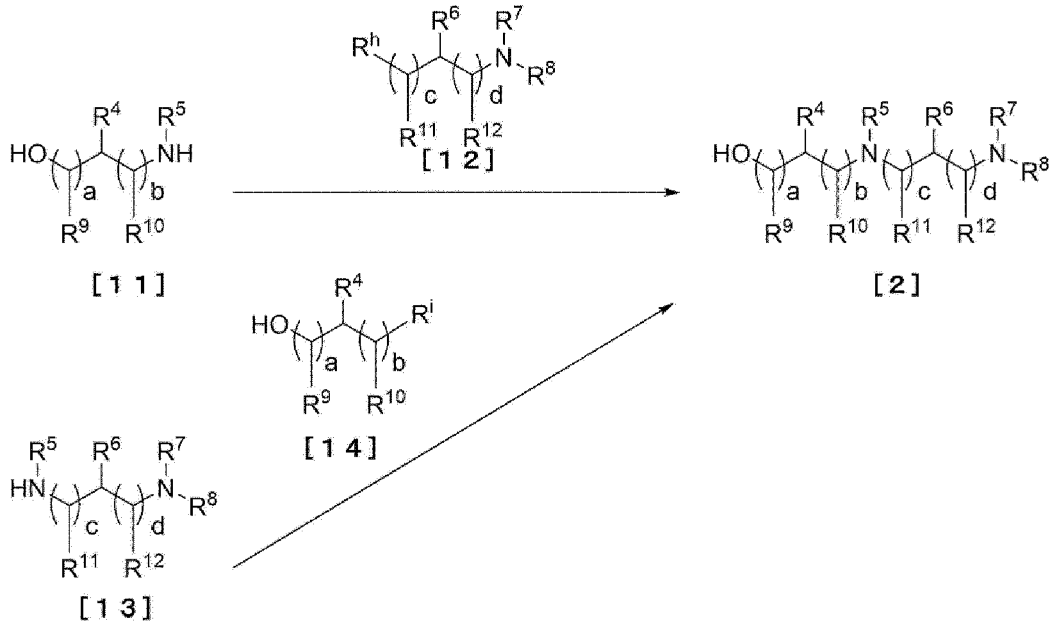

[12] [11] [2] [14] [13]

"In the formula, $R^h$ and $R^i$ each represent a leaving group; and $R^4$, $R^5$, $R^6$, $R^7$, $R^8$, $R^9$, $R^{10}$, $R^{11}$, and $R^{12}$ have the same definitions as $R^4$, $R^5$, $R^6$, $R^7$, $R^8$, $R^9$, $R^{10}$, $R^{11}$, and $R^{12}$ described above." Examples of the leaving group include a chloro group, a bromo group, an iodo group, a methanesulfonyl group, a 4-toluenesulfonyl group, a chloromethanesulfonyl group, a trifluoromethanesulfonyl group, and the like.

(4-1)

As the compound represented by Formula [12], for example, 2-chloro-N,N-dimethylethan-1-amine, 4-(2-chloroethyl)morpholine and 2-chloro-N,N-diethylethan-1-amine, 2-bromo-N,N-diethylethan-1-amine, 3-chloro-N,N-diethylethan-1-amine, and the like are known.

The compound represented by Formula [2] can be manufactured by reacting the compound represented by Formula [11] with the compound represented by Formula [12] in the presence or absence of a base.

The solvent used in this reaction is not particularly limited as long as the solvent does not affect the reaction. Examples of the solvent include alcohols, halogenated hydrocarbons, ethers, esters, amides, nitriles, sulfoxides, aromatic hydrocarbons, and water. These solvents may be used by being mixed together.

The used amount of the solvent is not particularly limited, but is only required to be 1 to 500 times (v/w) the amount of the compound represented by Formula [11].

Examples of the base used in this reaction include an inorganic base and an organic base. The used amount of the base is only required to be 1 to 10,000 times and preferably 1 to 5,000 times the molar amount of the compound represented by Formula [11].

The used amount of the compound represented by Formula [12] is not particularly limited, but is only required to be 1 to 10 times (v/w) the amount of the compound represented by Formula [11].

This reaction is required only to carry out at -30°C to 150°C, preferably at 0°C to 100°C for 5 minutes to 48 hours.

(4-2)

As the compound represented by Formula [14], for example, 2-bromoethan-1-ol, 3-bromopropan-1-ol, and the like are known.

The compound represented by Formula [2] can be manufactured by reacting the compound represented by Formula [13] with the compound represented by Formula [14] in the presence or absence of a base.

This reaction may be performed according to the manufacturing method (4-1).

[Manufacturing method 5]

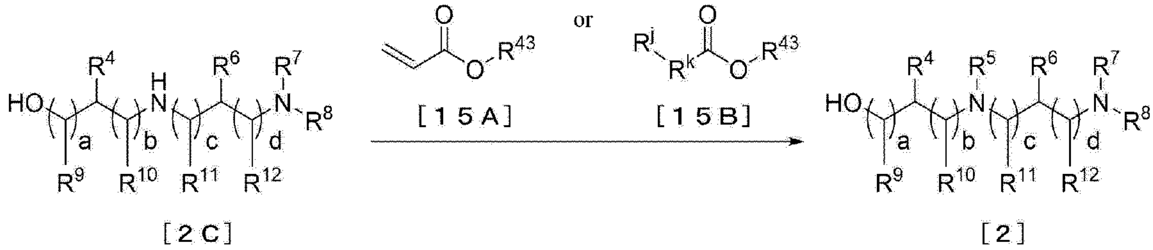

[2C] [15A] or [15B] [2]

"In the formula, $R^j$ represents a leaving group; $R^k$ represents an alkyl group having 1 to 18 carbon atoms; and $R^4$, $R^5$, $R^6$, $R^7$, $R^8$, $R^9$, $R^{10}$, $R^{11}$, $R^{12}$, and $R^{43}$ have the same definitions as $R^4$, $R^5$, $R^6$, $R^7$, $R^8$, $R^9$, $R^{10}$, $R^{11}$, $R^{12}$, and $R^{43}$ described above." Examples of the leaving group include a chloro group, a bromo group, an iodo group, a methanesulfonyl group, a 4-toluenesulfonyl group, a chloromethanesulfonyl group, a trifluoromethanesulfonyl group, and the like.

(5-1)
As the compound represented by Formula [15A], for example, heptyl acrylate and the like are known.

The compound represented by Formula [2] can be manufactured by reacting the compound represented by Formula [2C] with the compound represented by Formula [15A] in the presence or absence of a base.

The solvent used in this reaction is not particularly limited as long as the solvent does not affect the reaction. Examples of the solvent include alcohols, halogenated hydrocarbons, ethers, esters, amides, nitriles, sulfoxides, aromatic hydrocarbons, and water. These solvents may be used by being mixed together.

As the solvent, for example, ethers or nitriles are preferable, and tetrahydrofuran or acetonitrile is more preferable.

The used amount of the solvent is not particularly limited, but is only required to be 1 to 500 times (v/w) the amount of the compound represented by Formula [2C].

Examples of the base used in this reaction include an inorganic base and an organic base.

The used amount of the base is only required to be 1 to 10,000 times and preferably 1 to 5,000 times the molar amount of the compound represented by Formula [2C].

The used amount of the compound represented by Formula [15A] is not particularly limited, but is required to be 1 to 10 times (v/w) the amount of the compound represented by Formula [13].

This reaction is required only to carry out at -30°C to 150°C, preferably at 0°C to 100°C for 5 minutes to 48 hours.

(5-2)
As the compound represented by Formula [15B], for example, heptyl 3-chloropropanoate and the like are known.

The compound represented by Formula [2] can be manufactured by reacting the compound represented by Formula [2C] with the compound represented by Formula [15B] in the presence or absence of a base.

This reaction may be performed according to the manufacturing method (4-1).

[Manufacturing method 6]

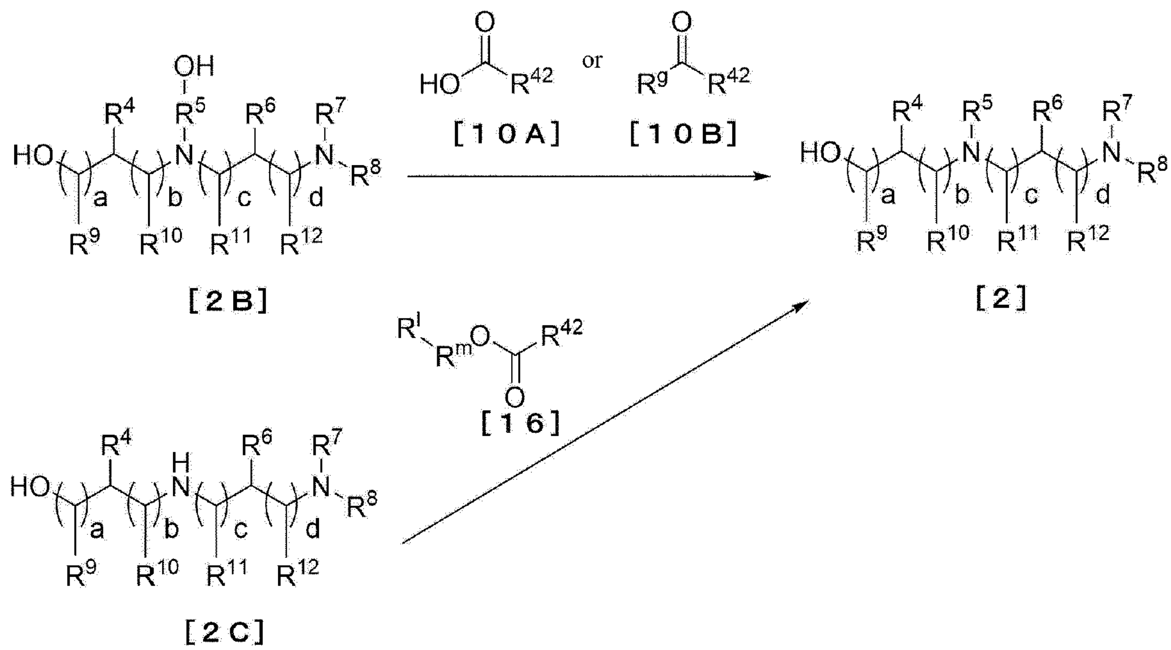

[10A] or [10B]

[2B]

[2]

[16]

[2C]

"In the formula, $R^9$ and $R^l$ each represent a leaving group; $R^m$ represents an alkyl group having 1 to 18 carbon atoms; and $R^4$, $R^5$, $R^6$, $R^7$, $R^8$, $R^9$, $R^{10}$, $R^{11}$, $R^{12}$, and $R^{42}$ have the same definitions as $R^4$, $R^5$, $R^6$, $R^7$, $R^8$, $R^9$, $R^{10}$, $R^{11}$, $R^{12}$, and $R^{42}$ described above." Examples of the leaving group include a chloro group, a bromo group, an iodo group, a methanesulfonyl group, a 4-toluenesulfonyl group, a chloromethanesulfonyl group, a trifluoromethanesulfonyl group, a trichloromethoxy group, a 4-nitro-phenoxy group, a 2,4-dinitrophenoxy group, a 2,4,6-trichlorophenoxy group, a pentafluorophenoxy group, a 2,3,5,6-tetrafluorophenoxy group, an imidazolyl group, a triazolyl group, a 3,5-dioxo-4-methyl-1,2,4-oxadiazolidyl group, a N-hydroxysuccinimidyl group, and the like.

(6-1)

As the compound represented by Formula [10A], for example, dodecanoic acid, decanoic acid, nonanoic acid, octanoic acid, and the like are known.

The compound represented by Formula [2] can be manufactured by reacting the compound represented by Formula [2B] with the compound represented by Formula [10A] in the presence of a condensing agent or an acid halide and in the presence of a base.

This reaction may be performed according to the manufacturing method (3-3).

(6-2)

As the compound represented by Formula [10B], for example, dodecanoic acid chloride, decanoic acid chloride, nonanoic acid chloride, octanoic acid chloride, and the like are known.

The compound represented by Formula [2] can be manufactured by reacting the compound represented by Formula [2B] with the compound represented by Formula [10B] in the presence of a base.

This reaction may be performed according to the manufacturing method (3-4).

(6-3)

As the compound represented by Formula [16], for example, heptyl 3-chloropropanoate and the like are known.

The compound represented by Formula [2] can be manufactured by reacting the compound represented by Formula [2C] with the compound represented by Formula [16] in the presence or absence of a base.

This reaction may be performed according to the manufacturing method (4-1).

[Manufacturing method 7]

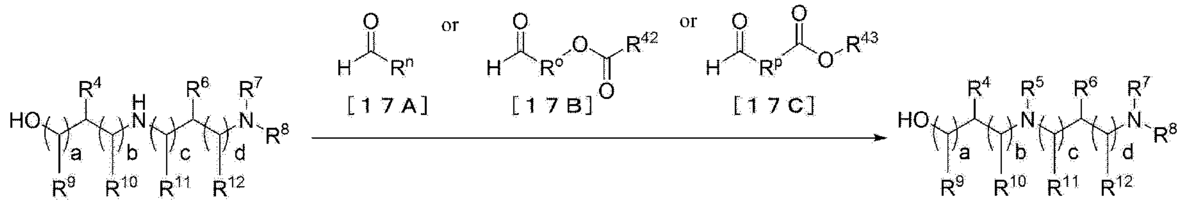

[17A] or [17B] or [17C]

[2C] [2]

"In the formula, $R^n$, $R^o$, and $R^p$ each represent an alkyl group having 1 to 17 carbon atoms; and $R^4$, $R^5$, $R^6$, $R^7$, $R^8$, $R^9$, $R^{10}$, $R^{11}$, $R^{12}$, $R^{42}$, and $R^{43}$ have the same definitions as $R^4$, $R^5$, $R^6$, $R^7$, $R^8$, $R^9$, $R^{10}$, $R^{11}$, $R^{12}$, $R^{42}$, and $R^{43}$ described above."

(7-1)

As the compound representd by Formula [17A], for example, formaldehyde, acetaldehyde, propanal, butanal, pentanal, hexanal, heptanal, octanal, and the like are known.

The compound represented by Formula [2] can be manufactured by reacting the compound represented by Formula [2C] with the compound represented by Formula [17A] in the presence of a reducing agent, in the presence or absence of a reducing catalyst, and in the presence or absence of an acid.

The solvent used in this reaction is not particularly limited as long as the solvent does not affect the reaction. Examples of the solvent include alcohols, halogenated hydrocarbons, ethers, esters, amides, nitriles, sulfoxides, aromatic hydrocarbons, and water. These solvents may be used by being mixed together.

The used amount of the solvent is not particularly limited, but is only required to be 1 to 500 times (v/w) the amount of the compound represented by Formula [2C].

Examples of the acid used in this reaction include an inorganic acid and an organic acid.

The used amount of the acid is only required to be 0.01 to 10,000 times and preferably 0.05 to 100 times the molar amount of the compound represented by Formula [2C].

Examples of the reducing agent used in this reaction include sodium triacetoxyborohydride, sodium cyanoborohydride, 2-picolineborane, formic acid, hydrogen, and the like.

Examples of the reducing catalyst used in this reaction include palladium-carbon, palladium hydroxide-carbon, platinum-carbon, rhodium-carbon, ruthenium-carbon, and the like.

The used amount of the compound represented by Formula [17A] is not particularly limited, but is required to be 1 to 10 times (v/w) the amount of the compound represented by Formula [13].

This reaction is required only to carry out at -30°C to 150°C, preferably at 0°C to 100°C for 5 minutes to 48 hours.

(7-2)

As the compound represented by Formula [17B], for example, 2-oxoethyloctanoate, 2-oxoethylnonanoate, and the like are known.

The compound represented by Formula [2] can be manufactured by reacting the compound represented by Formula [2C] with the compound represented by Formula [17B] in the presence of a reducing agent, in the presence or absence of a reducing catalyst, and in the presence or absence of an acid.

This reaction may be performed according to the manufacturing method (7-1).

(7-3)

As the compound represented by Formula [17C], for example, heptyl 3-oxopropanoate, octyl 3-oxopropanoate, and the like are known.

The compound represented by Formula [2] can be manufactured by reacting the compound represented by Formula [2C] with the compound represented by Formula [17C] in the presence of a reducing agent, in the presence or absence of a reducing catalyst, and in the presence or absence of an acid.

This reaction may be performed according to the manufacturing method (7-1).

In a case where the compounds used in the aforementioned manufacturing methods have isomers (for example, an optical isomer, a geometric isomer, a tautomer, and the like), these isomers can also be used.

Furthermore, in a case where the compounds are in the form of solvates, hydrates, and crystals of various shapes, these solvates, hydrates, and crystals of various shapes can also be used.

In a case where the compounds used in the aforementioned manufacturing methods have, for example, an amino group, a hydroxyl group, a carboxyl group, these groups can be protected with ordinary protecting groups in advance, and these protecting groups can be eliminated by known methods after the reaction.

The compounds obtained by the aforementioned manufacturing methods can be induced into other compounds by being subjected to known reaction such as condensation, addition, oxidation, reduction, transition, substitution, halogenation, dehydration, or hydrolysis, or by being subjected to these reactions that are appropriately combined.

In the lipid composition according to the embodiment of the present invention, the content of the lipid represented

by Formula (1) or a salt thereof with respect to the total lipids is preferably 20 mol% or more and 55 mol% or less, and more preferably 22 mol% or more and 55 mol% or less. In a case where the nucleic acid is mRNA, the content of the lipid represented by Formula (1) or a salt thereof with respect to the total lipids is more preferably 25 mol% or more and 52 mol% or less, and still more preferably 32 mol% or more and 48 mol% or less. In a case where the nucleic acid is siRNA, the content of the lipid represented by Formula (1) or a salt thereof with respect to the total lipids is more preferably 32 mol% or more and 55 mol% or less, still more preferably 37 mol% or more and 55 mol% or less, and particularly preferably 47 mol% or more and 55 mol% or less.

<Sterols>

The lipid composition according to the embodiment of the present invention contains sterols.

In the lipid composition according to the embodiment of the present invention, since the oil phase contains sterols, the membrane fluidity can be reduced and the effect to stabilize the lipid particles can be obtained.

The sterols are not particularly limited, and examples thereof include cholesterol, phytosterol (sitosterol, stigmasterol, fucosterol, spinasterol, brassicasterol, and the like), ergosterol, cholestanone, cholestenone, coprostanol, cholesteryl-2'-hydroxyethyl ether, cholesteryl-4'-hydroxybutyl ether, and the like. Among these, cholesterol or a derivative thereof is preferable.

In the lipid composition according to the embodiment of the present invention, the content of the sterols with respect to the total lipids is preferably 20 mol% to 70 mol%. In a case where the nucleic acid is mRNA, the content of the sterols with respect to the total lipids is more preferably 30 mol% to 66 mol%, still more preferably 30 to 60 mol%. In a case where the nucleic acid is siRNA, the content of the sterols with respect to the total lipids is more preferably 45 mol% to 68 mol%, still more preferably 45 mol% to 63 mol%, and particularly preferably 45 mol% to 52 mol%.

<Lipid having nonionic hydrophilic polymer structure>

The lipid composition according to the embodiment of the present invention contains a lipid having a nonionic hydrophilic polymer structure.

Since the oil phase contains the lipid having a nonionic hydrophilic polymer structure, the effect to stably disperse the lipid particles can be obtained.

The nonionic hydrophilic polymer is not particularly limited, and examples thereof include a nonionic vinyl-based polymer, a nonionic polyamino acid, a nonionic polyester, a nonionic polyether, a nonionic natural polymer, a nonionic modified natural polymer, and a block polymer or a graft copolymer having two or more kinds of these polymers as constitutional units.

Among these nonionic hydrophilic polymers, a nonionic polyether, a nonionic polyester, a nonionic polyamino acid, or a nonionic synthetic polypeptide is preferable, a nonionic polyether or a nonionic polyester is more preferable, a nonionic polyether or a nonionic monoalkoxy polyether is still more preferable, and polyethylene glycol (hereinafter, polyethylene glycol will be also called PEG) is particularly preferable. That is, as the lipid having a nonionic hydrophilic polymer structure, a lipid having a polyethylene glycol structure is preferable.

The lipid having a nonionic hydrophilic polymer is not particularly limited, and examples thereof include PEG-modified phosphoethanolamine, a diacylglycerol PEG derivative, a dialkylglycerol PEG derivative, a cholesterol PEG derivative, a ceramide PEG derivative, and the like. Among these, diacylglycerol PEG is preferable. That is, as the lipid having a polyethylene glycol structure, a lipid having a diacylglycerol structure and a polyethylene glycol structure is preferable. The acyl group of the diacylglycerol moiety is more preferably an acyl group having 12 to 22 carbon atoms.

In a case where the lipid having a nonionic hydrophilic polymer has a PEG chain, the weight-average molecular weight of the PEG chain is preferably 500 to 5,000, and more preferably 750 to 3,000.

The nonionic hydrophilic polymer chain may be branched and may have a substituent such as a hydroxymethyl group.

In the lipid composition according to the embodiment of the present invention, the content of the lipid having a nonionic hydrophilic polymer structure with respect to the total lipids is preferably 0.2 mol% to 10 mol%, more preferably 0.2 mol% to 5 mol%. In a case where the nucleic acid is mRNA, the content of the lipid having a nonionic hydrophilic polymer structure with respect to the total lipids is more preferably 0.2 mol% to 5 mol%, still more preferably 0.2 to 3 mol%, and most preferably 0.5 mol% to 2.5 mol%. In a case where the nucleic acid is siRNA, the content of the lipid having a nonionic hydrophilic polymer structure with respect to the total lipids is more preferably 0.2 mol% to 2.3 mol%, still more preferably 1.2 mol% to 2.3 mol%.

<Zwitterionic lipid>

The lipid composition according to the embodiment of the present invention may or may not contain a zwitterionic

lipid.

As the zwitterionic lipid, phospholipid is preferable. The phospholipid is not particularly limited, and examples thereof include phosphatidylcholine, phosphatidylethanolamine, sphingomyelin, and the like. Among these, phosphatidylcholine and phosphatidylethanolamine are preferable. The zwitterionic lipid may be used either singly or in combination of two or more different zwitterionic lipids.

The phosphatidylcholine is not particularly limited, and examples thereof include soybean lecithin (SPC), hydrogenated soybean lecithin (HSPC), egg yolk lecithin (EPC), hydrogenated egg yolk lecithin (HEPC), 1,2-dimyristoyl-sn-glycero-3-phosphocholine (DMPC), 1,2-dipalmitoyl-sn-glycero-3-phosphocholine (DPPC), 1,2-distearoyl-sn-glycero-3-phosphocholine (DSPC), 1-palmitoyl-2-oleoyl-sn-glycero-3-phosphocholine (POPC), 1,2-dioleoyl-sn-glycero-3-phosphocholine (DOPC), and the like.

Among the above, 1,2-dimyristoyl-sn-glycero-3-phosphocholine (DMPC), 1,2-dipalmitoyl- sn-glycero-3 -pho sphocholine (DPPC), 1,2-distearoyl-sn-glycero-3-phosphocholine (DSPC), and 1,2-dioleoyl-sn-glycero-3-phosphocholine (DOPC) are more preferable.

The phosphatidylethanolamine is not particularly limited, and examples thereof include 1,2-dimyristoyl- sn-glycero-3 -pho sphoethanolamine (DMPE), 1,2-dipalmitoyl-sn-glycero-3-phosphoethanolamine (DPPE), 1,2-distearoyl-sn-glycero-3-phosphoethanolamine (DSPE), 1,2-dioleoyl-sn-glycero-3-phosphoethanolamine (DOPE), 1,2-dilinoleoyl-sn-glycero-3-phosphoethanolamine (DLoPE), 1,2-diphytanoyl-sn-glycero-3-phosphoethanolamine (D(Phy)PE), 1-palmitoyl-2-oleoyl-sn-glycero-3-phosphoethanolamine (POPE), 1,2-ditetradecyl-sn-glycero-3-phosphoethanolamine, 1,2-dihexadecyl-sn-glycero-3-phosphoethanolamine, 1,2-dioctadecyl-sn-glycero-3-phosphoethanolamine, 1,2-diphytanyl-sn-glycero-3-phosphoethanolamine, and the like.

Among the above, 1,2-dioleoyl-sn-glycero-3-phosphoethanolamine (DOPE) is more preferable.

The sphingomyelin is not particularly limited, and examples thereof include egg yolk-derived sphingomyelin, milk-derived sphingomyelin, and the like.

In the lipid composition according to the embodiment of the present invention, the content of the zwitterionic lipid with respect to the total lipids is preferably 0 mol% to 35 mol%. In a case where the nucleic acid is mRNA, the content of the zwitterionic lipid with respect to the total lipids is preferably 0 mol% to 35 mol%, more preferably 0 mol% to 30 mol%, and still more preferably 0 mol% to 25 mol%. In a case where the nucleic acid is siRNA, the content of the zwitterionic lipid with respect to the total lipids is preferably 0 mol%.

In a case where the lipid composition according to the embodiment of the present invention contains the zwitterionic lipid, the lower limit of the content of the zwitterionic lipid with respect to the total lipids is not particularly limited, but is generally 0.5 mol% or more, preferably 1 mol% or more, and more preferably 2 mol% or more.

<Nucleic acid>

The lipid composition according to the embodiment of the present invention contains a nucleic acid. Examples of the nucleic acid include a plasmid, single-stranded DNA, double-stranded DNA, small interfering RNA (siRNA), micro RNA (miRNA), mRNA, an antisense nucleic acid, ribozyme, an aptamer, saRNA, sgRNA, and the like. The lipid composition may contain any of these. In addition, the lipid composition may contain a modified nucleic acid.

In the lipid composition according to the embodiment of the present invention, the content of nucleic acid with respect to the total lipids is preferably 0.5% to 50% by mass, more preferably 1% to 25% by mass, still more preferably 1.5% to 20% by mass, and particularly preferably 2% to 15% by mass. The content rate of the total lipids to the nucleic acid is preferably 2 to 200, more preferably 4 to 200, still more preferably 6 to 100, and particularly preferably 8 to 75.

<Method for manufacturing lipid composition>

The method for manufacturing the lipid composition according to the embodiment of the present invention will be described.

The method for manufacturing the lipid composition is not limited. For example, the lipid composition can be manufactured by a method in which all of the constituent components of the lipid composition or some of oil-soluble components of the lipid composition are dissolved in an organic solvent or the like to form an oil phase, water-soluble components of the lipid composition are dissolved in water to form a water phase, and then the oil phase and the water phase are mixed together. A micromixer may be used for mixing, or an emulsification using an emulsifying machine such as a homogenizer, an ultrasonic emulsifying machine, a high-pressure injection emulsifying machine, or the like may be performed.

Alternatively, the lipid composition can also be manufactured by a method in which a lipid-containing solution is subjected to evaporation to dryness using an evaporator under reduced pressure or subjected to spray drying using a spray drier, so that a dried mixture containing a lipid is prepared, and then the mixture is added to an aqueous solvent and further emulsified using the aforementioned emulsifying machine or the like.

One example of the method for manufacturing the lipid composition containing a nucleic acid is a method including

Step (a) of dissolving the constituent components of the lipid composition in an organic solvent to obtain an oil phase;
Step (b) of mixing the oil phase obtained in Step (a) with a water phase containing a water-soluble component such as a nucleic acid;
Step (c) of diluting the mixed solution containing the oil phase and the water phase obtained in Step (b) to obtain a dispersion liquid of lipid particles; and
Step (d) of removing the organic solvent from the dispersion liquid of lipid particles obtained in Step (c); and
Step (e) of adjusting the concentration of the dispersion liquid of lipid particles obtained in Step (d).

Step (a) includes a process of dissolving the constituent components classified as lipids in an organic solvent (an alcohol such as ethanol, an ester, or the like). The total lipid concentration after the dissolution of lipids in an organic solvent is not particularly limited, but is generally 1 mmol/L to 100 mmol/L, preferably 5 mmol/L to 50 mmol/L, and more preferably 10 mmol/L to 30 mmol/L.
In Step (b), the water phase can be obtained by dissolving a nucleic acid (for example, siRNA, an antisense nucleic acid, micro RNA (miRNA), mRNA) in water or a buffer solution. If necessary, a component such as an antioxidant can be added. The mixing ratio (mass ratio) of water phase:oil phase is preferably 5:1 to 1:1 and more preferably 4:1 to 2:1.
In Step (d), the method of removing the organic solvent from the dispersion liquid of lipid particles is not particularly limited, and a general method can be used. For example, the organic solvent can be removed by dialyzing the dispersion liquid with the phosphate buffered saline, a sucrose-Tris buffer solution, or the like.
In Step (e), the concentration of the dispersion liquid obtained in Step (d) can be adjusted. In a case of diluting the dispersion liquid, the dispersion liquid can be diluted to an appropriate concentration using phosphate buffered saline, physiological saline, sucrose-Tris buffer solution, or the like as a diluent. In a case of concentrating the dispersion liquid, the dispersion liquid obtained in Step (d) can be concentrated by such as ultrafiltration using an ultrafiltration membrane. It is preferable to use the concentrated dispersion as it is. Alternatively, it is preferable to adjust the concentrated dispersion liquid to a desired concentration by using the aforementioned diluent after the concentration.
An excipient and a buffer may be added as a solution that can be used for dialysis in Step (d) and dilution in Step (e). Examples of the excipient include saccharides. Examples of the saccharides include sucrose, trehalose, maltose, glucose, lactose, fructose, mannitol, sorbitol, inositol, xylitol and the like. Examples of the buffer include ACES, BES, Bicine, CAPS, CHES, DIPSO, EPPS, HEPES, HEPPSO, MES, MOPS, MOPSO, TAPS, TAPSO, TES, Tricine and the like.
In order to make a pharmaceutical composition of the dispersion liquid of lipid particles of the present invention, it is preferable to perform sterile filtration. As a filtration method, a hollow fiber membrane, a reverse osmosis membrane, a membrane filter, or the like can be used to remove unnecessary substances from the dispersion liquid of lipid particles. In the present invention, the filtration method is not particularly limited, but it is preferable to filter the dispersion liquid through a filter having a pore diameter capable of sterilization (preferably a filtration sterilization filter with a pore diameter of 0.2 μm). Furthermore, it is preferable that the sterile filtration be performed after Step (c) or Step (d).
In addition, if necessary, the dispersion liquid of lipid particles of the present invention can be frozen or freeze-dried. The dispersion liquid of the lipid particles of the present invention can be frozen or freeze-dried by a general method, and the method is not particularly limited.

<Lipid composition>

It is preferable that the composition according to the embodiment of the present invention be composed of lipid particles. Lipid particles mean particles composed of a lipid, and include a composition having any structure selected from a lipid aggregate in which lipids are aggregated, a micelle, and a liposome. The structure of the lipid particles is not limited to these as long as the lipid particles are a composition containing a lipid. The liposome has a lipid bilayer structure and has a water phase in the inside, and includes a liposome which has a single bilayer membrane, and a multilamellar liposome which has multiple layers stacked together. The present invention may include any of these liposomes.
The form of the lipid particles can be checked by electron microscopy, structural analysis using X-rays, or the like. For example, by a method using Cryo transmission electron microscopy (CryoTEM method), it is possible to check, for example, whether or not a lipid particle is, such as a liposome, a bimolecular lipid membrane structure (lamella structure) and a structure having an inner water layer, and whether or not a lipid particle has a structure having an inner core with a high electron density and packed with constituent components including a lipid. The X-ray small angle scattering (SAXS) analysis also makes it possible to check whether or not a lipid particle has a bimolecular lipid membrane structure (lamella structure).

The particle size of the lipid particles is not particularly limited, but is preferably 10 to 1,000 nm, more preferably 30 to 500 nm, still more preferably 50 to 250 nm, and particularly preferably 50 to 200 nm. The particle size of the lipid particles can be measured by a general method (for example, a dynamic light scattering method, a laser diffraction method, or the like).

<Use of lipid composition>

As one example for utilizing the lipid composition according to the embodiment of the present invention, a nucleic acid (for example, a gene) can be introduced into a cell by introducing the lipid composition containing the nucleic acid into the cell. In addition, in a case where the lipid composition according to the embodiment of the present invention contains a nucleic acid for a pharmaceutical use, the lipid composition can be administered to a living body as a nucleic acid drug. That is, the lipid composition according to the embodiment of the present invention is preferably a composition for introducing nucleic acids into cells.

In a case where the lipid composition according to the embodiment of the present invention is used as a nucleic acid drug, the lipid composition according to the embodiment of the present invention can be administered to a living body singly or by being mixed with a pharmaceutically acceptable carrier (also referred to dosing medium, such as physiological saline or a phosphate buffer solution).

The concentration of the lipid composition (lipid particles) in the mixture with a pharmaceutically acceptable carrier is not particularly limited, but can be set to 0.05% by mass to 90% by mass in general. In addition, other pharmaceutically acceptable additives, for example, a pH adjusting buffer and an osmotic pressure adjusting agent, may be added to the nucleic acid drug containing the lipid composition according to the embodiment of the present invention.

The route of administration when the nucleic acid drug containing the lipid composition according to the embodiment of the present invention is administered is not particularly limited. The nucleic acid drug can be administered by any method. Examples of the administration method include oral administration and parenteral administration (intraarticular administration, intravenous administration, intraarterial administration, subcutaneous administration, intracutaneous administration, intravitreal administration, intraperitoneal administration, intramuscular administration, intravaginal administration, intravesical administration, intrathecal administration, pulmonary administration, rectal administration, colonic administration, buccal administration, nasal administration, intracisternal administration, inhalation, and the like). Parenteral administration is preferable. As the method of administration, intravenous injection, subcutaneous injection, intracutaneous injection, or intramuscular injection is preferable. The nucleic acid drug containing the lipid composition according to the embodiment of the present invention can also be administered by being directly injected into the affected area.

The dosage form of the lipid composition according to the embodiment of the present invention is not particularly limited. In a case of oral administration, the lipid composition according to the embodiment of the present invention can be used in the form of tablets, troches, capsules, pills, suspension, syrup, or the like by being combined with an appropriate excipient. In addition, in a case of parenteral administration, the lipid composition according to the embodiment of the present invention can be appropriately combined with an antioxidant, a buffer, a bacteriostat, and additives such as an isotonic sterile injection, a suspending agent, a solubilizer, a thickener, a stabilizer, and a preservative.

<Nucleic acid delivery carrier>

The lipid composition according to the embodiment of the present invention can retain a nucleic acid at a high encapsulation rate and thereby is extremely useful as a nucleic acid delivery carrier. According to the nucleic acid delivery carrier using the present invention, the nucleic acid and the like can be introduced into the cells, for example, by transfecting cells with the lipid composition in vitro or in vivo. In addition, the nucleic acid delivery carrier using the present invention is also useful as a nucleic acid delivery carrier in nucleic acid drugs. That is, the lipid composition according to the embodiment of the present invention is useful as a composition for in vitro or in vivo (preferably in vivo) delivery of nucleic acids.

Next, the present invention will be described based on examples, but the present invention is not limited thereto.

Examples

Unless otherwise specified, for the purification by column chromatography, an automatic purification device ISOLERA (Biotage) or a medium pressure liquid chromatograph YFLC W-prep 2XY (Yamazen Corporation) was used.

Unless otherwise specified, as a carrier for silica gel column chromatography, Chromatorex Q-Pack SI 50 (FUJI SILYSIA CHEMICAL LTD.) or HIGH FLASH COLUMN W001, W002, W003, W004, or W005 (Yamazen Corporation) was used.

As an NH silica gel, Chromatorex Q-Pack NH 60 (FUJI SILYSIA CHEMICAL LTD.) was used.

NMR spectra were measured using tetramethylsilane as an internal standard and using Bruker AV300 (manufactured by Bruker) or Bruker AV400 (manufactured by Bruker), and all δ values are indicated in ppm.

MS spectra were measured using an ACQUITY SQD LC/MS System (manufactured by Waters Corporation).

<Synthesis of compound>

[Example 1]

(1)

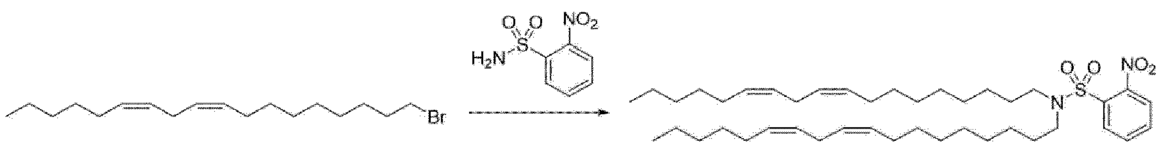

Potassium carbonate (70.4 g) and sodium iodide (2.54 g) were added to a N,N-dimethylformamide (830 mL) solution of (6Z,9Z)-18-bromooctadeca-6,9-diene (131 g) and 2-nitrobenzenesulfonamide (34.4 g), and the mixture was stirred at 80°C for 5 hours. The reaction mixture was cooled to room temperature, and hexane (300 mL) and water (600 mL) were added thereto. The organic layer was separated, and then the obtained mixture was purified by silica gel column chromatography (ethyl acetate-hexane), thereby obtaining 2-nitro-N,N-di((9Z,12Z)-octadeca-9,12-dien-1-yl)benzenesulfonamide (96.7 g).
$^1$H-NMR ($CDCl_3$) δ: 8.03-7.99 (1H, m), 7.69-7.58 (3H, m), 5.43-5.28 (8H, m), 3.26 (4H, t, J = 6.0 Hz), 2.77 (4H, t, J = 6.0 Hz), 2.09-2.00 (8H, m), 1.56-1.45 (4H, m), 1.40-1.19 (32H, m), 0.89 (6H, t, J = 6.0 Hz).

(2)

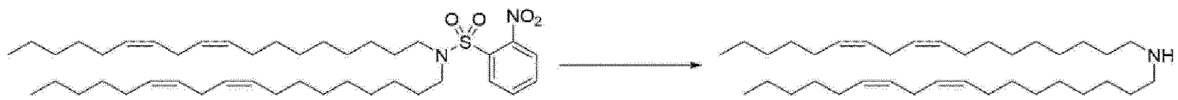

A 10.0 mol/L aqueous potassium hydroxide solution (47.5 mL) was added to a mixture of 2-nitro-N,N-di((9Z,12Z)-octadeca-9,12-dien-1-yl)benzenesulfonamide (96.7 g), dodecanethiol (54.9 mL), acetonitrile (400 mL), and tetrahydrofuran (400 mL), and the mixture was stirred at 40°C for 2 hours. The reaction mixture was cooled to room temperature, hexane (400 mL), tert-butyl methyl ether (100 mL), and water (200 mL) were added thereto, the organic layer was separated and then dried over anhydrous magnesium sulfate, and the solvent was distilled away under reduced pressure. The obtained residue was purified by silica gel column chromatography (ethyl acetate-hexane), thereby obtaining di((9Z,12Z)-octadeca-9,12-dien-1-yl)amine (57.7 g).
$^1$H-NMR ($CDCl_3$) δ: 5.43-5.28 (8H, m), 2.77 (4H, t, J = 6.0 Hz), 2.58 (4H, t, J = 6.0 Hz), 2.09-1.99 (8H, m), 1.56-1.45 (4H, m), 1.40-1.19 (32H, m), 0.89 (6H, t, J = 6.0 Hz).
MS m/z (M + H): 514.

(3)

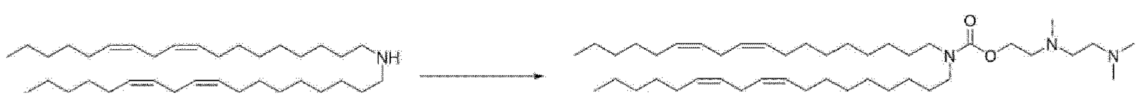

2-((2-(Dimethylamino)ethyl)(methyl)amino)ethan-1-ol (9.36 mL) was added to a tetrahydrofuran (150 mL) solution of 4-nitrophenyl chloroformate (11.7 g), and the mixture was stirred at room temperature for 1 hour. di((9Z,12Z)-octadeca-9,12-dien-1-yl)amine (15.0 g) and triethylamine (16.3 mL) were added to the reaction mixture, and the reaction mixture was stirred at 50°C for 4 hours. The reaction mixture was cooled to room temperature, ethyl acetate (150 mL) and water (100 mL) were added thereto, the organic layer was separated and then dried over anhydrous magnesium sulfate, the solvent was distilled away under reduced pressure, and the obtained residue was purified by a silica gel column chromatography (methanol-chloroform). The obtained oily substance was purified by silica gel column chromatography (ethyl acetate-hexane, NH silica gel), thereby obtaining 2-((2-(dimethylamino)ethyl)(methyl)amino)ethyl di((9Z,12Z)-octadeca -9,12-dien-1-yl)carbamate (11.2 g).
$^1$H-NMR ($CDCl_3$) $\delta$: 5.42-5.23 (8H, m), 4.17 (2H, t, J = 6.0 Hz), 3.26-3.08 (4H, m), 2.77 (4H, t, J = 6.0 Hz), 2.67 (2H, t, J = 6.0 Hz), 2.54 (2H, t, J = 6.0 Hz), 2.39 (2H, t, J = 6.0 Hz), 2.32 (3H, s), 2.24 (6H, s), 2.12-1.97 (8H, m), 1.57-1.43 (4H, m), 1.42-1.18 (32H, m), 0.89 (6H, t, J = 6.0 Hz).
MS m/z (M + H): 687.

[Example 2]

(1)

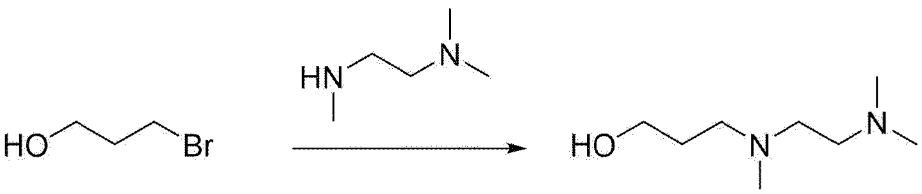

N,N,N'-trimethylethane-1,2-diamine (5 mL) was added to an ethanol (10 mL) solution of 3-bromopropan-1-ol (1.67 mL), and the mixture was stirred at 60°C for 8 hours. The solvent of the reaction mixture was distilled away under reduced pressure, and the obtained residue was purified by silica gel column chromatography (ethyl acetate-hexane, NH silica gel), thereby obtaining 3-((2-(dimethylamino)ethyl)(methyl)amino)propan-1-ol (1.2 g).
MS m/z (M + H): 161.

(2)

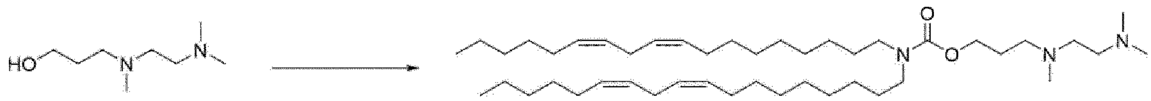

3-((2-(Dimethylamino)ethyl)(methyl)amino)propyl di((9Z,12Z)-octadeca-9,12-dien-1-yl)carbamate was obtained by the same method as that in (3) of Example 1, except that 3-((2-(dimethylamino)ethyl)(methyl)amino)propan-1-ol was used instead of 2-((2-(dimethylamino)ethyl)(methyl)amino)ethan-1-ol in (3) of Example 1. $^1$H-NMR ($CDCl_3$) $\delta$: 5.44-5.27 (8H, m), 4.09 (2H, t, J = 6.0 Hz), 3.25-3.09 (4H, m), 2.77 (4H, t, J = 6.0 Hz), 2.50-2.34 (6H, m), 2.25 (3H, s), 2.24 (6H, s), 2.10-1.99 (8H, m), 1.86-1.74 (2H, m), 1.58-1.43 (4H, m), 1.42-1.18 (32H, m), 0.89 (6H, t, J = 6.0 Hz)
MS m/z (M + H): 701.

[Example 3]

(1)

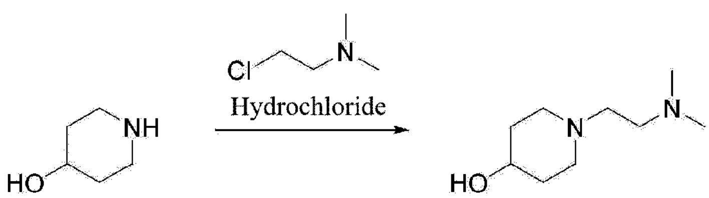


A 12.0 mol/L aqueous sodium hydroxide solution (5 mL) was added to an aqueous solution (5 mL) of piperidin-4-ol (2.0 g) and 2-chloro-N,N-dimethylethan-1-amine hydrochloride (5.69 g), and the mixture was stirred at room temperature for 9 hours. Dichloromethane and water were added to the reaction mixture, the organic layer was separated, and the aqueous layer was extracted with dichloromethane. The organic layer and the extract were combined and dried over anhydrous sodium sulfate, and the solvent was distilled away under reduced pressure. The obtained residue was

purified by silica gel column chromatography (ethyl acetate-hexane, NH silica gel), thereby obtaining 1-(2-(dimethylamino)ethyl)piperidin-4-ol (1.3 g).
MS m/z (M + H): 173.

(2)

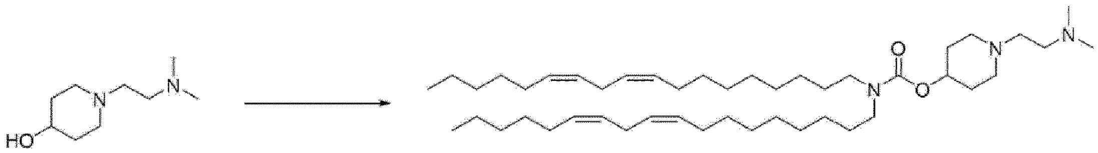

1-(2-(Dimethylamino)ethyl)piperidin-4-yl di((9Z,12Z)-octadeca-9,12-dien-1-yl)carbamate was obtained by the same method as that in (3) of Example 1, except that 1-(2-(dimethylamino)ethyl)piperidin-4-ol was used instead of 2-((2-(dimethylamino)ethyl)(methyl)amino)ethan-1-ol in (3) of Example 1.
$^1$H-NMR ($CDCl_3$) $\delta$: 5.43-5.28 (8H, m), 4.75-4.66 (1H, m), 3.24-3.10 (4H, m), 2.77 (4H, t, J = 6.0 Hz), 2.72-2.60 (2H, m), 2.50-2.39 (4H, m), 2.37-2.27 (2H, m), 2.24 (6H, s), 2.09-1.99 (8H, m), 1.97-1.85 (2H, m), 1.76-1.65 (2H, m), 1.66-1.58 (8H, m), 1.56-1.43 (4H, m), 1.41-1.19 (32H, m), 0.89 (6H, t, J = 6.0 Hz).
MS m/z (M + H): 713.

[Example 4]

(1)

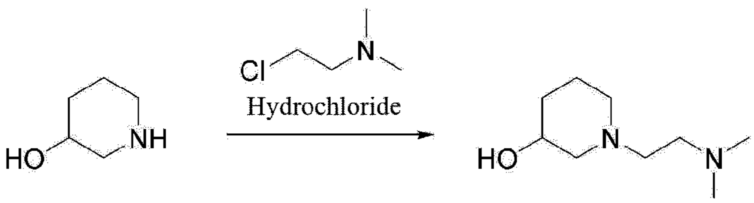


1-(2-(Dimethylamino)ethyl)piperidin-3-ol was obtained by the same method as that in (1) of Example 3, except that piperidin-3-ol was used instead of piperidin-4-ol in (1) of Example 3.
MS m/z (M + H): 173.

(2)

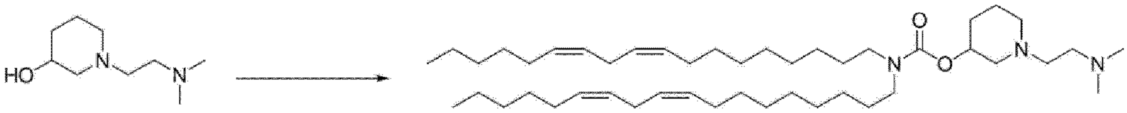

1-(2-(Dimethylamino)ethyl)piperidin-3-yl di((9Z,12Z)-octadeca-9,12-dien-1-yl)carbamate was obtained by the same method as that in (3) of Example 1, except that 1-(2-(dimethylamino)ethyl)piperidin-3-ol was used instead of 2-((2-(dimethylamino)ethyl)(methyl)amino)ethan-1-ol in (3) of Example 1.

$^1$H-NMR ($CDCl_3$) $\delta$: 5.43-5.28 (8H, m), 4.78-4.68 (1H, m), 3.26-3.06 (4H, m), 2.94-2.87 (1H, m), 2.77 (4H, t, J = 6.0 Hz), 2.70-2.61 (1H, m), 2.52-2.38 (4H, m), 2.24 (6H, s), 2.16-1.99 (10H, m), 1.97-1.87 (1H, m), 1.77-1.43 (7H, m), 1.41-1.19 (32H, m), 0.89 (6H, t, J = 6.0 Hz).
MS m/z (M + H): 713.

[Example 5]

(1)

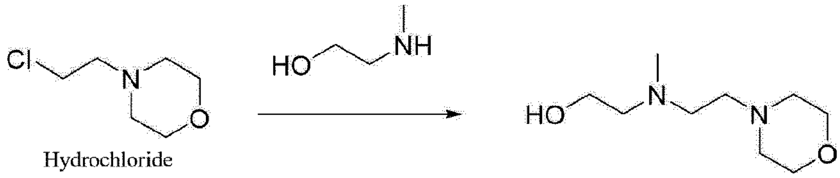

4-(2-Chloroethyl)morpholine hydrochloride (14.9 g) was added to an ethanol (60 mL) suspension of 2-(methylamino)ethan-1-ol (3.0 g) and potassium carbonate (22.1 g), and the mixture was stirred at 60°C for 4 hours and stirred under reflux for 3 hours. The reaction mixture was cooled to room temperature, then insoluble matters were filtered off, and the solvent was distilled away under reduced pressure. The obtained residue was purified by silica gel column chromatography (ethyl acetate-hexane, NH silica gel), thereby obtaining 2-(methyl(2-morpholinoethyl)amino)ethan-1-ol (5.5 g).
MS m/z (M + H): 189.

(2)

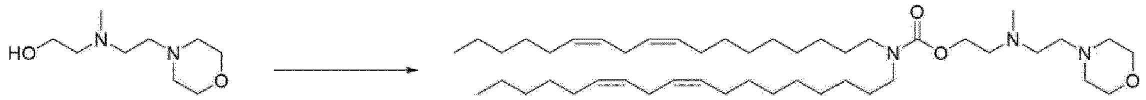

2-(Methyl(2-morpholinoethyl)amino)ethyl di((9Z,12Z)-octadeca-9,12-diene-1-yl)carbamate was obtained by the same method as that in (3) of Example 1, except that 2-(methyl(2-morpholinoethyl)amino)ethan-1-ol was used instead of 2-((2-(dimethylamino)ethyl)(methyl)amino)ethan-1-ol in (3) of Example 1.
$^1$H-NMR ($CDCl_3$) δ: 5.46-5.25 (8H, m), 4.16 (2H, t, J = 6.0 Hz), 3.71 (4H, t, J = 6.0 Hz), 3.25-3.09 (4H, m), 2.27 (4H, t, J = 6.0 Hz), 2.67 (2H, t, J = 6.0 Hz), 2.62-2.53 (2H, m), 2.52-2.42 (6H, m), 2.32 (3H, s), 2.11-1.97 (8H, m), 1.55-1.44 (4H, m), 1.42-1.17 (32H, m), 0.89 (6H, t, J = 6.0 Hz).
MS m/z (M + H): 729.

[Example 6]

(1)

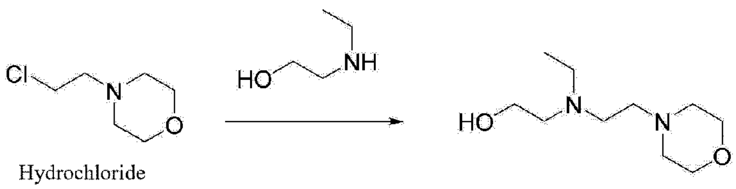

2-(Ethyl(2-morpholinoethyl)amino)ethan-1-ol was obtained by the same method as that in (1) of Example 5, except that 2-(ethylamino)ethan-1-ol was used instead of 2-(methylamino)ethan-1-ol in (1) of Example 5.
MS m/z (M + H): 203.

(2)

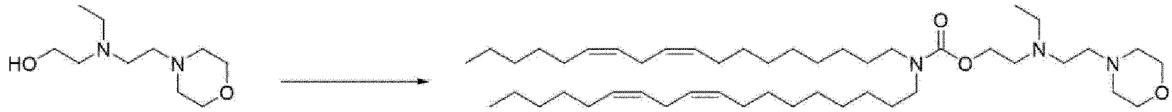

2-(Ethyl(2-morpholinoethyl)amino)ethyl di((9Z,12Z)-octadeca-9,12-dien-1-yl)carbamate was obtained by the same method as that in (3) of Example 1, except that 2-(ethyl(2-morpholinoethyl)amino)ethan-1-ol was used instead of

2-((2-(dimethylamino)ethyl)(methyl)amino)ethan-1-ol in (3) of Example 1.

$^1$H-NMR ($CDCl_3$) δ: 5.45-5.24 (8H, m), 4.12 (2H, t, J = 6.0 Hz), 3.70 (4H, t, J = 6.0 Hz), 3.27-3.06 (4H, m), 2.82-2.69 (6H, m), 2.69-2.54 (4H, m), 2.52-2.39 (6H, m), 2.12-1.97 (8H, m), 1.55-1.42 (4H, m), 1.41-1.17 (32H, m), 1.03 (3H, t, J = 6.0 Hz), 0.89 (6H, t, J = 6.0 Hz).
MS m/z (M + H): 743.

[Example 7]

(1)

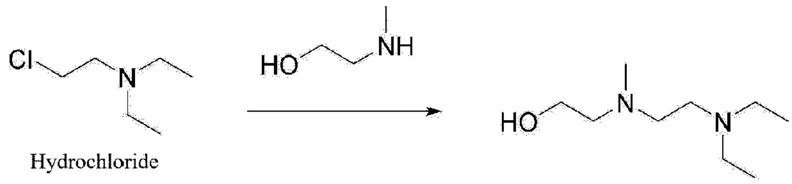


2-((2-(Diethylamino)ethyl)(methyl)amino)ethan-1-ol was obtained by the same method as that in (1) of Example 5, except that 2-chloro-N,N-diethylethan-1-amine hydrochloride was used instead of 4-(2-chloroethyl)morpholine hydrochloride in (1) of Example 5.
MS m/z (M + H): 175.

(2)

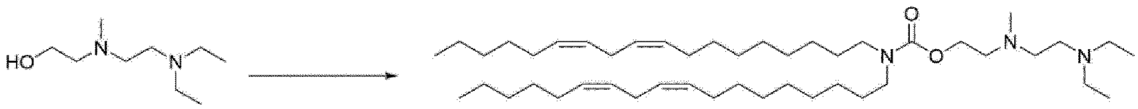

2-((2-(Diethylamino)ethyl)(methyl)amino)ethyl di((9Z,12Z)-octadeca-9,12-dien-1-yl)carbamate was obtained by the same method as that in (3) of Example 1, except that 2-((2-(diethylamino)ethyl)(methyl)amino)ethan-1-ol was used instead of 2-((2-(dimethylamino)ethyl)(methyl)amino)ethan-1-ol in (3) of Example 1.

$^1$H-NMR ($CDCl_3$) δ: 5.45-5.26 (8H, m), 4.16 (2H, t, J = 6.0 Hz), 3.25-3.09 (4H, m), 2.77 (4H, t, J = 6.0 Hz), 2.67 (2H, t, J = 6.0 Hz), 2.60-2.49 (8H, m), 2.32 (3H, s), 2.12-1.96 (8H, m), 1.56-1.44 (4H, m), 1.42-1.17 (32H, m), 1.02 (6H, t, J = 6.0 Hz), 0.89 (6H, t, J = 6.0 Hz).
MS m/z (M + H): 715.

[Example 8]

(1)

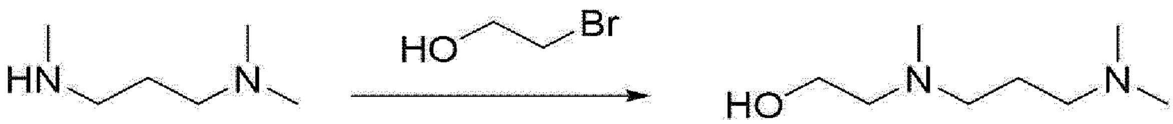

2-((3-(Dimethylamino)propyl)(methyl)amino)ethan-1-ol was obtained by the same method as that in (1) of Example 2, except that 2-bromoethan-1-ol and N,N,N'-trimethylpropane-1,3-diamine were used instead of 3-bromopropan-1-ol and N,N,N'-trimethylethane-1,2-diamine in (1) of Example 2, respectively.
MS m/z (M + H): 161.

(2)

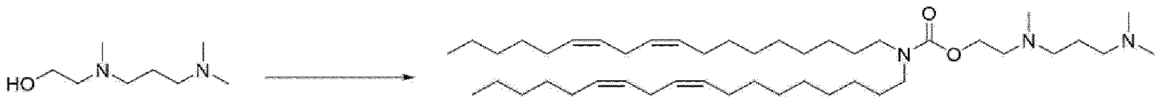

2-((3-(Dimethylamino)propyl)(methyl)amino)ethyl di((9Z,12Z)-octadeca-9,12-dien-1-yl)carbamate was obtained by the same method as that in (3) of Example 1, except that 2-((3-(dimethylamino)propyl)(methyl)amino)ethan-1-ol was used instead of 2-((2-(dimethylamino)ethyl)(methyl)amino)ethan-1-ol in (3) of Example 1.

$^1$H-NMR ($CDCl_3$) δ:5.43-5.28 (8H, m), 4.16 (2H, t, J = 6.0 Hz), 3.25-3.10 (4H, m), 2.77 (4H, t, J = 6.0 Hz), 2.63 (2H, t, J = 6.0 Hz), 2.42 (2H, t, J = 6.0 Hz), 2.28 (3H, s), 2.27 (2H, t, J = 6.0 Hz), 2.21 (6H, s), 2.04 (8H, q, J = 6.0 Ha), 1.67-1.58 (2H, m), 1.56-1.43 (4H, m), 1.40-1.19 (32H, m), 0.89 (6H, t, J = 6.0 Hz).
MS m/z (M + H): 701.

[Example 9]

(1)

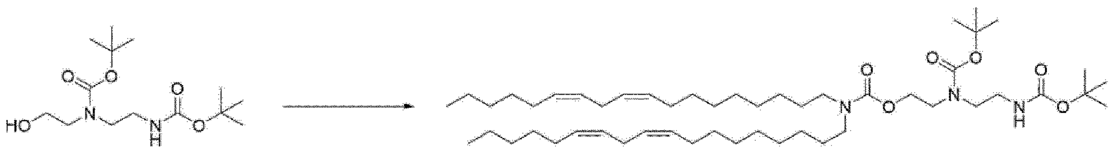

2-((tert-Butoxycarbonyl)(2-((tert-butoxycarbonyl)amino)ethyl)amino)ethyl di((9Z,12Z)-octadeca-9,12-dien-1-yl)carbamate was obtained by the same method as that in (3) of Example 1, except that tert-butyl(2-((tert-butoxycarbonyl)amino)ethyl)(2-hydroxyethyl)carbamate was used instead of 2-((2-(dimethylamino)ethyl)(methyl)amino)ethan-1-ol in (3) of Example 1.
$^1$H-NMR ($CDCl_3$) δ: 5.44-5.27 (8H, m), 4.20-4.09 (1H, m), 3.51-3.10 (10H, m), 2.77 (4H, t, J = 6.0 Hz), 2.10-1.99 (8H, m), 1.64-1.48 (4H, m), 1.41-1.23 (32H, m), 0.89 (6H, t, J = 6.0 Hz).

(2)

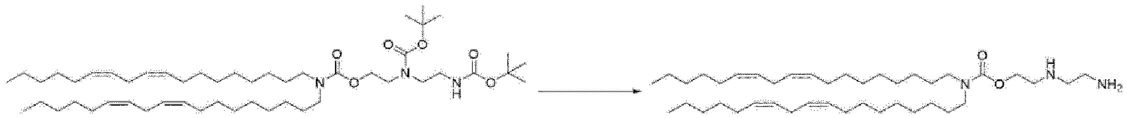

Trifluoroacetic acid (2 mL) was added to a mixture of 2-((tert-butoxycarbonyl)(2-((tert-butoxycarbonyl)amino)ethyl)amino)ethyl di((9Z,12Z)-octadeca-9,12-dien-1-yl)carbamate (0.6 g), water (0.2 mL), and dichloromethane (0.5 mL), and the mixture was stirred at room temperature for 30 minutes. Toluene was added to the reaction mixture, and the solvent was distilled away under reduced pressure. The obtained residue was purified by silica gel column chromatography (methanol-chloroform, NH silica gel), thereby obtaining 2-((2-aminoethyl)amino)ethyl di((9Z,12Z)-octadeca-9,12-dien-1-yl)carbamate (0.3 g).

$^1$H-NMR ($CDCl_3$) δ: 5.43-5.28 (8H, m), 4.18 (2H, t, J = 6.0 Hz), 3.24-3.11 (4H, m), 2.87 (2H, t, J = 6.0 Hz), 2.80 (2H, t, J = 6.0 Hz), 2.77 (4H, t, J = 6.0 Hz), 2.70 (2H, t, J = 6.0 Hz), 2.09-2.00 (8H, m), 1.59-1.44 (4H, m), 1.40-1.19 (32H, m), 0.89 (6H, t, J = 6.0 Hz).
MS m/z (M + H): 645.

[Example 10]

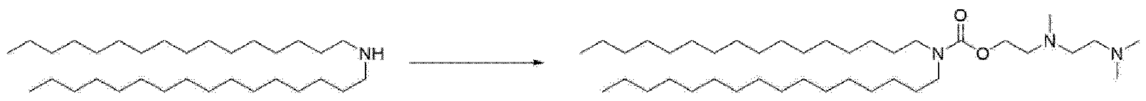

2-((2-(Dimethylamino)ethyl)(methyl)amino)ethyl dihexadecylcarbamate was obtained by the same method as that in (3) of Example 1, except that dihexadecylamine was used instead of di((9Z,12Z)-octadeca-9,12-dien-1-yl)amine in (3) of Example 1.

$^1$H-NMR ($CDCl_3$) δ: 4.17 (2H, t, J = 6.0 Hz), 3.23-3.12 (4H, m), 2.67 (2H, t, J = 6.0 Hz), 2.54 (2H, t, J = 6.0 Hz), 2.39 (2H, t, J = 6.0 Hz), 2.32 (3H, s), 2.24 (6H, s), 1.55-1.38 (4H, m), 1.35-1.18 (52H, m), 0.88 (6H, t, J = 6.0 Hz).
MS m/z (M + H): 639.

[Example 11]

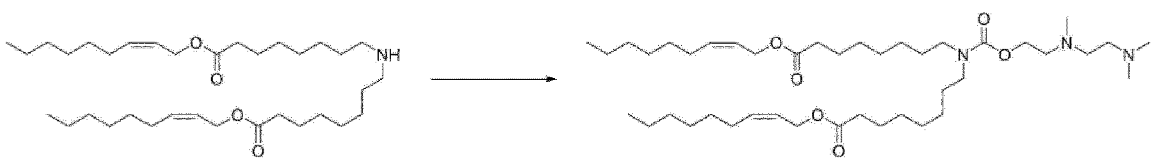

(Z)-non-2-en-1-yl

2,5-dimethyl-10-(8-(((Z)-non-2-en-1-yl)oxy)-8-oxooctyl)-9-oxo-8-oxa-2,5,10-triazaoctadecan-18-oate was obtained by the same method as that in (3) of Example 1, except that di((Z)-non-2-en-1-yl)8,8'-azanedyl dioctanoate synthesized according to the method described in WO2016/081029A1 was used instead of di((9Z,12Z)-octadeca-9,12-dien-1-yl)amine in (3) of Example 1.

$^1$H-NMR ($CDCl_3$) δ: 5.70-5.46 (4H, m), 4.61 (4H, d, J = 6.0 Hz), 4.16 (2H, t, J = 6.0 Hz), 3.23-3.09 (4H, m), 2.66 (2H, t, J = 6.0 Hz), 2.61-2.45 (2H, m), 2.42-2.25 (2H, m), 2.31 (3H, s), 2.23 (6H, s), 2.15-2.05 (4H, m), 1.65-1.56 (4H, m), 1.55-1.43 (4H, m), 1.39-1.20 (32H, m), 0.88 (6H, t, J = 6.0 Hz).
MS m/z (M + H): 723.

[Example 12]

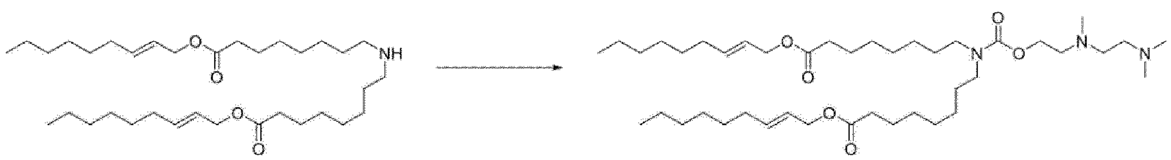

(E)-non-2-en-1-yl

2,5-dimethyl-10-(8-(((E)-non-2-en-1-yl)oxy)-8-oxooctyl)-9-oxo-8-oxa-2,5,10-triazaoctadecan-18-oate was obtained by the same method as that in (3) of Example 1, except that di((E)-non-2-en-1-yl)8,8'-azanedyl dioctanoate synthesized according to the method described in WO2016/081029A was used instead of di((9Z,12Z)-octadeca-9,12-dien-1-yl)amine in (3) of Example 1.

$^1$H-NMR ($CDCl_3$) δ: 5.83-5.70 (2H, m), 5.61-5.49 (2H, m), 4.50 (4H, d, J = 6.0 Hz), 4.16 (2H, t, J = 6.0 Hz), 3.24-3.09 (4H, m), 2.67 (2H, t, J = 6.0 Hz), 2.54 (2H, t, J = 6.0 Hz), 2.38 (2H, t, J = 6.0 Hz), 2.31 (3H, s), 2.24 (6H, s), 2.09-2.00 (4H, m), 1.65-1.56 (4H, m), 1.55-1.44 (4H, m), 1.41-1.23 (32H, m), 0.88 (6H, t, J = 6.0 Hz).
MS m/z (M + H): 723.

[Example 13]

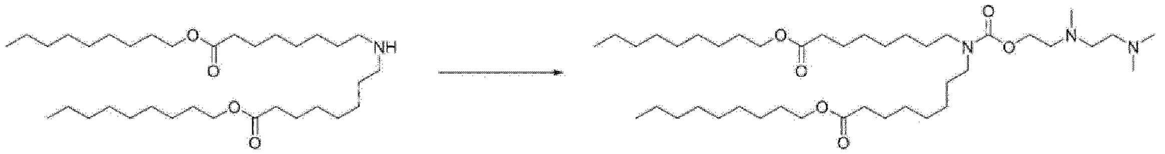

Nonyl

2,5-dimethyl-10-(8-(nonyloxy)-8-oxooctyl)-9-oxo-8-oxa-2,5,10-triazaoctadecan-18-oate was obtained by the same method as that in (3) of Example 1, except that dinonyl 8,8'-azanedyl dioctanoate synthesized according to the method described in WO2016/081029A was used instead of di((9Z,12Z)-octadeca-9,12-dien-1-yl)amine in (3) of Example 1.

$^1$H-NMR ($CDCl_3$) $\delta$: 4.20-4.01 (6H, m), 3.24-3.09 (4H, m), 2.71-2.51 (4H, m), 2.44-2.38 (2H, m), 2.31 (3H, s), 2.26 (6H, s), 1.79-1.43 (12H, m), 1.37-1.23 (40H, m), 0.88 (6H, t, J = 6.0 Hz).
MS m/z (M + H): 727.

[Example 14]

(1)

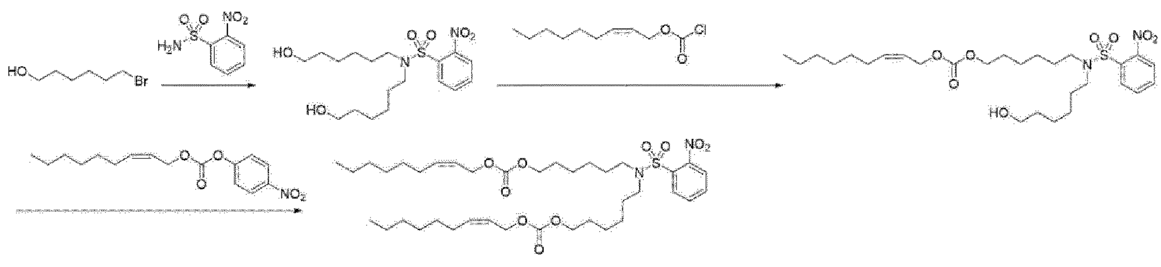

N,N-bis(6-hydroxyhexyl)-2-nitrobenzenesulfonamide was obtained by the same method as that in (1) of Example 1, except that 6-bromohexan-1-ol was used instead of (6Z,9Z)-18-bromooctadeca-6,9-diene in (1) of Example 1.
(Z)-non-2-en-1-yl carbonochloridate (3.15 g) was added to a mixture of the obtained N,N-bis(6-hydroxyhexyl)-2-nitrobenzenesulfonamide (2.13 g), triethylamine (0.58 mL), and tetrahydrofuran (5 mL), and the mixture was stirred at room temperature for 1 hour. Water and ethyl acetate were added to the reaction mixture, the organic layer was separated, washed with water, and then dried over anhydrous sodium sulfate, and the solvent was distilled away under reduced pressure, thereby obtaining (Z)-6-((N-(6-hydroxyhexyl)-2-nitrophenyl)sulfonamido)hexyl non-2-en-1-yl carbonate (1.67 g).
$^1$H-NMR ($CDCl_3$) $\delta$: 8.04-7.97 (1H, m), 7.71-7.59 (3H, m), 5.72-5.51 (2H, m), 4.68 (2H, d, J = 6.0 Hz), 4.12 (2H, t, J = 6.0 Hz), 3.65-3.59 (2H, m), 3.30-3.24 (4H, m), 2.14-2.07 (2H, m), 1.66-1.48 (8H, m), 1.40-1.22 (16H, m), 0.88 (3H, t, J = 6.0 Hz).
4-Dimethylaminopyridine (0.37 g) was added to a mixture of the obtained (Z)-6-((N-(6-hydroxyhexyl)-2-nitrophenyl)sulfonamido)hexyl non-2-en-1-yl carbonate (1.67 g), (Z)-4-nitrophenyl non-2-en-1-yl carbonate (1.84 g), triethylamine (1.7 mL), and tetrahydrofuran (17 mL), and the mixture was stirred at 50°C for 6 hours. The reaction mixture was cooled to room temperature, and then the solvent was distilled away under reduced pressure. The obtained residue was purified by silica gel column chromatography (ethyl acetate-hexane), thereby obtaining (((2-nitrophenyl)sulfonyl)azanedyl)bis(hexane-6,1-diyl) di((Z)-non-2-en-1-yl)bis(carbonate) (1.96 g).
$^1$H-NMR ($CDCl_3$) $\delta$: 8.04-7.97 (1H, m), 7.71-7.59 (3H, m), 5.72-5.51 (4H, m), 4.68 (4H, d, J = 6.0 Hz), 4.12 (4H, t, J = 6.0 Hz), 3.27 (4H, t, J = 6.0 Hz), 2.14-2.07 (4H, m), 1.66-1.48 (8H, m), 1.40-1.22 (24H, m), 0.88 (6H, t, J = 6.0 Hz).

(2)

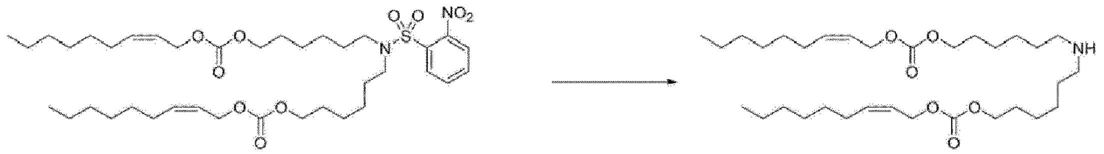

Cesium carbonate (2.51 g) was added to a mixture of (((2-nitrophenyl)sulfonyl)azanedyl)bis(hexane-6,1-diyl) di((Z)-non-2-en-1-yl)bis(carbonate) (1.01 g), dodecane-1-thiol (1.05 mL), and acetonitrile (10 mL), and the mixture was stirred at 50°C for 10 hours. The reaction mixture was cooled to room temperature, water and ethyl acetate were added thereto, the organic layer was separated and dried over anhydrous sodium sulfate, and then the solvent was distilled away under reduced pressure. The obtained residue was purified by silica gel column chromatography (ethyl acetate-hexane), thereby obtaining azanedylbis(hexane-6,1-diyl) di((Z)-non-2-en-1-yl)bis(carbonate) (1.59 g).

$^1$H-NMR ($CDCl_3$) δ: 5.73-5.50 (4H, m), 4.68 (4H, d, J = 6.0 Hz), 4.12 (4H, t, J = 6.0 Hz), 2.61 (4H, t, J = 6.0 Hz), 2.15-2.05 (4H, m), 1.73-1.46 (8H, m), 1.42-1.24 (24H, m), 0.88 (6H, t, J = 6.0 Hz).

(3)

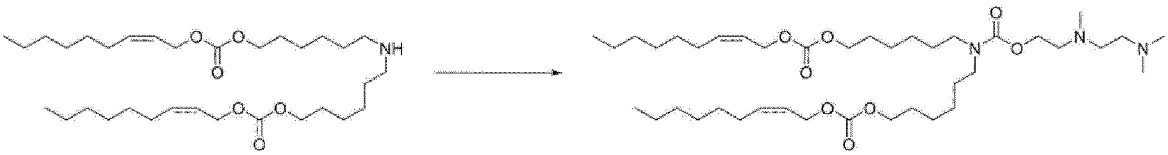

2-((2-(Dimethylamino)ethyl)(methyl)amino)ethyl bis(6-(((((Z)-non-2-en-1-yl)oxy)carbonyl)oxy)hexyl)carbamate was obtained by the same method as that in (3) of Example 1, except that azanedylbis(hexane-6,1-diyl) di((Z)-non-2-en-1-yl)bis(carbonate) was used instead of di((9Z,12Z)-octadeca-9,12-dien-1-yl)amine in (3) of Example 1.

$^1$H-NMR ($CDCl_3$) δ: 5.73-5.50 (4H, m), 4.67 (4H, d, J = 6.0 Hz), 4.20-4.08 (6H, m), 3.24-3.10 (4H, m), 2.66 (2H, d, J = 6.0 Hz), 2.53 (2H, t, J = 6.0 Hz), 2.38 (2H, t, J = 6.0 Hz), 2.31 (3H, s), 2.24 (6H, s), 2.15-2.06 (4H, m), 1.72-1.45 (8H, m), 1.42-1.23 (24H, m), 0.88 (6H, t, J = 6.0 Hz).
MS m/z (M + H): 727.

[Example 15]

(1)

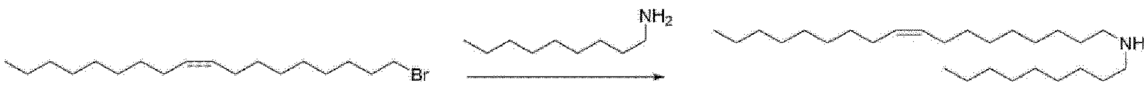

(Z)-1-bromooctadec-9-ene (4.53 g) was added to a N,N-dimethylformamide (20 mL) suspension of nonan-1-amine (1.95 g) and potassium carbonate (1.87 g), and the mixture was stirred at 80°C for 9 hours. The reaction mixture was cooled to room temperature, and water (40 mL) and hexane (40 mL) were added thereto. The organic layer was separated, then the solvent was distilled away under reduced pressure, and the obtained residue was purified by silica gel column chromatography (ethyl acetate-hexane), thereby obtaining (Z)-N-nonyloctadec-9-en-1-amine (1.72 g).
MS m/z (M + H): 394.

(2)

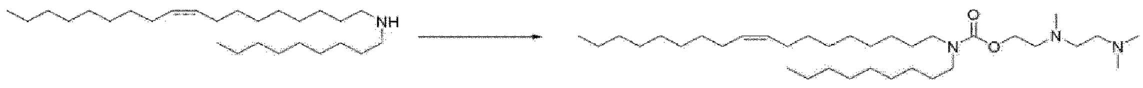

2-((2-(Dimethylamino)ethyl)(methyl)amino)ethyl (Z)-nonyl(octadec-9-en-1-yl)carbamate was obtained by the same method as that in (3) of Example 1, except that (Z)-N-nonyloctadec-9-en-1-amine was used instead of di((9Z,12Z)-octadeca-9,12-dien-1-yl)amine in (3) of Example 1.

> $^1$H-NMR ($CDCl_3$) δ: 5.41-5.29 (2H, m), 4.17 (2H, t, J = 6.0 Hz), 3.24-3.11 (4H, m), 2.68 (2H, t, J = 6.0 Hz), 2.54 (2H, t, J = 6.0 Hz), 2.38 (2H, t, J = 6.0 Hz), 2.32 (3H, s), 2.24 (6H, s), 2.08-1.93 (4H, m), 1.56-1.43 (4H, m), 1.38-1.18 (34H, m), 0.89 (6H, t, J = 6.0 Hz).
> MS m/z (M + H): 567.

[Example 16]

(1)

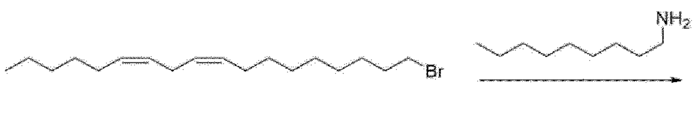

(9Z,12Z)-N-nonyloctadeca-9,12-dien-1-amine was obtained by the same method as that in (1) of Example 15, except that (6Z,9Z)-18-bromooctadeca-6,9-diene was used instead of (Z)-1-bromooctadec-9-ene in (1) of Example 15. MS m/z (M + H): 392.

(2)

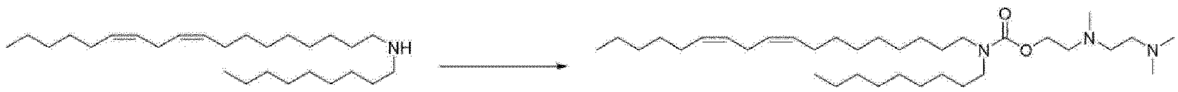

2-((2-(Dimethylamino)ethyl)(methyl)amino)ethyl nonyl((9Z,12Z)-octadeca-9,12-dien-1-yl)carbamate was obtained by the same method as that in (3) of Example 1, except that (9Z,12Z)-N-nonyloctadeca-9,12-dien-1-amine was used instead of di((9Z,12Z)-octadeca-9,12-dien-1-yl)amine in (3) of Example 1.

> $^1$H-NMR ($CDCl_3$) δ: 5.43-5.29 (4H, m), 4.17 (2H, t, J = 6.0 Hz), 3.25-3.11 (4H, m), 2.77 (2H, t, J = 6.0 Hz), 2.68 (2H, t, J = 6.0 Hz), 2.54 (2H, t, J = 6.0 Hz), 2.38 (2H, t, J = 6.0 Hz), 2.32 (3H, s), 2.24 (6H, s), 2.10-1.99 (4H, m), 1.56-1.43 (4H, m), 1.41-1.19 (28H, m), 0.92-0.85 (6H, m).
> MS m/z (M + H): 565.

[Example 17]

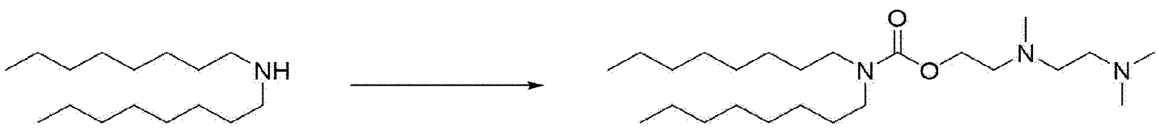

2-((2-(Dimethylamino)ethyl)(methyl)amino)ethyl dioctylcarbamate was obtained by the same method as that in (3) of Example 1, except that dioctylamine was used instead of di((9Z,12Z)-octadeca-9,12-dien-1-yl)amine in (3) of Example 1.

> $^1$H-NMR ($CDCl_3$) δ: 4.17 (2H, t, J = 6.0 Hz), 3.24-3.12 (4H, m), 2.68 (2H, t, J = 6.0 Hz), 2.54 (2H, t, J = 6.0 Hz), 2.39 (2H, t, J = 6.0 Hz), 2.32 (3H, s), 2.24 (6H, s), 1.55-1.43 (4H, m), 1.34-1.19 (20H, m), 0.88 (6H, t, J = 6.0 Hz).
> MS m/z (M + H): 414.

[Example 18]

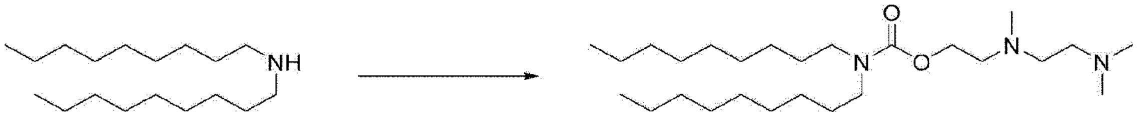

2-((2-(Dimethylamino)ethyl)(methyl)amino)ethyl dinonylcarbamate was obtained by the same method as that in (3) of Example 1, except that dinonylamine was used instead of di((9Z,12Z)-octadeca-9,12-dien-1-yl)amine in (3) of Example 1.

$^1$H-NMR ($CDCl_3$) δ: 4.17 (2H, t, J = 6.0 Hz), 3.24-3.12 (4H, m), 2.68 (2H, t, J = 6.0 Hz), 2.54 (2H, t, J = 6.0 Hz), 2.39 (2H, t, J = 6.0 Hz), 2.32 (3H, s), 2.24 (6H, s), 1.55-1.43 (4H, m), 1.34-1.19 (24H, m), 0.88 (6H, t, J = 6.0 Hz).
MS m/z (M + H): 442.

[Example 19]

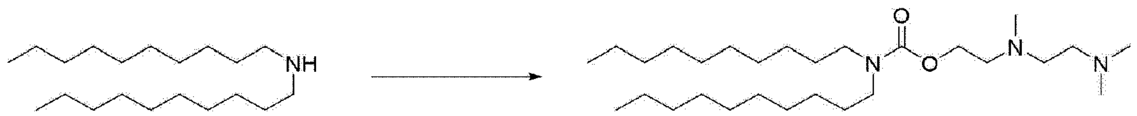

2-((2-(Dimethylamino)ethyl)(methyl)amino)ethyl didecylcarbamate was obtained by the same method as that in (3) of Example 1, except that didecylamine was used instead of di((9Z,12Z)-octadeca-9,12-dien-1-yl)amine in (3) of Example 1.

$^1$H-NMR ($CDCl_3$) δ: 4.17 (2H, t, J = 6.0 Hz), 3.23-3.12 (4H, m), 2.67 (2H, t, J = 6.0 Hz), 2.54 (2H, t, J = 6.0 Hz), 2.39 (2H, t, J = 6.0 Hz), 2.32 (3H, s), 2.24 (6H, s), 1.55-1.38 (4H, m), 1.35-1.18 (28H, m), 0.88 (6H, t, J = 6.0 Hz).
MS m/z (M + H): 470.

[Example 20]

(1)

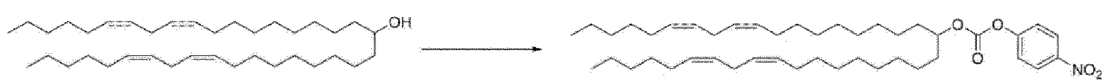

4-Nitrophenyl chloroformate (3.8 g) was added to a mixture of (6Z,9Z,28Z,31Z)-heptatriaconta-6,9,28,31-tetraen-19-ol (5.0 g) synthesized according to the method described in WO2010/054401A, triethylamine (4.0 mL), and tetrahydrofuran (25 mL), and the mixture was stirred at room temperature for 6 hours. Water and ethyl acetate were added to the reaction mixture, the organic layer was separated, washed with water, and then dried over anhydrous sodium sulfate, and the solvent was distilled away under reduced pressure. The obtained residue was purified by silica gel column chromatography (ethyl acetate-hexane), thereby obtaining (6Z,9Z,28Z,31Z)-heptatriaconta-6,9,28,31-tetraen-19-yl(4-nitrophenyl)carbonate (6.25 g).

$^1$H-NMR ($CDCl_3$) δ: 8.31-8.24 (2H, m), 7.42-7.35 (2H, m), 5.44-5.27 (8H, m), 4.87-4.76 (1H, m), 2.77 (4H, t, J = 6.0 Hz), 2.11-1.99 (8H, m), 1.74-1.57 (4H, m), 1.44-1.21 (36H, m), 0.89 (6H, t, J = 6.0 Hz).

(2)

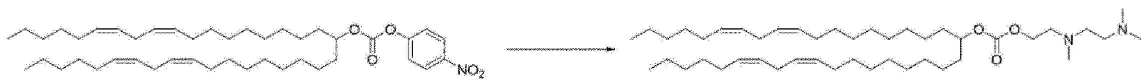

4-Dimethylaminopyridine (0.23 g) was added to a mixture of (6Z,9Z,28Z,31Z)-heptatriaconta-6,9,28,31-tetraen-19-yl(4-nitrophenyl)carbonate (0.89 g), 2-((2-(dimethylamino)ethyl)(methyl)amino)ethan-1-ol (0.30 mL), triethylamine (0.27 mL), and tetrahydrofuran (5 mL), and the mixture was stirred at 60°C for 6 hours. The reaction mixture was cooled

to room temperature, water and ethyl acetate were added thereto, the organic layer was separated, washed with water, and then dried over anhydrous sodium sulfate, and the solvent was distilled away under reduced pressure. The obtained residue was purified by silica gel column chromatography (ethyl acetate-hexane), thereby obtaining 2-((2-(dimethylamino)ethyl)(methyl)amino)ethyl((6Z,9Z,28Z,31Z)-heptatriaconta-6,9,28,31-te traen-19-yl)carbonate (0.36 g).

$^1$H-NMR ($CDCl_3$) δ: 5.44-5.27 (8H, m), 4.73-4.62 (1H, m), 4.22 (2H, t, J = 6.0 Hz), 2.77 (4H, t, J = 6.0 Hz), 2.71 (2H, t, J = 6.0 Hz), 2.58-2.50 (2H, m), 2.43-2.35 (2H, m), 2.32 (3H, s), 2.24 (6H, s), 2.11-1.97 (8H, m), 1.63-1.48 (4H, m), 1.42-1.19(36H, m), 0.89 (6H, t, J = 6.0 Hz).
MS m/z (M + H): 702.

[Example 21]

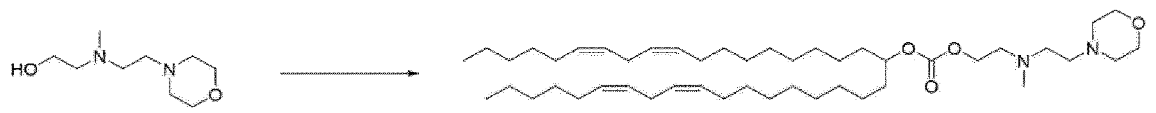

2-(Methyl(2-morpholinoethyl)amino)ethyl((6Z,9Z,28Z,31Z)-heptatriaconta-6,9,28,31 -tetraen-19-yl)carbonate was obtained by the same method as that in (2) of Example 20, except that 2-(methyl(2-morpholinoethyl)amino)ethan-1-ol synthesized in (1) of Example 5 was used instead of 2-((2-(dimethylamino)ethyl)(methyl)amino)ethan-1-ol in (2) of Example 20.

$^1$H-NMR ($CDCl_3$) δ: 5.46-5.25 (8H, m), 4.73-4.61 (1H, m), 4.21 (2H, t, J = 6.0 Hz), 3.71 (4H, t, J = 6.0 Hz), 2.77 (4H, t, J = 6.0 Hz), 2.71 (2H, t, J = 6.0 Hz), 2.62-2.54 (2H, m), 2.51-2.43 (6H, m), 2.32 (3H, s), 2.13-1.98 (8H, m), 1.65-1.46 (4H, m), 1.43-1.20 (36H, m), 0.89 (6H, t, J = 6.0 Hz).
MS m/z (M + H): 744.

[Example 22]

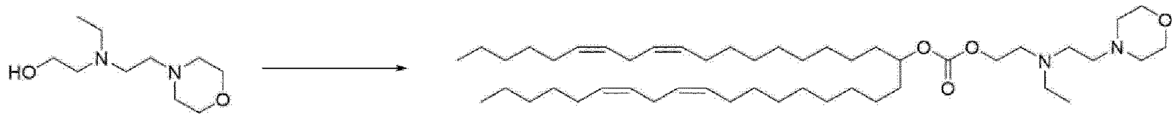

2-(Ethyl(2-morpholinoethyl)amino)ethyl((6Z,9Z,28Z,31Z)-heptatriaconta-6,9,28,31-t etraen-19-yl)carbonate was obtained by the same method as that in (2) of Example 20, except that 2-(ethyl(2-morphofinoethyl)amino)ethan-1-ol synthesized in (1) of Example 6 was used instead of 2-((2-(dimethylamino)ethyl)(methyl)amino)ethan-1-ol in (2) of Example 20.

$^1$H-NMR ($CDCl_3$) δ: 5.45-5.26 (8H, m), 4.74-4.60 (1H, m), 4.17 (2H, t, J = 6.0 Hz), 3.71 (4H, t, J = 6.0 Hz), 2.84-2.72 (6H, m), 2.70-2.54 (4H, m), 2.52-2.39 (6H, m), 2.12-1.94 (8H, m), 1.66-1.47 (4H, m), 1.44-1.18 (36H, m), 1.03 (3H, t, J = 6.0 Hz), 0.89 (6H, t, J = 6.0 Hz).
MS m/z (M + H): 758.

[Example 23]

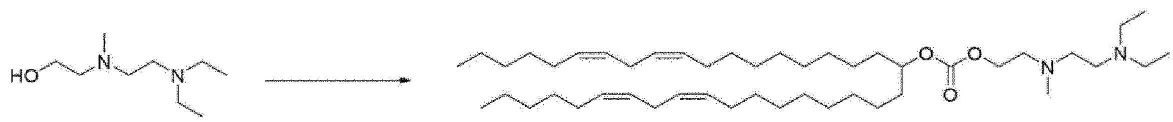

2-((2-(Diethylamino)ethyl)(methyl)amino)ethyl((6Z,9Z,28Z,31Z)-heptatriaconta-6,9, 28,31-tetraen-19-yl)carbonate was obtained by the same method as that in (2) of Example 20, except that 2-((2-(diethylamino)ethyl)(methyl)amino)ethan-1-ol synthesized in (1) of Example 7 was used instead of 2-((2-(dimethylamino)ethyl)(methyl)amino)ethan-1-ol in (2) of Example 20.

$^1$H-NMR ($CDCl_3$) δ: 5.44-5.27 (8H, m), 4.72-4.61 (1H, m), 4.21 (2H, t, J = 6.0 Hz), 2.77 (4H, t, J = 6.0), 2.70 (2H, t, J = 6.0 Hz), 2.59-2.49 (8H, m), 2.31 (3H, s), 2.14-1.94 (8H, m), 1.64-1.47 (4H, m), 1.43-1.19 (36H, m), 1.02 (6H, t, J = 6.0 Hz), 0.89 (6H, t, J = 6.0 Hz).
MS m/z (M + H): 730.

[Example 24]

(1)

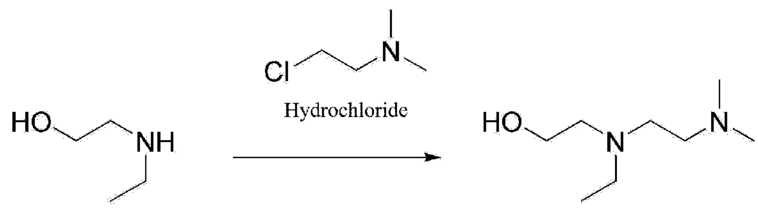


2-((2-(Dimethylamino)ethyl)(ethyl)amino)ethan-1-ol was obtained by the same method as that in (1) of Example 5, except that 2-chloro-N,N-dimethylethan-1-amine hydrochloride and 2-(ethylamino)ethan-1-ol were used instead of 4-(2-chloroethyl)morpholine hydrochloride and 2-(methylamino)ethan-1-ol in (1) of Example 5, respectively.
MS m/z (M + H): 161.

(2)

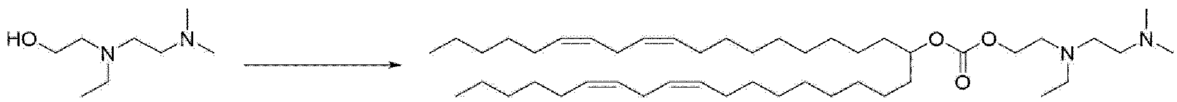

2-((2-(Dimethylamino)ethyl)(ethyl)amino)ethyl((6Z,9Z,28Z,31Z)-heptatriaconta-6,9, 28,31-tetraen-19-yl)carbonate was obtained by the same method as that in (2) of Example 20, except that 2-((2-(dimethylamino)ethyl)(ethyl)amino)ethan-1-ol was used instead of 2-((2-(dimethylamino)ethyl)(methyl)amino)ethan-1-ol in (2) of Example 20.

$^1$H-NMR ($CDCl_3$) δ: 5.45-5.26 (8H, m), 4.73-4.61 (1H, m), 4.18 (2H, t, J = 6.0 Hz), 2.83-2.71 (6H, m), 2.67-2.55 (4H, m), 2.42-2.33 (2H, m), 2.24 (6H, s), 2.12-1.98 (8H, m), 1.64-1.50 (4H, m), 1.45-1.19 (36H, m), 1.03 (3H, t, J = 6.0 Hz), 0.89 (6H, t, J = 6.0 Hz).
MS m/z (M + H): 716.

[Example 25]

(1)

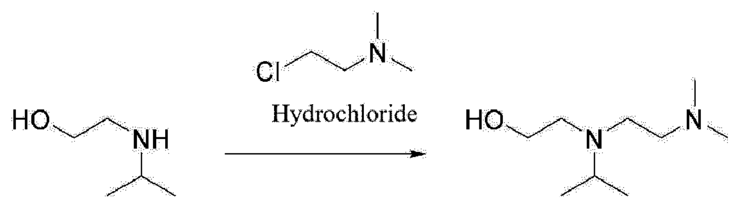

2-((2-(Dimethylamino)ethyl)(isopropyl)amino)ethan-1-ol was obtained by the same method as that in (1) of Example 5, except that 2-chloro-N,N-dimethylethan-1-amine hydrochloride and 2-(isopropylamino)ethan-1-ol were used instead of 4-(2-chloroethyl)morpholine hydrochloride and 2-(methylamino)ethan-1-ol in (1) of Example 5, respectively.
MS m/z (M + H): 175.

(2)

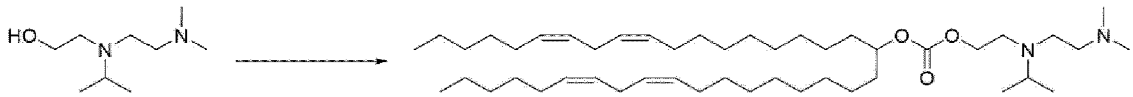

2-((2-(Dimethylamino)ethyl)(isopropyl)amino)ethyl((6Z,9Z,28Z,31Z)-heptatriaconta-6,9,28,31-tetraen-19-yl)carbonate was obtained by the same method as that in (2) of Example 20, except that 2-((2-(dimethylamino)ethyl)(isopropyl)amino)ethan-1-ol was used instead of 2-((2-(dimethylamino)ethyl)(methyl)amino)ethan-1-ol in (2) of Example 20.

$^1$H-NMR ($CDCl_3$) $\delta$: 5.46-5.26 (8H, m), 4.73-4.61 (1H, m), 4.10 (2H, t, J = 6.0 Hz), 2.98-2.85 (1H, m), 2.77 (4H, t, J = 6.0 Hz), 2.69 (2H, t, J = 6.0 Hz), 2.60-2.52 (2H, m), 2.37-2.29 (2H, m), 2.24 (6H, s), 2.10-1.99 (8H, m), 1.58-1.49 (4H, m), 1.45-1.20 (36H, m), 0.99 (6H, d, J = 6.0 Hz), 0.89 (6H, t, J = 6.0 Hz).
MS m/z (M + H): 730.

[Example 26]

(1)

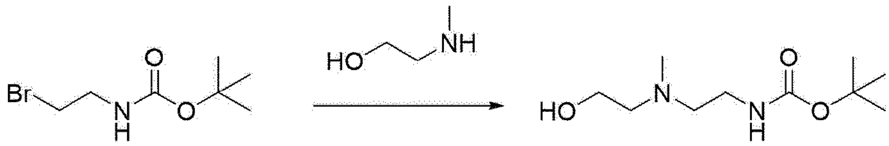

tert-Butyl(2-((2-hydroxyethyl)(methyl)amino)ethyl)carbamate was obtained by the same method as that in (1) of Example 5, except that tert-butyl(2-bromoethyl)carbamate was used instead of 4-(2-chloroethyl)morpholine hydrochloride in (1) of Example 5.
MS m/z (M + H): 219.

(2)

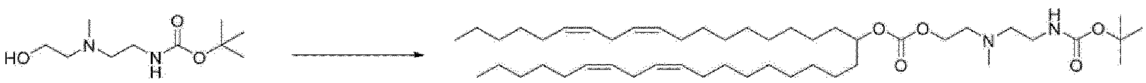

tert-Butyl

(2-((2-(((6Z,9Z,28Z,31Z)-heptatriaconta-6,9,28,31-tetraen-19-yl)oxy)ethyl)(methyl)amino)eth yl)carbamate was obtained by the same method as that in (2) of Example 20, except that tert-butyl (2-((2-hydroxyethyl)(methyl)amino)ethyl)carbamate was used instead of 2-((2-(dimethylamino)ethyl)(methyl)amino)ethan-1-ol in (2) of Example 20.

$^1$H-NMR ($CDCl_3$) $\delta$: 5.45-5.26 (8H, m), 5.04 (1H, bs), 4.76-4.62 (1H, m), 4.20 (2H, t, J = 6.0 Hz), 3.25-3.12 (2H, m), 2.77 (4H, t, J = 6.0 Hz), 2.68 (2H, t, J = 6.0 Hz), 2.52 (2H, t, J = 6.0 Hz), 2.28 (3H, s), 2.12-1.96 (8H, m), 1.62-1.50 (4H, m), 1.45 (9H, s), 1.62-1.50 (36H, m), 0.89 (6H, t, J = 6.0 Hz).
MS m/z (M + H): 774.

(3)

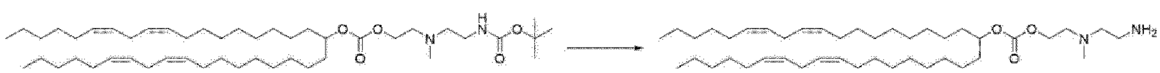

2-((2-Aminoethyl)(methyl)amino)ethyl((6Z,9Z,28Z,31Z)-heptatriaconta-6,9,28,31-tet raen-19-yl)carbonate was obtained by the same method as that in (2) of Example 9, except that tert-butyl

(2-((2-(((((6Z,9Z,28Z,31Z)-heptatriaconta-6,9,28,31-tetraen-19-yl)oxy)ethyl)(methyl)amino)et hyl)carbamate synthesized in (2) of Example 26 was used instead of 2-((tert-butoxycarbonyl)(2-((tert-butoxycarbonyl)amino)ethyl)amino)ethyl di((9Z,12Z)-octadeca-9,12-dien-1-yl)carbamate in (2) of Example 9.

> $^1$H-NMR ($CDCl_3$) δ: 5.45-5.26 (8H, m), 4.73-4.61 (1H, m), 4.22 (2H, t, J = 6.0 Hz), 2.82-2.72 (6H, m), 2.68 (2H, t, J = 6.0 Hz), 2.47 (2H, t, J = 6.0 Hz), 2.29 (3H, s), 2.11-1.98 (8H, m), 1.62-1.44 (4H, m), 1.42-1.19 (36H, m), 0.89 (6H, t, J = 6.0 Hz).
> MS m/z (M + H): 674.

[Example 27]

(1)

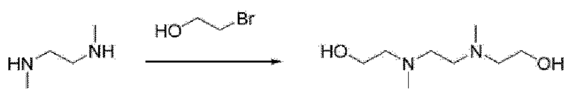

2-Bromoethan-1-ol (14.2 g) was added to an ethanol (50 mL) suspension of N,N'-dimethylethane-1,2-diamine (5.0 g) and potassium carbonate (17.2 g), and the mixture was stirred at 60°C for 5 hours. The reaction mixture was cooled to room temperature, the insoluble matters were filtered off, and then the solvent was distilled away under reduced pressure, thereby obtaining 2,2'-(ethane-1,2-diylbis(methylazanedyl))bis(ethan-1-ol) (10.2 g).
MS m/z (M + H): 177.

(2)

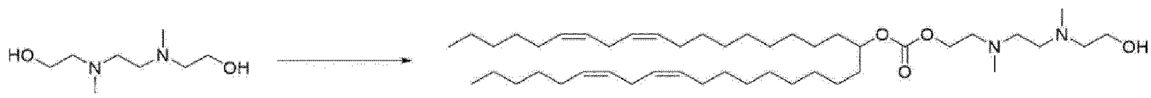

(6Z,9Z,28Z,31Z)-heptatriaconta-6,9,28,31-tetraen-19-yl(2-((2-((2-hydroxyethyl)(met hyl)amino)ethyl)(methyl)amino)ethyl)carbonate was obtained by the same method as that in (2) of Example 20, except that 2,2'-(ethane-1,2-diylbis(methylazanedyl))bis(ethan-1-ol) was used instead of 2-((2-(dimethylamino)ethyl)(methyl)amino)ethan-1-ol in (2) of Example 20.

> $^1$H-NMR ($CDCl_3$) δ: 5.44-5.27 (8H, m), 4.73-4.61 (1H, m), 4.23 (2H, t, J = 6.0 Hz), 3.56 (2H, t, J = 6.0 Hz), 2.82-2.67 (6H, m), 2.58-2.52 (6H, m), 2.31 (6H, s), 2.11-1.99 (8H, m), 1.63-1.46 (4H, m), 1.42-1.20 (36H, m), 0.89 (6H, t, J = 6.0 Hz).
> MS m/z (M + H): 732.

[Example 28]

(1)

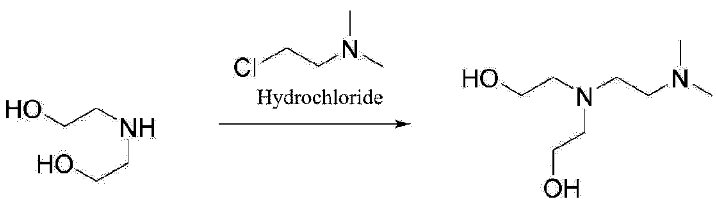

2,2'-((2-(Dimethylamino)ethyl)azanedyl)bis(ethan-1-ol) was obtained by the same method as that in (1) of Example 5, except that 2,2'-azanedylbis(ethan-1-ol) and 2-chloro-N,N-dimethylethan-1-amine hydrochloride were used instead of 2-(methylamino)ethan-1-ol and 4-(2-chloroethyl)morpholine hydrochloride in (1) of Example 5, respectively. MS m/z (M + H): 177.

(2)

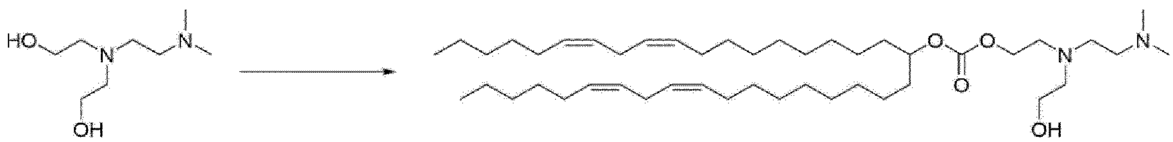

2-((2-(Dimethylamino)ethyl)(2-hydroxyethyl)amino)ethyl)((6Z,9Z,28Z,31Z)-heptatri aconta-6,9,28,31-tetraen-19-yl)carbonate was obtained by the same method as that in (2) of Example 20, except that 2,2'-((2-(dimethylamino)ethyl)azanedyl)bis(ethan-1-ol) was used instead of 2-((2-(dimethylamino)ethyl)(methyl)amino)ethan-1-ol in (2) of Example 20.

$^1$H-NMR ($CDCl_3$) δ: 5.45-5.25 (8H, m), 4.73-4.62 (1H, m), 4.21 (2H, t, J = 6.0 Hz), 3.53 (2H, t, J = 6.0 Hz), 2.89 (2H, t, J = 6.0 Hz), 2.77 (4H, t, J = 6.0 Hz), 2.73-2.64 (4H, m), 2.37 (2H, t, J = 6.0 Hz), 2.23 (6H, s), 2.10-1.98 (8H, m), 1.65-1.46 (4H, m), 1.43-1.18 (36H, m), 0.89 (6H, t, J = 6.0 Hz).
MS m/z (M + H): 732.

[Example 29]

(1)

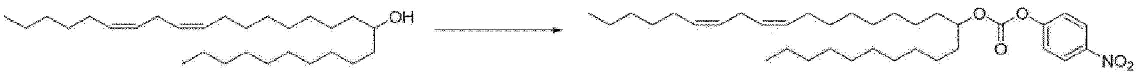

4-Nitrophenyl chloroformate (1.0 g) was added to a mixture of ((19Z,22Z)-octacosa-19,22-dien-11-ol (1.0 g) synthesized according to the method described in WO2015/005253A, triethylamine (1.0 mL), and tetrahydrofuran (5.0 mL), and the mixture was stirred at room temperature for 2 hours. Water and ethyl acetate were added to the reaction mixture, the organic layer was separated and dried over anhydrous sodium sulfate, and then the solvent was distilled away under reduced pressure. The obtained residue was purified by silica gel column chromatography (ethyl acetate-hexane), thereby obtaining 4-nitrophenyl((19Z,22Z)-octacosa-19,22-dien-11-yl)carbonate (2.0 g).

$^1$H-NMR ($CDCl_3$) δ: 8.28 (2H, d, J = 9.0 Hz), 7.38 (2H, d, J = 9.0 Hz), 5.43-5.28 (4H, m), 4.87-4.77 (1H, m), 2.77 (2H, t, J = 6.0 Hz), 2.10-1.99 (4H, m), 1.76-1.60 (4H, m), 1.43-1.20 (32H, m), 0.92-0.83 (6H, m).

(2)

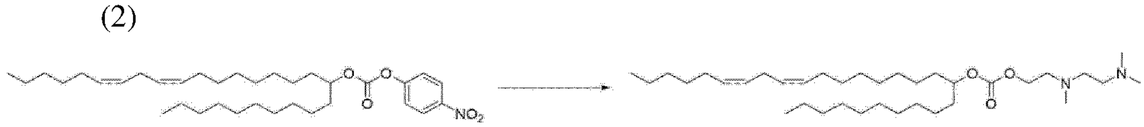

2-((2-(Dimethylamino)ethyl)(methyl)amino)ethyl((19Z,22Z)-octacosa-19,22-dien-11-yl)carbonate was obtained by the same method as that in (2) of Example 20, except that 4-nitrophenyl((19Z,22Z)-octacosa-19,22-dien-11-yl)carbonate was used instead of (6Z,9Z,28Z,31Z)-heptatriaconta-6,9,28,31-tetraen-19-yl(4-nitrophenyl)carbonate in (2) of Example 20.

$^1$H-NMR ($CDCl_3$) δ: 5.44-5.26 (4H, m), 4.73-4.62 (1H, m), 4.22 (2H, t, J = 6.0 Hz), 2.77 (2H, t, J = 6.0 Hz), 2.71 (2H, t, J = 6.0 Hz), 2.54 (2H, t, J = 6.0 Hz), 2.39 (2H, t, J = 6.0 Hz), 2.31 (3H, s), 2.24 (6H, s), 2.11-1.97 (4H, m), 1.65-1.45 (4H, m), 1.42-1.19 (32H, m), 0.93-0.84 (6H, m).
MS m/z (M + H): 580.

[Example 30]

(1)

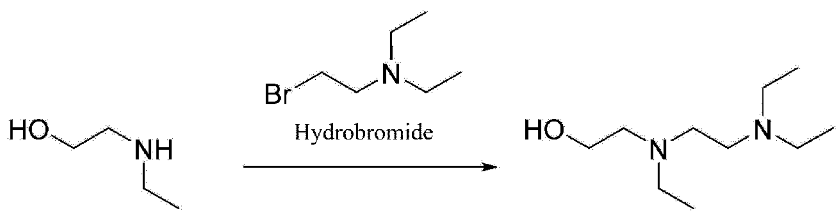


Potassium carbonate (18.6 g) was added to a mixture of 2-(ethylamino)ethan-1-ol (4.0 g), 2-bromo-N,N-diethylethan-1-amine hydrobromide (17.6 g), and ethanol (80 mL), and the mixture was stirred under reflux with heating for 7 hours. The reaction mixture was cooled to room temperature, the insoluble matters were filtered off, and the solvent was distilled away under reduced pressure. The obtained residue was purified by silica gel column chromatography (ethyl acetate-hexane, NH silica gel), thereby obtaining 2-((2-(diethylamino)ethyl)(ethyl)amino)ethan-1-ol (6.5 g) as a light yellow oily substance.
MS m/z (M + H): 189.

(2)

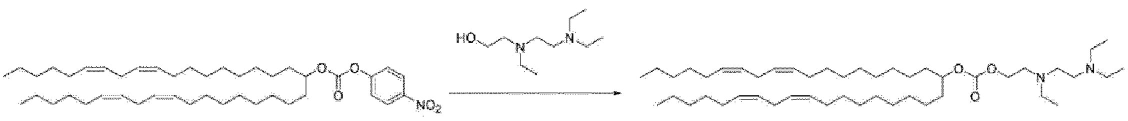

2-((2-(Diethylamino)ethyl)(ethyl)amino)ethyl((6Z,9Z,28Z,31Z)-heptatriaconta-6,9,28 ,31-tetraen-19-yl)carbonate as a colorless oily substance was obtained by the same method as that in (2) of Example 20, except that 2-((2-(diethylamino)ethyl)(ethyl)amino)ethan-1-ol was used instead of 2-((2-(dimethylamino)ethyl)(methyl)amino)ethan-1-ol in (2) of Example 20.

$^1$H-NMR ($CDCl_3$) δ: 5.45-5.26 (8H, m), 4.72-4.60 (1H, m), 4.17 (2H, t, J = 6.6 Hz), 2.83-2.69 (6H, m), 2.65-2.46 (10H, m), 2.13-1.96 (8H, m), 1.65-1.47 (4H, m), 1.43-1.20 (36H, m), 1.09-0.98 (9H, m), 0.89 (6H, t, J = 6.6 Hz).
MS m/z (M + H): 744.

[Example 31]

(1)

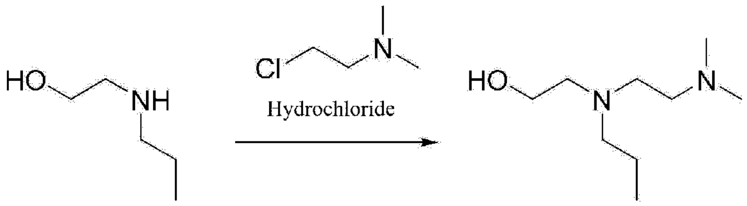


Potassium carbonate (8.0 g) was added to a mixture of 2-(propylamino)ethan-1-ol (2.0 g), 2-chloro-N,N-dimethylethan-1-amine hydrochloride (4.2 g), and ethanol (40 mL), and the mixture was stirred under reflux with heating for 9 hours. The reaction mixture was cooled to room temperature, the insoluble matters were filtered off, and the solvent was distilled away under reduced pressure. The obtained residue was purified by silica gel column chromatography (ethyl acetate-hexane, NH silica gel), thereby obtaining 2-((2-(dimethylamino)ethyl)(propyl)amino)ethan-1-ol (0.87 g) as

a yellow oily substance.
MS m/z (M + H): 175.

(2)

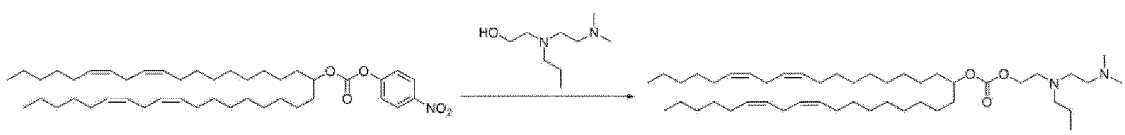

2-((2-(Dimethylamino)ethyl)(propyl)amino)ethyl((6Z,9Z,28Z,31Z)-heptatriaconta-6,9 ,28,31-tetraen-19-yl)carbonate as a colorless oily substance was obtained by the same method as that in (2) of Example 20, except that 2-((2-(dimethylamino)ethyl)(propyl)amino)ethan-1-ol was used instead of 2-((2-(dimethylamino)ethyl)(methyl)amino)ethan-1-ol in (2) of Example 20.

$^1$H-NMR ($CDCl_3$) $\delta$: 5.45-5.26 (8H, m), 4.73-4.61 (1H, m), 4.17 (2H, t, J = 6.0 Hz), 2.85-2.70 (6H, m), 2.66-2.56 (2H, m), 2.51-2.41 (2H, m), 2.41-2.32 (2H, m), 2.24 (6H, s), 2.12-1.95 (8H, m), 1.66-1.18(42H, m), 0.96-0.81 (9H, m).
MS m/z (M + H): 730.

[Example 32]

(1)

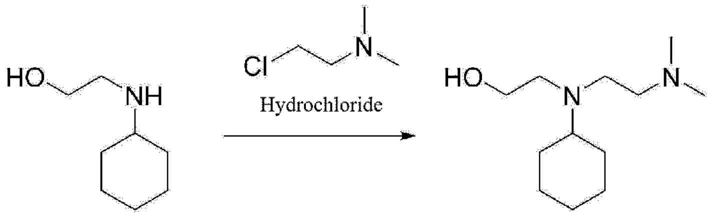


2-(Cyclohexyl(2-(dimethylamino)ethyl)amino)ethan-1-ol as a yellow oily substance was obtained by the same method as that in (1) of Example 31, except that 2-(cyclohexylamino)ethan-1-ol was used instead of 2-(propylamino)ethan-1-ol in (1) of Example 31.
MS m/z (M + H): 215.

(2)

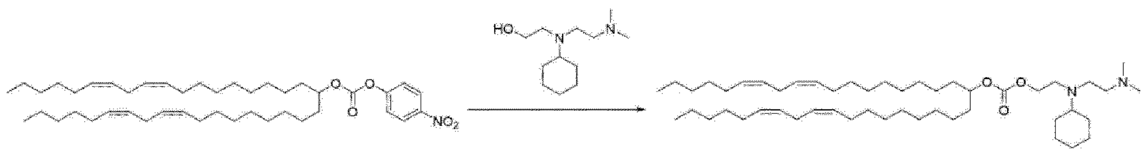

2-(Cyclohexyl(2-(dimethylamino)ethyl)amino)ethyl((6Z,9Z,28Z,31Z)-heptatriaconta-6,9,28,31-tetraen-19-yl)carbonate as a colorless oily substance was obtained by the same method as that in (2) of Example 20, except that 2-(cyclohexyl(2-(dimethylamino)ethyl)amino)ethan-1-ol was used instead of 2-((2-(dimethylamino)ethyl)(methyl)amino)ethan-1-ol in (2) of Example 20.

$^1$H-NMR ($CDCl_3$) $\delta$: 5.45-5.25 (8H, m), 4.74-4.59 (1H, m), 4.08 (2H, t, J = 6.6 Hz), 2.85-2.70 (6H, m), 2.68-2.57 (2H, m), 2.48-2.37 (1H, m), 2.37-2.29 (2H, m), 2.24 (6H, s), 2.13-1.94 (8H, m), 1.85-1.69 (4H, m), 1.66-1.49 (4H, m), 1.46-1.09 (42H, m), 0.89 (6H, t, J = 6.6 Hz).
MS m/z (M + H): 770.

[Example 33]

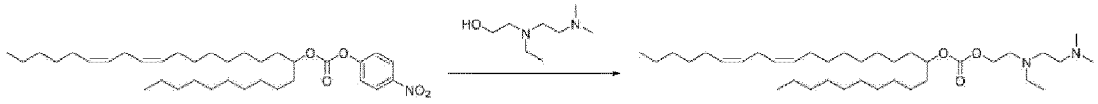

2-((2-(Dimethylamino)ethyl)(ethyl)amino)ethyl((19Z,22Z)-octacosa-19,22-dien-11-yl )carbonate as a colorless oily substance was obtained by the same method as that in (2) of Example 29, except that 2-((2-(dimethylamino)ethyl)(ethyl)amino)ethan-1-ol was used instead of 2-((2-(dimethylamino)ethyl)(methyl)amino)ethan-1-ol in (2) of Example 29.

$^1$H-NMR ($CDCl_3$) $\delta$: 5.45-5.27 (4H, m), 4.73-4.62 (1H, m), 4.18 (2H, t, J = 4.8 Hz), 2.83-2.71 (4H, m), 2.67-2.55 (4H, m), 2.42-2.34 (2H, m), 2.24 (6H, s), 2.12-1.97 (4H, m), 1.67-1.47 (4H, m), 1.43-1.19 (32H, m), 1.03 (3H, t, J = 5.4 Hz), 0.95-0.82 (6H, m).
MS m/z (M + H): 594.

[Example 34]

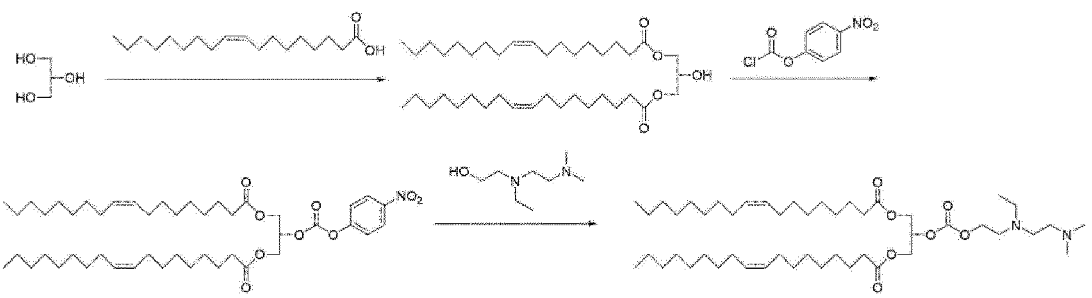

N,N'-dicyclohexylcarbodiimide (9.0 g) was added to a mixture of propane-1,2,3-triol (2.0 g), oleic acid (12.3 g), 4-dimethylaminopyridine (5.3 g), and tetrahydrofuran (100 mL), and the mixture was stirred at room temperature for 12 hours. Water and ethyl acetate were added to the reaction mixture, the organic layer was separated, washed with a saturated aqueous sodium chloride solution, and then dried over anhydrous sodium sulfate, and the solvent was distilled away under reduced pressure. The obtained residue was purified by silica gel column chromatography (ethyl acetate-hexane), thereby obtaining 2-hydroxypropane-1,3-diyldioleate (2.5 g) as a colorless oily substance.

$^1$H-NMR ($CDCl_3$) $\delta$: 5.41-5.28 (4H, m), 4.22-4.04 (5H, m), 2.35 (4H, t, J = 7.2 Hz), 2.05-1.97 (8H, m), 1.68-1.56 (4H, m), 1.40-1.23 (40H, m), 0.88 (6H, t, J = 7.5 Hz).

4-Nitrophenyl chloroformate (246 mg) was added to a mixture of 2-hydroxypropane-1,3-diyldioleate (500 mg), triethylamine (0.34 mL), and tetrahydrofuran (5 mL), and the mixture was stirred at room temperature for 5 hours. 2-((2-(Diethylamino)ethyl)(ethyl)amino)ethan-1-ol (0.26 g), triethylamine (0.23 mL), and 4-dimethylaminopyridine (0.20 g) were added to the reaction mixture, and the reaction mixture was stirred at 70°C for 5 hours. Water and ethyl acetate were added to the reaction mixture, the organic layer was separated, washed with a saturated aqueous sodium chloride solution, and then dried over anhydrous sodium sulfate, and the solvent was distilled away under reduced pressure. The obtained residue was purified by silica gel column chromatography (methanol-ethyl acetate) and silica gel column chromatography (ethyl acetate-hexane, NH silica gel), thereby obtaining 2-(((2-((2-(dimethylamino)ethyl)(ethyl)amino)ethoxy)carbonyl)oxy)propane-1,3-diyldioleate (74 mg) as a colorless oily substance.

$^1$H-NMR ($CDCl_3$) $\delta$: 5.42-5.27 (4H, m), 5.13-5.04 (1H, m), 4.38-4.27 (2H, m), 4.25-4.10 (4H, m), 2.83-2.73 (2H, m), 2.67-2.54 (4H, m), 2.43-2.29 (6H, m), 2.24 (6H, s), 2.08-1.93 (8H, m), 1.68-1.46 (4H, m), 1.40-1.18 (40H, m), 1.03 (3H, t, J = 5.1 Hz), 0.88 (6H, t, J = 5.4 Hz).
MS m/z (M + H): 808.

[Example 35]

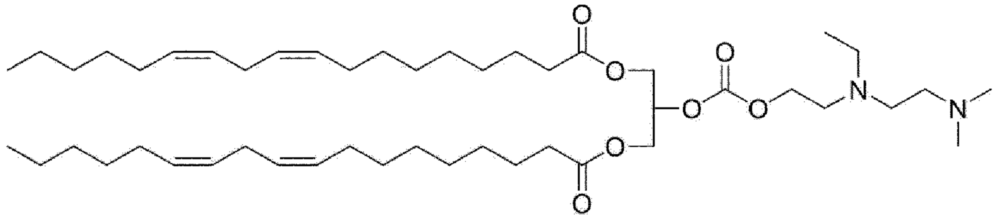

2-(((2-((2-(Dimethylamino)ethyl)(ethyl)amino)ethoxy)carbonyl)oxy)propane-1,3-diyl (9Z,9'Z,12Z,12'Z)-bis(octadeca-9,12-dienoate) as a colorless oily substance was obtained by the same method as that in Example 34, except that (9Z,12Z)-octadeca-9,12-dienoic acid was used instead of oleic acid used in Example 34.

$^1$H-NMR ($CDCl_3$) δ: 5.44-5.28 (8H, m), 5.13-5.03 (1H, m), 4.38-4.29 (2H, m), 4.25-4.13 (4H, m), 2.83-2.72 (6H, m), 2.66-2.55 (4H, m), 2.42-2.28 (6H, m), 2.24 (6H, s), 2.13-1.95 (8H, m), 1.68-1.50 (4H, m), 1.42-1.23 (28H, m), 1.03 (3H, t, J = 5.4 Hz), 0.89 (6H, t, J = 5.4 Hz).
MS m/z (M + H): 804.

[Example 36]

(1)

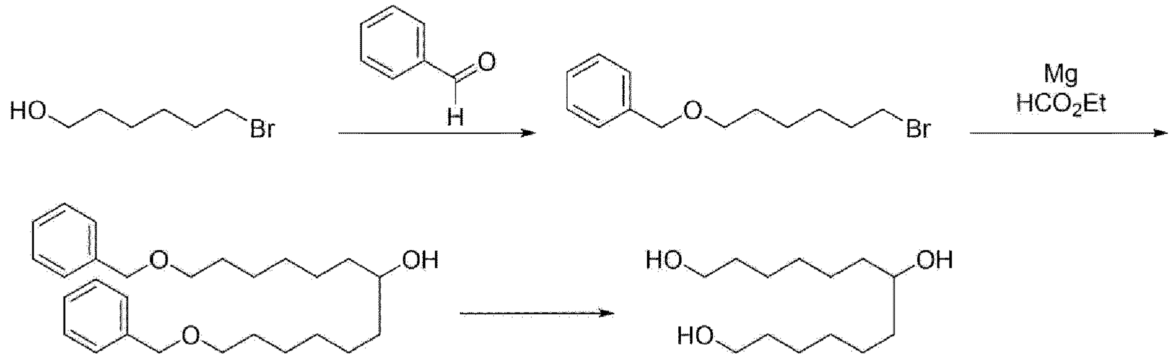


A boron trifluoride-diethyl ether complex (46.2 mL) was added to a mixture of benzaldehyde (30.0 g), 6-bromohexan-1-ol (56.1 g), triethylsilane (67.5 mL), and toluene (300 mL) under ice cooling, and the mixture was stirred at the same temperature for 40 minutes. Water was added to the reaction mixture, the organic layer was separated and washed with a saturated aqueous sodium hydrogen carbonate solution, and then the solvent was distilled away under reduced pressure. The obtained residue was purified by silica gel column chromatography (ethyl acetate-hexane), thereby obtaining (((6-bromohexyl)oxy)methyl)benzene (73.5 g) as a colorless oily substance.

$^1$H-NMR ($CDCl_3$) δ: 7.38-7.23 (5H, m), 4.50 (2H, s), 3.47 (2H, t, J = 6.6 Hz), 3.40 (2H, t, J = 6.6 Hz), 1.92-1.81 (2H, m), 1.68-1.58 (2H, m), 1.52-1.35 (4H, m).

A mixture of (((6-bromohexyl)oxy)methyl)benzene (66.7 g) and tetrahydrofuran (200 mL) was added dropwise to a mixture of magnesium (7.5 g) and tetrahydrofuran (40 mL), and the mixture was stirred at room temperature for 1 hour. A mixture of ethyl formate (8.3 g) and tetrahydrofuran (100 mL) was added to the reaction mixture under ice cooling, and the reaction mixture was stirred at the same temperature for 1 hour. The reaction mixture was poured into a 10% aqueous sulfuric acid solution (330 mL) under ice cooling, then hexane (300 mL) was added thereto, the organic layer was separated and dried over anhydrous magnesium sulfate, and then the solvent was distilled away under reduced pressure. Tetrahydrofuran (200 mL), ethanol (100 mL), and a 10 mol/L aqueous potassium hydroxide solution were added to the obtained residue, and the mixture was stirred at 40°C for 1 hour. Hexane (200 mL) and water (100 mL) were added to the reaction mixture, the organic layer was separated and then dried over anhydrous magnesium sulfate, and the solvent was distilled away under reduced pressure. The obtained residue was purified by silica gel column

chromatography (ethyl acetate-hexane), thereby obtaining 1,13-bis(benzyloxy)tridecan-7-ol (25.3 g) as a colorless oily substance.

$^1$H-NMR ($CDCl_3$) δ: 7.36-7.24 (10H, m), 4.50 (4H, s), 3.61-3.54 (1H, m), 3.46 (4H, t, J = 6.6 Hz), 1.68-1.56 (4H, m), 1.48-1.26 (16H, m).

A mixture of 1,13-bis(benzyloxy)tridecan-7-ol (24.0 g), 10% palladium hydroxide-carbon (10.0 g), and methanol (240 mL) was stirred at 50°C for 3 hours in a hydrogen atmosphere. The reaction mixture was cooled to room temperature, the insoluble matters were filtered off through celite, and then the solvent was distilled away under reduced pressure. Ethyl acetate (40 mL) was added to the obtained residue, and solids were collected by filtration, washed with ethyl acetate, and then dried under reduced pressure, thereby obtaining tridecane-1,7,13-triol (11.7 g) as white solids.

$^1$H-NMR ($CDCl_3$) δ: 3.70-3.55 (5H, m), 1.64-1.24 (20H, m).

(2)

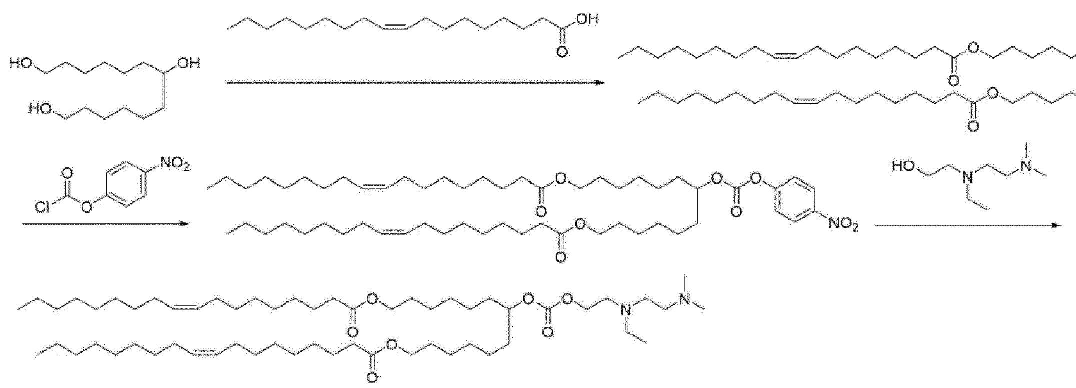

1-Ethyl-3-(3-dimethylaminopropyl)carbodiimide hydrochloride (10.3 g) was added to a mixture of tridecane-1,7,13-triol (5.0 g), oleic acid (13.4 g), triethylamine (18.2 mL), 4-dimethylaminopyridine (0.26 g), and N,N-dimethylformamide (25 mL), and the mixture was stirred at room temperature for 15 hours. Water and ethyl acetate were added to the reaction mixture, the organic layer was separated, washed with a saturated aqueous sodium chloride solution, and then dried over anhydrous sodium sulfate, and the solvent was distilled away under reduced pressure. The obtained residue was purified by silica gel column chromatography (ethyl acetate-hexane), thereby obtaining 7-hydroxytridecane-1,3-diyldioleate (3.6 g) as a colorless oily substance.

$^1$H-NMR ($CDCl_3$) δ: 5.41-5.28 (4H, m), 4.06 (4H, t, J = 6.6 Hz), 3.63-3.53 (1H, m), 2.29 (4H, t, J = 7.2 Hz), 2.06-1.96 (8H, m), 1.68-1.20 (64H, m), 0.88 (6H, t, J = 7.2 Hz).

4-Nitrophenyl chloroformate (161 mg) was added to a mixture of 7-hydroxytridecane-1,3-diyldioleate (400 mg), triethylamine (0.22 mL), and tetrahydrofuran (4 mL), and the mixture was stirred at room temperature for 5 hours. 2-((2-(Dimethylamino)ethyl)(ethyl)amino)ethan-1-ol (0.26 g), triethylamine (0.22 mL), and 4-dimethylaminopyridine (0.19 g) were added to the reaction mixture, and the reaction mixture was stirred at 70°C for 4 hours. Water and ethyl acetate were added to the reaction mixture, the organic layer was separated and washed with a saturated aqueous sodium chloride solution, and then the solvent was distilled away under reduced pressure. The obtained residue was purified by silica gel column chromatography (methanol-ethyl acetate) and silica gel column chromatography (ethyl acetate-hexane, NH silica gel), thereby obtaining 7-(((2-((2-(dimethylamino)ethyl)(ethyl)amino)ethoxy)carbonyl)oxy)tridecane-1,13-diyldioleat e (138 mg) as a colorless oily substance.

$^1$H-NMR ($CDCl_3$) δ: 5.41-5.26 (4H, m), 4.72-4.63 (1H, m), 4.18 (2H, t, J = 6.4 Hz), 4.04 (4H, t, J = 6.8 Hz), 2.77 (2H, t, J = 6.8 Hz), 2.66-2.56 (4H, m), 2.43-2.34 (2H, m), 2.34-2.25 (4H, m), 2.24 (6H, s), 2.09-1.94 (8H, m), 1.70-1.47 (12H, m), 1.44-1.19 (52H, m), 1.03 (3H, t, J = 7.2), 0.88 (6H, t, J = 6.8 Hz).
MS m/z (M + H): 948.

[Example 37]

(1)

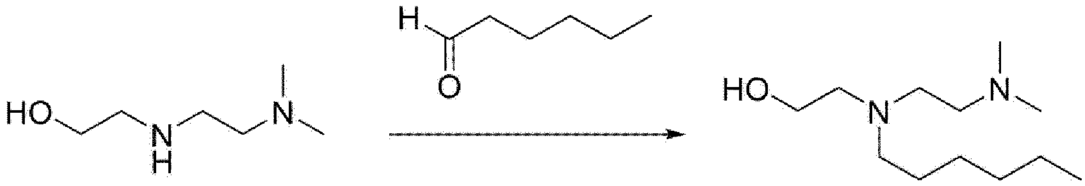

Sodium triacetoxyborohydride (1.8 g) was added to a mixture of 2-((2-(dimethylamino)ethyl)amino)ethan-1-ol (250 mg), hexanal (0.35 mL), acetic acid (0.16 mL), and tetrahydrofuran (2.5 mL), and the mixture was stirred at room temperature for 2 hours. Methanol was added to the reaction mixture under ice cooling, and the reaction mixture was purified by silica gel column chromatography (ethyl acetate-hexane, NH silica gel), thereby obtaining 2-((2-(dimethylamino)ethyl)(hexyl)amino)ethane-1-ol (400 mg) as a colorless oily substance.
MS m/z (M + H): 217.

(2)

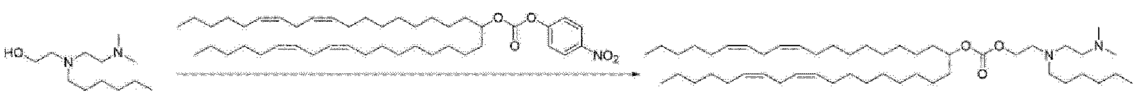

2-((2-(Dimethylamino)ethyl)(hexyl)amino)ethyl((6Z,9Z,28Z,31Z)-heptatriaconta-6,9, 28,31-tetraen-19-yl)carbonate as a colorless oily substance was obtained by the same method as that in (2) of Example 20, except that 2-((2-(dimethylamino)ethyl)(hexyl)amino)ethan-1-ol was used instead of 2-((2-(dimethylamino)ethyl)(methyl)amino)ethan-1-ol in (2) of Example 20.

$^1$H-NMR ($CDCl_3$) $\delta$: 5.45-5.27 (8H, m), 4.72-4.62 (1H, m), 4.17 (2H, t, J = 6.4 Hz), 2.84-2.71 (6H, m), 2.65-2.57 (2H, m), 2.53-2.54 (2H, m), 2.41-2.32 (2H, m), 2.23 (6H, s), 2.12-1.97 (8H, m), 1.68-1.49 (4H, m), 1.48-1.20 (44H, m), 0.97-0.83 (9H, m).
MS m/z (M + H): 772.

[Example 38]

(1)

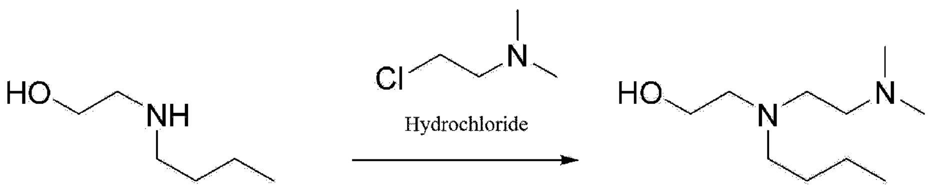


2-(Butyl(2-(dimethylamino)ethyl)amino)ethan-1-ol as a yellow oily substance was obtained by the same method as that in (1) of Example 31, except that 2-(butylamino)ethan-1-ol was used instead of 2-(propylamino)ethan-1-ol in (1) of Example 31.
MS m/z (M + H): 189.

(2)

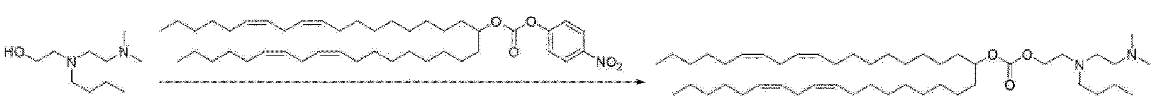

2-(Butyl(2-(dimethylamino)ethyl)amino)ethyl((6Z,9Z,28Z,31Z)-heptatriaconta-6,9,28 ,31-tetraen-19-yl)carbonate as a colorless oily substance was obtained by the same method as that in (2) of Example 20, except that 2-(butyl(2-(dimethylamino)ethyl)amino)ethan-1-ol was used instead of 2-((2-(dimethylamino)ethyl)(methyl)amino)ethan-1-ol in (2) of Example 20.

$^1$H-NMR ($CDCl_3$) $\delta$: 5.44-5.26 (8H, m), 4.72-4.61 (1H, m), 4.17 (2H, t, J = 6.4 Hz), 2.84-2.71 (6H, m), 2.67-2.57 (2H, m), 2.54-2.44 (2H,m), 2.42-2.33 (2H, m), 2.23 (6H, s), 2.12-1.96 (8H, m), 1.67-1.48 (4H, m), 1.48-1.19 (40H, m), 0.97-0.84 (9H, m).
MS m/z (M + H): 744.

[Example 39]

(1)

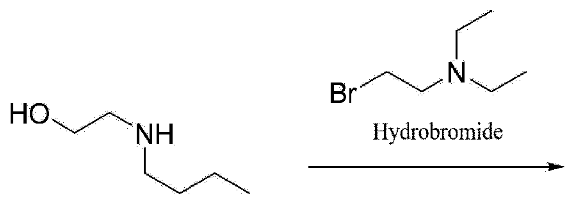


2-(Butyl(2-(diethylamino)ethyl)amino)ethan-1-ol as a light yellow oily substance was obtained by the same method as that in (1) of Example 30, except that 2-(butylamino)ethan-1-ol was used instead of 2-(ethylamino)ethan-1-ol in (1) of Example 30.
MS m/z (M + H): 217.

(2)

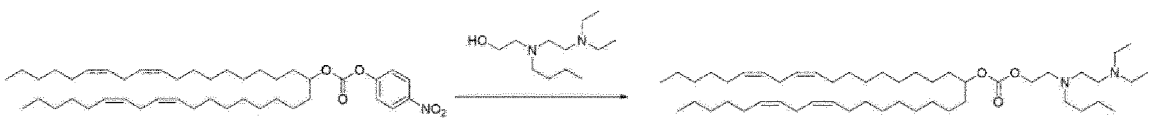

2-(Butyl(2-(diethylamino)ethyl)amino)ethyl((6Z,9Z,28Z,31Z)-heptatriaconta-6,9,28,3 1-tetraen-19-yl)carbonate as a colorless oily substance was obtained by the same method as that in (2) of Example 20, except that 2-(butyl(2-(diethylamino)ethyl)amino)ethan-1-ol was used instead of 2-((2-(dimethylamino)ethyl)(methyl)amino)ethan-1-ol in (2) of Example 20.

$^1$H-NMR ($CDCl_3$) $\delta$: 5.43-5.28 (8H, m), 4.71-4.62 (1H, m), 4.16 (2H, t, J = 6.4 Hz), 2.83-2.70 (6H, m), 2.65-2.43 (10H, m), 2.11-1.96 (8H, m), 1.65-1.49 (4H, m), 1.46-1.19 (40H, m), 1.02 (6H, t, J = 7.2 Hz), 0.96-0.83 (9H, m).
MS m/z (M + H): 772.

[Example 40]

(1)

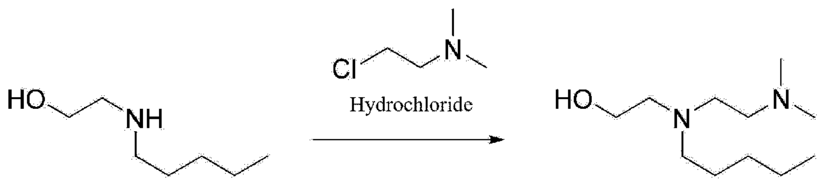


2-((2-(Dimethylamino)ethyl)(pentyl)amino)ethan-1-ol as a brown oily substance was obtained by the same method as that in (1) of Example 31, except that 2-(pentylamino)ethan-1-ol was used instead of 2-(propylamino)ethan-1-ol in (1) of Example 31.
MS m/z (M + H): 203.

(2)

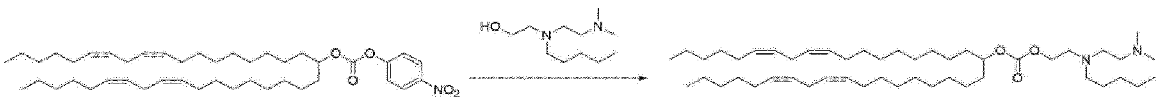

2-((2-(Dimethylamino)ethyl)(pentyl)amino)ethyl((6Z,9Z,28Z,31Z)-heptatriaconta-6,9 ,28,31-tetraen-19-yl)carbonate as a colorless oily substance was obtained by the same method as that in (2) of Example 20, except that 2-((2-(dimethylamino)ethyl)(pentyl)amino)ethan-1-ol was used instead of 2-((2-(dimethylamino)ethyl)(methyl)amino)ethan-1-ol in (2) of Example 20.

> $^1$H-NMR ($CDCl_3$) $\delta$: 5.43-5.26 (8H, m), 4.72-4.61 (1H, m), 4.17 (2H, t, J = 6.0 Hz), 2.83-2.70 (6H, m), 2.65-2.57 (2H, m), 2.53-2.43 (2H, m), 2.41-2.32 (2H, m), 2.23 (6H, s), 2.11-1.97 (8H, m), 1.65-1.49 (4H, m), 1.48-1.19 (42H, m), 0.95-0.83 (9H, m).
> MS m/z (M + H): 758.

[Example 41]

(1)

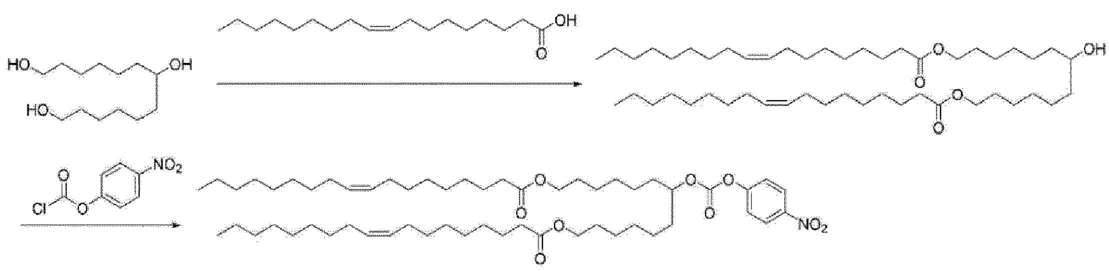

1-Ethyl-3-(3-dimethylaminopropyl)carbodiimide hydrochloride (10.3 g) was added to a mixture of tridecane-1,7,13-triol (5.0 g), oleic acid (13.4 g), triethylamine (18.2 mL), 4-dimethylaminopyridine (0.26 g), and N,N-dimethylformamide (25 mL), and the mixture was stirred at room temperature for 15 hours. Water and ethyl acetate were added to the reaction mixture, the organic layer was separated, washed with a saturated aqueous sodium chloride solution, and then dried over anhydrous sodium sulfate, and the solvent was distilled away under reduced pressure. The obtained residue was purified by silica gel column chromatography (ethyl acetate-hexane), thereby obtaining 7-hydroxytridecane-1,3-diyldioleate (3.6 g) as a colorless oily substance.
$^1$H-NMR ($CDCl_3$) $\delta$: 5.41-5.28 (4H, m), 4.06 (4H, t, J = 6.6 Hz), 3.63-3.53 (1H, m), 2.29 (4H, t, J = 7.2 Hz), 2.06-1.96 (8H, m), 1.68-1.20 (64H, m), 0.88 (6H, t, J = 7.2 Hz).

4-Nitrophenyl chloroformate (1.4 g) was added to a mixture of 7-hydroxytridecane-1,3-diyldioleate (3.6 g), triethylamine (2.0 mL), and tetrahydrofuran (36 mL), and the mixture was stirred at room temperature for 1 hour. Water and ethyl acetate were added to the reaction mixture, the organic layer was separated, washed with a saturated aqueous sodium chloride solution, and then dried over anhydrous sodium sulfate, and the solvent was distilled away under reduced pressure. The obtained residue was purified by silica gel column chromatography (ethyl acetate-hexane), thereby obtaining 7-(((4-nitrophenoxy)carbonyl)oxy)tridecane-1,13-diyldioleate (4.1 g) as a light yellow oily substance.

$^1$H-NMR ($CDCl_3$) $\delta$: 8.28 (2H, dd, J = 7.2Hz, 2.1 Hz), 7.39 (2H, dd, J = 7.2 Hz, 2.1 Hz), 5.40-5.28 (4H, m), 4.86-4.76 (1H, m), 4.06 (4H, t, J = 6.6 Hz), 2.29 (4H, t, J = 7.2 Hz), 2.05-1.96 (8H, m), 1.74-1.56 (12H, m), 1.42-1.21 (52H, m), 0.88 (6H, t, J = 7.2 Hz).

(2)

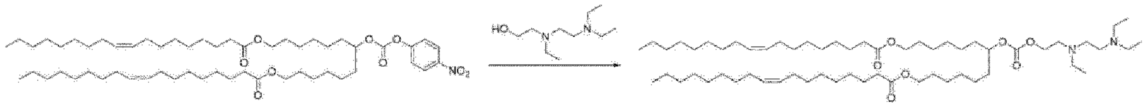

4-Dimethylaminopyridine (0.79 g) was added to a mixture of 7-(((4-nitrophenoxy)carbonyl)oxy)tridecane-1,13-diyldioleate (2.0 g), 2-((2-(diethylamino)ethyl)(ethyl)amino)ethan-1-ol (1.2 g), triethylamine (0.91 mL), and tetrahydrofuran (20 mL), and the mixture was stirred under reflux with heating for 8 hours. The reaction mixture was cooled to room temperature, water and ethyl acetate were added thereto, the organic layer was separated, washed with a saturated aqueous sodium chloride solution, and then dried over anhydrous sodium sulfate, and the solvent was distilled away under reduced pressure. The obtained residue was purified by silica gel column chromatography (methanol-ethyl acetate) and silica gel column chromatography (ethyl acetate-hexane, NH silica gel), thereby obtaining 7-(((2-((2-(diethylamino)ethyl)(ethyl)amino)ethoxy)carbonyl)oxy)tridecane-1,13-diyldioleate (1.7 g) as a colorless oily substance.

$^1$H-NMR ($CDCl_3$) $\delta$: 5.39-5.27 (4H, m), 4.71-4.62 (1H, m), 4.17 (2H, t, J = 6.4 Hz), 4.04 (4H, t, J = 6.8 Hz), 2.76 (2H, t, J = 6.0 Hz), 2.66-2.46 (10H, m), 2.29 (4H, t, J = 7.6 Hz), 2.08-1.94 (8H, m), 1.69-1.48 (12H, m), 1.41-1.19 (52H, m), 1.07-0.97 (9H, m), 0.88 (6H, t, J = 7.2 Hz).
MS m/z (M + H): 976.

[Example 42]

(1)

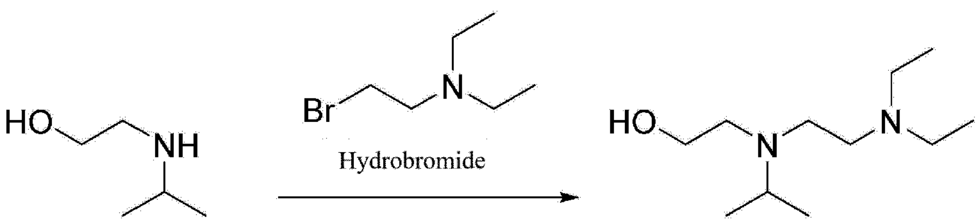


Potassium carbonate (8.0 g) was added to a mixture of 2-(isopropylamino)ethan-1-ol (2.0 g), 2-bromo-N,N-diethylethan-1-amine hydrobromide (7.6 g), and ethanol (20 mL), and the mixture was stirred under reflux with heating for 7 hours. The reaction mixture was cooled to room temperature, the insoluble matters were filtered off, and the solvent was distilled away under reduced pressure. The obtained residue was purified by silica gel column chromatography (ethyl acetate-hexane, NH silica gel), thereby obtaining 2-((2-(diethylamino)ethyl)(isopropyl)amino)ethan-1-ol (3.5 g) as a light yellow oily substance.
MS m/z (M + H): 203.

(2)

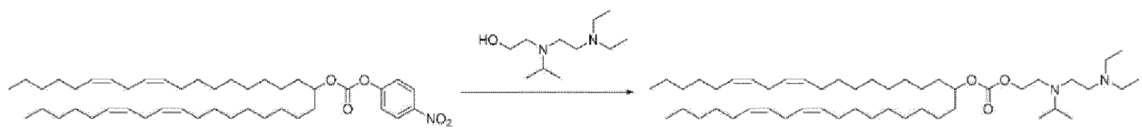

2-((2-(Diethylamino)ethyl)(isopropyl)amino)ethyl((6Z,9Z,28Z,31Z)-heptatriaconta-6, 9,28,31-tetraen-19-yl)carbonate as a colorless oily substance was obtained by the same method as that in (2) of Example 20, except that 2-((2-(diethylamino)ethyl)(isopropyl)amino)ethan-1-ol was used instead of 2-((2-(dimethylamino)ethyl)(methyl)amino)ethan-1-ol in (2) of Example 20.
$^1$H-NMR ($CDCl_3$) δ: 5.45-5.27 (8H, m), 4.72-4.61 (1H, m), 4.10 (2H, t, J = 6.8 Hz), 2.96-2.85 (1H, m), 2.83-2.74 (4H, m), 2.68 (2H, t, J = 6.8 Hz), 2.60-2.41 (8H, m), 2.12-1.96 (8H, m), 1.65-1.48 (4H, m), 1.45-1.19 (36H, m), 1.10-0.95 (12H, m), 0.89 (6H, t, J = 6.8 Hz).
MS m/z (M + H): 758.

[Example 43]

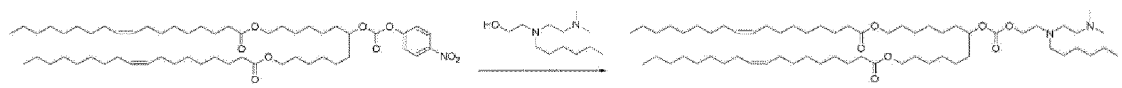

7-(((2-((2-(Dimethylamino)ethyl)(hexyl)amino)ethoxy)carbonyl)oxy)tridecane-1,13-d iyldioleate as a colorless oily substance was obtained by the same method as that in (2) of Example 41, except that 2-((2-(dimethylamino)ethyl)(hexyl)amino)ethan-1-ol was used instead of 2-((2-(diethylamino)ethyl)(ethyl)amino)ethan-1-ol in (2) of Example 41.

$^1$H-NMR ($CDCl_3$) δ: 5.42-5.26 (4H, m), 4.73-4.60 (1H, m), 4.17 (2H, t, J = 5.7 Hz), 4.04 (4H, t, J = 6.6 Hz), 2.76 (2H, t, J = 6.6 Hz), 2.67-2.56 (2H, m), 2.55-2.44 (2H, m), 2.42-2.34 (2H, m), 2.29 (4H, t, J = 7.5 Hz), 2.23 (6H, s), 2.10-1.93 (8H, m), 1.69-1.49 (12H, m), 1.48-1.19 (60H, m), 0.95-0.81 (9H, m).
MS m/z (M + H): 1004.

[Example 44]

(1)

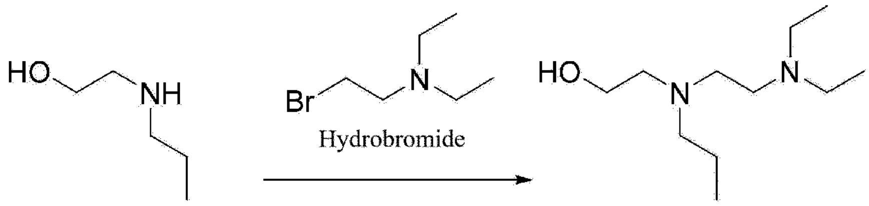


2-((2-(Diethylamino)ethyl)(propyl)amino)ethan-1-ol as a light yellow oily substance was obtained by the same method as that in (1) of Example 30, except that 2-(propylamino)ethan-1-ol was used instead of 2-(ethylamino)ethan-1-ol in (1) of Example 30.
MS m/z (M + H): 203.

(2)

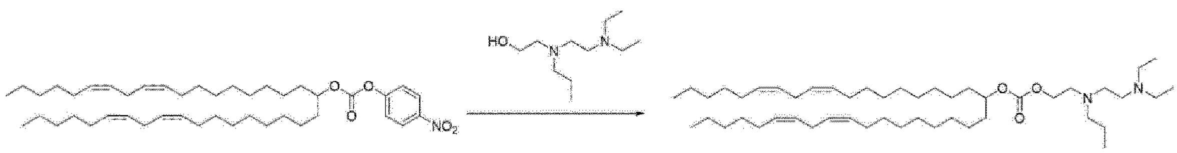

2-((2-(Diethylamino)ethyl)(propyl)amino)ethyl((6Z,9Z,28Z,31Z)-heptatriaconta-6,9,2 8,31-tetraen-19-yl)carbonate as a colorless oily substance was obtained by the same method as that in (2) of Example 20, except that 2-((2-(diethylamino)ethyl)(propyl)amino)ethan-1-ol was used instead of 2-((2-(dimethylamino)ethyl)(methyl)amino)ethan-1-ol in (2) of Example 20.

$^1$H-NMR ($CDCl_3$) δ: 5.46-5.24 (8H, m), 4.73-4.61 (1H, m), 4.16 (2H, t, J = 6.6 Hz), 2.83-2.70 (6H, m), 2.65-2.41 (10H, m), 2.11-1.96 (8H, m), 1.64-1.51 (4H, m), 1.49-1.21 (38H, m), 1.02 (6H, t, J = 7.2 Hz), 0.95-0.81 (9H, m).
MS m/z (M + H): 758.

[Example 45]

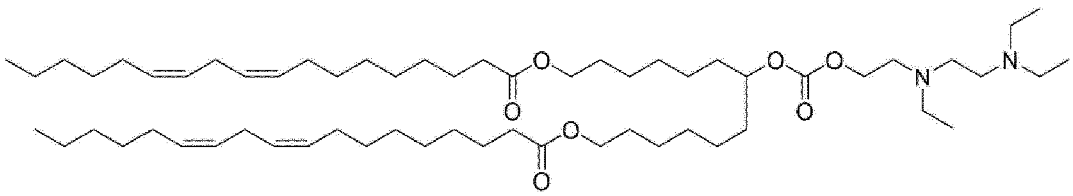

7-(((2-((2-(Diethylamino)ethyl)(ethyl)amino)ethoxy)carbonyl)oxy)tridecane-1,13-diyl (9Z,9'Z,12Z,12'Z)-bis(octadeca-9,12-dienoate) as a colorless oily substance was obtained by the same method as that in (1) and (2) of Example 41, except that (9Z,12Z)-octadeca-9,12-dienoic acid was used instead of oleic acid in (1) and (2) of Example 41.

$^1$H-NMR ($CDCl_3$) δ: 5.46-5.24 (8H, m), 4.73-4.61 (1H, m), 4.17 (2H, t, J = 6.6 Hz), 4.04 (4H, t, J = 6.6 Hz), 2.83-2.71 (6H, m), 2.66-2.47 (10H, m), 2.29 (4H, t, J = 8.1 Hz), 2.13-1.96 (8H, m), 1.69-1.50 (12H, m), 1.44-1.21 (40H, m), 1.08-0.97 (9H, m), 0.89 (6H, t, J = 6.6 Hz).
MS m/z (M + H): 972.

[Example 46]

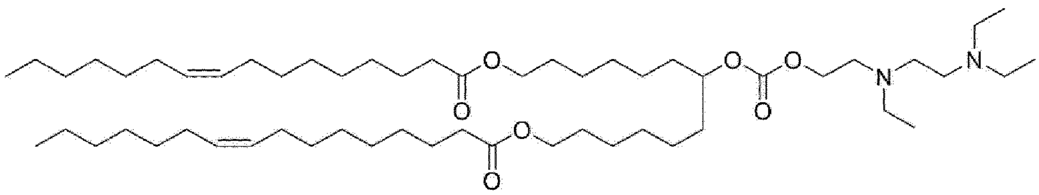

7-(((2-((2-(Diethylamino)ethyl)(ethyl)amino)ethoxy)carbonyl)oxy)tridecane-1,13-diyl (9Z,9'Z)-bis(hexadec-9-enoate) as a colorless oily substance was obtained by the same method as that in (1) and (2) of Example 41, except that (Z)-hexadec-9-enoic acid was used instead of oleic acid in (1) and (2) of Example 41.

$^1$H-NMR ($CDCl_3$) δ: 5.40-5.27 (4H, m), 4.73-4.61 (1H, m), 4.17 (2H, t, J = 6.6 Hz), 4.04 (4H, t, J = 6.6 Hz), 2.76 (2H, t, J = 6.6 Hz), 2.66-2.45 (10H, m), 2.29 (4H, t, J = 7.2 Hz), 2.09-1.93 (8H, m), 1.70-1.48 (12H, m), 1.43-1.20 (44H, m), 1.11-0.97 (9H, m), 0.88 (6H, t, J = 6.6 Hz).
MS m/z (M + H): 920.

[Example 47]

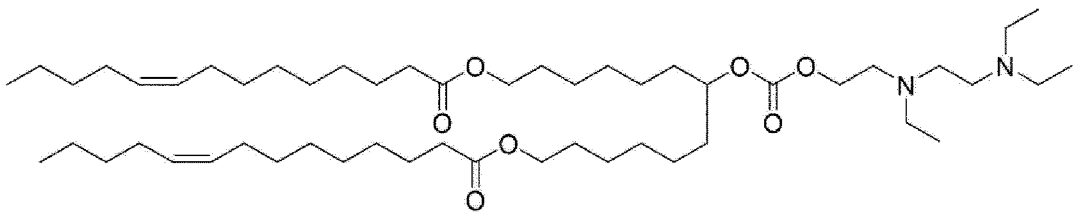

7-(((2-((2-(Diethylamino)ethyl)(ethyl)amino)ethoxy)carbonyl)oxy)tridecane-1,13-diyl (9Z,9'Z)-bis(tetradec-9-enoate) as a colorless oily substance was obtained by the same method as that in (1) and (2) of Example 41, except that (Z)-tetradec-9-enoic acid was used instead of oleic acid in (1) and (2) of Example 41.

$^1$H-NMR ($CDCl_3$) $\delta$: 5.44-5.24 (4H, m), 4.73-4.61 (1H, m), 4.17 (2H, t, J = 6.0 Hz), 4.04 (4H, t, J = 6.6 Hz), 2.76 (2H, t, J = 6.6 Hz), 2.67-2.46 (10H, m), 2.29 (4H, t, J = 7.8 Hz), 2.11-1.92 (8H, m), 1.71-1.47 (12H, m), 1.45-1.21 (36H, m), 1.09-0.96 (9H, m), 0.95-0.83 (6H, m).
MS m/z (M + H): 864.

[Example 48]

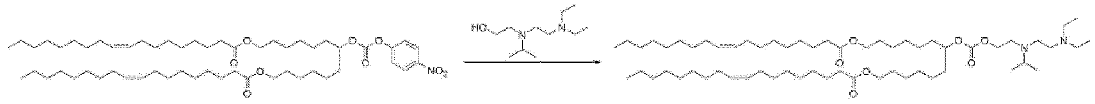

7-(((2-((2-(Diethylamino)ethyl)(isopropyl)amino)ethoxy)carbonyl)oxy)tridecane-1,13 -diyldioleate as a colorless oily substance was obtained by the same method as that in (1) and (2) of Example 41, except that 2-((2-(diethylamino)ethyl)(isopropyl)amino)ethan-1-ol was used instead of 2-((2-(diethylamino)ethyl)(ethyl)amino)ethan-1-ol in (1) and (2) of Example 41.

$^1$H-NMR ($CDCl_3$) $\delta$: 5.41-5.27 (4H, m), 4.72-4.61 (1H, m), 4.17-3.99 (6H, m), 2.95-2.86 (1H, m), 2.68 (2H, t, J = 6.4 Hz), 2.60-2.42 (8H, m), 2.28 (4H, t, J = 8.0 Hz), 2.08-1.93 (8H, m), 1.69-1.48 (12H, m), 1.43-1.20 (52H, m), 1.09-0.95 (12H, m), 0.88 (6H, t, J = 6.8 Hz).
MS m/z (M + H): 990.

[Example 49]

(1)

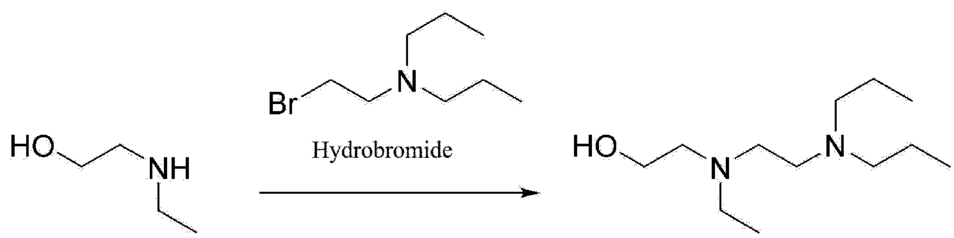


2-((2-(Dipropylamino)ethyl)(ethyl)amino)ethan-1-ol as a colorless oily substance was obtained by the same method as that in (1) of Example 30, except that N-(2-bromoethyl)-N-propylpropan-1-amine hydrobromide was used instead of 2-bromo-N,N-diethylethan-1-amine hydrobromide in (1) of Example 30.

MS m/z (M + H): 217.

(2)

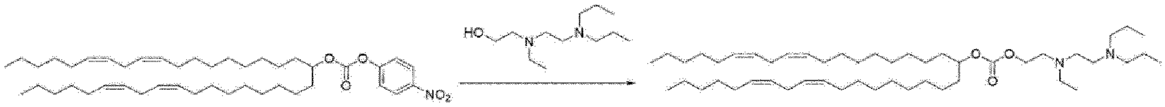

2-((2-(Dipropylamino)ethyl)(ethyl)amino)ethyl((6Z,9Z,28Z,31Z)-heptatriaconta-6,9,2 8,31-tetraen-19-yl)carbonate as a colorless oily substance was obtained by the same method as that in (2) of Example 20, except that 2-((2-(dipropylamino)ethyl)(ethyl)amino)ethan-1-ol was used instead of 2-((2-(dimethylamino)ethyl)(methyl)amino)ethan-1-ol in (2) of Example 20.

$^1$H-NMR ($CDCl_3$) δ: 5.45-5.26 (8H, m), 4.74-4.61 (1H, m), 4.17 (2H, t, J = 6.0 Hz), 2.84-2.70 (6H, m), 2.65-2.46 (6H, m), 2.43-2.31 (4H, m), 2.13-1.97 (8H, m), 1.66-1.52 (4H, m), 1.50-1.21 (40H, m), 1.03 (3H, t, J = 6.6 Hz), 0.95-0.80 (12H, m).
MS m/z (M + H): 772.

[Example 50]

(1)

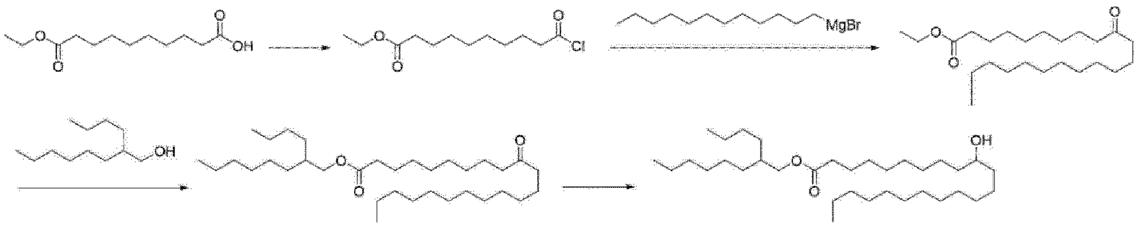

A mixture of 10-ethoxy-10-oxodecanoic acid (22.0 g), thionyl chloride (22.0 mL), and N,N-dimethylformamide (0.1 mL) was stirred under reflux with heating for 1 hour and 30 minutes. The solvent was distilled away under reduced pressure, thereby obtaining ethyl 10-chloro-10-oxodecanoate as a light yellow oily substance and as a crude product.

A 1.0 mol/L dodecyl magnesium bromide-diethyl ether solution (190 mL) was added dropwise to a tetrahydrofuran (284 mL) suspension of zinc (II) chloride (13.0 g) at -78°C, and the mixture was heated to 0°C and then stirred at the same temperature for 30 minutes. Tetrakis(triphenylphosphine)palladium(0) (2.8 g) and ethyl 10-chloro-10-oxodecanoate were added to the reaction mixture, and the reaction mixture was stirred at 0°C for 1 hour. A 1.0 mol/L aqueous hydrochloric acid solution (50 mL) and ethyl acetate were added to the reaction mixture, the organic layer was separated, washed with a saturated aqueous sodium chloride solution, and then dried over anhydrous sodium sulfate, and the solvent was distilled away under reduced pressure. The obtained residue was purified by silica gel column chromatography (ethyl acetate-hexane), thereby obtaining ethyl 10-oxodocosanoate (13.2 g) as a brown oily substance.

Tetraisopropyl orthotitanate (1.7 g) was added to a mixture of ethyl 10-oxodocosanoate (22.0 g) and 2-butyloctan-1-ol (31.9 g), and the mixture was stirred at 110°C for 17 hours. Water and ethyl acetate were added to the reaction mixture, the organic layer was separated and dried over anhydrous sodium sulfate, and then the solvent was distilled away under reduced pressure. The obtained residue was purified by silica gel column chromatography (ethyl acetate-hexane), thereby obtaining 2-butyloctyl 10-oxodocosanoate (11.7 g) as light yellow solids.

Sodium borohydride (4.2 g) was added to a mixture of 2-butyloctyl 10-oxodocosanoate (11.7 g), methanol (47 mL), and tetrahydrofuran (47 mL) under ice cooling, and the mixture was stirred at room temperature for 1 hour. The reaction mixture was poured into a mixture of ice and water, then a 1.0 mol/L aqueous hydrochloric acid solution (22 mL) was added thereto, the organic layer was separated and dried over anhydrous sodium sulfate, and then the solvent was distilled away under reduced pressure. The obtained residue was purified by silica gel column chromatography (ethyl acetate-hexane), thereby obtaining 2-butyloctyl 10-hydroxydocosanoate (7.8 g) as white solids.

$^1$H-NMR ($CDCl_3$) δ: 3.96-3.98 (2H, d), 3.58 (1H, s), 2.27-2.31 (2H, t), 1.60-1.63 (2H, t), 1.38-1.43 (6H, d), 1.26-1.29 (46H, m), 0.86-0.89 (9H, m).

(2)

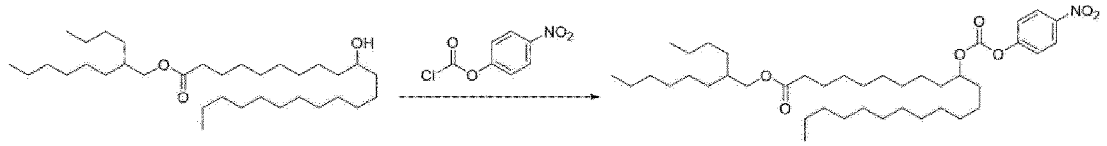

4-Nitrophenyl chloroformate (408 mg) was added to a mixture of 2-butyloctyl 10-hydroxydocosanoate (500 mg), triethylamine (0.43 mL), and tetrahydrofuran (5 mL), and the mixture was stirred at room temperature for 3 hours. Water and ethyl acetate were added to the reaction mixture, the organic layer was separated, washed with a saturated aqueous sodium chloride solution, and then dried over sodium sulfate, and the solvent was distilled away under reduced pressure. The obtained residue was purified by silica gel column chromatography (ethyl acetate-hexane), thereby obtaining 2-butyloctyl 10-(((4-nitrophenoxy)carbonyl)oxy)docosanoate (750 mg) as a colorless oily substance.

$^1$H-NMR ($CDCl_3$) $\delta$: 8.28 (2H, dd, J = 7.2Hz, 2.1 Hz), 7.39 (2H, dd, J = 7.2 Hz, 2.1 Hz), 4.86-4.77 (1H, m), 3.97 (2H, d, J = 6.0 Hz), 2.30 (2H, t, J = 7.2 Hz), 1.74-1.55 (7H, m), 1.40-1.21 (46H, m), 0.92-0.85 (9H, m).

(3)

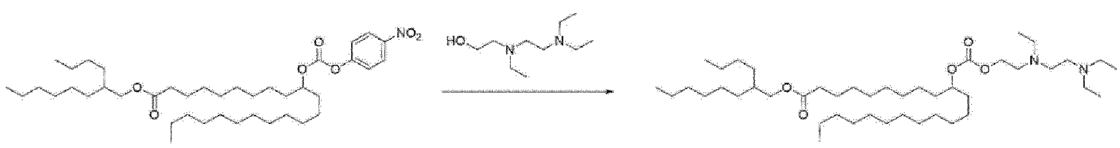

2-Butyloctyl 12-dodecyl-3,6-diethyl-10-oxo-9,11-dioxa-3,6-diazahenicosan-21-oate as a colorless oily substance was obtained by the same method as that in (2) of Example 41, except that 2-butyloctyl 10-(((4-nitrophenoxy)carbonyl)oxy)docosanoate was used instead of 7-(((4-nitrophenoxy)carbonyl)oxy)tridecane-1,13-diyldioleate in (2) of Example 41.

$^1$H-NMR ($CDCl_3$) $\delta$: 4.73-4.61 (1H, m), 4.17 (2H, t, J = 6.6 Hz), 3.97 (2H, d, J = 6.0 Hz), 2.76 (2H, t, J = 6.6 Hz), 2.67-2.46 (10H, m), 2.29 (2H, t, J = 7.8 Hz), 1.67-1.48 (7H, m), 1.39-1.18 (46H, m), 1.10-0.98 (9H, m), 0.96-0.82 (9H, m).
MS m/z (M + H): 740.

[Example 51]

(1)

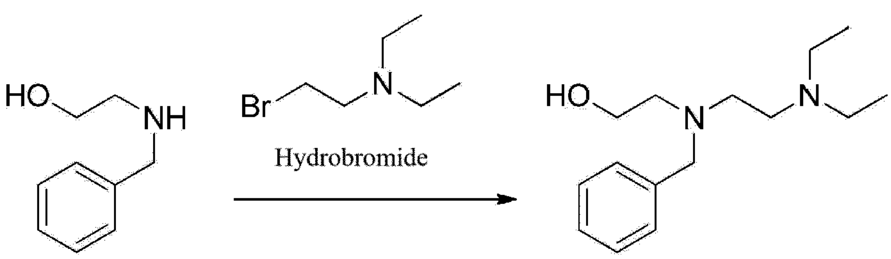


2-(Benzyl(2-(diethylamino)ethyl)amino)ethan-1-ol as a light yellow oily substance was obtained by the same method as that in (1) of Example 30, except that 2-(benzylamino)ethan-1-ol was used instead of 2-(ethylamino)ethan-1-ol in (1) of Example 30.
MS m/z (M + H): 251.

(2)

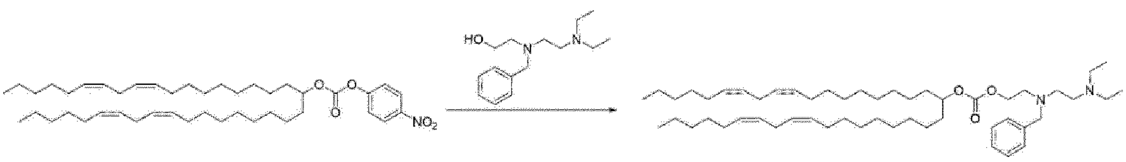

2-(Benzyl(2-(diethylamino)ethyl)amino)ethyl((6Z,9Z,28Z,31Z)-heptatriaconta-6,9,28 ,31-tetraen-19-yl)carbonate as a colorless oily substance was obtained by the same method as that in (2) of Example 20, except that 2-(benzyl(2-(diethylamino)ethyl)amino)ethan-1-ol was used instead of 2-((2-(dimethylamino)ethyl)(methyl)amino)ethan-1-ol in (2) of Example 20.

$^1$H-NMR ($CDCl_3$) $\delta$: 7.36-7.19 (5H, m), 5.46-5.27 (8H, m), 4.72-4.61 (1H, m), 4.18 (2H, t, J = 6.0 Hz), 3.68 (2H, s), 2.84-2.73 (6H, m), 2.69-2.42 (8H, m), 2.13-1.97 (8H, m), 1.65-1.49 (4H, m), 1.42-1.19 (36H, m), 0.98 (6H, t, J = 7.2 Hz), 0.89 (6H, t, J = 6.6 Hz).
MS m/z (M + H): 806.

[Example 52]

(1)

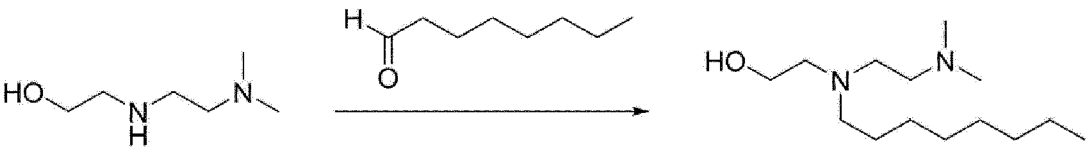

2-((2-Dimethylamino)ethyl)(octyl)amino)ethan-1-ol as a colorless oily substance was obtained by the same method as that in (1) of Example 37, except that octanal was used instead of hexanal in (1) of Example 37.
MS m/z (M + H): 245.

(2)

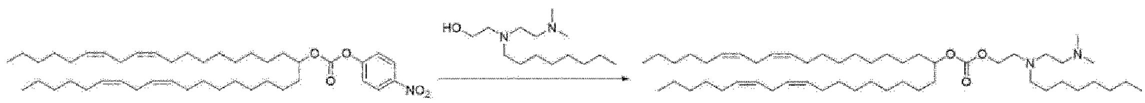

2-((2-(Dimethylamino)ethyl)(octyl)amino)ethyl((6Z,9Z,28Z,31Z)-heptatriaconta-6,9, 28,31-tetraen-19-yl)carbonate as a colorless oily substance was obtained by the same method as that in(2) of Example 20, except that 2-((2-dimethylamino)ethyl)(octyl)amino)ethan-1-ol was used instead of 2-((2-(dimethylamino)ethyl)(methyl)amino)ethan-1-ol in (2) of Example 20.

$^1$H-NMR ($CDCl_3$) $\delta$: 5.45-5.24 (8H, m), 4.73-4.62 (1H, m), 4.17 (2H, t, J = 6.0 Hz), 2.84-2.71 (6H, m), 2.67-2.56 (2H, m), 2.53-2.43 (2H, m), 2.43-2.31 (2H, m), 2.23 (6H, s), 2.12-1.96 (8H, m), 1.66-1.51 (4H, m), 1.47-1.19 (48H, m), 0.96-0.80 (9H, m).
MS m/z (M + H): 800.

[Example 53]

(1)

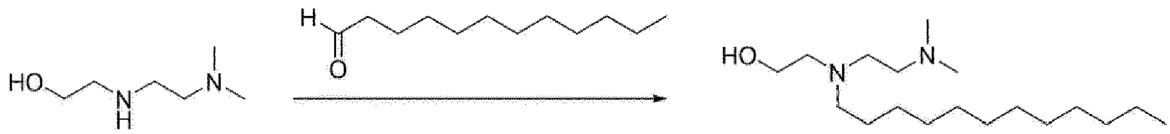

2-((2-(Dimethylamino)ethyl)(dodecyl)amino)ethan-1-ol as a colorless oily substance was obtained by the same method as that in (1) of Example 37, except that dodecanal was used instead of hexanal in (1) of Example 37. MS m/z (M + H): 301.

(2)

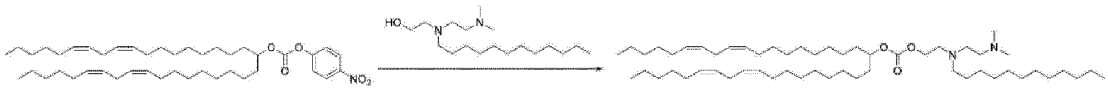

2-((2-(Dimethylamino)ethyl)(dodecyl)amino)ethyl ((6Z,9Z,28Z,31Z)-heptatriaconta-6,9,28,31-tetraen-19-yl)carbonate as a colorless oily substance was obtained by the same method as that in (2) of Example 20, except that 2-((2-(dimethylamino)ethyl)(dodecyl)amino)ethan-1-ol was used instead of 2-((2-(dimethylamino)ethyl)(methyl)amino)ethan-1-ol in (2) of Example 20.

$^1$H-NMR ($CDCl_3$) δ: 5.46-5.25 (8H, m), 4.72-4.61 (1H, m), 4.17 (2H, t, J = 6.6 Hz), 2.85-2.70 (6H, m), 2.66-2.57 (2H, m), 2.54-2.43 (2H, m), 2.42-2.32 (2H, m), 2.23 (6H, s), 2.11-1.97 (8H, m), 1.66-1.50 (4H, m), 1.47-1.17 (56H, m), 0.97-0.81 (9H, m).
MS m/z (M + H): 856.

[Example 54]

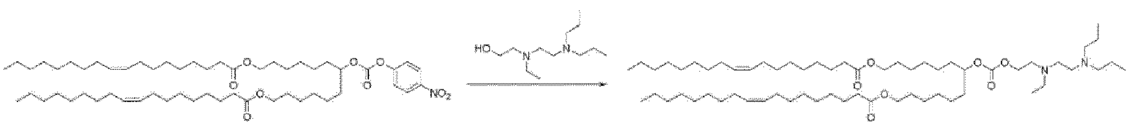

7-(((2-((2-(Dipropylamino)ethyl)(ethyl)amino)ethoxy)carbonyl)oxy)tridecane-1,13-di yldioleate as a colorless oily substance was obtained by the same method as that in (2) of Example 41, except that 2-((2-(dipropylamino)ethyl)(ethyl)amino)ethan-1-ol was used instead of 2-((2-(diethylamino)ethyl)(ethyl)amino)ethan-1-ol in (2) of Example 41.

$^1$H-NMR ($CDCl_3$) δ: 5.41-5.26 (4H, m), 4.73-4.60 (1H, m), 4.17 (2H, t, J = 6.0 Hz), 4.04 (4H, t, J = 6.6 Hz), 2.75 (2H, t, J = 6.6 Hz), 2.65-2.46 (6H, m), 2.43-2.34 (4H, m), 2.28 (4H, t, J = 7.2 Hz), 2.10-1.95 (8H, m), 1.69-1.51 (12H, m), 1.50-1.19 (56H, m), 1.03 (3H, t, J = 7.5 Hz), 0.94-0.81 (12H, m).
MS m/z (M + H): 1004.

[Example 55]

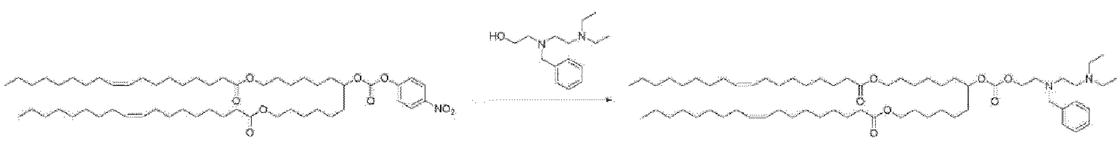

7-(((2-(Benzyl(2-(diethylamino)ethyl)amino)ethoxy)carbonyl)oxy)tridecane-1,13-diyl dioleate as a colorless oily substance was obtained by the same method as that in (2) of Example 41, except that 2-(benzyl(2-(diethylamino)ethyl)amino)ethan-1-ol was used instead of 2-((2-(diethylamino)ethyl)(ethyl)amino)ethan-1-ol in (2) of Example 41.

$^1$H-NMR ($CDCl_3$) δ: 7.36-7.17 (5H, m), 5.42-5.27 (4H, m), 4.71-4.61 (1H, m), 4.19 (2H, t, J = 6.6 Hz), 4.04 (4H, t, J = 7.2 Hz), 3.68 (2H, s), 2.79 (2H, t, J = 6.0 Hz), 2.67-2.42 (8H, m), 2.28 (4H, t, J = 8.1 Hz), 2.08-1.93 (8H, m), 1.69-1.49 (12H, m), 1.42-1.20 (52H, m), 0.97 (6H, t, J = 7.2 Hz), 0.88 (6H, t, J = 6.6 Hz).
MS m/z (M + H): 1038.

[Example 56]

(1)

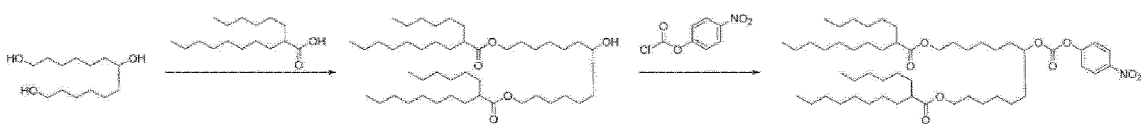

7-(((4-Nitrophenoxy)carbonyl)oxy)tridecane-1,13-diylbis(2-hexyldecanoate) as a colorless oily substance was obtained by the same method as that in (1) of Example 41, except that 2-hexyldecanoic acid was used instead of oleic acid in (1) of Example 41.

$^1$H-NMR ($CDCl_3$) δ: 8.28 (2H, dd, J = 7.2Hz, 2.1 Hz), 7.39 (2H, dd, J = 7.2 Hz, 2.1 Hz), 4.86-4.76 (1H, m), 4.07 (4H, t, J = 6.6 Hz), 2.36-2.25 (2H, m), 1.72-1.20 (68H, m), 0.87 (12H, t, J = 6.0 Hz).

(2)

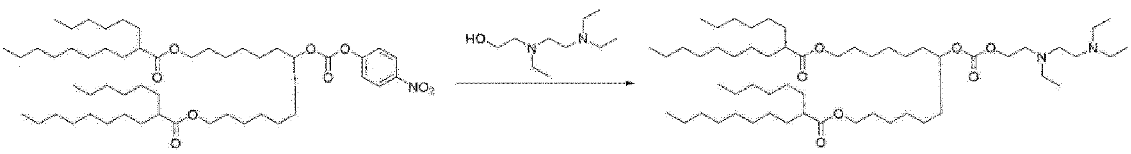

7-(((2-((2-(Diethylamino)ethyl)(ethyl)amino)ethoxy)carbonyl)oxy)tridecane-1,13-diyl bis(2-hexyldecanoate) as a colorless oily substance was obtained by the same method as that in (2) of Example 41, except that 7-(((4-nitrophenoxy)carbonyl)oxy)tridecane-1,13-diylbis(2-hexyldecanoate) was used instead of 7-(((4-nitrophenoxy)carbonyl)oxy)tridecane-1,13-diyldioleate in (2) of Example 41.

$^1$H-NMR ($CDCl_3$) δ: 4.73-4.61 (1H, m), 4.17 (2H, t, J = 6.6 Hz), 4.05 (4H, t, J = 6.6 Hz), 2.76 (2H, t, J = 6.6 Hz), 2.67-2.46 (10H, m), 2.36-2.23 (2H, m), 1.68-1.16 (68H, m), 1.09-0.97 (9H, m), 0.94-0.81 (12H, m).
MS m/z (M + H): 924.

[Example 57]

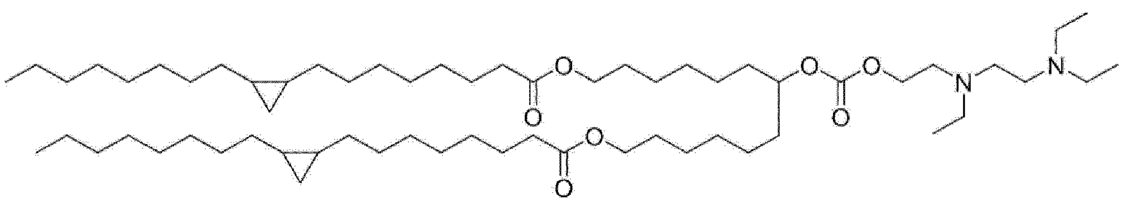

7-(((2-((2-(Diethylamino)ethyl)(ethyl)amino)ethoxy)carbonyl)oxy)tridecane-1,13-diyl bis(8-(2-octylcyclopropyl)octanoate)) as a colorless oily substance was obtained by the same method as that in (1) and (2) of Example 41, except that 8-(2-octylcyclopropyl)octanoic acid synthesized according to the method described in European Journal of Medicinal Chemistry, 2016, 109, p134-145 was used instead of oleic acid in (1) and (2) of Example 41.

$^1H$-NMR ($CDCl_3$) δ: 4.72-4.62 (1H, m), 4.18 (2H, t, J = 6.6 Hz), 4.05 (4H, t, J = 6.6 Hz), 2.76 (2H, t, J = 6.6 Hz), 2.66-2.47 (10H, m), 2.29 (4H, t, J = 8.1 Hz), 1.69-1.48 (12H, m), 1.45-1.08 (60H, m), 1.08-0.97 (9H, m), 0.88 (6H, t, J = 7.2 Hz), 0.71-0.51 (6H, m), -0.29--0.38 (2H, m).
MS m/z (M + H): 1004.

[Example 58]

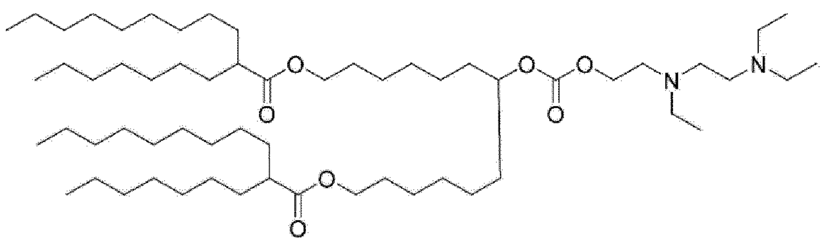

7-(((2-((2-(Diethylamino)ethyl)(ethyl)amino)ethoxy)carbonyl)oxy)tridecane-1,13-diyl bis(2-heptylundecanoate) as a colorless oily substance was obtained by the same method as that in (1) and (2) of Example 41, except that 2-heptylundecanoic acid was used instead of oleic acid in (1) and (2) of Example 41.

$^1H$-NMR ($CDCl_3$) δ: 4.72-4.62 (1H, m), 4.17 (2H, t, J = 6.6 Hz), 4.05 (4H, t, J = 6.6 Hz), 2.76 (2H, t, J = 5.7 Hz), 2.65-2.47 (10H, m), 2.36-2.24 (2H, m), 1.69-1.17 (76H, m), 1.08-0.98 (9H, m), 0.88 (12H, t, J = 7.5 Hz).
MS m/z (M + H): 980.

[Example 59]

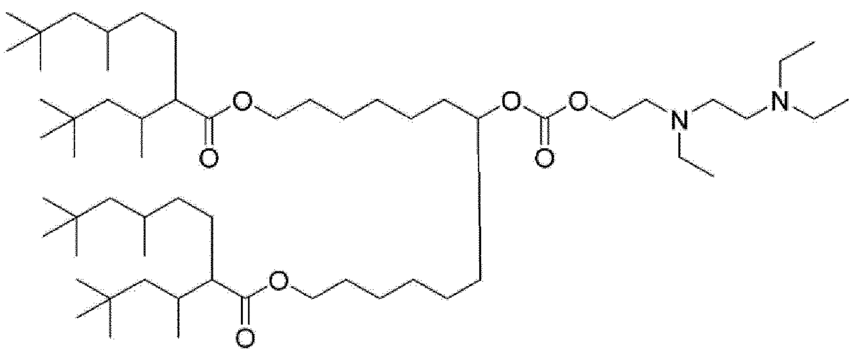

7-(((2-((2-(Diethylamino)ethyl)(ethyl)amino)ethoxy)carbonyl)oxy)tridecane-1,13-diyl bis(2-(4,4-dimethylpentan-2-yl)-5,7,7-trimethyloctanoate) as a colorless oily substance was obtained by the same method as that in (1) and (2) of Example 41, except that 2-(4,4-dimethylpentan-2-yl)-5,7,7-trimethyloctanoic acid was used instead of oleic acid in (1) and (2) of Example 41.

$^1H$-NMR ($CDCl_3$) δ: 4.73-4.62 (1H, m), 4.17 (2H, t, J = 6.6 Hz), 4.11-3.95 (4H, m), 2.76 (2H, t, J = 6.0 Hz), 2.65-2.46 (10H, m), 2.19-2.06 (2H, m), 1.86-1.13 (40H, m), 1.10-0.79 (57H, m).
MS m/z (M + H): 980.

[Example 60]

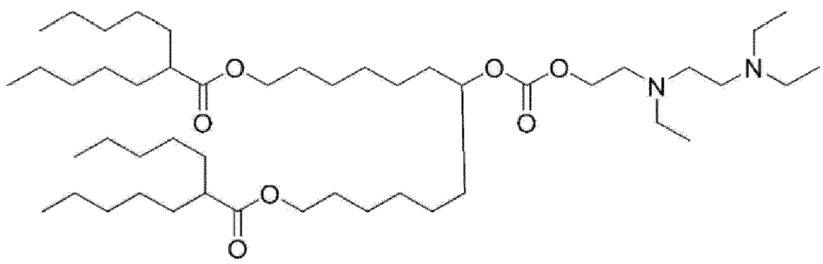

7-(((2-((2-(Diethylamino)ethyl)(ethyl)amino)ethoxy)carbonyl)oxy)tridecane-1,13-diyl bis(2-pentylheptanoate) as a colorless oily substance was obtained by the same method as that in (1) and (2) of Example 41, except that 2-pentylheptanoic acid was used instead of oleic acid in (1) and (2) of Example 41.

$^1$H-NMR ($CDCl_3$) $\delta$: 4.73-4.61 (1H, m), 4.17 (2H, t, J = 6.6 Hz), 4.05 (4H, t, J = 6.6 Hz), 2.76 (2H, t, J = 6.0 Hz), 2.65-2.47 (10H, m), 2.37-2.25 (2H, m), 1.69-1.19 (52H, m), 1.07-0.98 (9H, m), 0.87 (12H, t, J = 6.6 Hz).
MS m/z (M + H): 812.

[Example 61]

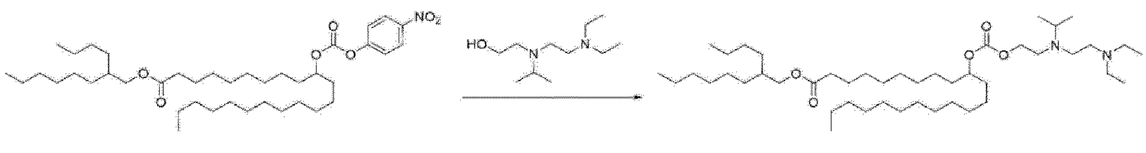

2-Butyloctyl

12-dodecyl-3-ethyl-6-isopropyl-10-oxo-9,11-dioxa-3,6-diazahenicosan-21-oate as a colorless oily substance was obtained by the same method as that in (3) of Example 50, except that 2-((2-(diethylamino)ethyl)(isopropyl)amino)ethan-1-ol was used instead of 2-((2-(diethylamino)ethyl)(ethyl)amino)ethan-1-ol in (3) of Example 50.

$^1$H-NMR ($CDCl_3$) $\delta$: 4.73-4.60 (1H, m), 4.10 (2H, t, J = 6.6 Hz), 3.97 (2H, d, J = 6.0 Hz), 2.97-2.85 (1H, m), 2.68 (2H, t, J = 7.2 Hz), 2.60-2.41 (8H, m), 2.29 (2H, t, J = 7.8 Hz), 1.66-1.48 (7H, m), 1.40-1.20 (46H, m), 1.07-0.95 (12H, m), 0.94-0.81 (9H, m).
MS m/z (M + H): 754.

[Example 62]

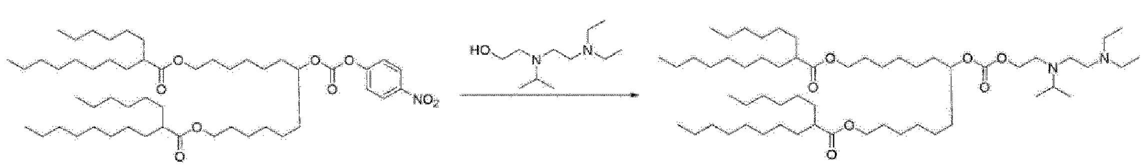

7-(((2-((2-(Diethylamino)ethyl)(isopropyl)amino)ethoxy)carbonyl)oxy)tridecane-1,13 -diylbis(2-hexyldecanoate) as a colorless oily substance was obtained by the same method as that in (2) of Example 56, except that 2-((2-(diethylamino)ethyl)(isopropyl)amino)ethan-1-ol was used instead of 2-((2-(diethylamino)ethyl)(ethyl)amino)ethan-1-ol in (2) of Example 56.

$^1$H-NMR ($CDCl_3$) $\delta$: 4.73-4.61 (1H, m), 4.15-3.99 (6H, m), 2.97-2.84 (1H, m), 2.68 (2H, t, J = 6.6 Hz), 2.60-2.41 (8H, m), 2.37-2.23 (2H, m), 1.69-1.16 (68H, m), 1.10-0.95 (12H, m), 0.87 (12H, t, J = 6.6 Hz).
MS m/z (M + H): 938.

[Example 63]

(1)

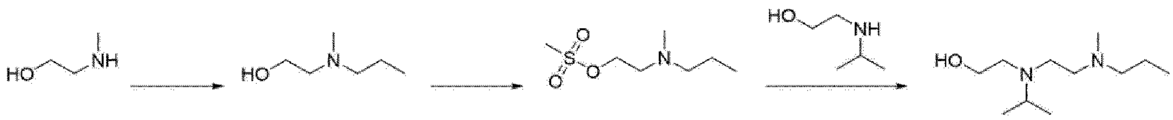

A mixture of 2-(methylamino)ethan-1-ol (3 g), potassium carbonate (6.6 g), 1-bromopropane (5.6 mL), and acetonitrile (30 mL) was stirred at 60°C for 9 hours and 30 minutes. A saturated aqueous sodium hydrogen carbonate solution was added to the reaction mixture, and extraction was performed with chloroform. The organic layer was washed with saturated saline and dried over anhydrous sodium sulfate. The solvent was distilled away under reduced pressure, thereby obtaining 2-(methyl(propyl)amino)ethan-1-ol (4.3 g) as a colorless oily substance.
MS m/z (M + H): 118.

Methanesulfonic anhydride (1.9 g) was added dropwise to a mixture of 2-(methyl(propyl)amino)ethan-1-ol (1.2 g) and acetonitrile (10 mL) under ice cooling, and the mixture was stirred at 0°C for 30 minutes and then stirred at room temperature for 30 minutes. 2-(Isopropylamino)ethan-1-ol (2.0 g) and N,N-diisopropylethylamine (2.0 mL) were added to the reaction mixture, and the reaction mixture was stirred at 70°C for 25 hours and 30 minutes. The reaction mixture was cooled to room temperature, then potassium carbonate and water were added thereto, and extraction was performed with ethyl acetate. The organic layer was washed with saturated saline and dried over anhydrous sodium sulfate. The solvent was distilled away under reduced pressure, and the obtained residue was purified by silica gel column chromatography (methanol-chloroform), thereby obtaining 2-(isopropyl(2-(methyl(propyl)amino)ethyl)amino)ethan-1-ol (0.3g) as a yellow oily substance.
MS m/z (M + H): 203.

(2)

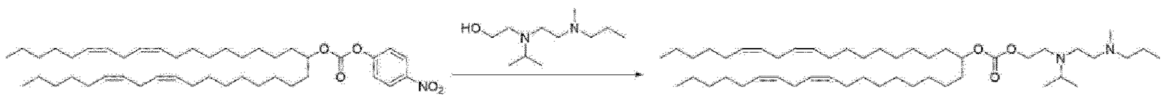

(6Z,9Z,28Z,3 1Z)-heptatriaconta-6,9,28,3 1-tetraen- 19-yl(2-(isopropyl(2-(methyl(prop yl)amino)ethyl)amino)ethyl)carbonate as a colorless oily substance was obtained by the same method as that in (2) of Example 20, except that 2-(isopropyl(2-(methyl(propyl)amino)ethyl)amino)ethan-1-ol was used instead of 2-((2-(dimethylamino)ethyl)(methyl)amino)ethan-1-ol in (2) of Example 20.

$^1$H-NMR ($CDCl_3$) δ: 5.45-5.26 (8H, m), 4.73-4.62 (1H, m), 4.09 (2H, t, J = 6.6 Hz), 2.97-2.86 (1H, m), 2.77 (4H, t, J = 6.0 Hz), 2.69 (2H, t, J = 7.2 Hz), 2.62-2.51 (2H, m), 2.44-2.35 (2H, m), 2.35-2.27 (2H, m), 2.23 (3H, s), 2.11-1.96 (8H, m), 1.66-1.20 (42H, m), 0.98 (6H, d, J = 6.6 Hz), 0.94-0.82 (9H, m).
MS m/z (M + H): 758.

[Example 64]

(1)

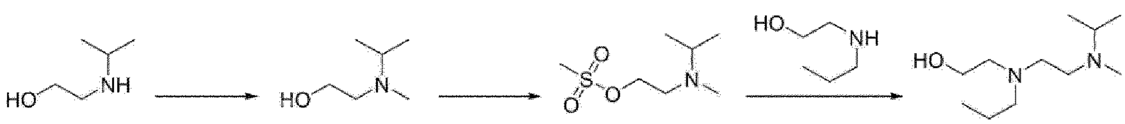

Methyl iodide (1.9 mL) was added dropwise to a dichloromethane (30 mL) solution of 2-(isopropylamino)ethan-1-ol (3 g) under ice cooling. The mixture was stirred at the same temperature for 1 hour and 15 minutes and then stirred at room temperature for 6 hours and 50 minutes. Potassium carbonate and water were added to the reaction mixture,

and extraction was performed with chloroform. The organic layer was washed with saturated saline and dried over anhydrous sodium sulfate. The solvent was distilled away under reduced pressure, and the obtained residue was purified by silica gel column chromatography (methanol-chloroform, NH silica gel), thereby obtaining 2-(isopropyl(methyl)amino)ethan-1-ol (2.2 g) as a colorless oily substance.
MS m/z (M + H): 118.

Methanesulfonic anhydride (2.6 g) was added to a mixture of 2-(isopropyl(methyl)amino)ethan-1-ol (1.5 g), N,N-diisopropylethylamine (2.5 mL), and acetonitrile (15 mL) under ice cooling, and the mixture was stirred at room temperature for 4 hours and 50 minutes. 2-(Propylamino)ethan-1-ol (4.3 mL) was added to the reaction mixture, and the reaction mixture was stirred at 70°C for 23 hours and 30 minutes. The reaction mixture was cooled to room temperature, then a saturated aqueous sodium hydrogen carbonate solution was added thereto, and extraction was performed with ethyl acetate. The organic layer was washed with saturated saline and dried over anhydrous sodium sulfate. The solvent was distilled away under reduced pressure, and the obtained residue was purified by silica gel column chromatography (methanol-chloroform), thereby obtaining 2-((2-(isopropyl(methyl)amino)ethyl)(propyl)amino)ethan-1-ol (0.7 g) as a yellow oily substance.
MS m/z (M + H): 203.

(2)

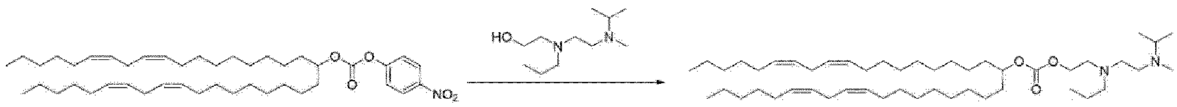

(6Z,9Z,28Z,31Z)-heptatriaconta-6,9,28,31-tetraen-19-yl(2-((2-isopropyl(methyl)amin o)ethyl)(propyl)amino)ethyl)carbonate as a colorless oily substance was obtained by the same method as that in (2) of Example 20, except that 2-((2-(isopropyl(methyl)amino)ethyl)(propyl)amino)ethan-1-ol was used instead of 2-((2-(dimethylamino)ethyl)(methyl)amino)ethan-1-ol in (2) of Example 20.

$^1$H-NMR ($CDCl_3$) δ: 5.46-5.26 (8H, m), 4.74-4.60 (1H, m), 4.17 (2H, t, J = 6.6 Hz), 2.87-2.70 (7H, m), 2.65-2.54 (2H, m), 2.51-2.40 (4H, m), 2.21 (3H, s), 2.12-1.95 (8H, m), 1.64-1.20 (42H, m), 1.00 (6H, d, J = 6.6 Hz), 0.94-0.81 (9H, m).
MS m/z (M + H): 758.

[Example 65]

(1)

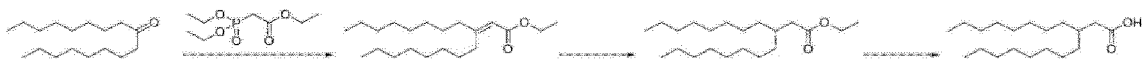

Ethyl 2-(diethoxyphosphoryl)acetate (9.4 mL) was added dropwise to a tetrahydrofuran (60 mL) suspension of 60 wt% sodium hydride (1.7 g) under ice cooling, and the mixture was stirred at the same temperature for 30 minutes. Heptadecan-9-one (1.5 g) was added to the reaction mixture, and the reaction mixture was stirred under reflux with heating for 16 hours. The reaction mixture was cooled to room temperature and poured into ice water, and then ethyl acetate was added thereto. The organic layer was separated and washed with a saturated aqueous sodium chloride solution, then the solvent was distilled away under reduced pressure, and the obtained residue was purified by silica gel column chromatography (ethyl acetate-hexane), thereby obtaining ethyl 3-octylundec-2-enoate (1.2 g) as a colorless oily substance.

$^1$H-NMR ($CDCl_3$) δ: 5.61 (1H, s), 4.14 (2H, q, J = 6.6 Hz), 2.58 (2H, t, J = 7.2 Hz), 2.12 (2H, t, J = 7.2 Hz), 1.50-1.20 (27H, m), 0.91-0.85 (6H, m).

Ammonium formate (1.4 g) was added to a mixture of ethyl 3-octylundec-2-enoate (1.2 g), 10% palladium-carbon (0.35 g), and methanol (24 mL), and the mixture was stirred under reflux with heating for 4 hours. The reaction mixture was cooled to room temperature, the insoluble matters were filtered off through celite, and then the solvent was distilled away under reduced pressure. The obtained residue was purified by silica gel column chromatography (ethyl acetate-hexane), thereby obtaining ethyl 3-octylundecanoate (1.1 g) as a colorless oily substance.

$^1$H-NMR ($CDCl_3$) δ: 4.12 (2H, q, J = 7.2 Hz), 2.21 (2H, d, J = 6.6 Hz), 2.05-2.04 (1H, m), 1.34-1.20 (31H, m), 0.88 (6H, 6.6 Hz).

A 5 mol/L aqueous sodium hydroxide solution (5 mL) was added to a mixture of ethyl 3-octylundecanoate (1.1

g) and ethanol (10 mL), and the mixture was stirred at 80°C for 5 hours. The reaction mixture was cooled to room temperature, a 1 mol/L aqueous hydrochloric acid solution was added until the reaction mixture became acidic, and then ethyl acetate was added thereto. The organic layer was separated and washed with a saturated aqueous sodium chloride solution, then the solvent was distilled away under reduced pressure, and the obtained residue was purified by silica gel column chromatography (ethyl acetate-hexane), thereby obtaining 3-octylundecanoic acid (1.1 g) as a colorless oily substance.

$^1$H-NMR ($CDCl_3$) $\delta$: 2.28 (2H, d, J = 6.6 Hz), 1.90-1.79 (1H, m), 1.35-1.19 (28H, m), 0.88 (6H, t, J = 6.6 Hz).

(2)

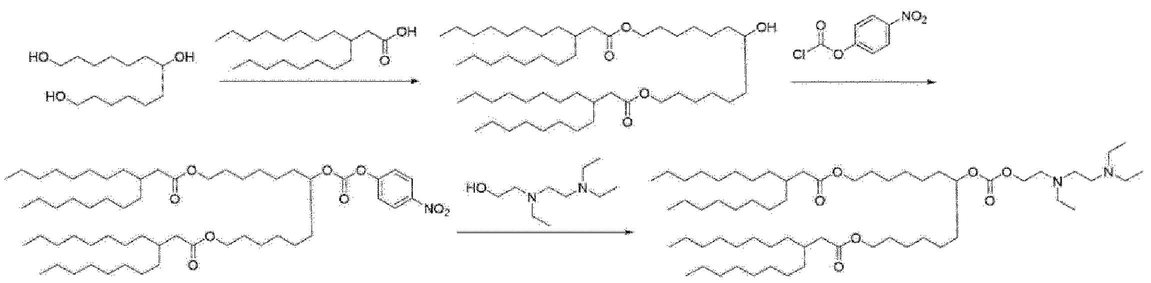

7-(((2-((2-(Diethylamino)ethyl)(ethyl)amino)ethoxy)carbonyl)oxy)tridecane-1,13-diyl bis(3-octylundecanoate) as a colorless oily substance was obtained by the same method as that in (1) and (2) of Example 41, except that 3-octylundecanoic acid was used instead of oleic acid in (1) and (2) of Example 41.

$^1$H-NMR ($CDCl_3$) $\delta$: 4.74-4.62 (1H, m), 4.17 (2H, t, J = 6.6 Hz), 4.04 (4H, t, J = 6.6 Hz), 2.76 (2H, t, J = 6.6 Hz), 2.66-2.47 (10H, m), 2.22 (4H, d, J = 6.6 Hz), 1.90-1.76 (2H, m), 1.70-1.17 (76H, m), 1.10-0.97 (9H, m), 0.88 (12H, t, J = 6.6 Hz).
MS m/z (M + H): 1008.

[Example 66]

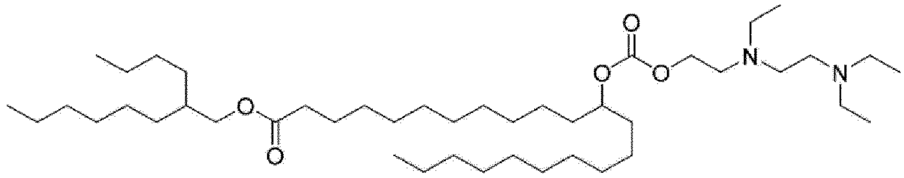

2-Butyloctyl 12-decyl-3,6-diethyl-10-oxo-9,11-dioxa-3,6-diazatricosan-23-oate as a colorless oily substance was obtained by the same method as that in (1), (2), and (3) of Example 50, except that 12-ethoxy-12-oxododecanoic acid and a 1.0 mol/L decyl magnesium bromide-diethyl ether solution were used instead of 10-ethoxy-10-oxodecanoic acid and a 1.0 mol/L dodecyl magnesium bromide-diethyl ether solution in (1), (2), and (3) of Example 50, respectively.

$^1$H-NMR ($CDCl_3$) $\delta$: 4.73-4.61 (1H, m), 4.17 (2H, t, J = 6.6 Hz), 3.97 (2H, d, J = 5.7 Hz), 2.76 (2H, t, J = 6.6 Hz), 2.66-2.46 (10H, m), 2.30 (2H, t, J = 7.2 Hz), 1.70-1.47 (7H, m), 1.41-1.20 (46H, m), 1.11-0.98 (9H, m), 0.95-0.82 (9H, m).
MS m/z (M + H): 740.

[Example 67]

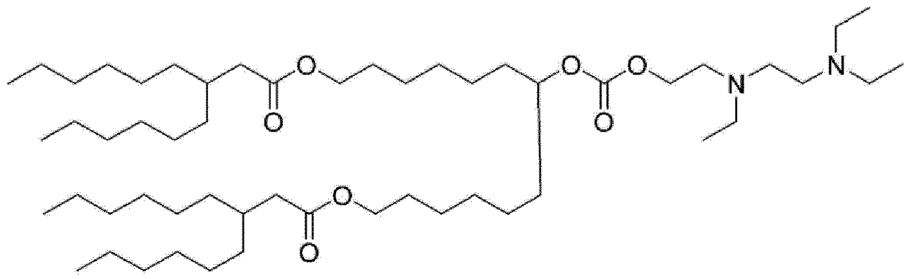

7-(((2-((2-(Diethylamino)ethyl)(ethyl)amino)ethoxy)carbonyl)oxy)tridecane-1,13-diyl bis(3-hexylnonanoate) as a colorless oily substance was obtained by the same method as that in (1) and (2) of Example 65, except that tridecan-7-one was used instead of heptadecan-9-one in (1) and (2) of Example 65.

$^1$H-NMR ($CDCl_3$) $\delta$: 4.73-4.62 (1H, m), 4.17 (2H, t, J = 6.6 Hz), 4.04 (4H, t, J = 6.6 Hz), 2.76 (2H, t, J = 6.6 Hz), 2.67-2.45 (10H, m), 2.22 (4H, d, J = 6.6 Hz), 1.89-1.77 (2H, m), 1.67-1.17 (60H, m), 1.08-0.98 (9H, m), 0.88 (12H, t, J = 6.6 Hz).
MS m/z (M + H): 896.

[Example 68]

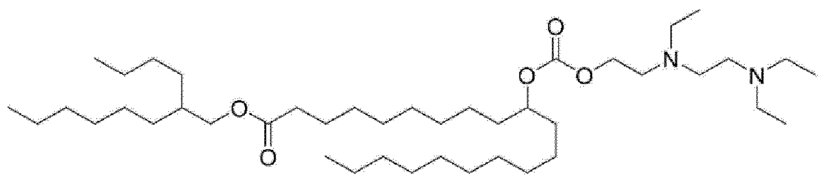

2-Butyloctyl 12-decyl-3,6-diethyl-10-oxo-9,11-dioxa-3,6-diazahenicosan-21-oate as a colorless oily substance was obtained by the same method as that in (1), (2), and (3) of Example 50, except that a 1.0 mol/L decyl magnesium bromide-diethyl ether solution was used instead of a 1.0 mol/L dodecyl magnesium bromide-diethyl ether solution in (1), (2), and (3) of Example 50.

$^1$H-NMR ($CDCl_3$) $\delta$: 4.73-4.60 (1H, m), 4.17 (2H, t, J = 6.6 Hz), 3.97 (2H, J = 5.4 Hz), 2.76 (2H, t, J = 6.0 Hz), 2.67-2.46 (10H, m), 2.29 (2H, t, J = 7.8 Hz), 1.68-1.50 (7H, m), 1.39-1.20 (42H, m), 1.07-0.98 (9H, m), 0.94-0.83 (9H, m).
MS m/z (M + H): 712.

[Example 69]

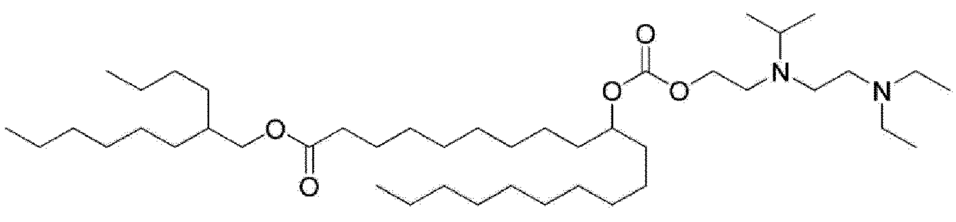

2-Butyloctyl

12-decyl-3-ethyl-6-isopropyl-10-oxo-9,11-dioxa-3,6-diazahenicosan-21-oate as a colorless oily substance was obtained by the same method as that in Example 68, except that 2-((2-(diethylamino)ethyl)(isopropyl)amino)ethan-1-ol was used instead of 2-((2-(diethylamino)ethyl)(ethyl)amino)ethan-1-ol in Example 68.

$^1$H-NMR ($CDCl_3$) δ: 4.73-4.61 (1H, m), 4.10 (2H, t, J = 6.6 Hz), 3.97 (2H, d, J = 6.0 Hz), 2.99-2.83 (1H, m), 2.68 (2H, t, J = 6.6 Hz), 2.62-2.41 (8H, m), 2.29 (2H, t, J = 7.2 Hz), 1.69-1.47 (7H, m), 1.40-1.19 (42H, m), 1.10-0.96 (12H, m), 0.94-0.83 (9H, m).
MS m/z (M + H): 726.

[Example 70]

(1)

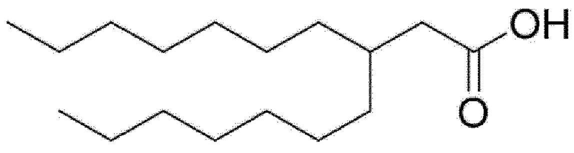

3-Heptyldecanoic acid as a colorless oily substance was obtained by the same method as that in (1) of Example 65, except that pentadecan-8-one was used instead of heptadecane-9-one in (1) of Example 65.
$^1$H-NMR ($CDCl_3$) δ: 2.28 (2H, d, J = 6.6 Hz), 1.90-1.79 (1H, m), 1.35-1.19 (24H, m), 0.88 (6H, t, J = 6.6 Hz).

(2)

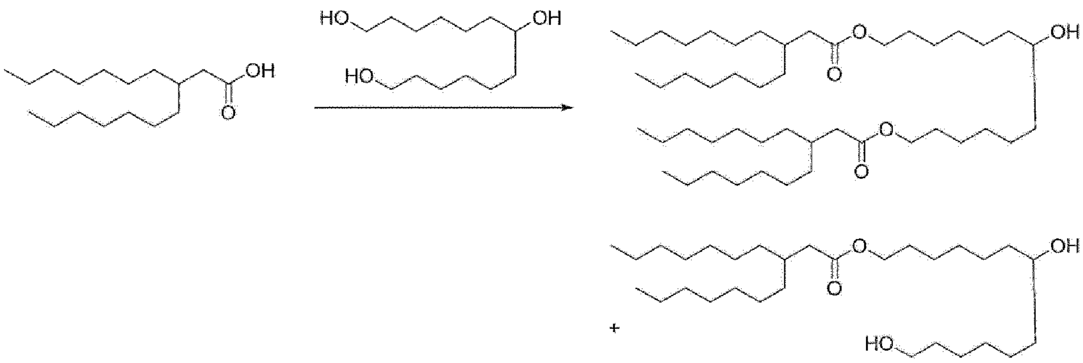

1-Ethyl-3-(3-dimethylaminopropyl)carbodiimide hydrochloride (1.07 g) was added to a mixture of 3-heptyldecanoic acid (974 mg), tridecane-1,7,13-triol (2.49 g), triethylamine (3.5 mL), 4-dimethylaminopyridine (51 mg), and dichloromethane (20 mL), and the mixture was stirred at room temperature for 4 days. Water and ethyl acetate were added to the reaction mixture, the organic layer was separated, washed with a saturated aqueous sodium chloride solution, and then dried over anhydrous sodium sulfate, and the solvent was distilled away under reduced pressure. The obtained residue was purified by silica gel column chromatography (ethyl acetate-hexane), thereby obtaining 7-hydroxytridecane-1,13-diylbis(3-heptyldecanoate) (1.03 g) as a colorless oily substance and 7,13-dihydroxytridecyl 3-heptyldecanoate (1.03 g) in a colorless oily substance.
7-Hydroxytridecane-1,13-diylbis(3-heptyldecanoate) $^1$H-NMR ($CDCl_3$) δ: 4.05 (4H, t, J = 6.6 Hz), 3.61-3.54 (1H, m), 2.22 (4H, d, J = 7.2 Hz), 1.88-1.20 (70H, m), 0.88 (12H, t, J = 6.6 Hz).
7,13-Dihydroxytridecyl 3-heptyldecanoate $^1$H-NMR ($CDCl_3$) δ: 4.05 (2H, t, J = 6.6 Hz), 3.68-3.55 (3H, m), 2.22 (2H, d, J = 6.6 Hz), 1.88-1.77 (1H, m), 1.68-1.20 (44H, m), 0.88 (6H, t, J = 6.6 Hz).

(3)

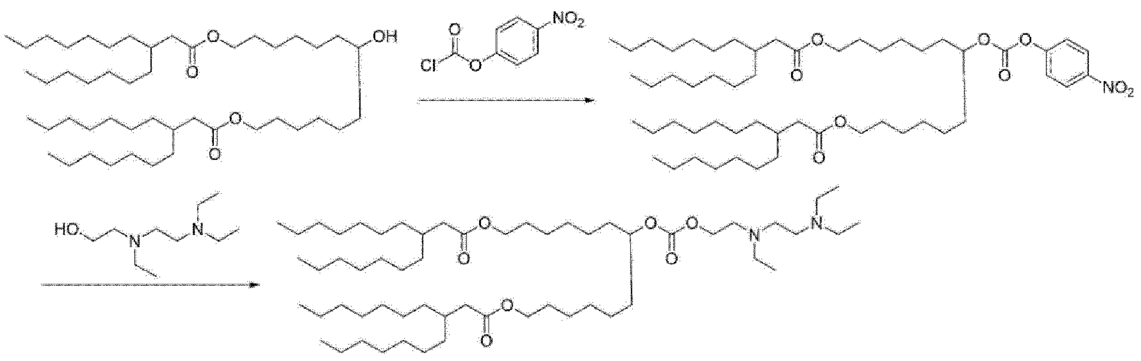

7 -(((2- ((2- (diethylamino)ethyl) (ethyl) amino)ethoxy)carbonyl)oxy)tridecane- 1, 13-diyl bis(3-heptyldecanoate) as a colorless oily substance was obtained by the same method as that in (1) and (2) of Example 20, except that 7-hydroxytridecane-1,13-diylbis(3-heptyldecanoate) and 2-((2-(diethylamino)ethyl)(ethyl)amino)ethan-1-ol were used instead of (6Z,9Z,28Z,31Z)-heptatriaconta-6,9,28,31-tetraen-19-ol and 2-((2-(dimethylamino)ethyl)(methyl)amino)ethan-1-ol in (1) and (2) of Example 20, respectively.

$^1$H-NMR ($CDCl_3$) $\delta$: 4.73-4.61 (1H, m), 4.17 (2H, t, J = 6.6 Hz), 4.04 (4H, t, J = 7.2 Hz), 2.76 (2H, t, J = 6.0 Hz), 2.66-2.46 (10H, m), 2.22 (4H, d, J = 7.2 Hz), 1.91-1.76 (2H, m), 1.67-1.15 (68H, m), 1.08-0.97 (9H, m), 0.88 (12H, t, J = 6.6 Hz).
MS m/z (M + H): 952.

[Example 71]

(1)

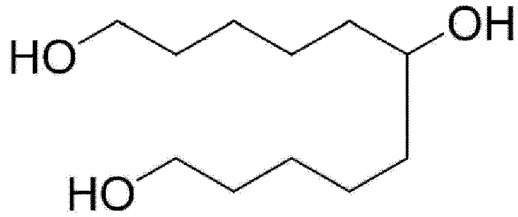

Undecane-1,6,11-triol as white solids was obtained by the same method as that in (1) of Example 36, except that 5-bromopentan-1-ol was used instead of 6-bromohexan-1-ol in (1) of Example 36.
$^1$H-NMR ($CDCl_3$) $\delta$: 3.70-3.55 (5H, m), 1.64-1.24 (16H, m).

(2)

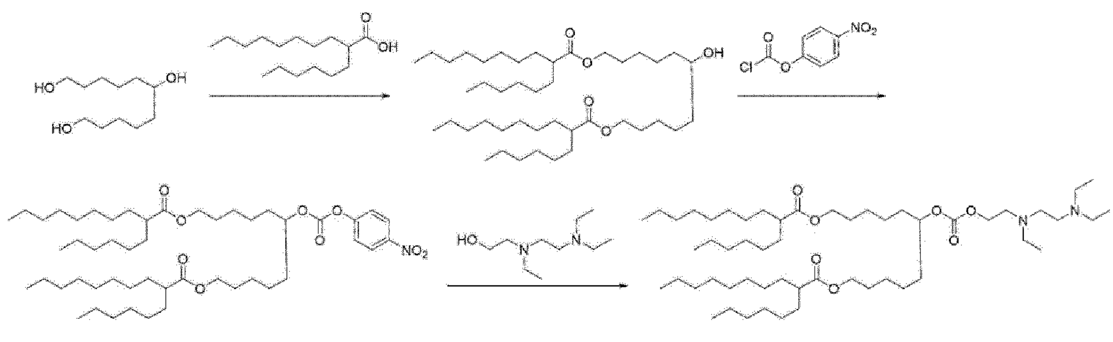

6-(((2-((2-(Diethylamino)ethyl)(ethyl)amino)ethoxy)carbonyl)oxy)undecane-1,11-diy lbis(2-hexyldecanoate) as

a colorless oily substance was obtained by the same method as that in (1) and (2) of Example 56, except that undecane-1,6,11-triol was used instead of tridecane-1,7,13-triol in (1) and (2) of Example 56.

$^1$H-NMR ($CDCl_3$) δ: 4.72-4.63 (1H, m), 4.17 (2H, t, J = 6.6 Hz), 4.05 (4H, t, J = 6.6 Hz), 2.76 (2H, t, J = 6.6 Hz), 2.66-2.47 (10H, m), 2.37-2.23 (2H, m), 1.71-1.18 (64H, m), 1.10-0.98 (9H, m), 0.88 (12H, t, J = 7.2 Hz).
MS m/z (M + H): 896.

[Example 72]

(1)

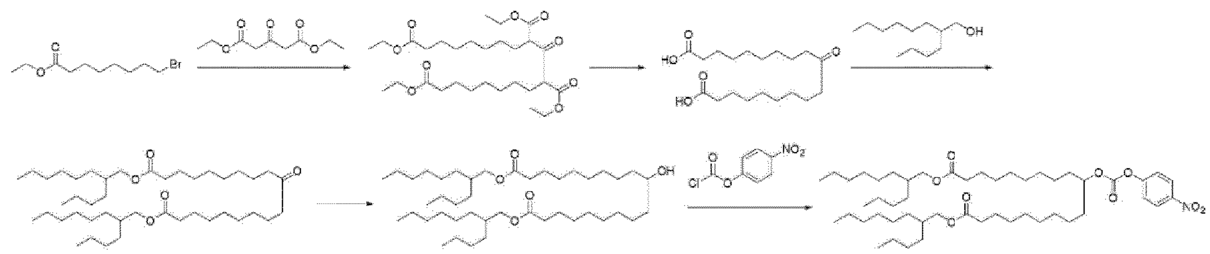

A mixture of diethyl 3-oxopentanedioate (4.0 g) and a 20% sodium ethoxide-ethanol solution (6.7 g) was stirred at 80°C for 20 minutes, then ethyl 8-bromooctanoate (5.0 g) was added thereto, and the mixture was stirred for 4 hours. A 20% sodium ethoxide-ethanol solution (6.7 g) was added to the reaction mixture, the reaction mixture was stirred for 5 minutes, then ethyl 8-bromooctanoate (5.0 g) was added thereto, and the mixture was stirred for 3 hours. The reaction mixture was cooled to room temperature, then hexane and a 20% aqueous ammonium chloride solution (10 mL) were added thereto, the organic layer was separated, and then the solvent was distilled away under reduced pressure, thereby obtaining tetraethyl 9-oxoheptadecane-1,8,10,17-tetracarboxylate (10.3 g) as a crude product.
A mixture of the obtained tetraethyl 9-oxoheptadecane-1,8,10,17-tetracarboxylate (2.5 g), acetic acid (4.0 mL), and a 30% aqueous hydrochloric acid solution (8.0 mL) was stirred at 115°C for 6 hours. The reaction mixture was cooled to room temperature, then the solvent was distilled away under reduced pressure, and water and acetone were added to the residue. Solids were collected by filtration, washed with water and acetone, and then dried under reduced pressure, thereby obtaining 10-oxononadecanedioic acid (0.6 g) as white solids.
$^1$H-NMR (DMSO-$d_6$) δ: 2.38 (4H, t, J = 7.2 Hz), 2.18 (4H, t, J = 7.2 Hz), 1.54-1.38 (8H, m), 1.31-1.18 (16H, m).
1-Ethyl-3-(3-dimethylaminopropyl)carbodiimide hydrochloride (853 mg) was added to a mixture of 10-oxononadecanedioic acid (610 mg), 2-butyloctan-1-ol (663 mg), triethylamine (1.25 mL), 4-dimethylaminopyridine (217 mg), and dichloromethane (6 mL), and the mixture was stirred at room temperature for 2 days. A 10% aqueous potassium hydrogen sulfate solution (12 mL), hexane (6 mL), and ethyl acetate (6 mL) were added to the reaction mixture, the organic layer was separated and then dried over anhydrous sodium sulfate, and the solvent was distilled away under reduced pressure. The obtained residue was purified by silica gel column chromatography (ethyl acetate-hexane), thereby obtaining bis(2-butyloctyl)10-oxononadecanedioate (612 mg) as a colorless oily substance.
$^1$H-NMR ($CDCl_3$) δ: 3.97 (4H, d, J = 6.0 Hz), 2.38 (4H, t, J = 7.2 Hz), 2.30 (4H, t, J = 7.2 Hz), 1.66-1.49 (10H, m), 1.36-1.23 (48H, m), 0.92-0.83 (12H, m).
Sodium borohydride (35 mg) was added to a mixture of bis(2-butyloctyl)10-oxononadecanedioate (612 mg) and methanol (6 mL) under ice cooling, and the mixture was stirred at the same temperature for 1 hour. A 10% aqueous potassium hydrogen sulfate solution (6 mL) and hexane (6 mL) were added to the reaction mixture under ice cooling, the organic layer was separated and dried over anhydrous sodium sulfate, and then the solvent was distilled away under reduced pressure. The obtained residue was purified by silica gel column chromatography (ethyl acetate-hexane), thereby obtaining bis(2-butyloctyl)10-hydroxynonadecanedioate (369 mg) as a colorless oily substance.
$^1$H-NMR ($CDCl_3$) δ: 3.97 (4H, d, J = 6.0 Hz), 3.62-3.52 (1H, m), 2.30 (4H, t, J = 7.2 Hz), 1.66-1.53 (10H, m), 1.45-1.20 (52H, m), 0.92-0.83 (12H, m).
4-Nitrophenyl chloroformate (218 mg) was added to a mixture of bis(2-butyloctyl)10-hydroxynonadecanedioate (369 mg), triethylamine (0.30 mL), and tetrahydrofuran (2 mL), and the mixture was stirred at room temperature for 17 hours. Water and ethyl acetate were added to the reaction mixture, the organic layer was separated, washed with water, and then dried over anhydrous sodium sulfate, and the solvent was distilled away under reduced pressure. The obtained residue was purified by silica gel column chromatography (ethyl acetate-hexane), thereby obtaining bis(2-butyloctyl)10-(((4-nitrophenoxy)carbonyl)oxy)nonadecanedioate (436 mg) as a colorless oily substance.

$^1$H-NMR ($CDCl_3$) $\delta$: 8.28 (2H, dd, J = 7.2 Hz, 1.8 Hz), 7.38 (2H, dd, J = 7.2 Hz, 1.8 Hz), 4.86-4.74 (1H, m), 3.97 (4H, d, J = 6.0 Hz), 2.30 (4H, t, J = 7.2 Hz), 1.66-1.53 (10H, m), 1.45-1.20 (52H, m), 0.92-0.83 (12H, m).

(2)

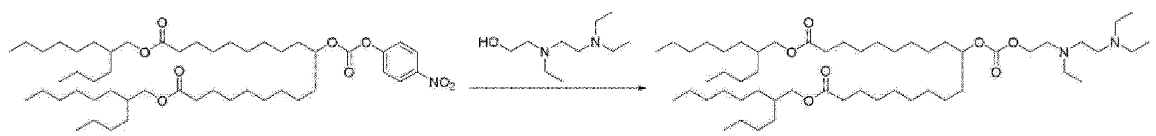

Bis(2-butyloctyl)10-(((2-((2-(diethylamino)ethyl)(ethyl)amino)ethoxy)carbonyl)oxy) nonadecanedioate as a colorless oily substance was obtained by the same method as that in (2) of Example 41, except that bis(2-butyloctyl)10-(((4-nitrophenoxy)carbonyl)oxy)nonadecanedioate was used instead of 7-(((4-nitrophenoxy)carbonyl)oxy)tridecane-1,13-diyldioleate in (2) of Example 41.

$^1$H-NMR ($CDCl_3$) $\delta$: 4.71-4.62 (1H, m), 4.17 (2H, t, J = 6.6 Hz), 3.96 (4H, d, J = 6.0 Hz), 2.76 (2H, t, J = 6.6 Hz), 2.64-2.48 (10H, m), 2.29 (4H, t, J = 7.2 Hz), 1.66-1.50 (10H, m), 1.36-1.20 (52H, m), 1.03 (3H, t, J = 7.2 Hz), 1.02 (6H, t, J = 7.2 Hz), 0.93-0.84 (12H, m).
MS m/z (M + H): 896.

[Example 73]

(1)

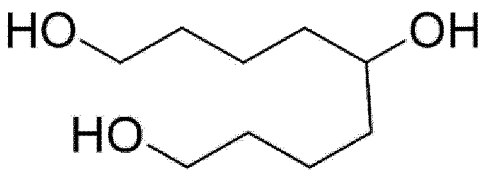

Nonane-1,5,9-triol as white solids was obtained by the same method as that in (1) of Example 36, except that 4-bromobutan-1-ol was used instead of 6-bromohexan-1-ol in (1) of Example 36.

$^1$H-NMR ($CDCl_3$) $\delta$: 3.70-3.55 (5H, m), 1.64-1.24 (12H, m).

(2)

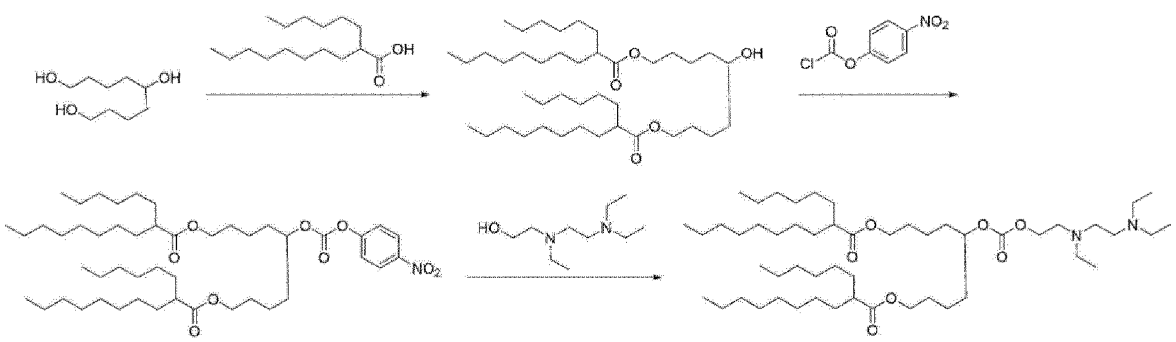

5-(((2-((2-(Diethylamino)ethyl)(ethyl)amino)ethoxy)carbonyl)oxy)nonane-1,9-diylbis (2-hexyldecanoate) as a colorless oily substance was obtained by the same method as that in (1) and (2) of Example 56, except that nonane-1,5,9-triol was used instead of tridecane-1,7,13-triol in (1) and (2) of Example 56.

$^1$H-NMR ($CDCl_3$) $\delta$: 4.74-4.63 (1H, m), 4.17 (2H, t, J = 5.7 Hz), 4.05 (4H, t, J = 6.6 Hz), 2.76 (2H, t, J = 6.6 Hz),

2.66-2.48 (10H, m), 2.36-2.24 (2H, m), 1.70-1.16 (60H, m), 1.09-0.98 (9H, m), 0.88 (12H, t, J = 6.6 Hz).
MS m/z (M + H): 868.

[Example 74]

(1)

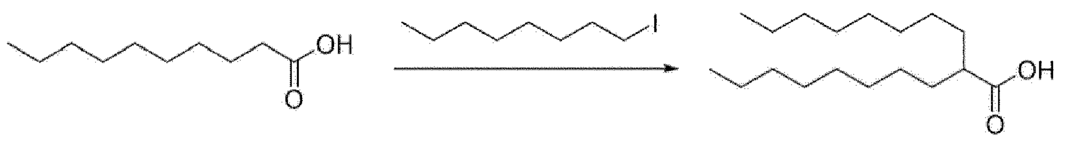

Decanoic acid (3.0 g) was added dropwise to a tetrahydrofuran (30 mL) suspension of 60 wt% sodium hydride under ice cooling, and the mixture was stirred at the same temperature for 30 minutes. A 1.5 mol/L lithium diisopropylamide-tetrahydrofuran-heptane-ethyl benzene solution (13.9 mL) was added to the reaction mixture at the same temperature, the reaction mixture was stirred at room temperature for 30 minutes. Then 1-iodooctane (3.8 mL) was added dropwise thereto, and the reaction mixture was stirred at 45°C for 6 hours.
The reaction mixture was poured into a mixture of a 1 mol/L aqueous hydrochloric acid solution and ethyl acetate under ice cooling, then the organic layer was separated, washed with a saturated aqueous sodium chloride solution, and dried over anhydrous magnesium sulfate, and then the solvent was distilled away under reduced pressure. The obtained residue was purified by silica gel column chromatography (ethyl acetate-hexane), thereby obtaining 2-octyl decanoic acid (2.62 g) as a yellow oily substance.
$^1$H-NMR ($CDCl_3$) $\delta$: 2.43-2.30 (1H, m), 1.72-1.20 (28H, m), 0.88 (6H, t, J = 6.6 Hz).

(2)

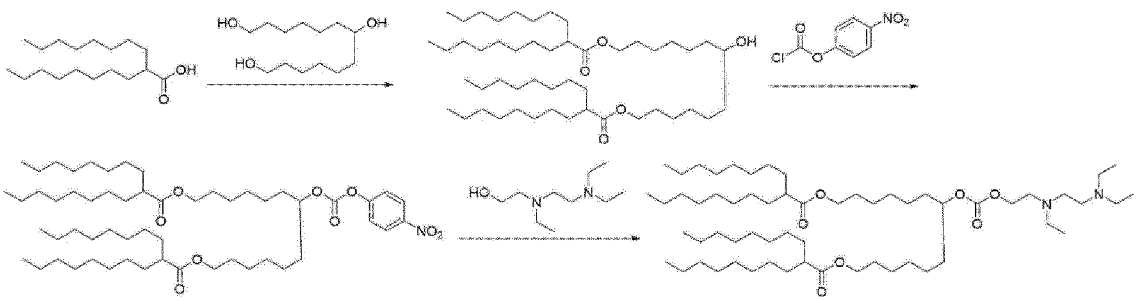

7-(((2-((2-(Diethylamino)ethyl)(ethyl)amino)ethoxy)carbonyl)oxy)tridecane-1,13-diyl bis(2-octyldecanoate) as a colorless oily substance was obtained by the same method as that in (1) and (2) of Example 41, except that 2-octyldecanoic acid was used instead of oleic acid in (1) and (2) of Example 41.

$^1$H-NMR ($CDCl_3$) $\delta$: 4.73-4.60 (1H, m), 4.17 (2H, t, J = 6.6 Hz), 4.05 (4H, t, J = 6.6 Hz), 2.76 (2H, t, J = 6.0 Hz), 2.66-2.47 (10H, m), 2.37-2.24 (2H, m), 1.70-1.16 (76H, m), 1.11-0.98 (9H, m), 0.88 (12H, t, J = 6.6 Hz).
MS m/z (M + H): 980.

[Example 75]

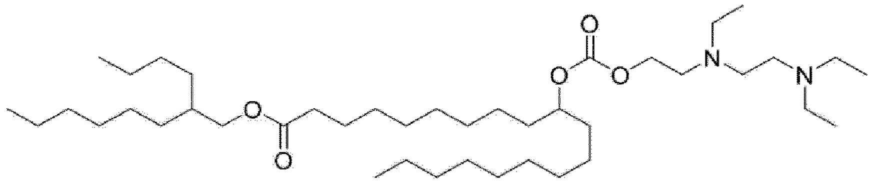

2-Butyloctyl 3,6-diethyl-12-nonyl-10-oxo-9,11-dioxa-3,6-diazahenicosan-21-oate as a colorless oily substance was obtained by the same method as that in Example 50, except that a 1.0 mol/L nonyl magnesium bromide-diethyl ether solution was used instead of a 1.0 mol/L dodecyl magnesium bromide-diethyl ether solution in Example 50.

$^1$H-NMR ($CDCl_3$) δ: 4.73-4.60 (1H, m), 4.18 (2H, t, J = 6.6 Hz), 3.97 (2H, d, J = 5.7 Hz), 2.76 (2H, t, J = 6.6 Hz), 2.66-2.47 (10H, m), 2.30 (2H, t, J = 7.8 Hz), 1.69-1.47 (7H, m), 1.41-1.19 (40H, m), 1.09-0.97 (9H, m), 0.94-0.83 (9H, m).
MS m/z (M + H): 698.

[Example 76]

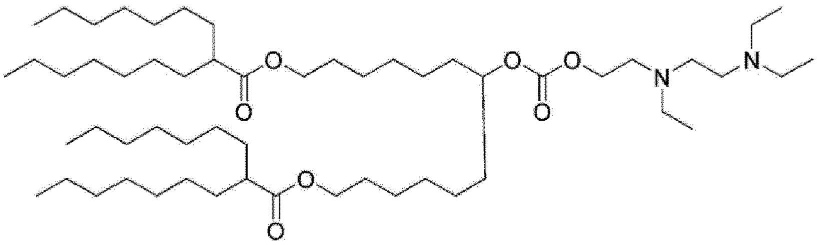

7-(((2-((2-(Diethylamino)ethyl)(ethyl)amino)ethoxy)carbonyl)oxy)tridecane-1,13-diyl bis(2-heptylnonanoate) as a colorless oily substance was obtained by the same method as that in (1) and (2) of Example 74, except that nonanoic acid and 1-iodoheptane were used instead of decanoic acid and 1-iodooctane in (1) and (2) of Example 74, respectively.

$^1$H-NMR ($CDCl_3$) δ: 4.73-4.61 (1H, m), 4.18 (2H, t, J = 6.6 Hz), 4.05 (4H, t, J = 6.6 Hz), 2.76 (2H, t, J = 6.6 Hz), 2.66-2.48 (10H, m), 2.37-2.23 (2H, m), 1.68-1.16 (68H, m), 1.08-0.97 (9H, m), 0.87 (12H, t, J = 6.6 Hz).
MS m/z (M + H): 924.

[Example 77]

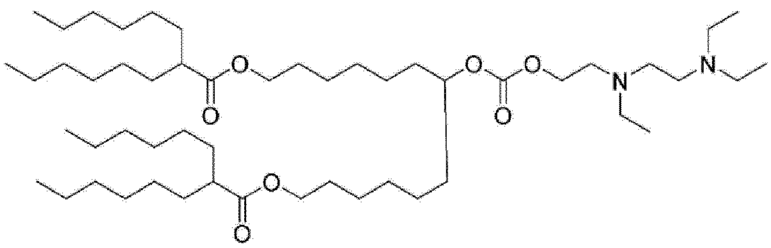

7-(((2-((2-(Diethylamino)ethyl)(ethyl)amino)ethoxy)carbonyl)oxy)tridecane-1,13-diyl bis(2-hexyloctanoate) as a colorless oily substance was obtained by the same method as that in (1) and (2) of Example 74, except that octanoic acid and 1-iodohexane were used instead of decanoic acid and 1-iodooctane in (1) and (2) of Example 74, respectively.

$^1$H-NMR ($CDCl_3$) δ: 4.73-4.60 (1H, m), 4.17 (2H, t, J = 6.6 Hz), 4.05 (4H, t, J = 6.6 Hz), 2.76 (2H, t, J = 6.6 Hz), 2.67-2.45 (10H, m), 2.37-2.24 (2H, m), 1.72-1.15 (60H, m), 1.12-0.96 (9H, m), 0.87 (12H, t, J = 6.6 Hz).
MS m/z (M + H): 868.

[Example 78]

(1)

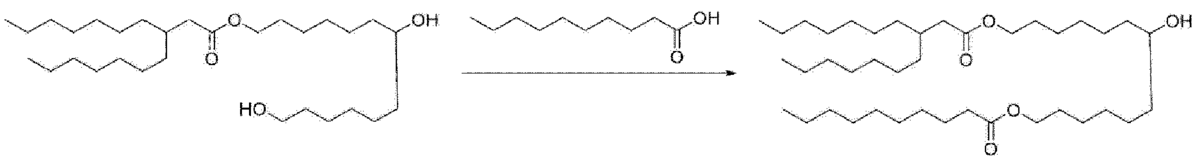

1-Ethyl-3-(3-dimethylaminopropyl)carbodiimide hydrochloride (126 mg) was added to a mixture of 7,13-dihydroxytridecyl 3-heptyldecanoate (500 mg) synthesized in (1) and (2) of Example 70, decanoic acid (195 mg), triethylamine (0.43 mL), 4-dimethylaminopyridine (38 mg), and dichloromethane (10 mL), and the mixture was stirred at room temperature for 18 hours. Water and ethyl acetate were added to the reaction mixture, the organic layer was separated, washed with a saturated aqueous sodium chloride solution, and then dried over anhydrous sodium sulfate, and the solvent was distilled away under reduced pressure. The obtained residue was purified by silica gel column chromatography (ethyl acetate-hexane), thereby obtaining 13-(decanoyloxy)-7-hydroxytridecyl 3-heptyldecanoate (469 mg) as a colorless oily substance.

$^1$H-NMR ($CDCl_3$) δ: 4.06 (4H, t, J = 6.6 Hz), 3.63-3.53 (1H, m), 2.29 (2H, t, J = 7.2 Hz), 2.22 (2H, d, J = 7.2 Hz), 1.88-1.78 (1H, m), 1.68-1.20 (60H, m), 0.88 (9H, t, J = 6.6 Hz).

(2)

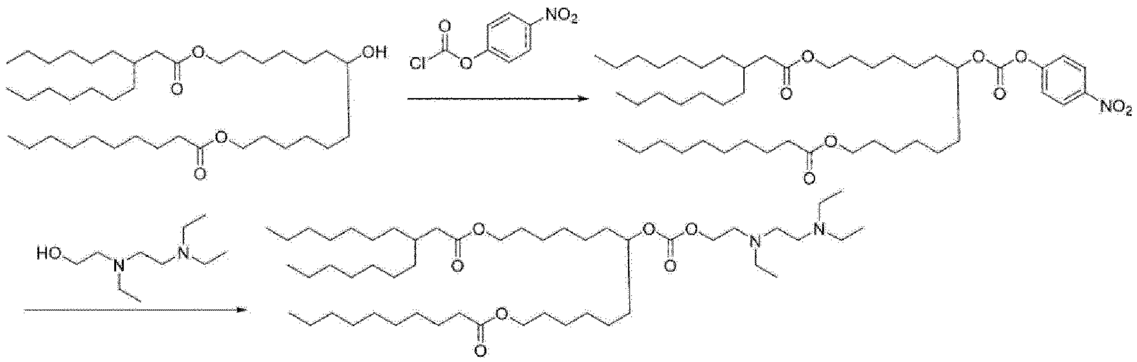

12-(6-(Decanoyloxy)hexyl)-3,6-diethyl-10-oxo-9,11-dioxa-3,6-diazooctadecan-18-yl 3-heptyldecanoate as a colorless oily substance was obtained by the same method as that in (1) and (2) of Example 20, except that 13-(decanoyloxy)-7-hydroxytridecyl 3-heptyldecanoate and 2-((2-(diethylamino)ethyl)(ethyl)amino)ethan-1-ol were used instead of (6Z,9Z,28Z,31Z)-heptatriaconta-6,9,28,31-tetraen-19-ol and 2-((2-(dimethylamino)ethyl)(methyl)amino)ethan-1-ol in (1) and (2) of Example 20, respectively.

$^1$H-NMR ($CDCl_3$) δ: 4.72-4.61 (1H, m), 4.17 (2H, t, J = 6.0 Hz), 4.04 (4H, t, J = 6.6 Hz), 2.76 (2H, t, J = 6.6 Hz), 2.67-2.45 (10H, m), 2.29 (2H, t, J = 8.1 Hz), 2.22 (2H, d, J = 7.2 Hz), 1.87-1.78 (1H, m), 1.70-1.18 (58H, m), 1.11-0.97 (9H, m), 0.93-0.82 (9H, m).
MS m/z (M + H): 854.

[Example 79]

(1)

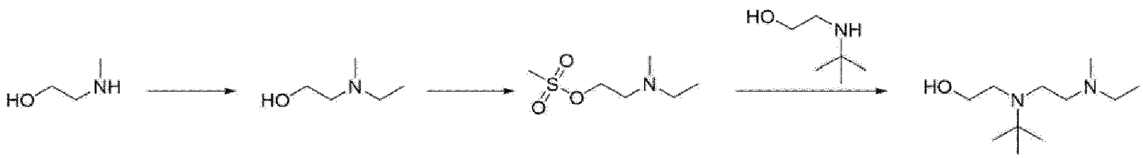

Ethyl iodide (3.4 mL) was added dropwise to an acetonitrile solution (30 mL) of 2-(methylamino)ethan-1-ol (3.0

g) under ice cooling, and the mixture was stirred at the same temperature for 1 hour and 45 minutes and then stirred at 60°C for 3 hours and 10 minutes. Potassium carbonate and water were added to the reaction mixture, and extraction was performed with chloroform. The organic layer was washed with saturated saline and dried over anhydrous sodium sulfate. The solvent was distilled away under reduced pressure, thereby obtaining 2-(ethyl(methyl)amino)ethan-1-ol (3.4 g) as a colorless oily substance.
MS m/z (M + H): 104.

A tetrahydrofuran solution (20 mL) of methanesulfonic anhydride (7.6 g) was added dropwise to a mixture of 2-(ethyl(methyl)amino)ethan-1-ol (3.0 g), 4-dimethylaminopyridine (0.36 g), N,N-diisopropylethylamine (9.9 mL), and tetrahydrofuran (60 mL) under ice cooling. The mixture was stirred at 0°C for 15 minutes and then stirred at room temperature for 3 hours and 45 minutes. 2-(tert-Butylamino)ethan-1-ol (6.0 g), sodium iodide (0.45 g), and water (1 mL) were added to the reaction mixture, and the reaction mixture was stirred at 75°C for 30 hours. The reaction mixture was cooled to room temperature, then the solvent was distilled away under reduced pressure, then water and a 2 mol/L aqueous sodium hydroxide solution were added thereto, and extraction was performed with ethyl acetate. The organic layer was washed with saturated saline and dried over anhydrous sodium sulfate. The solvent was distilled away under reduced pressure, and the obtained residue was purified by silica gel column chromatography (ethyl acetate-hexane, NH silica gel), thereby obtaining 2-(tert-butyl(2-(ethyl(methyl)amino)ethyl)amino)ethan-1-ol (0.15 g) as a yellow oily substance.
MS m/z (M + H): 203.

(2)

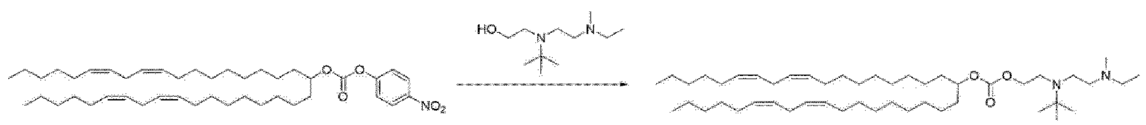

2-(tert-Butyl(2-(ethyl(methyl)amino)ethyl)amino)ethyl((6Z,9Z,28Z,31Z)-heptatriacon ta-6,9,28,31-tetraen-19-yl)carbonate as a colorless oily substance was obtained by the same method as that in (2) of Example 20, except that 2-(tert-butyl(2-(ethyl(methyl)amino)ethyl)amino)ethan-1-ol was used instead of 2-((2-(dimethylamino)ethyl)(methyl)amino)ethan-1-ol in (2) of Example 20.

$^1$H-NMR ($CDCl_3$) δ: 5.44-5.25 (8H, m), 4.73-4.62 (1H, m), 4.06 (2H, t, J = 7.5 Hz), 2.84-2.73 (6H, m), 2.72-2.59 (2H, m), 2.50-2.34 (4H, m), 2.25 (3H, s), 2.11-1.97 (8H, m), 1.65-1.48 (4H, m), 1.43-1.19 (36H, m), 1.12-1.01 (12H, m), 0.89 (6H, t, J = 6.6 Hz).
MS m/z (M + H): 758.

[Example 80]

(1)

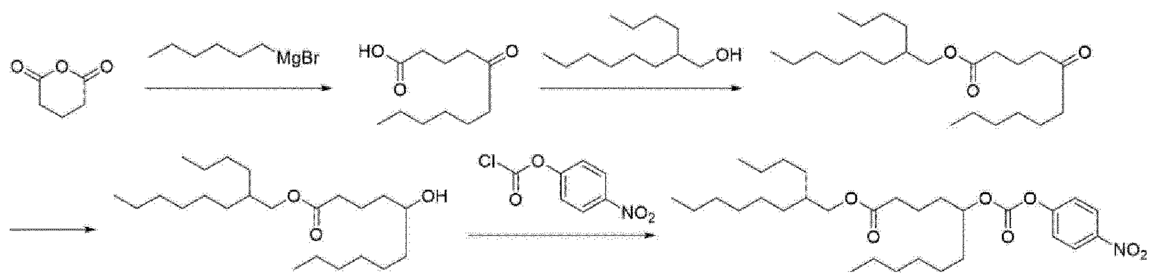

A 1 mol/L hexyl magnesium bromide-tetrahydrofuran solution (200 mL) was added dropwise to a tetrahydrofuran (273 mL) solution of glutaric anhydride (27.3 g) under ice cooling, and the mixture was stirred at the same temperature for 1 hour. A 2 mol/L aqueous hydrochloric acid solution (240 mL) was added to the reaction mixture under ice cooling, then ethyl acetate (270 mL) was added thereto, the organic layer was separated, washed with water and a saturated aqueous sodium chloride solution, and dried over anhydrous magnesium sulfate, and then the solvent was distilled away under reduced pressure. The obtained residue was purified by silica gel column chromatography (ethyl acetate-hexane),

then hexane (10 mL) was added thereto, and solids were collected by filtration, washed with hexane, and then dried under reduced pressure, thereby obtaining 5-oxoundecanoic acid (16.0 g) as white solids.

$^1$H-NMR ($CDCl_3$) δ: 2.50 (2H, t, J = 7.2 Hz), 2.40 (4H, t, J = 7.2 Hz), 2.02-1.80 (2H, m), 1.63-1.48 (2H, m), 1.37-1.20 (6H, m), 0.88 (3H, t, J = 6.6 Hz).

1-Ethyl-3-(3-dimethylaminopropyl)carbodiimide hydrochloride (5.8g) was added to a mixture of 5-oxoundecanoic acid (4.0 g), 2-butyloctan-1-ol (3.7 g), triethylamine (8.4 mL), 4-dimethylaminopyridine (1.22 g), and dichloromethane (40 mL), and the mixture was stirred at 40°C for 3 hours. Water and ethyl acetate were added to the reaction mixture, the organic layer was separated, washed with water, and then dried over anhydrous magnesium sulfate, and the solvent was distilled away under reduced pressure. The obtained residue was purified by silica gel column chromatography (ethyl acetate-hexane), thereby obtaining 2-butyloctyl 5-oxoundecanoate (7.3 g) as a colorless oily substance.

$^1$H-NMR ($CDCl_3$) δ: 3.97 (2H, d, J = 5.1 Hz), 2.47 (2H, t, J = 7.2 Hz), 2.39 (2H, t, J = 7.2 Hz), 2.33 (2H, t, J = 7.2 Hz), 1.95-1.83 (2H, m), 1.66-1.49 (3H, m), 1.36-1.20 (22H, m), 0.92-0.82 (9H, m).

Sodium borohydride (1.1 g) was added to a mixture of 2-butyloctyl 5-oxoundecanoate (7.3 g), tetrahydrofuran (35 mL), and methanol (35 mL) under ice cooling, and the mixture was stirred at the same temperature for 30 minutes. A 2.0 mol/L aqueous hydrochloric acid solution (35 mL) and hexane (35 mL) were added to the reaction mixture under ice cooling, the organic layer was separated, then washed with a saturated aqueous sodium chloride solution, and dried over anhydrous sodium sulfate, and then the solvent was distilled away under reduced pressure. The obtained residue was purified by silica gel column chromatography (ethyl acetate-hexane), thereby obtaining 2-butyloctyl 5-hydroxyundecanoate (6.3 g) as a colorless oily substance.

$^1$H-NMR ($CDCl_3$) δ: 3.97 (2H, d, J = 5.7 Hz), 3.65-3.53 (1H, m), 2.35 (2H, t, J = 7.2 Hz), 1.87-1.20 (32H, m), 0.92-0.84 (9H, m).

4-Nitrophenyl chloroformate (1.71 g) was added to a mixture of 2-butyloctyl 5-hydroxyundecanoate (1.62 g), triethylamine (2.38 mL), and tetrahydrofuran (16 mL), and the mixture was stirred at room temperature for 4 hours. Water and ethyl acetate were added to the reaction mixture, the organic layer was separated, washed with water, and then dried over anhydrous sodium sulfate, and the solvent was distilled away under reduced pressure. The obtained residue was purified by silica gel column chromatography (ethyl acetate-hexane), thereby obtaining 2-butyloctyl 5-(((4-nitrophenoxy)carbonyl)oxy)undecanoate (1.99 g) as a colorless oily substance.

$^1$H-NMR ($CDCl_3$) δ: 8.28 (2H, d, J = 9.3 Hz), 7.39 (2H, d, J = 9.3 Hz), 4.88-4.77 (1H, m), 3.99 (2H, d, J = 6.0 Hz), 2.41-2.31 (2H, m), 1.80-1.48 (7H, m), 1.44-1.20 (24H, m), 0.92-0.83 (9H, m).

(2)

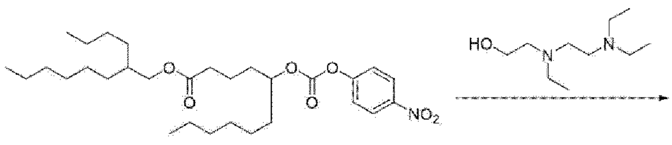

4-Dimethylaminopyridine (342 mg) was added to a mixture of 2-butyloctyl 5-(((4-nitrophenoxy)carbonyl)oxy)undecanoate (500 mg), 2-((2-(diethylamino)ethyl)(ethyl)amino)ethan-1-ol (527 mg), triethylamine (0.787 mL), and tetrahydrofuran (2.5 mL), and the mixture was stirred at 60°C for 10 hours. The reaction mixture was cooled to room temperature, water and ethyl acetate were added thereto, the organic layer was separated, washed with a saturated aqueous sodium chloride solution, and then dried over anhydrous sodium sulfate, and the solvent was distilled away under reduced pressure. The obtained residue was purified by silica gel column chromatography (methanol-ethyl acetate) and silica gel column chromatography (ethyl acetate-hexane, NH silica gel), thereby obtaining 2-butyloctyl 3,6-diethyl-12-hexyl-10-oxo-9,11-dioxa-3,6-diazahexadecan-16-oate (356 mg) as a colorless oily substance.

$^1$H-NMR ($CDCl_3$) δ: 4.73-4.64 (1H, m), 4.22-4.12 (2H, m), 3.97 (2H, d, J = 5.1 Hz), 2.76 (2H, t, J = 6.6 Hz), 2.64-2.49 (10H, m), 2.32 (2H, t, J = 6.6 Hz), 1.73-1.50 (7H, m), 1.36-1.20 (24H, m), 1.06-0.99 (9H, m), 0.92-0.84 (9H, m).
MS m/z (M + H): 586.

[Example 81]

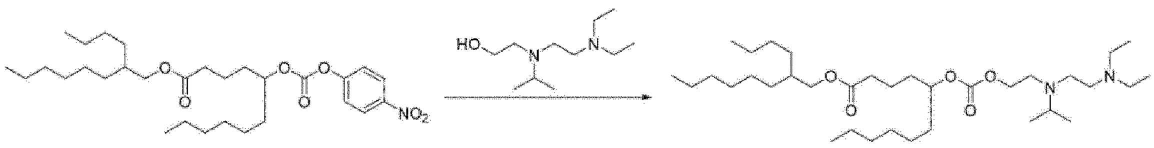

2-Butyloctyl

3-ethyl-12-hexyl-6-isopropyl-10-oxo-9,11-dioxa-3,6-diazahexadecan-16-oate as a colorless oily substance was obtained by the same method as that in (2) of Example 80, except that 2-((2-(diethylamino)ethyl)(isopropyl)amino)ethan-1-ol was used instead of 2-((2-(diethylamino)ethyl)(ethyl)amino)ethan-1-ol in (2) of Example 80.

$^1$H-NMR ($CDCl_3$) $\delta$: 4.73-4.64 (1H, m), 4.15-4.04 (2H, m), 3.97 (2H, d, J = 5.4 Hz), 2.97-2.83 (1H, m), 2.68 (2H, t, 6.6 Hz), 2.58-2.43 (8H, m), 2.32 (2H, t, J = 6.6 Hz), 1.73-1.50 (7H, m), 1.36-1.20 (24H, m), 1.06-0.96 (12H, m), 0.92-0.84 (9H, m).
MS m/z (M + H): 600.

[Example 82]

(1)

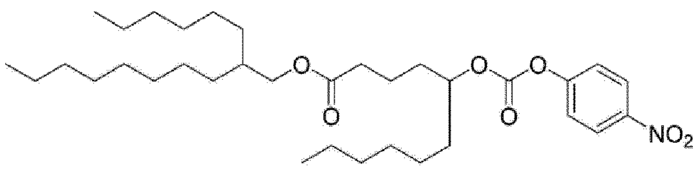

2-Hexyldecyl 5-(((4-nitrophenoxy)carbonyl)oxy)undecanoate as a colorless oily substance was obtained by the same method as that in (1) of Example 80, except that 2-hexyldecan-1-ol was used instead of 2-butyloctan-1-ol in (1) of Example 80.
$^1$H-NMR ($CDCl_3$) $\delta$: 8.27 (2H, dd, J = 6.6 Hz, 1.8 Hz), 7.38 (2H, dd, J = 6.6 Hz, 1.8 Hz), 4.88-4.78 (1H, m), 3.98 (2H, d, J = 6.0 Hz), 2.41-2.30 (2H, m), 1.79-1.53 (7H, m), 1.42-1.20 (32H, m), 0.92-0.83 (9H, m).

(2)

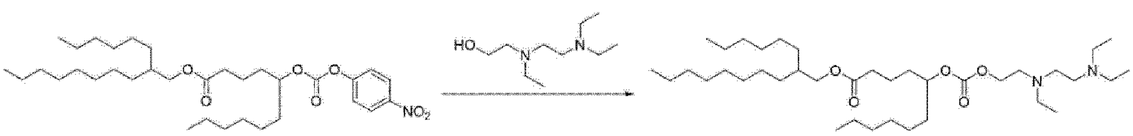

2-Hexyldecyl 3,6-diethyl-12-hexyl-10-oxo-9,11-dioxa-3,6-diazahexadecan-16-oate as a colorless oily substance was obtained by the same method as that in (2) of Example 80, except that 2-hexyldecyl 5-(((4-nitrophenoxy)carbonyl)oxy)undecanoate was used instead of 2-butyloctyl 5-(((4-nitrophenoxy)carbonyl)oxy)undecanoate in (2) of Example 80.

$^1$H-NMR ($CDCl_3$) $\delta$: 4.73-4.64 (1H, m), 4.23-4.12 (2H, m), 3.97 (2H, d, J = 5.7 Hz), 2.76 (2H, t, J = 6.6 Hz), 2.64-2.48 (10H, m), 2.32 (2H, t, J = 6.6 Hz), 1.75-1.50 (7H, m), 1.36-1.20 (32H, m), 1.06-0.99 (9H, m), 0.92-0.84 (9H, m).
MS m/z (M + H): 642.

[Example 83]

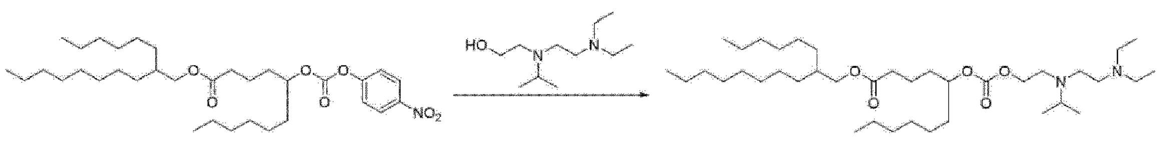

2-Hexyldecyl 3-ethyl-12-hexyl-6-isopropyl-10-oxo-9,11-dioxa-3,6-diazahexadecan-16-oate as a colorless oily substance was obtained by the same method as that in (2) of Example 82, except that 2-((2-(diethylamino)ethyl)(isopropyl)amino)ethan-1-ol was used instead of 2-((2-(diethylamino)ethyl)(ethyl)amino)ethan-1-ol in (2) of Example 82.

$^1$H-NMR ($CDCl_3$) δ: 4.73-4.64 (1H, m), 4.17-4.03 (2H, m), 3.97 (2H, d, J = 6.0 Hz), 2.97-2.84 (1H, m), 2.68 (2H, t, J = 6.6 Hz), 2.57-2.42 (8H, m), 2.32 (2H, t, J = 6.6 Hz), 1.73-1.50 (7H, m), 1.38-1.19 (32H, m), 1.06-0.96 (12H, m), 0.92-0.84 (9H, m).
MS m/z (M + H): 656.

[Example 84]

(1)

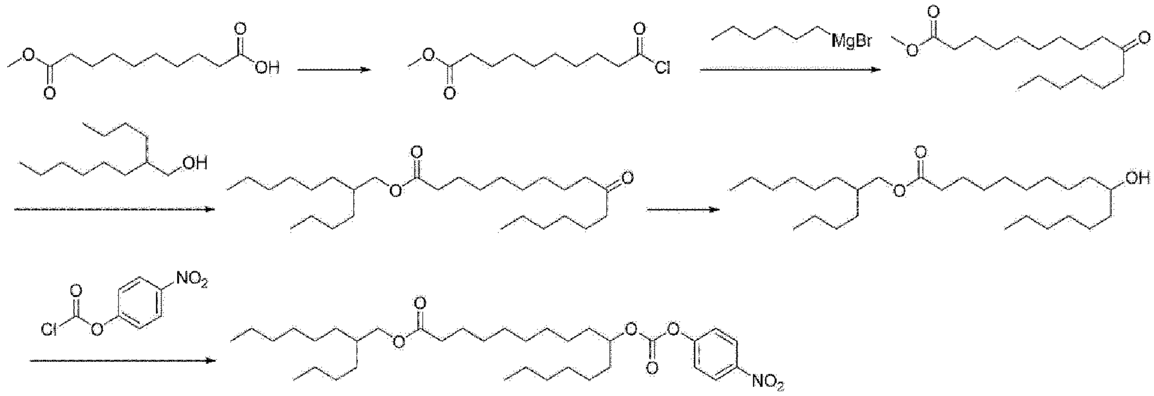

A mixture of 10-methoxy-10-oxodecanoic acid (47.6 g), thionyl chloride (47.6 mL), and N,N-dimethylformamide (0.1 mL) was stirred under reflux with heating for 1 hour. The solvent was distilled away under reduced pressure, thereby obtaining methyl 10-chloro-10-oxodecanoate (59.7 g) as a brown oily substance.

$^1$H-NMR ($CDCl_3$) δ: 3.67 (3H, s), 2.88 (2H, t, J = 7.2 Hz), 2.30 (2H, t, J = 7.2 Hz), 1.75-1.57 (4H, m), 1.38-1.25 (8H, m).

A 1.0 mol/L hexyl magnesium bromide-diethyl ether solution (440 mL) was added dropwise to a tetrahydrofuran (500 mL) suspension of zinc (II) chloride (30.0 g) at -78°C, and the mixture was heated to 0°C and then stirred at the same temperature for 30 minutes. Tetrakis(triphenylphosphine)palladium(0) (6.4g) was added to the reaction mixture under ice cooling, then methyl 10-chloro-10-oxodecanoate (59.7g) was added dropwise thereto at the same temperature, and the reaction mixture was stirred at the same temperature for 1 hour. A 1.0 mol/L aqueous hydrochloric acid solution (200 mL) and ethyl acetate (600 mL) were added to the reaction mixture, the organic layer was separated, washed with a saturated aqueous sodium chloride solution (560 mL), and then dried over anhydrous sodium sulfate, and the solvent was distilled away under reduced pressure. The obtained residue was purified by silica gel column chromatography (ethyl acetate-hexane), thereby obtaining methyl 10-oxohexadecanoate (50.6 g) as white solids.

$^1$H-NMR ($CDCl_3$) δ: 3.67 (3H, s), 2.38 (4H, t, J = 7.2 Hz), 2.30 (2H, t, 7.2 Hz), 1.65-1.49 (6H, m), 1.35-1.20 (14H, m), 0.88 (3H, t, J = 7.2 Hz).

Tetraisopropyl orthotitanate (1.5 g) was added to a mixture of methyl 10-oxohexadecanoate (15.0 g) and 2-butyloctan-1-ol (14.7 g), and the mixture was stirred at 110°C for 1 hour. Water (1 mL) was added to the reaction mixture, and the reaction mixture was stirred at room temperature for 15 minutes and then purified by silica gel column chromatography (ethyl acetate-hexane), thereby obtaining 2-butyloctyl 10-oxohexadecanoate (21.6 g) as a colorless oily substance.

$^1$H-NMR ($CDCl_3$) δ: 3.97 (2H, d, J = 5.6 Hz), 2.38 (4H, t, J = 7.6 Hz), 2.29 (2H, t, J = 7.6 Hz), 1.65-1.50 (7H, m), 1.35-1.20 (30H, m), 0.92-0.83 (9H, m).

Sodium borohydride (2.8 g) was added to a mixture of 2-butyloctyl 10-oxohexadecanoate (21.6 g), methanol (86 mL), and tetrahydrofuran (86 mL) under ice cooling, and the mixture was stirred at the same temperature for 30 minutes. The reaction mixture was poured into a mixture of ice (80 g) and water (80 g), a 1.0 mol/L aqueous hydrochloric acid solution (110 mL) and ethyl acetate (200 mL) were added thereto, the organic layer was separated, then washed with a saturated aqueous sodium chloride solution (200 mL), and dried over anhydrous sodium sulfate, and then the solvent was distilled away under reduced pressure. The obtained residue was purified by silica gel column chromatography (ethyl acetate-hexane), thereby obtaining 2-butyloctyl 10-hydroxyhexadecanoate (18.0 g) as a colorless oily substance.

$^1$H-NMR ($CDCl_3$) δ: 3.97 (2H, d, J = 6.0 Hz), 3.61-3.54 (1H, m), 2.30 (2H, t, J = 7.6 Hz), 1.65-1.56 (3H, m), 1.48-1.22 (38H, m), 0.92-0.83 (9H, m).

4-Nitrophenyl chloroformate (1.03 g) was added to a mixture of 2-butyloctyl 10-hydroxyhexadecanoate (1.50 g), triethylamine (1.43 mL), and tetrahydrofuran (15 mL), and the mixture was stirred at room temperature for 4 hours. Water and ethyl acetate were added to the reaction mixture, the organic layer was separated, washed with water, and then dried over anhydrous sodium sulfate, and the solvent was distilled away under reduced pressure. The obtained residue was purified by silica gel column chromatography (ethyl acetate-hexane), thereby obtaining 2-butyloctyl 10-(((4-nitrophenoxy)carbonyl)oxy)hexadecanoate (2.07 g) as a colorless oily substance.

$^1$H-NMR ($CDCl_3$) δ: 8.28 (2H, dd, J = 7.2 Hz, 2.1 Hz), 7.39 (2H, dd, J = 7.2 Hz, 2.1 Hz), 4.86-4.76 (1H, m), 3.97 (2H, d, J = 5.7 Hz), 2.30 (2H, t, J = 7.2 Hz), 1.74-1.20 (41H, m), 0.92-0.85 (9H, m).

(2)

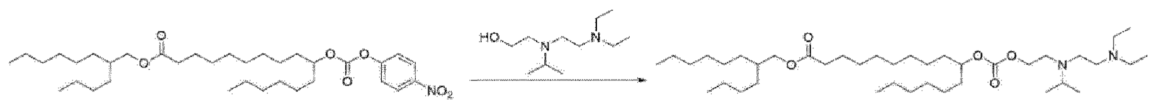

4-Dimethylaminopyridine (183 mg) was added to a mixture of 2-butyloctyl 10-(((4-nitrophenoxy)carbonyl)oxy)hexadecanoate (300 mg), 2-((2-(diethylamino)ethyl)(isopropyl)amino)ethan-1-ol (304 mg), triethylamine (0.211 mL), and tetrahydrofuran (6 mL), and the mixture was stirred at 80°C for 8 hours. The reaction mixture was cooled to room temperature, water and ethyl acetate were added thereto, the organic layer was separated, washed with a saturated aqueous sodium chloride solution, and then dried over anhydrous sodium sulfate, and the solvent was distilled away under reduced pressure. The obtained residue was purified by silica gel column chromatography (methanol-ethyl acetate) and silica gel column chromatography (ethyl acetate-hexane, NH silica gel), thereby obtaining 2-butyloctyl 3-ethyl-12-hexyl-6-isopropyl-10-oxo-9,11-dioxa-3,6-diazahenicosan-21-oate (296 mg) as a colorless oily substance.

$^1$H-NMR ($CDCl_3$) δ: 4.73-4.61 (1H, m), 4.10 (2H, t, J = 6.6 Hz), 3.97 (2H, d, J = 6.0 Hz), 2.97-2.85 (1H, m), 2.68 (2H, t, J = 7.2 Hz), 2.63-2.40 (8H, m), 2.29 (2H, t, J = 7.2 Hz), 1.68-1.47 (7H, m), 1.40-1.19 (34H, m), 1.10-0.96 (12H, m), 0.95-0.79 (9H, m).
MS m/z (M + H): 670.

[Example 85]

(1)

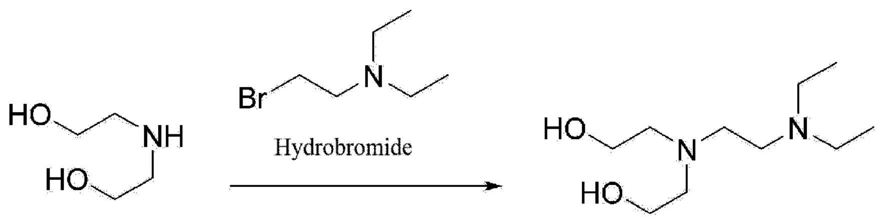


Potassium carbonate (7.9 g) was added to a mixture of 2,2'-azanediylbis(ethan-1-ol) (2.0 g), 2-bromo-N,N-diethylethan-1-amine hydrobromide (7.4 g), and ethanol (40 mL), and the mixture was stirred under reflux with heating for 8 hours. The reaction mixture was cooled to room temperature, the insoluble matters were filtered off, and the solvent was distilled away under reduced pressure. The obtained residue was purified by silica gel column chromatography

(ethyl acetate-hexane, NH silica gel), thereby obtaining 2,2'-((2-(diethylamino)ethyl)azanediyl)bis(ethan-1-ol) (2.3 g) as a light yellow oily substance.
MS m/z (M + H): 205.

(2)

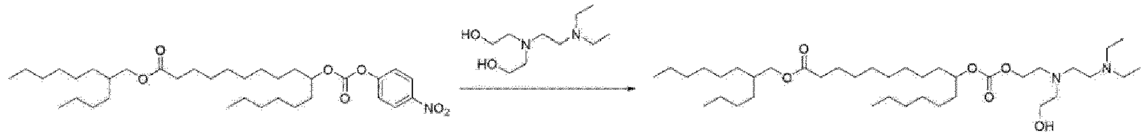

2-Butyloctyl

3-ethyl-12-hexyl-6-(2-hydroxyethyl)-10-oxo-9,11-dioxa-3,6-diazahenicosan-21-oate as a colorless oily substance was obtained by the same method as that in (2) of Example 84, except that 2,2'-((2-(diethylamino)ethyl)azanediyl)bis(ethan-1-ol) was used instead of 2-((2-(diethylamino)ethyl)(isopropyl)amino)ethan-1-ol in (2) of Example 84.

$^1$H-NMR ($CDCl_3$) $\delta$: 4.75-4.61 (1H, m), 4.21 (2H, t, J = 6.6 Hz), 3.97 (2H, d, J = 5.7 Hz), 3.55 (2H, t, J = 5.1 Hz), 2.89 (2H, t, J = 6.6 Hz), 2.76-2.65 (4H, m), 2.64-2.41 (6H, m), 2.30 (2H, t, J = 8.1 Hz), 1.72-1.45 (7H, m), 1.40-1.20 (34H, m), 1.13-0.98 (6H, m), 0.96-0.81 (9H, m).
MS m/z (M + H): 672.

[Example 86]

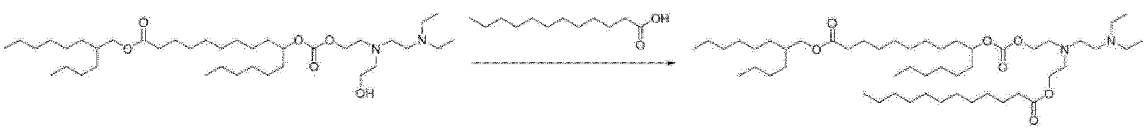

1-Ethyl-3-(3-dimethylaminopropyl)carbodiimide hydrochloride (142 mg) was added to a mixture of 2-butyloctyl 3-ethyl-12-hexyl-6-(2-hydroxyethyl)-10-oxo-9,11-dioxa-3,6-diazahenicosan-21-oate (250 mg) synthesized in (2) of Example 85, dodecanoic acid (112 mg), triethylamine (0.31 mL), 4-dimethylaminopyridine (136 mg), and dichloromethane (5 mL), and the mixture was stirred at room temperature for 6 hours. Water and ethyl acetate were added to the reaction mixture, the organic layer was separated, washed with water, and then dried over anhydrous magnesium sulfate, and the solvent was distilled away under reduced pressure. The obtained residue was purified by silica gel column chromatography (methanol-ethyl acetate) and silica gel column chromatography (ethyl acetate-hexane, NH silica gel), thereby obtaining 2-butyloctyl 6-(2-(dodecanoyloxy)ethyl)-3-ethyl-12-hexyl-10-oxo-9,11-dioxa-3,6-diazahenicosan-21-oate (177 mg) as a colorless oily substance.

$^1$H-NMR ($CDCl_3$) $\delta$: 4.72-4.60 (1H, m), 4.21-4.08 (4H, m), 3.97 (2H, d, J = 6.0 Hz), 2.88-2.75 (4H, m), 2.73-2.43 (8H, m), 2.29 (4H, t, J = 7.5 Hz), 1.70-1.46 (9H, m), 1.39-1.18 (50H, m), 1.12-0.97 (6H, m), 0.95-0.81 (12H, m).
MS m/z (M + H): 854.

[Example 87]

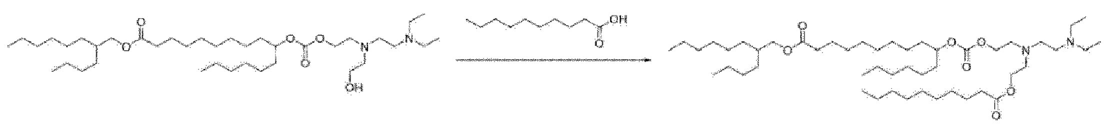

2-Butyloctyl

6-(2-(decanoyloxy)ethyl)-3-ethyl-12-hexyl-10-oxo-9,11-dioxa-3,6-diazahenicosan-21-oate as a colorless oily substance was obtained by the same method as that in Example 86, except that decanoic acid was used instead of dodecanoic acid in Example 86.

$^1$H-NMR ($CDCl_3$) $\delta$: 4.72-4.61 (1H, m), 4.22-4.07 (4H, m), 3.97 (2H, d, J = 6.0 Hz), 2.89-2.77 (4H, m), 2.74-2.43 (8H, m), 2.30 (4H, t, J = 8.1 Hz), 1.68-1.46 (9H, m), 1.40-1.18 (46H, m), 1.13-0.97 (6H, m), 0.95-0.80 (12H, m). MS m/z (M + H): 826.

[Example 88]

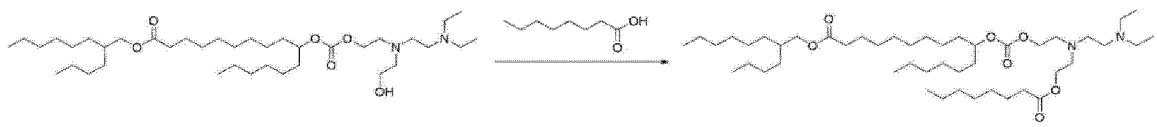

2-Butyloctyl

3-ethyl-12-hexyl-6-(2-(octanoyloxy)ethyl)-10-oxo-9,11-dioxa-3,6-diazahenicosan-21-oate as a colorless oily substance was obtained by the same method as that in Example 86, except that octanoic acid was used instead of dodecanoic acid in Example 86.

$^1$H-NMR ($CDCl_3$) $\delta$: 4.71-4.62 (1H, m), 4.20-4.08 (4H, m), 3.97 (2H, d, J = 5.6 Hz), 2.89-2.77 (4H, m), 2.73-2.42 (8H, m), 2.29 (4H, t, J = 7.6 Hz), 1.68-1.48 (9H, m), 1.39-1.18 (42H, m), 1.10-0.98 (6H, m), 0.94-0.81 (12H, m). MS m/z (M + H): 798.

[Example 89]

(1)

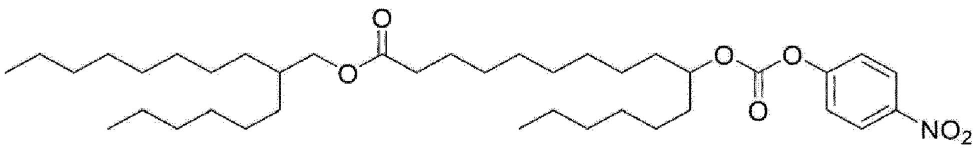

2-Hexyldecyl 10-(((4-nitrophenoxy)carbonyl)oxy)hexadecanoate as a colorless oily substance was obtained by the same method as that in (1) of Example 84, except that 2-hexyldecan-1-ol was used instead of 2-butyloctan-1-ol in (1) of Example 84.

$^1$H-NMR ($CDCl_3$) $\delta$: 8.28 (2H, dd, J = 7.2 Hz, 2.4 Hz), 7.39 (2H, dd, J = 7.2 Hz, 2.4 Hz), 4.85-4.77 (1H, m), 3.97 (2H, d, J = 5.6 Hz), 2.30 (2H, t, J = 7.6 Hz), 1.72-1.20 (49H, m), 0.92-0.85 (9H, m).

(2)

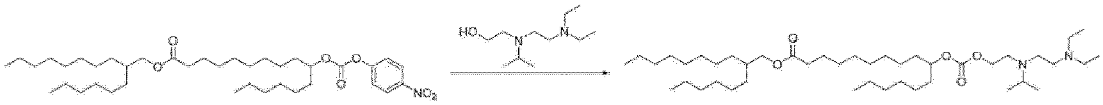

2-Hexyldecyl

3-ethyl-12-hexyl-6-isopropyl-10-oxo-9,11-dioxa-3,6-diazahenicosan-21-oate as a colorless oily substance was obtained by the same method as that in (2) of Example 84, except that 2-hexyldecyl 10-(((4-nitrophenoxy)carbonyl)oxy)hexadecanoate was used instead of 2-butyloctyl 10-(((4-nitrophenoxy)carbonyl)oxy)hexadecanoate in (2) of Ex-

ample 84.

$^1$H-NMR ($CDCl_3$) δ: 4.72-4.61 (1H, m), 4.10 (2H, t, J = 6.6 Hz), 3.97 (2H, d, J = 5.7 Hz), 2.97-2.87 (1H, m), 2.68 (2H, t, J = 6.6 Hz), 2.62-2.40 (8H, m), 2.29 (2H, t, J = 7.2 Hz), 1.69-1.49 (7H, m), 1.40-1.19 (42H, m), 1.12-0.95 (12H, m), 0.93-0.82 (9H, m).
MS m/z (M + H): 726.

[Example 90]

(1)

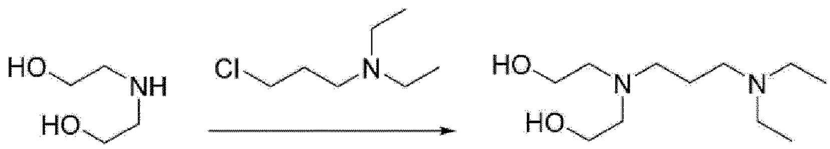

2,2'-((3-(Diethylamino)propyl)azanediyl)bis(ethan-1-ol) as a colorless oily substance was obtained by the same method as that in (1) of Example 85, except that 3-chloro-N,N-diethylpropan-1-amine was used instead of 2-bromo-N,N-diethylethan-1-amine hydrobromide in (1) of Example 85.
MS m/z (M + H): 219.

(2)

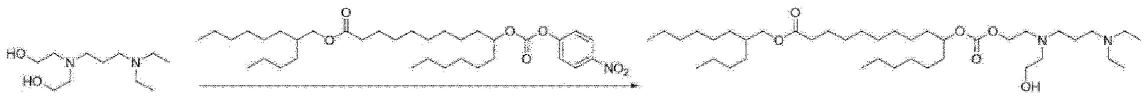

2-Butyloctyl

3-ethyl-13-hexyl-7-(2-hydroxyethyl)-11-oxo-10,12-dioxa-3,7-diazadocosan-22-oate as a colorless oily substance was obtained by the same method as that in (2) of Example 85, except that 2,2'-((3-(diethylamino)propyl)azanediyl)bis(ethan-1-ol) was used instead of 2,2'-((2-(diethylamino)ethyl)azanediyl)bis(ethan-1-ol) in (2) of Example 85.

$^1$H-NMR ($CDCl_3$) δ: 4.73-4.61 (1H, m), 4.17 (2H, t, J = 6.0 Hz), 3.97 (2H, d, J = 6.0 Hz), 3.58 (2H, t, J = 5.4 Hz), 2.76 (2H, t, J = 5.7 Hz), 2.67-2.40 (10H, m), 2.30 (2H, t, J = 8.1 Hz), 1.76-1.46 (9H, m), 1.38-1.19 (34H, m), 1.12-0.98 (6H, m), 0.94-0.82 (9H, m).
MS m/z (M + H): 686.

[Example 91]

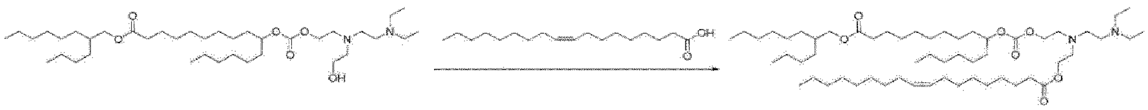

2-Butyloctyl

3-ethyl-12-hexyl-6-(2-(oleoyloxy)ethyl)-10-oxo-9,11-dioxa-3,6-diazahenicosan-21-oate as a colorless oily substance was obtained by the same method as that in Example 86, except that oleic acid was used instead of dodecanoic acid in Example 86.

$^1$H-NMR ($CDCl_3$) δ: 5.38-5.28 (2H, m), 4.72-4.63 (1H, m), 4.21-4.06 (4H, m), 3.97 (2H, d, J = 6.0 Hz), 2.90-2.76 (4H, m), 2.74-2.44 (8H, m), 2.29 (4H, t, J = 7.8 Hz), 2.07-1.93 (4H, m), 1.68-1.45 (9H, m), 1.38-1.17 (54H, m),

1.11-0.96 (6H, m), 0.94-0.81 (12H, m).
MS m/z (M + H): 936.

[Example 92]

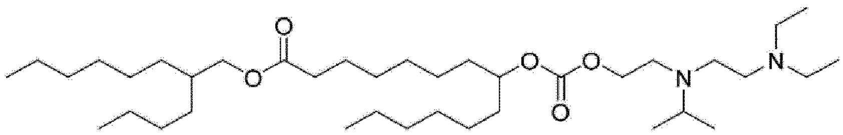

2-Butyloctyl

3-ethyl-12-hexyl-6-isopropyl-10-oxo-9,11-dioxa-3,6-diazanonadecan-19-oate as a colorless oily substance was obtained by the same method as that in (1) and (2) of Examples 84, except that 8-methoxy-8-oxooctanoic acid was used instead of 10-methoxy-10-oxodecanoic acid in (1) and (2) of Example 84.

$^1$H-NMR ($CDCl_3$) δ: 4.72-4.59 (1H, m), 4.17-4.04 (2H, m), 3.97 (2H, d, J = 5.4 Hz), 2.97-2.84 (1H, m), 2.69 (2H, t, J = 6.6 Hz), 2.64-2.42 (8H, m), 2.29 (2H, t, J = 7.2 Hz), 1.68-1.46 (7H, m), 1.40-1.18 (30H, m), 1.14-0.94 (12H, m), 0.93-0.82 (9H, m).
MS m/z (M + H): 642.

[Example 93]

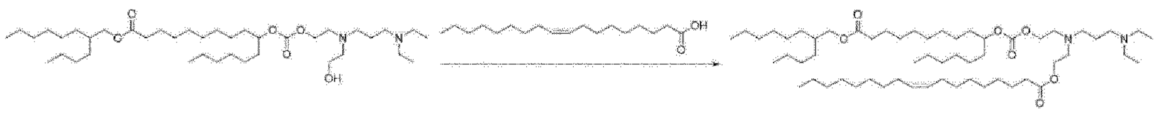

2-Butyloctyl

3-ethyl-13-hexyl-7-(2-(oleoyloxy)ethyl)-11-oxo-10,12-dioxa-3,7-diazadocosan-22-oate as a colorless oily substance was obtained by the same method as that in Example 86, except that 2-butyloctyl

3-ethyl-13-hexyl-7-(2-hydroxyethyl)-11-oxo-10,12-dioxa-3,7-diazadocosan-22-oate and oleic acid were used instead of 2-butyloctyl 3-ethyl-12-hexyl-6-(2-hydroxyethyl)-10-oxo-9,11-dioxa-3,6-diazahenicosan-21-oate and dodecanoic acid in Example 86, respectively.

$^1$H-NMR ($CDCl_3$) δ: 5.42-5.27 (2H, m), 4.72-4.59 (1H, m), 4.21-4.07 (4H, m), 3.97 (2H, d, J = 6.0 Hz), 2.86-2.71 (4H, m), 2.65-2.35 (8H, m), 2.29 (4H, t, J = 7.2H), 2.07-1.94 (4H, m), 1.70-1.48 (11H, m), 1.41-1.19 (54H, m), 1.11-0.97 (6H, m), 0.96-0.82 (12H, m).
MS m/z (M + H): 950.

[Example 94]

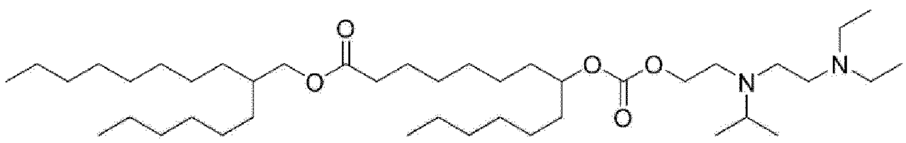

2-Hexyldecyl

3-ethyl-12-hexyl-6-isopropyl-10-oxo-9,11-dioxa-3,6-diazanonadecan-19-oate as a colorless oily substance was obtained by the same method as that in Example 92, except that 2-hexyldecan-1-ol was used instead of 2-butyloctan-1-ol in Example 92.

$^1$H-NMR ($CDCl_3$) δ: 4.71-4.62 (1H, m), 4.16-4.04 (2H, m), 3.96 (2H, d, J = 6.0 Hz), 2.97-2.85 (1H, m), 2.68 (2H, t, J = 6.6 Hz), 2.64-2.41 (8H, m), 2.29 (2H, t, J = 7.5 Hz), 1.70-1.47 (7H, m), 1.41-1.19 (38H, m), 1.11-0.95 (12H, m), 0.93-0.83 (9H, m).
MS m/z (M + H): 698.

[Example 95]

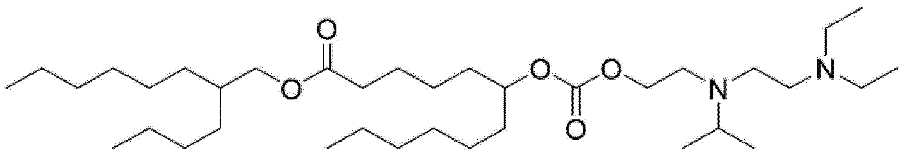

2-Butyloctyl

3-ethyl-12-hexyl-6-isopropyl-10-oxo-9,11-dioxa-3,6-diazaheptadecan-17-oate as a colorless oily substance was obtained by the same method as that in (1) and (2) of Examples 84, except that 6-methoxy-6-oxohexanoic acid was used instead of 10-methoxy-10-oxodecanoic acid in (1) and (2) of Example 84.

$^1$H-NMR ($CDCl_3$) δ: 4.73-4.61 (1H, m), 4.16-4.04 (2H, m), 3.96 (2H, d, J = 5.7 Hz), 2.97-2.85 (1H, m), 2.68 (2H, t, J = 6.6 Hz), 2.63-2.42 (8H, m), 2.30 (2H, t, J = 8.1 Hz), 1.69-1.49 (7H, m), 1.44-1.20 (26H, m), 1.12-0.95 (12H, m), 0.94-0.82 (9H, m).
MS m/z (M + H): 614.

[Example 96]

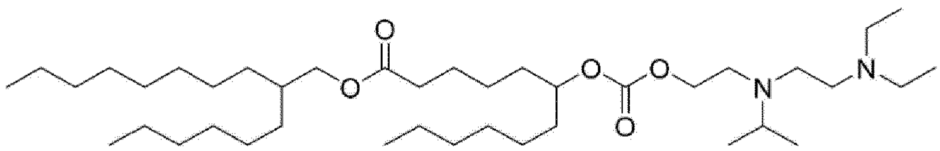

2-Hexyldecyl

3-ethyl-12-hexyl-6-isopropyl-10-oxo-9,11-dioxa-3,6-diazaheptadecan-17-oate as a colorless oily substance was obtained by the same method as that in Example 95, except that 2-hexyldecan-1-ol was used instead of 2-butyloctan-1-ol in Example 95.

$^1$H-NMR ($CDCl_3$) δ: 4.73-4.62 (1H, m), 4.17-4.04 (2H, m), 3.96 (2H, d, J = 5.7 Hz), 2.98-2.83 (1H, m), 2.68 (2H, t, J = 6.6 Hz), 2.62-2.41 (8H, m), 2.30 (2H, t, J = 7.8 Hz), 1.69-1.49 (7H, m), 1.42-1.18 (34H, m), 1.12-0.96 (12H, m), 0.93-0.81 (9H, m).
MS m/z (M + H): 670.

[Example 97]

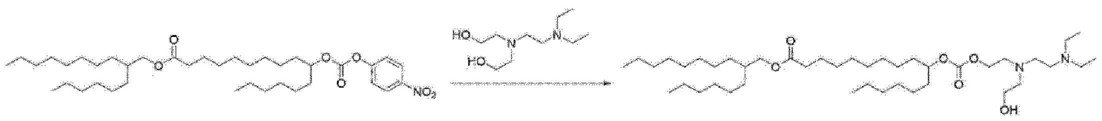

2-Hexyldecyl

3-ethyl-12-hexyl-6-(2-hydroxyethyl)-10-oxo-9,11-dioxa-3,6-diazahenicosan-21-oate as a colorless oily substance was obtained by the same method as that in Example 85, except that 2-hexyldecyl 10-(((4-nitrophenoxy)carbonyl)oxy)hexadecanoate was used instead of 2-butyloctyl 10-(((4-nitrophenoxy)carbonyl)oxy)hexadecanoate in Example 85.

$^1$H-NMR ($CDCl_3$) δ: 4.73-4.61 (1H, m), 4.20 (2H, t, J = 6.6 Hz), 3.96 (2H, d, J = 5.4 Hz), 3.54 (2H, t, J = 5.4 Hz), 2.89 (2H, t, J = 6.0 Hz), 2.76-2.63 (4H, m), 2.62-2.42 (6H, m), 2.29 (2H, t, J = 7.5 Hz), 1.72-1.46 (7H, m), 1.39-1.18 (42H, m), 1.04 (6H, t, J = 7.2 Hz), 0.94-0.80 (9H, m).
MS m/z (M + H): 728.

[Example 98]

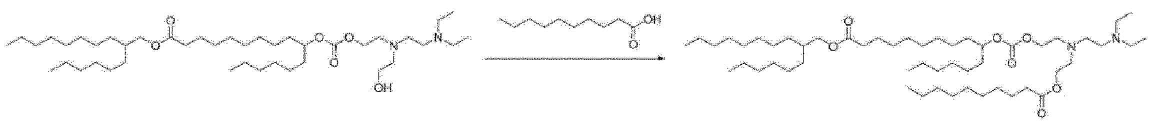

2-Hexyldecyl

6-(2-(decanoyloxy)ethyl)-3-ethyl-12-hexyl-10-oxo-9,11-dioxa-3,6-diazahenicosan-21-oate as a colorless oily substance was obtained by the same method as that in Example 86, except that 2-hexyldecyl

3-ethyl-12-hexyl-6-(2-hydroxyethyl)-10-oxo-9,11-dioxa-3,6-diazahenicosan-21-oate and decanoic acid were used instead of 2-butyloctyl 3-ethyl-12-hexyl-6-(2-hydroxyethyl)-10-oxo-9,11-dioxa-3,6-diazahenicosan-21-oate and dodecanoic acid in Example 86, respectively.

$^1$H-NMR ($CDCl_3$) δ: 4.72-4.63 (1H, m), 4.22-4.08 (4H, m), 3.96 (2H, d, J = 5.4 Hz), 2.88-2.76 (4H, m), 2.75-2.43 (8H, m), 2.29 (4H, t, J = 7.2 Hz), 1.68-1.50 (9H, m), 1.39-1.16 (54H, m), 1.03 (6H, t, J = 6.6 Hz), 0.95-0.82 (12H, m).
MS m/z (M + H): 882.

[Example 99]

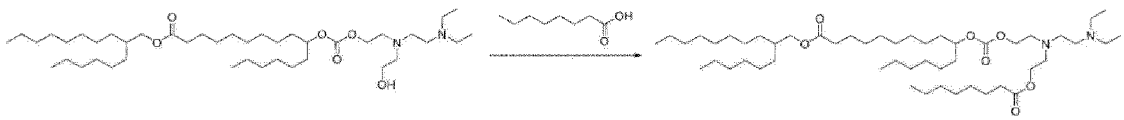

2-Hexyldecyl

3-ethyl-12-hexyl-6-(2-(octanoyloxy)ethyl)-10-oxo-9,11-dioxa-3,6-diazahenicosan-21-oate as a colorless oily substance was obtained by the same method as that in Example 98, except that octanoic acid was used instead of decanoic acid in Example 98.

$^1$H-NMR ($CDCl_3$) δ: 4.72-4.61 (1H, m), 4.21-4.07 (4H, m), 3.96 (2H, d, J = 5.1 Hz), 2.90-2.76 (4H, m), 2.76-2.42 (8H, m), 2.29 (4H, t, J = 7.8 Hz), 1.68-1.47 (9H, m), 1.39-1.19 (50H, m), 1.12-0.96 (6H, m), 0.95-0.82 (12H, m).
MS m/z (M + H): 854.

[Example 100]

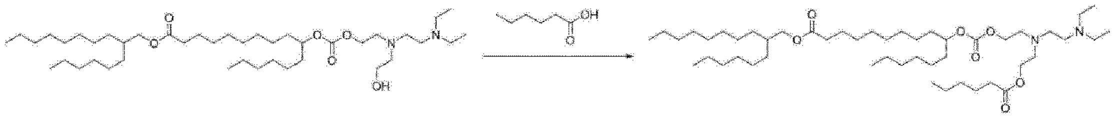

2-Hexyldecyl

3-ethyl-6-(2-(hexanoyloxy)ethyl)-12-hexyl-10-oxo-9,11-dioxa-3,6-diazahenicosan-21-oate as a colorless oily substance was obtained by the same method as that in Example 98, except that hexanoic acid was used instead of decanoic acid in Example 98.

$^1$H-NMR ($CDCl_3$) $\delta$: 4.72-4.61 (1H, m), 4.21-4.07 (4H, m), 3.96 (2H, d, J = 5.7 Hz), 2.90-2.77 (4H, m), 2.73-2.41 (8H, m), 2.29 (4H, t, J = 7.2 Hz), 1.70-1.46 (9H, m), 1.42-1.18 (46H, m), 1.13-0.97 (6H, m), 0.95-0.81 (12H, m).
MS m/z (M + H): 826.

[Example 101]

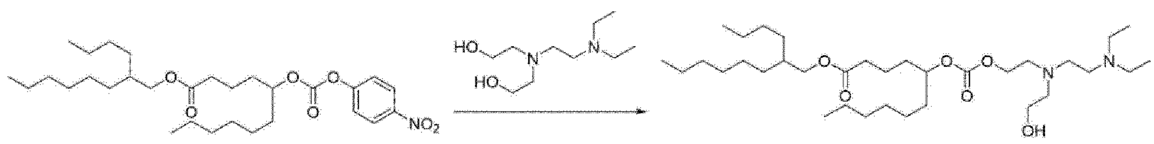

2-Butyloctyl

3-ethyl-12-hexyl-6-(2-hydroxyethyl)-10-oxo-9,11-dioxa-3,6-diazahexadecan-16-oate as a colorless oily substance was obtained by the same method as that in (2) of Example 80, except that 2,2'-((2-(diethylamino)ethyl)azanediyl)bis(ethan-1-ol) was used instead of 2-((2-(diethylamino)ethyl)(ethyl)amino)ethan-1-ol in (2) of Example 80.

$^1$H-NMR ($CDCl_3$) $\delta$: 4.75-4.64 (1H, m), 4.25-4.15 (2H, m), 3.97 (2H, d, J = 6.0 Hz), 3.54 (2H, t, J = 5.4 Hz), 2.89 (2H, t, J = 6.6 Hz), 2.75-2.63 (4H, m), 2.60-2.42 (6H, m), 2.33 (2H, t, J = 6.6 Hz), 1.73-1.50 (7H, m), 1.39-1.20 (24H, m), 1.03 (6H, t, J = 7.2 Hz), 0.95-0.81 (9H, m).
MS m/z (M + H): 602.

[Example 102]

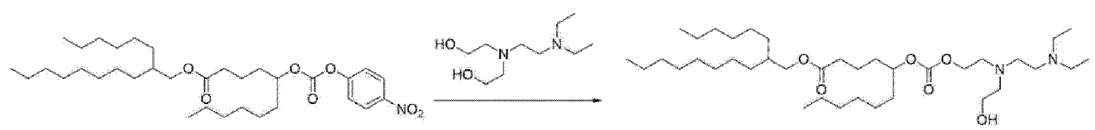

2-Hexyldecyl

3-ethyl-12-hexyl-6-(2-hydroxyethyl)-10-oxo-9,11-dioxa-3,6-diazahexadecan-16-oate as a colorless oily substance was obtained by the same method as that in (2) of Example 85, except that 2-hexyldecyl 5-(((4-nitrophenoxy)carbonyl)oxy)undecanoate was used instead of 2-butyloctyl 10-(((4-nitrophenoxy)carbonyl)oxy)hexadecanoate in (2) of Example 85.

$^1$H-NMR ($CDCl_3$) $\delta$: 4.75-4.63 (1H, m) 4.25-4.14 (2H, m), 3.97 (2H, d, J = 6.0 Hz), 3.54 (2H, t, J = 4.8 Hz), 2.89 (2H, t, J = 6.0 Hz), 2.76-2.63 (4H, m), 2.60-2.43 (6H, m), 2.33 (2H, t, J = 7.5 Hz), 1.73-1.48 (7H, m), 1.40-1.17 (32H, m),

1.03 (6H, t, J = 7.2 Hz), 0.96-0.78 (9H, m).
MS m/z (M + H): 658.

[Example 103]

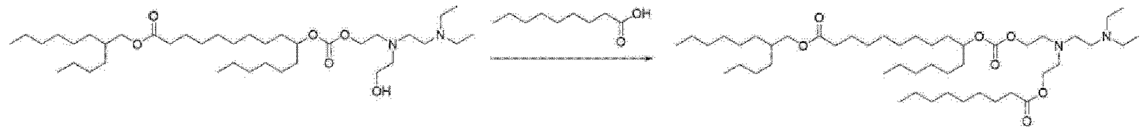

2-Butyloctyl

3-ethyl-12-hexyl-6-(2-(nonanoyloxy)ethyl)-10-oxo-9,11-dioxa-3,6-diazahenicosan-21-oate as a colorless oily substance was obtained by the same method as that in Example 86, except that nonanoic acid was used instead of dodecanoic acid in Example 86.

$^1$H-NMR ($CDCl_3$) δ: 4.72-4.61 (1H, m), 4.21-4.09 (4H, m), 3.97 (2H, d, J = 6.0 Hz), 2.88-2.47 (4H, m), 2.72-2.62 (2H, m), 2.58-2.46 (6H, m), 2.29 (4H, t, J = 7.8 Hz), 1.69-1.50 (9H, m), 1.40-1.19 (44H, m), 1.01 (6H, t, J = 7.2 Hz), 0.95-0.82 (12H, m).
MS m/z (M + H): 812.

[Example 104]

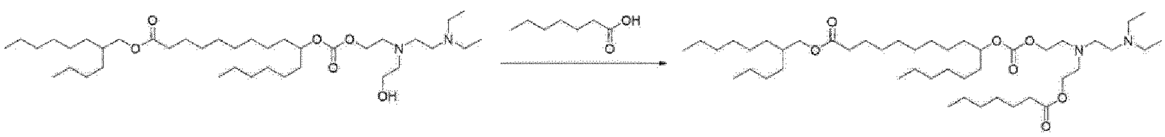

2-Butyloctyl

3-ethyl-6-(2-(heptanoyloxy)ethyl)-12-hexyl-10-oxo-9,11-dioxa-3,6-diazahenicosan-21-oate as a colorless oily substance was obtained by the same method as that in Example 86, except that heptanoic acid was used instead of dodecanoic acid in Example 86.

$^1$H-NMR ($CDCl_3$) δ: 4.72-4.60 (1H, m), 4.20-4.06 (4H, m), 3.97 (2H, d, J = 5.1 Hz), 2.89-2.76 (4H, m), 2.71-2.62 (2H, m), 2.58-2.46 (6H, m), 2.30 (4H, t, J = 8.1 Hz), 1.68-1.47 (9H, m), 1.39-1.19 (40H, m), 1.02 (6H, t, J = 6.6 Hz), 0.95-0.83 (12H, m).
MS m/z (M + H): 784.

[Example 105]

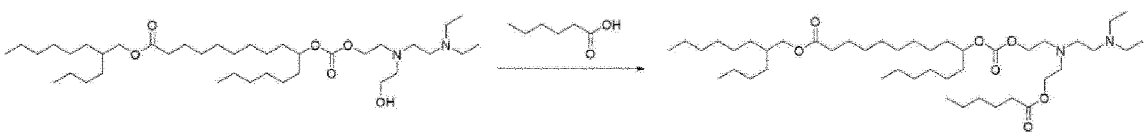

2-Butyloctyl

3-ethyl-6-(2-(hexanoyloxy)ethyl)-12-hexyl-10-oxo-9,11-dioxa-3,6-diazahenicosan-21-oate as a colorless oily

substance was obtained by the same method as that in Example 86, except that hexanoic acid was used instead of dodecanoic acid in Example 86.

$^1$H-NMR ($CDCl_3$) δ: 4.73-4.61 (1H, m), 4.21-4.08 (4H, m), 3.97 (2H, d, J = 5.4 Hz), 2.89-2.76 (4H, m), 2.72-2.62 (2H, m), 2.59-2.45 (6H, m), 2.30 (4H, t, J = 8.1 Hz), 1.71-1.47 (9H, m), 1.40-1.19 (38H, m), 1.02 (6H, t, J = 6.6 Hz), 0.94-0.82 (12H, m).
MS m/z (M + H): 770.

[Example 106]

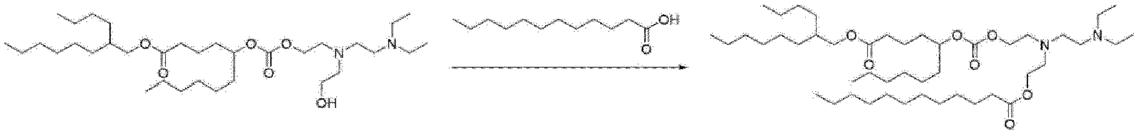

2-Butyloctyl

6-(2-(dodecanoyloxy)ethyl)-3-ethyl-12-hexyl-10-oxo-9,11-dioxa-3,6-diazahexadecan-16-oate as a colorless oily substance was obtained by the same method as that in Example 86, except that 2-butyloctyl 3-ethyl-12-hexyl-6-(2-hydroxyethyl)-10-oxo-9,11-dioxa-3,6-diazahexadecan-16-oate was used instead of 2-butyloctyl 3-ethyl-12-hexyl-6-(2-hydroxyethyl)-10-oxo-9,11-dioxa-3,6-diazahenicosan-21-oate in Example 86.

$^1$H-NMR ($CDCl_3$) δ: 4.74-4.64 (1H, m), 4.21-4.07 (4H, m), 3.97 (2H, d, J = 6.0 Hz), 2.89-2.76 (4H, m), 2.72-2.63 (2H, m), 2.58-2.46 (6H, m), 2.37-2.25 (4H, m), 1.74-1.50 (9H, m), 1.39-1.19 (40H, m), 1.02 (6H, t, J = 6.6 Hz), 0.95-0.83 (12H, m).
MS m/z (M + H): 784.

[Example 107]

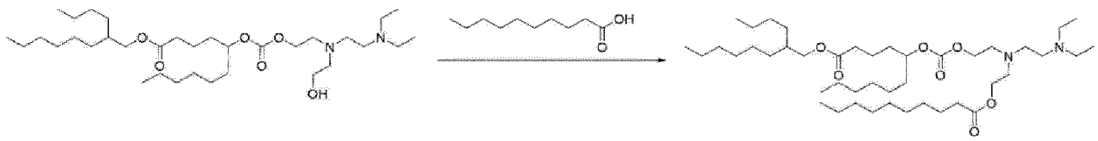

2-Butyloctyl

6-(2-(decanoyloxy)ethyl)-3-ethyl-12-hexyl-10-oxo-9,11-dioxa-3,6-diazahexadecan-16-oate as a colorless oily substance was obtained by the same method as that in Example 106, except that decanoic acid was used instead of dodecanoic acid in Example 106.

$^1$H-NMR ($CDCl_3$) δ: 4.74-4.64 (1H, m), 4.22-4.07 (4H, m), 3.97 (2H, d, J = 6.0 Hz), 2.88-2.75 (4H, m), 2.72-2.62 (2H, m), 2.58-2.46 (6H, m), 2.37-2.25 (4H, m), 1.74-1.52 (9H, m), 1.40-1.19 (36H, m), 1.02 (6H, t, J = 7.2 Hz), 0.94-0.82 (12H, m).
MS m/z (M + H): 756.

[Example 108]

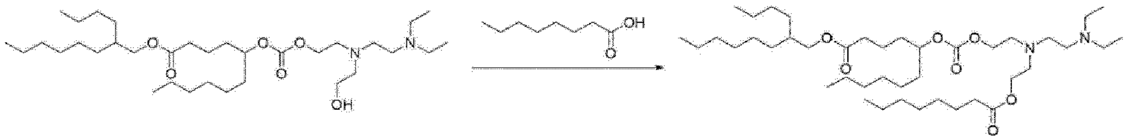

2-Butyloctyl

3-ethyl-12-hexyl-6-(2-(octanoyloxy)ethyl)-10-oxo-9,11-dioxa-3,6-diazahexadecan-16-oate as a colorless oily substance was obtained by the same method as that in Example 106, except that octanoic acid was used instead of dodecanoic acid in Example 106.

$^1$H-NMR ($CDCl_3$) $\delta$: 4.74-4.64 (1H, m), 4.21-4.08 (4H, m), 3.97 (2H, d, J = 6.0 Hz), 2.88-2.76 (4H, m), 2.72-2.62 (2H, m), 2.58-2.46 (6H, m), 2.37-2.27 (4H, m), 1.74-1.50 (9H, m), 1.40-1.19 (32H, m), 1.02 (6H, t, J = 7.2 Hz), 0.95-0.83 (12H, m).
MS m/z (M + H): 728.

[Example 109]

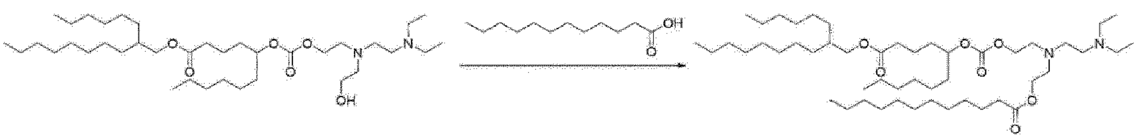

2-Hexyldecyl

6-(2-(dodecanoyloxy)ethyl)-3-ethyl-12-hexyl-10-oxo-9,11-dioxa-3,6-diazahexadecan-16-oate as a colorless oily substance was obtained by the same method as that in Example 86, except that 2-hexyldecyl 3-ethyl-12-hexyl-6-(2-hydroxyethyl)-10-oxo-9,11-dioxa-3,6-diazahexadecan-16-oate was used instead of 2-butyloctyl 3-ethyl-12-hexyl-6-(2-hydroxyethyl)-10-oxo-9,11-dioxa-3,6-diazahenicosan-21-oate in Example 86.

$^1$H-NMR ($CDCl_3$) $\delta$: 4.74-4.64 (1H, m), 4.21-4.06 (4H, m), 3.97 (2H, d, J = 5.4 Hz), 2.88-2.76 (4H, m), 2.71-2.63 (2H, m), 2.57-2.46 (6H, m), 2.36-2.25 (4H, m), 1.72-1.52 (9H, m), 1.39-1.20 (48H, m), 1.02 (6H, t, J = 7.5 Hz), 0.95-0.81 (12H, m).
MS m/z (M + H): 840.

[Example 110]

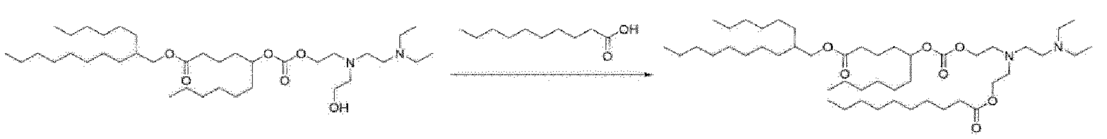

2-Hexyldecyl

6-(2-(decanoyloxy)ethyl)-3-ethyl-12-hexyl-10-oxo-9,11-dioxa-3,6-diazahexadecan-16-oate as a colorless oily substance was obtained by the same method as that in Example 109, except that decanoic acid was used instead of dodecanoic acid in Example 109.

$^1$H-NMR ($CDCl_3$) $\delta$: 4.75-4.63 (1H, m), 4.21-4.07 (4H, m), 3.97 (2H, d, J = 5.7 Hz), 2.88-2.76 (4H, m), 2.71-2.62 (2H, m), 2.58-2.45 (6H, m), 2.36-2.26 (4H, m), 1.73-1.52 (9H, m), 1.38-1.19 (44H, m), 1.02 (6H, t, J = 7.2 Hz), 0.95-0.81 (12H, m).
MS m/z (M + H): 812.

[Example 111]

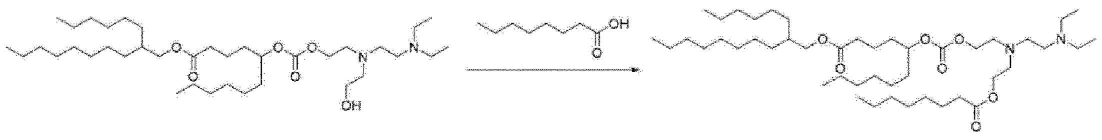

2-Hexyldecyl

3-ethyl-12-hexyl-6-(2-(octanoyloxy)ethyl)-10-oxo-9,11-dioxa-3,6-diazahexadecan-16-oate as a colorless oily substance was obtained by the same method as that in Example 109, except that octanoic acid was used instead of dodecanoic acid in Example 109.

$^{1}$H-NMR ($CDCl_3$) $\delta$: 4.75-4.63 (1H, m), 4.22-4.07 (4H, m), 3.96 (2H, d, J = 5.1 Hz), 2.88-2.76 (4H, m), 2.71-2.63 (2H, m), 2.58-2.45 (6H, m), 2.37-2.24 (4H, m), 1.74-1.52 (9H, m), 1.39-1.19 (40H, m), 1.02 (6H, t, J = 6.6 Hz), 0.96-0.83 (12H, m).
MS m/z (M + H): 784.

[Example 112]

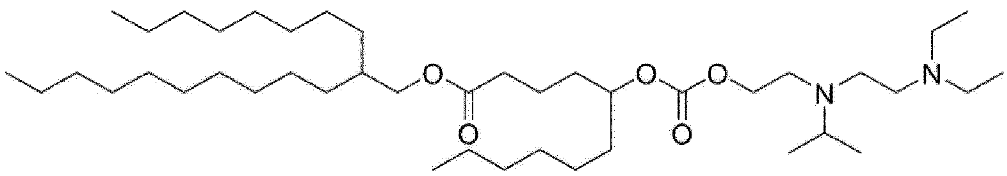

2-Octyldodecyl

3-ethyl-12-hexyl-6-isopropyl-10-oxo-9,11-dioxa-3,6-diazahexadecan-16-oate as a colorless oily substance was obtained by the same method as that in (1) and (2) of Example 80, except that 2-octyldodecan-1-ol and 2-((2-(diethylamino)ethyl)(isopropyl)amino)ethan-1-ol were used instead of 2-butyloctan-1-ol and 2-((2-(diethylamino)ethyl)(ethyl)amino)ethan-1-ol in (1) and (2) of Example 80, respectively.

$^{1}$H-NMR ($CDCl_3$) $\delta$: 4.75-4.63 (1H, m), 4.18-4.02 (2H, m), 3.96 (2H, d, J = 6.0 Hz), 2.97-2.83 (1H, m), 2.68 (2H, t, J = 7.2 Hz), 2.60-2.41 (8H, m), 2.32 (2H, t, J = 6.6 Hz), 1.74-1.50 (7H, m), 1.39-1.16 (40H, m), 1.09-0.95 (12H, m), 0.93-0.80 (9H, m).
MS m/z (M + H): 712.

[Example 113]

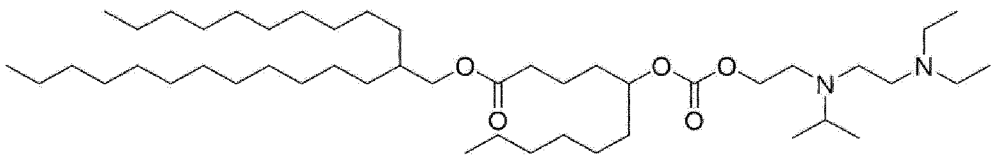

2-Decyltetradecyl

3-ethyl-12-hexyl-6-isopropyl-10-oxo-9,11-dioxa-3,6-diazahexadecan-16-oate as a colorless oily substance was obtained by the same method as that in (1) and (2) of Example 80, except that 2-decyltetradecan-1-ol and 2-((2-(diethylamino)ethyl)(isopropyl)amino)ethan-1-ol were used instead of 2-butyloctan-1-ol and 2-((2-(diethylamino)ethyl)(ethyl)amino)ethan-1-ol in (1) and (2) of Example 80, respectively.

$^1$H-NMR ($CDCl_3$) $\delta$: 4.75-4.63 (1H, m), 4.17-4.02 (2H, m), 3.96 (2H, d, J = 5.4 Hz), 2.97-2.85 (1H, m), 2.68 (2H, t, J = 6.6 Hz), 2.60-2.42 (8H, m), 2.32 (2H, t, J = 7.2 Hz), 1.74-1.49 (7H, m), 1.39-1.17 (48H, m), 1.09-0.95 (12H, m), 0.94-0.81 (9H, m).
MS m/z (M + H): 768.

[Example 114]

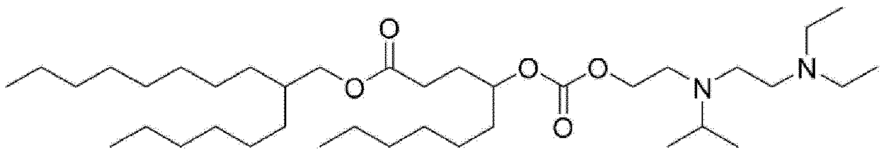

2-Hexyldecyl

3-ethyl-12-hexyl-6-isopropyl-10-oxo-9,11-dioxa-3,6-diazapentadecan-15-oate as a colorless oily substance was obtained by the same method as that in (1) and (2) of Example 84, except that 4-ethoxy-4-oxobutanoic acid and 2-hexyldecan-1-ol were used instead of 10-methoxy-10-oxodecanoic acid and 2-butyloctan-1-ol in (1) and (2) of Example 84, respectively.

$^1$H-NMR ($CDCl_3$) $\delta$: 4.77-4.67 (1H, m), 4.18-4.04 (2H, m), 3.97 (2H, d, J = 5.4 Hz), 2.97-2.84 (1H, m), 2.68 (2H, t, J = 7.5 Hz), 2.61-2.30 (10H, m), 2.02-1.78 (2H, m), 1.70-1.48 (3H, m), 1.41-1.17 (32H, m), 1.11-0.95 (12H, m), 0.94-0.81 (9H, m).
MS m/z (M + H): 642.

[Example 115]

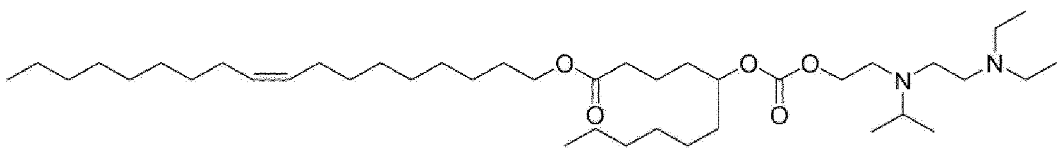

(Z)-octadec-9-en-1-yl 3-ethyl-12-hexyl-6-isopropyl-10-oxo-9,11-dioxa-3,6-diazahexadecan-16-oate as a colorless oily substance was obtained by the same method as that in (1) and (2) of Example 80, except that (Z)-octadec-9-en-1-ol and 2-((2-(diethylamino)ethyl)(isopropyl)amino)ethan-1-ol were used instead of 2-butyloctan-1-ol and 2-((2-(diethylamino)ethyl)(ethyl)amino)ethan-1-ol in (1) and (2) of Example 80, respectively.

$^1$H-NMR ($CDCl_3$) $\delta$: 5.41-5.26 (2H, m), 4.74-4.64 (1H, m), 4.15-4.01 (4H, m), 2.97-2.85 (1H, m), 2.68 (2H, t, J = 6.6 Hz), 2.60-2.42 (8H, m), 2.31 (2H, t, J = 7.2 Hz), 2.08-1.94 (4H, m), 1.74-1.50 (10H, m), 1.41-1.19 (28H, m), 1.07-0.95 (12H, m), 0.92-0.82 (6H, m).
MS m/z (M + H): 682.

[Example 116]

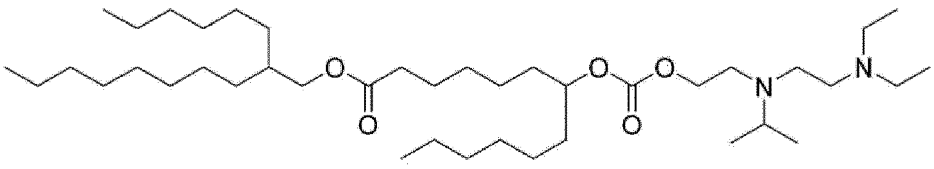

2-Hexyldecyl

3-ethyl-12-hexyl-6-isopropyl-10-oxo-9,11-dioxa-3,6-diazoctadecan-18-oate as a colorless oily substance was obtained by the same method as that in (1) and (2) of Example 84, except that 7-ethoxy-7-oxoheptanoic acid and 2-hexyldecan-1-ol were used instead of 10-methoxy-10-oxodecanoic acid and 2-butyloctan-1-ol in (1) and (2) of Example 84, respectively.

$^1$H-NMR ($CDCl_3$) $\delta$: 4.72-4.62 (1H, m), 4.17-4.03 (2H, m), 3.96 (2H, d, J = 6.0 Hz), 2.97-2.85 (1H, m), 2.68 (2H, t, J = 7.5 Hz), 2.60-2.41 (8H, m), 2.29 (2H, t, J = 7.8 Hz), 1.68-1.48 (7H, m), 1.41-1.18 (36H, m), 1.08-0.95 (12H, m), 0.93-0.81 (9H, m).
MS m/z (M + H): 684.

[Example 117]

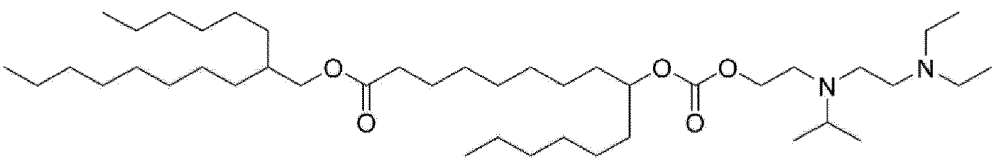

2-Hexyldecyl

3-ethyl-12-hexyl-6-isopropyl-10-oxo-9,11-dioxa-3,6-diazaicosan-20-oate as a colorless oily substance was obtained by the same method as that in (1) and (2) of Example 84, except that 9-methoxy-9-oxononanoic acid and 2-hexyldecan-1-ol were used instead of 10-methoxy-10-oxodecanoic acid and 2-butyloctan-1-ol in (1) and (2) of Example 84, respectively.

$^1$H-NMR ($CDCl_3$) $\delta$: 4.73-4.61 (1H, m), 4.15-4.03 (2H, m), 3.96 (2H, d, J = 5.1 Hz), 2.98-2.84 (1H, m), 2.68 (2H, t, J = 6.6 Hz), 2.60-2.42 (8H, m), 2.29 (2H, t, J = 7.2 Hz), 1.68-1.47 (7H, m), 1.40-1.19 (40H, m), 1.08-0.95 (12H, m), 0.93-0.81 (9H, m).
MS m/z (M + H): 712.

[Example 118]

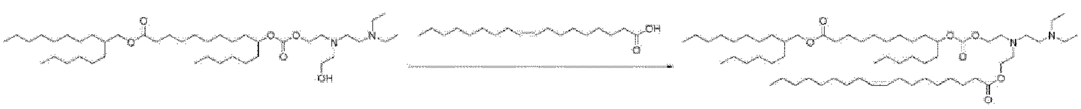

2-Hexyldecyl

3-ethyl-12-hexyl-6-(2-(oleoyloxy)ethyl)-10-oxo-9,11-dioxa-3,6-diazahenicosan-21-oate as a colorless oily substance was obtained by the same method as that in Example 98, except that oleic acid was used instead of decanoic acid in Example 98.

$^1$H-NMR ($CDCl_3$) $\delta$: 5.38-5.28 (2H, m), 4.71-4.61 (1H, m), 4.21-4.08 (4H, m), 3.96 (2H, d, J = 6.0 Hz), 2.87-2.76

(4H, m), 2.71-2.63 (2H, m), 2.57-2.45 (6H, m), 2.29 (4H, t, J = 7.2 Hz), 2.06-1.94 (4H, m), 1.67-1.49 (9H, m), 1.39-1.18 (62H, m), 1.02 (6H, t, J = 7.2 Hz), 0.95-0.82 (12H, m).
MS m/z (M + H): 992.

[Example 119]

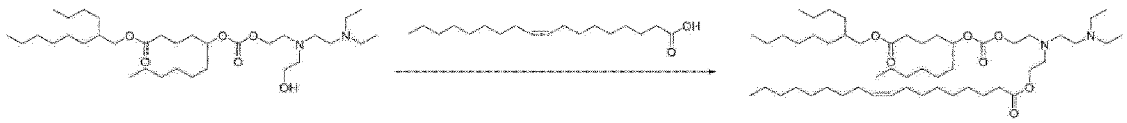

2-Butyloctyl

3-ethyl-12-hexyl-6-(2-(oleoyloxy)ethyl)-10-oxo-9,11-dioxa-3,6-diazahexadecan-16-oate as a colorless oily substance was obtained by the same method as that in Example 106, except that oleic acid was used instead of dodecanoic acid in Example 106.

$^1$H-NMR ($CDCl_3$) δ: 5.40-5.28 (2H, m), 4.74-4.63 (1H, m), 4.22-4.07 (4H, m), 3.97 (2H, d, J = 6.0 Hz), 2.88-2.76 (4H, m), 2.73-2.62 (2H, m), 2.59-2.45 (6H, m), 2.37-2.25 (4H, m), 2.08-1.94 (4H, m), 1.73-1.50 (9H, m), 1.41-1.18 (44H, m), 1.02 (6H, t, J = 6.6 Hz), 0.96-0.82 (12H, m).
MS m/z (M + H): 866.

[Example 120]

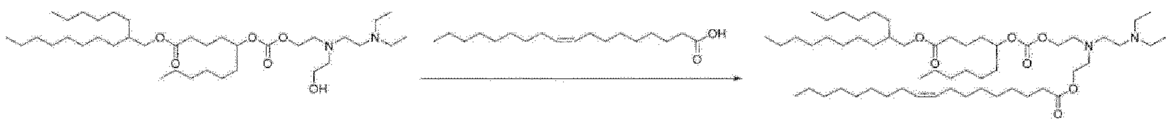

2-Hexyldecyl

3-ethyl-12-hexyl-6-(2-(oleoyloxy)ethyl)-10-oxo-9,11-dioxa-3,6-diazahexadecan-16-oate as a colorless oily substance was obtained by the same method as that in Example 109, except that oleic acid was used instead of dodecanoic acid in Example 109.

$^1$H-NMR ($CDCl_3$) δ: 5.40-5.28 (2H, m), 4.73-4.64 (1H, m), 4.21-4.07 (4H, m), 3.96 (2H, d, J = 5.1 Hz), 2.88-2.76 (4H, m), 2.72-2.62 (2H, m), 2.58-2.45 (6H, m), 2.37-2.24 (4H, m), 2.07-1.94 (4H, m), 1.73-1.51 (9H, m), 1.39-1.19 (52H, m), 1.02 (6H, t, J = 6.6 Hz), 0.94-0.81 (12H, m).
MS m/z (M + H): 922.

[Example 121]

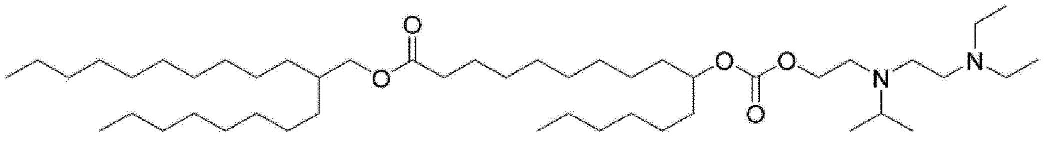

2-Octyldodecyl

3-ethyl-12-hexyl-6-isopropyl-10-oxo-9,11-dioxa-3,6-diazahenicosan-21-oate as a colorless oily substance was

obtained by the same method as that in (1) and (2) of Example 84, except that 2-octyldodecan-1-ol was used instead of 2-butyloctan-1-ol in (1) and (2) of Example 84.

$^1$H-NMR ($CDCl_3$) $\delta$: 4.73-4.61 (1H, m), 4.15-4.06 (2H, m), 3.96 (2H, d, J = 6.0 Hz), 2.97-2.84 (1H, m), 2.68 (2H, t, J = 6.6 Hz), 2.59-2.42 (8H, m), 2.29 (2H, t, J = 8.1 Hz), 1.68-1.48 (7H, m), 1.38-1.19 (50H, m), 1.09-0.96 (12H, m), 0.93-0.82 (9H, m).
MS m/z (M + H): 782.

[Example 122]

(1)

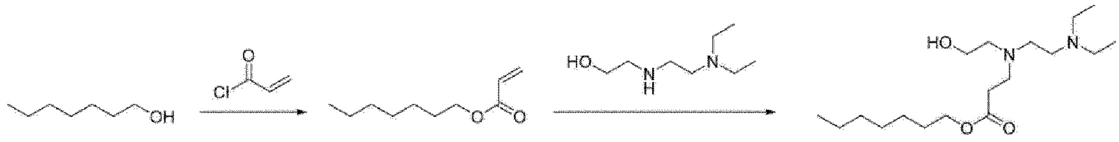

Acrylic acid chloride (0.45 mL) was added to a mixture of heptan-1-ol (0.86 mL), triethylamine (1.55 mL), and tetrahydrofuran (5.00 mL) under ice cooling, and the mixture was stirred at room temperature for 2 hours. Water and ethyl acetate were added to the reaction mixture, the organic layer was separated, washed with a saturated aqueous sodium chloride solution, and then dried over anhydrous sodium sulfate, and the solvent was distilled away under reduced pressure. The obtained residue was purified by silica gel column chromatography (ethyl acetate-hexane), thereby obtaining heptyl acrylate (0.57 g) as a colorless oily substance.
Triethylamine (1.24 mL) was added to a mixture of the obtained heptyl acrylate (0.57 g), 2-((2-(diethylamino)ethyl)amino)ethan-1-ol dihydrochloride (0.52 g), and tetrahydrofuran (10 mL), and the mixture was stirred under reflux with heating for 8 hours. The reaction mixture was cooled to room temperature, and the solvent was distilled away under reduced pressure. The obtained residue was purified by silica gel column chromatography (ethyl acetate-hexane, NH silica gel), thereby obtaining heptyl 3-((2-(diethylamino)ethyl)(2-hydroxyethyl)amino)propanoate (0.21 g) as a colorless oily substance.
MS m/z (M + H): 331.

(2)

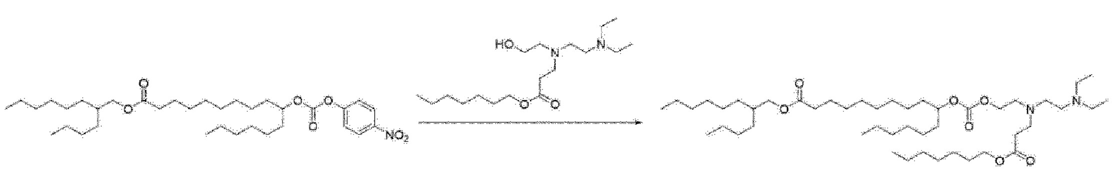

2-Butyloctyl

3-ethyl-6-(3-(heptyloxy)-3-oxopropyl)-12-hexyl-10-oxo-9,11-dioxa-3,6-diazahenicosan-21-oat e as a colorless oily substance was obtained by the same method as that in (2) of Example 84, except that heptyl 3-((2-(diethylamino)ethyl)(2-hydroxyethyl)amino)propanoate was used instead of 2-((2-(diethylamino)ethyl)(isopropyl)amino)ethan-1-ol in (2) of Example 84.

$^1$H-NMR ($CDCl_3$) $\delta$: 4.72-4.61 (1H, m), 4.20-4.11 (2H, m), 4.06 (2H, t, J = 6.6 Hz), 3.96 (2H, d, J = 6.0 Hz), 2.87 (2H, t, J = 6.6 Hz), 2.77 (2H, d, J = 6.0 Hz), 2.64-2.41 (10H, m), 2.29 (2H, t, J = 7.2 Hz), 1.66-1.50 (9H, m), 1.37-1.22 (42H, m), 1.02 (6H, t, J = 6.6 Hz), 0.92-0.84 (12H, m).
MS m/z (M + H): 798.

[Example 123]

(1)

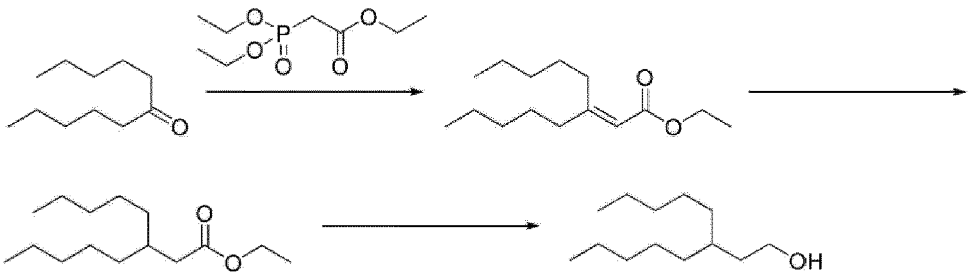

Ethyl 2-(diethoxyphosphoryl)acetate (18.8 mL) was added dropwise to a tetrahydrofuran (80 mL) suspension of 60 wt% sodium hydride (3.3 g) under ice cooling, and the mixture was stirred at the same temperature for 30 minutes. Undecan-6-one (2.0 g) was added to the reaction mixture, and the reaction mixture was stirred under reflux with heating for 5 hours. The reaction mixture was cooled to room temperature and poured into ice water, and then ethyl acetate was added thereto. The organic layer was separated and washed with a saturated aqueous sodium chloride solution, then the solvent was distilled away under reduced pressure, and the obtained residue was purified by silica gel column chromatography (ethyl acetate-hexane), thereby obtaining ethyl 3-pentyloct-2-enoate (2.8g) as a colorless oily substance.

$^1$H-NMR ($CDCl_3$) $\delta$: 5.61 (1H, s), 4.14 (2H, q, J = 6.6 Hz), 2.58 (2H, t, J = 7.2 Hz), 2.12 (2H, t, J = 7.2 Hz), 1.50-1.20 (15H, m), 0.89 (6H, t, J = 6.6 Hz).

Ammonium formate (4.4 g) was added to a mixture of ethyl 3-pentyloct-2-enoate (2.8 g), 10% palladium-carbon (0.84 g), and methanol (56 mL), and the mixture was stirred under reflux with heating for 3 hours. The reaction mixture was cooled to room temperature, the insoluble matters were filtered off through celite, and then the solvent was distilled away under reduced pressure. The obtained residue was purified by silica gel column chromatography (ethyl acetate-hexane), thereby obtaining ethyl 3-pentyloctanoate (2.8 g) as a colorless oily substance.

$^1$H-NMR ($CDCl_3$) $\delta$: 4.12 (2H, q, J = 7.2 Hz), 2.22 (2H, t, J = 6.6 Hz), 2.05-2.04 (1H, m), 1.34-1.20 (19H, m), 0.88 (6H, t, J = 6.6 Hz).

A tetrahydrofuran (10 mL) solution of ethyl 3-pentyloctanoate (2.8 g) was added dropwise to a mixture of a 2.5 mol/L lithium aluminum hydride-tetrahydrofuran solution (9.3 mL) and tetrahydrofuran (50 mL) under ice cooling, and the mixture was stirred at the same temperature for 30 minutes and then stirred at room temperature for 2 hours. Ethyl acetate was added to the reaction mixture, the reaction mixture was poured into ice water under ice cooling, and then the insoluble matters were filtered off through celite. The organic layer was separated, washed with a saturated aqueous sodium chloride solution, and then dried over anhydrous sodium sulfate, and the solvent was distilled away under reduced pressure. The obtained residue was purified by silica gel column chromatography (ethyl acetate-hexane), thereby obtaining 3-pentyloctan-1-ol (2.2 g) as a colorless oily substance.

$^1$H-NMR ($CDCl_3$) $\delta$: 3.71-3.62 (2H, m), 1.57-1.49 (2H, m), 1.35-1.20 (17H, m), 0.88 (6H, t, J = 6.6 Hz).

(2)

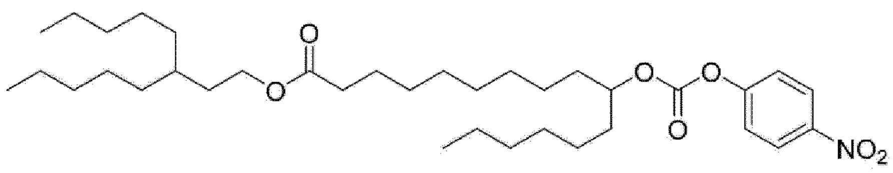

3-Pentyloctyl 10-(((4-nitrophenoxy)carbonyl)oxy)hexadecanoate as a colorless oily substance was obtained by the same method as that in (1) of Example 84, except that 3-pentyloctan-1-ol was used instead of 2-butyloctan-1-ol in (1) of Example 84.

$^1$H-NMR ($CDCl_3$) $\delta$: 8.31-8.25 (2H, m), 7.41-7.36 (2H, m), 4.86-4.77 (1H, m), 3.97 (2H, d, J = 5.4 Hz), 2.30 (2H, t, J = 7.5 Hz), 1.72-1.20 (43H, m), 0.92-0.85 (9H, m).

(3)

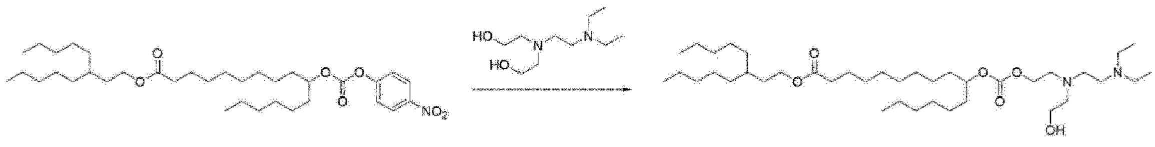

3-Pentyloctyl

3-ethyl-12-hexyl-6-(2-hydroxyethyl)-10-oxo-9,11-dioxa-3,6-diazahenicosan-21-oate as a colorless oily substance was obtained by the same method as that in (2) of Example 85, except that 3-pentyloctyl 10-(((4-nitrophenoxy)carbonyl)oxy)hexadecanoate was used instead of 2-butyloctyl 10-(((4-nitrophenoxy)carbonyl)oxy)hexadecanoate in (2) of Example 85.

$^1$H-NMR ($CDCl_3$) δ: 4.74-4.06 (1H, m), 4.20 (2H, t, J = 6.0 Hz), 4.08 (2H, t, J = 6.6 Hz), 3.54 (2H, t, J = 4.5 Hz), 2.88 (2H,, t, J = 5.7 Hz), 2.75-2.63 (4H, m), 2.60-2.41 (6H, m), 2.28 (2H, t, J = 7.8 Hz), 1.72-1.47 (8H, m), 1.44-1.14 (35H, m), 1.03 (6H, t, J = 7.2 Hz), 0.94-0.81 (9H, m).
MS m/z (M + H): 686.

[Example 124]

(1)

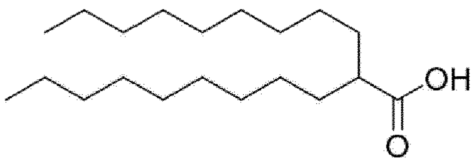

2-Nonylundecanoic acid as a colorless oily substance was obtained by the same method as that in (1) of Example 74, except that undecanoic acid and 1-iodononane were used instead of decanoic acid and 1-iodooctane in (1) of Example 74, respectively.
$^1$H-NMR ($CDCl_3$) δ: 2.29-2.41 (1H, m), 1.68-1.20 (32H, m), 0.88 (6H, t, J = 6.6 Hz).

(2)

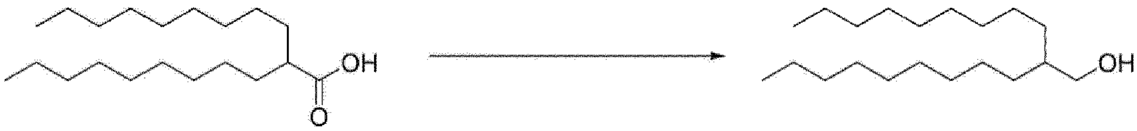

A tetrahydrofuran (10 mL) solution of 2-nonylundecanoic acid (3.0 g) was added dropwise to a mixture of a 2.5 mol/L lithium aluminum hydride-tetrahydrofuran solution (7.6 mL) and tetrahydrofuran (60 mL) under ice cooling, and the mixture was stirred at the same temperature for 30 minutes and then stirred at room temperature for 6 hours. Ethyl acetate was added to the reaction mixture, the reaction mixture was poured into ice water under ice cooling, and then the insoluble matters were filtered off through celite. The organic layer was separated, washed with a saturated aqueous sodium chloride solution, and then dried over anhydrous sodium sulfate, and the solvent was distilled away under reduced pressure. The obtained residue was purified by silica gel column chromatography (ethyl acetate-hexane), thereby obtaining 2-nonylundecan-1-ol (2.8 g) as a colorless oily substance.
$^1$H-NMR ($CDCl_3$) δ: 3.57-3.51 (2H, m), 1.50-1.20 (33H, m), 0.88 (6H, t, J = 6.6 Hz).

(3)

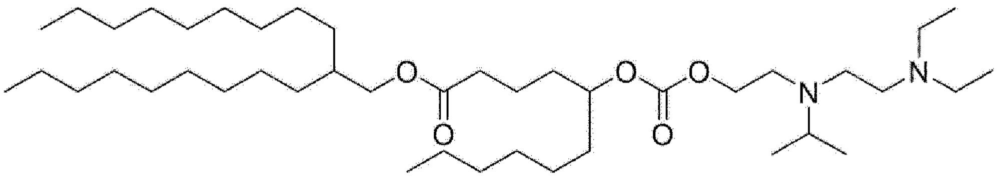

2-Nonylundecyl

3-ethyl-12-hexyl-6-isopropyl-10-oxo-9,11-dioxa-3,6-diazahexadecan-16-oate as a colorless oily substance was obtained by the same method as that in (1) and (2) of Example 84, except that 2-nonylundecan-1-ol was used instead of 2-butyloctan-1-ol in (1) and (2) of Example 84.

$^1$H-NMR ($CDCl_3$) δ: 4.76-4.62 (1H, m), 4.18-4.02 (2H, m), 3.96 (2H, d, J = 5.7 Hz), 2.97-2.84 (1H, m), 2.68 (2H, t, J = 7.2 Hz), 2.60-2.42 (8H, m), 2.36-2.27 (2H, m), 1.76-1.49 (7H, m), 1.39-1.19 (40H, m), 1.09-0.94 (12H, m), 0.93-0.83 (9H, m).
MS m/z (M + H): 712.

[Example 125]

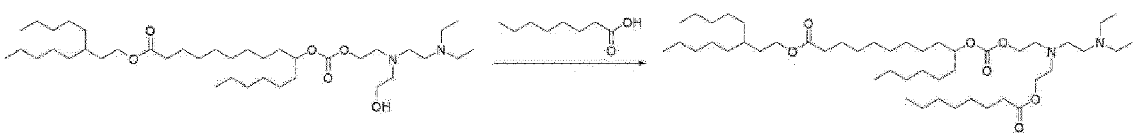

3-Pentyloctyl

3-ethyl-12-hexyl-6-(2-(octanoyloxy)ethyl)-10-oxo-9,11-dioxa-3,6-diazahenicosan-21-oate as a colorless oily substance was obtained by the same method as that in Example 86, except that 3-pentyloctyl
3-ethyl-12-hexyl-6-(2-hydroxyethyl)-10-oxo-9,11-dioxa-3,6-diazahenicosan-21-oate and octanoic acid were used instead of 2-butyloctyl 3-ethyl-12-hexyl-6-(2-hydroxyethyl)-10-oxo-9,11-dioxa-3,6-diazahenicosan-21-oate and dodecanoic acid in Example 86, respectively.

$^1$H-NMR ($CDCl_3$) δ: 4.72-4.61 (1H, m), 4.21-4.02 (6H, m), 2.88-2.75 (4H, m), 2.71-2.62 (2H, m), 2.58-2.45 (6H, m), 2.34-2.22 (4H, m), 1.68-1.48 (10H, m), 1.44-1.17 (43H, m), 1.02 (6H, t, J = 6.6 Hz), 0.94-0.81 (12H, m).
MS m/z (M + H): 812.

[Example 126]

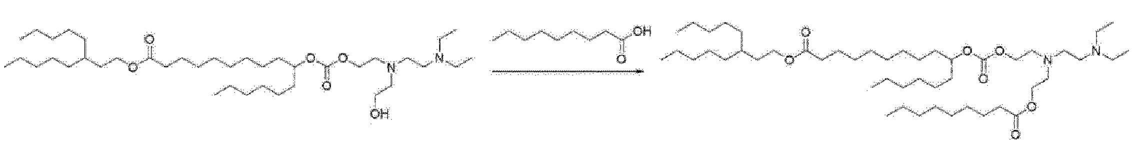

3-Pentyloctyl

3-ethyl-12-hexyl-6-(2-(nonanoyloxy)ethyl)-10-oxo-9,11-dioxa-3,6-diazahenicosan-21-oate as a colorless oily substance was obtained by the same method as that in Example 86, except that 3-pentyloctyl
3-ethyl-12-hexyl-6-(2-hydroxyethyl)-10-oxo-9,11-dioxa-3,6-diazahenicosan-21-oate and nonanoic acid were used instead of 2-butyloctyl 3-ethyl-12-hexyl-6-(2-hydroxyethyl)-10-oxo-9,11-dioxa-3,6-diazahenicosan-21-oate and dodecanoic acid in Example 86, respectively.

$^1$H-NMR ($CDCl_3$) δ: 4.73-4.59 (1H, m), 4.23-4.01 (6H, m), 2.90-2.76 (4H, m), 2.72-2.62 (2H, m), 2.58-2.45 (6H, m),

2.35-2.22 (4H, m), 1.69-1.47 (10H, m), 1.44-1.18 (45H, m), 1.02 (6H, t, J = 7.5 Hz), 0.96-0.80 (12H, m).
MS m/z (M + H): 826.

[Example 127]

(1)

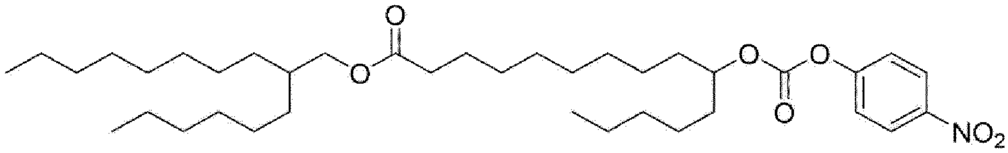

2-Hexyldecyl 10-(((4-nitrophenoxy)carbonyl)oxy)pentadecanoate as a colorless oily substance was obtained by the same method as that in (1) of Example 84, except that a 1.0 mol/L pentyl magnesium bromide-tetrahydrofuran solution and 2-hexyldecan-1-ol were used instead of a 1.0 mol/L hexyl magnesium bromide-diethyl ether solution and 2-butyloctan-1-ol in (1) of Example 84, respectively.

$^1$H-NMR ($CDCl_3$) δ: 8.31-8.25 (2H, m), 7.41-7.35 (2H, m), 4.87-4.75 (1H, m), 3.96 (2H, d, J = 6.0 Hz), 2.30 (2H, t, J = 7.2 Hz), 1.72-1.20 (47H, m), 0.93-0.83 (9H, m).

(2)

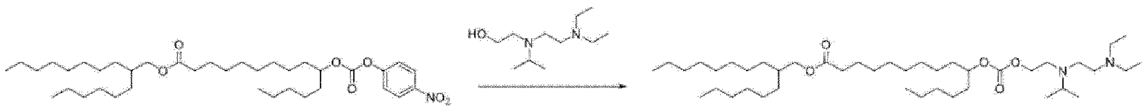

2-Hexyldecyl

3-ethyl-6-isopropyl-10-oxo-12-pentyl-9,11-dioxa-3,6-diazahenicosan-21-oate as a colorless oily substance was obtained by the same method as that in (2) of Example 84, except that 2-hexyldecyl 10-(((4-nitrophenoxy)carbonyl)oxy)pentadecanoate was used instead of 2-butyloctyl 10-(((4-nitrophenoxy)carbonyl)oxy)hexadecanoate in (2) of Example 84.

$^1$H-NMR ($CDCl_3$) δ: 4.72-4.62 (1H, m), 4.10 (2H, t, J = 6.6 Hz), 3.96 (2H, d, J = 6.0 Hz), 2.98-2.82 (1H, m), 2.68 (2H, t, J = 6.6 Hz), 2.59-2.42 (8H, m), 2.29 (2H, t, J = 7.2 Hz), 1.66-1.47 (7H, m), 1.40-1.18 (40H, m), 1.06-0.96 (12H, m), 0.92-0.84 (9H, m).
MS m/z (M + H): 712.

[Example 128]

(1)

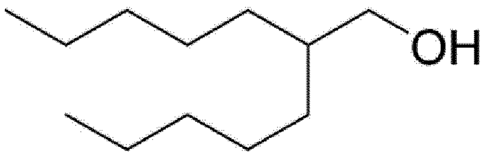

2-Pentylheptan-1-ol as a colorless oily substance was obtained by the same method as that in (2) of Example 124, except that 2-pentylheptanoic acid was used instead of 2-nonylundecanoic acid in (2) of Example 124.

$^1$H-NMR ($CDCl_3$) δ: 3.57-3.51 (2H, m), 1.50-1.20 (17H, m), 0.88 (6H, t, J = 6.6 Hz).

(2)

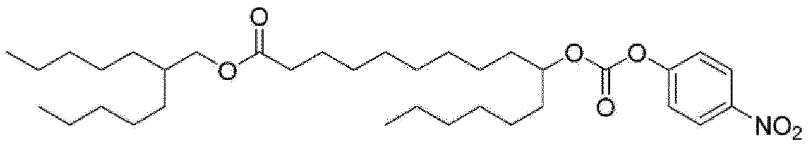

2-Pentylheptyl 10-(((4-nitrophenoxy)carbonyl)oxy)hexadecanoate as a colorless oily substance was obtained by the same method as that in (1) of Example 84, except that 2-pentylheptan-1-ol was used instead of 2-butyloctan-1-ol in (1) of Example 84.

$^1$H-NMR ($CDCl_3$) $\delta$: 8.28 (2H, dd, J = 7.2 Hz, 2.1 Hz), 7.39 (2H, dd, J = 7.2 Hz, 2.1 Hz), 4.86-4.76 (1H, m), 3.97 (2H, d, J = 6.0 Hz), 2.30 (2H, t, J = 7.2 Hz), 1.74-1.20 (41H, m), 0.92-0.85 (9H, m).

(3)

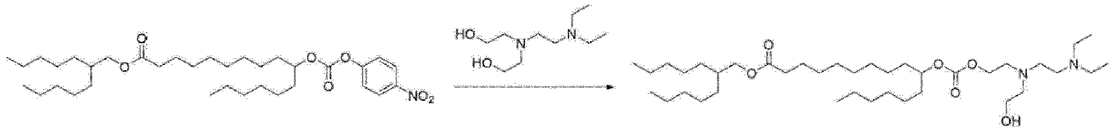

2-Pentylheptyl

3-ethyl-12-hexyl-6-(2-hydroxyethyl)-10-oxo-9,11-dioxa-3,6-diazahenicosan-21-oate as a colorless oily substance was obtained by the same method as that in (2) of Example 85, except that 2-pentylheptyl 10-(((4-nitrophenoxy)carbonyl)oxy)hexadecanoate was used instead of 2-butyloctyl 10-(((4-nitrophenoxy)carbonyl)oxy)hexadecanoate in (2) of Example 85.

$^1$H-NMR ($CDCl_3$) $\delta$: 4.73-4.61 (1H, m), 4.20 (2H, t, J = 6.0 Hz), 3.97 (2H, d, J = 5.4 Hz), 3.54 (2H, t, J = 4.5 Hz), 2.88 (2H, t, J = 6.6 Hz), 2.74-2.64 (4H, m), 2.59-2.44 (6H, m), 2.29 (2H, t, J = 7.2 Hz), 1.75-1.45 (7H, m), 1.40-1.19 (34H, m), 1.02 (6H, t, J = 7.2 Hz), 0.92-0.84 (9H, m).
MS m/z (M + H): 672.

[Example 129]

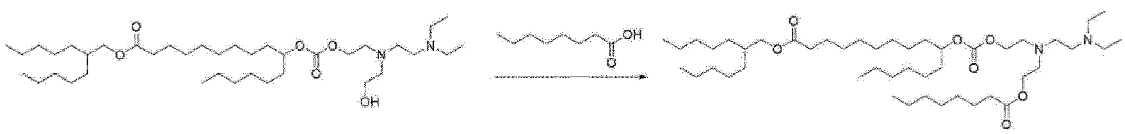

2-Pentylheptyl

3-ethyl-12-hexyl-6-(2-(octanoyloxy)ethyl)-10-oxo-9,11-dioxa-3,6-diazahenicosan-21-oate as a colorless oily substance was obtained by the same method as that in Example 86, except that 2-pentylheptyl

3-ethyl-12-hexyl-6-(2-hydroxyethyl)-10-oxo-9,11-dioxa-3,6-diazahenicosan-21-oate and octanoic acid were used instead of 2-butyloctyl 3-ethyl-12-hexyl-6-(2-hydroxyethyl)-10-oxo-9,11-dioxa-3,6-diazahenicosan-21-oate and dodecanoic acid in Example 86, respectively.

$^1$H-NMR ($CDCl_3$) $\delta$: 4.73-4.61 (1H, m), 4.21-4.07 (4H, m), 3.97 (2H, d, J = 5.4 Hz), 2.88-2.77 (4H, m), 2.72-2.62 (2H, m), 2.58-2.45 (6H, m), 2.29 (4H, t, J = 7.2 Hz), 1.69-1.48 (9H, m), 1.41-1.18 (42H, m), 1.02 (6H, t, J = 7.2 Hz), 0.94-0.82 (12H, m).
MS m/z (M + H): 798.

[Example 130]

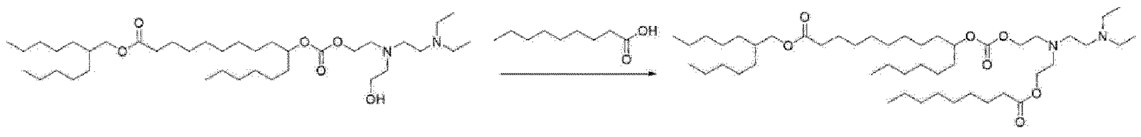

2-Pentylheptyl

3-ethyl-12-hexyl-6-(2-(nonanoyloxy)ethyl)-10-oxo-9,11-dioxa-3,6-diazahenicosan-21-oate as a colorless oily substance was obtained by the same method as that in Example 86, except that 2-pentylheptyl

3-ethyl-12-hexyl-6-(2-hydroxyethyl)-10-oxo-9,11-dioxa-3,6-diazahenicosan-21-oate and nonanoic acid were used instead of 2-butyloctyl 3-ethyl-12-hexyl-6-(2-hydroxyethyl)-10-oxo-9,11-dioxa-3,6-diazahenicosan-21-oate and dodecanoic acid in Example 86, respectively.

$^1$H-NMR ($CDCl_3$) $\delta$: 4.72-4.61 (1H, m), 4.22-4.08 (4H, m), 3.97 (2H, d, J = 6.0 Hz), 2.88-2.75 (4H, m), 2.72-2.62 (2H, m), 2.60-2.46 (6H, m), 2.29 (4H, t, J = 7.5 Hz), 1.70-1.47 (9H, m), 1.41-1.18 (44H, m), 1.02 (6H, t, J = 6.6 Hz), 0.95-0.81 (12H, m).
MS m/z (M + H): 812.

[Example 131]

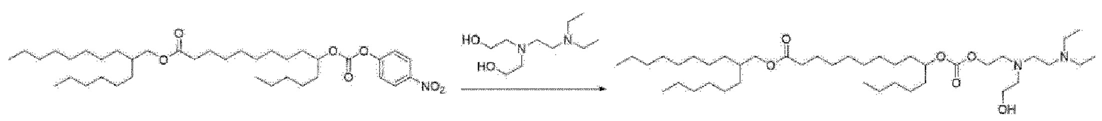

2-Hexyldecyl

3-ethyl-6-(2-hydroxyethyl)-10-oxo-12-pentyl-9,11-dioxa-3,6-diazahenicosan-21-oate as a colorless oily substance was obtained by the same method as that in (2) of Example 85, except that 2-hexyldecyl 10-(((4-nitrophenoxy)carbonyl)oxy)pentadecanoate was used instead of 2-butyloctyl 10-(((4-nitrophenoxy)carbonyl)oxy)hexadecanoate in (2) of Example 85.

$^1$H-NMR ($CDCl_3$) $\delta$: 4.74-4.62 (1H, m), 4.20 (2H, t, J = 6.0 Hz), 3.96 (2H, d, J = 5.7 Hz), 3.54 (2H, t, J = 4.5 Hz), 2.89 (2H, t, J = 6.0 Hz), 2.75-2.64 (4H, m), 2.60-2.43 (6H, m), 2.29 (2H, t, J = 7.8 Hz), 1.67-1.49 (7H, m), 1.41-1.19 (40H, m), 1.02 (6H, t, J = 7.2 Hz), 0.94-0.82 (9H, m).
MS m/z (M + H): 714.

[Example 132]

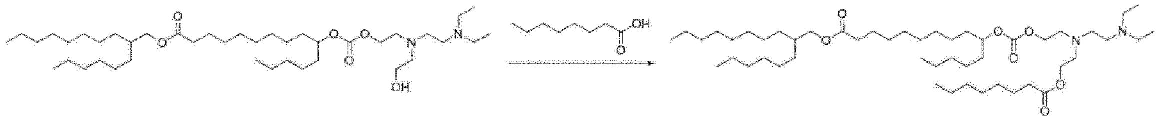

2-Hexyldecyl

3-ethyl-6-(2-(octanoyloxy)ethyl)-10-oxo-12-pentyl-9,11-dioxa-3,6-diazahenicosan-21-oate as a colorless oily

substance was obtained by the same method as that in Example 86, except that 2-hexyldecyl

3-ethyl-6-(2-hydroxyethyl)-10-oxo-12-pentyl-9,11-dioxa-3,6-diazahenicosan-21-oate and octanoic acid were used instead of 2-butyloctyl 3-ethyl-12-hexyl-6-(2-hydroxyethyl)-10-oxo-9,11-dioxa-3,6-diazahenicosan-21-oate and dodecanoic acid in Example 86, respectively.

$^1$H-NMR ($CDCl_3$) δ: 4.72-4.61 (1H, m), 4.22-4.06 (4H, m), 3.96 (2H, d, J = 6.0 Hz), 2.88-2.76 (4H, m), 2.72-2.62 (2H, m), 2.58-2.45 (6H, m), 2.29 (4H, t, J = 7.2 Hz), 1.68-1.48 (9H, m), 1.39-1.18 (48H, m), 1.02 (6H, t, J = 6.6 Hz), 0.94-0.82 (12H, m).
MS m/z (M + H): 840.

[Example 133]

(1)

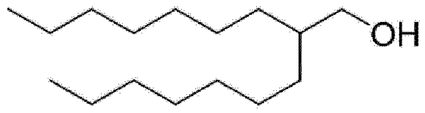

2-Heptylnonan-1-ol as a colorless oily substance was obtained by the same method as that in (1) and (2) of Example 124, except that nonanoic acid and 1-iodoheptane were used instead of undecanoic acid and 1-iodononane in (1) and (2) of Example 124, respectively.

$^1$H-NMR ($CDCl_3$) δ: 3.57-3.51 (2H, m), 1.50-1.20 (25H, m), 0.88 (6H, t, J = 6.6 Hz).

(2)

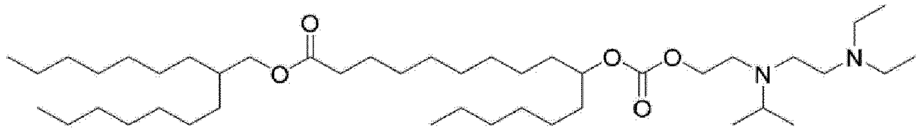

2-Heptylnonyl

3-ethyl-12-hexyl-6-isopropyl-10-oxo-9,11-dioxa-3,6-diazahenicosan-21-oate as a colorless oily substance was obtained by the same method as that in (1) and (2) of Example 84, except that 2-heptylnonan-1-ol was used instead of 2-butyloctan-1-ol in (1) and (2) of Example 84.

$^1$H-NMR ($CDCl_3$) δ: 4.72-4.62 (1H, m), 4.10 (2H, t, J = 6.6 Hz), 3.96 (2H, d, J = 6.0 Hz), 2.97-2.85 (1H, m), 2.68 (2H, t, J = 6.9 Hz), 2.60-2.41 (8H, m), 2.29 (2H, t, J = 7.2 Hz), 1.67-1.47 (7H, m), 1.39-1.19 (42H, m), 1.08-0.95 (12H, m), 0.94-0.82 (9H, m).
MS m/z (M + H): 726.

[Example 134]

(1)

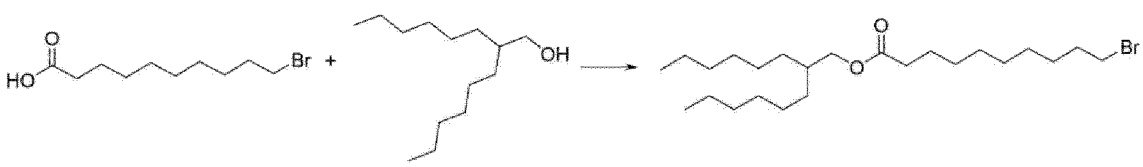

2-Hexyl-1-octanol (4.4 g), 1-(3-dimethylaminopropyl)-3-ethylcarbodiimide hydrochloride (3.9 g), 4-dimethylaminopyridine (0.9 g), and triethylamine (8.6 mL) were added to a dichloromethane (80 mL) solution of 10-bromodecanoic acid (4.0 g) at room temperature, and the mixture was stirred overnight at the same temperature. Ethyl acetate and water were added to the reaction mixture. The organic layer was separated, washed with a saturated aqueous sodium chloride solution, and then dried over anhydrous sodium sulfate, and the solvent was distilled away under reduced pressure. The obtained residue was purified by silica gel column chromatography (ethyl acetate-hexane), thereby obtaining 2-hexyloctyl 10-bromodecanoate (3.4 g) as a colorless oily substance.

$^1$H-NMR ($CDCl_3$) $\delta$: 3.97 (2H, d, J = 6.0 Hz), 3.41 (2H, t, J = 6.6 Hz), 2.30 (2H, t, J = 7.5 Hz), 1.91-1.78 (2H, m), 1.69-1.19 (33H, m), 0.89 (6H, t, J = 7.5 Hz).

(2)

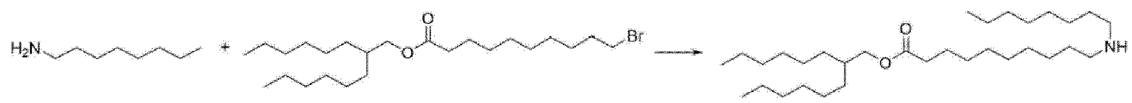

N-octylamine (1.1 mL) and potassium carbonate (1.9 g) were added to a N,N-dimethylformamide (5 mL) solution of 2-hexyloctyl 10-bromodecanoate (1.0 g) at room temperature, and the mixture was stirred for 4 hours at 60°C. The reaction mixture was cooled to room temperature, and then ethyl acetate and water were added thereto. The organic layer was separated, washed with a saturated aqueous sodium chloride solution, and then dried over anhydrous sodium sulfate, and the solvent was distilled away under reduced pressure. The obtained residue was purified by silica gel column chromatography (methanol-ethyl acetate), thereby obtaining 2-hexyloctyl 10-(octylamino)decanoate (806 mg) as a light yellow oily substance.

$^1$H-NMR ($CDCl_3$) $\delta$: 3.97 (2H, d, J = 6.0 Hz), 2.58 (4H, t, J = 7.2 Hz), 2.29 (2H, t, J = 7.2 Hz), 1.68-1.20 (47H, m), 0.92-0.84 (9H, m).

(3)

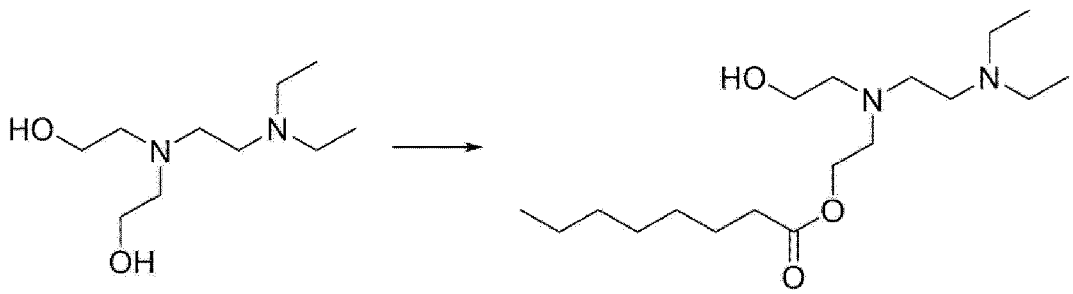

Octanoic acid (0.79 mL), 1-(3-dimethylaminopropyl)-3-ethylcarbodiimide hydrochloride (1.4 g), 4-dimethylaminopyridine (1.2 g), and triethylamine (2.1 mL) were added to a dichloromethane (20 mL) solution of 2,2'-((2-(diethylamino)ethyl)azanediyl)bis(ethan-1-ol) (1.0 g) at room temperature, and the mixture was stirred at the same temperature for 12 hours. Ethyl acetate and water were added to the reaction mixture. The organic layer was separated, washed with a saturated aqueous sodium chloride solution, and then dried over anhydrous sodium sulfate, and the solvent was distilled away under reduced pressure. The obtained residue was purified by silica gel column chromatography (ethyl acetate-hexane, NH silica gel), thereby obtaining 2-((2-(diethylamino)ethyl)(2-hydroxyethyl)amino)ethyl octanoate (541 mg) as a colorless oily substance.

$^1$H-NMR ($CDCl_3$) $\delta$: 4.15 (2H, t, J = 5.4 Hz), 3.54 (2H, t, J = 5.4 Hz), 2.84 (2H, t, J = 6.0 Hz), 2.72-2.63 (4H, m), 2.59-2.44 (6H, m), 2.30 (2H, t, J = 7.2 Hz), 1.78-1.19 (10H, m), 1.03 (6H, t, J = 7.2Hz), 0.88 (3H, t, J = 6.6Hz).

(4)

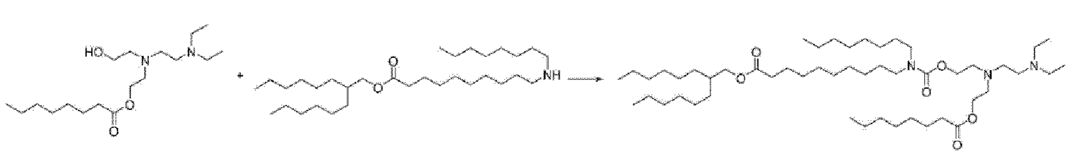

4-Nitrophenylchloroformate (311 mg) was added to a tetrahydrofuran (6 mL) solution of 2-((2-(diethylamino)ethyl)(2-hydroxyethyl)amino)ethyl octanoate (500 mg) at room temperature, and the mixture was stirred at the same temperature for 1 hour. 2-Hexyloctyl 10-(octylamino)decanoate (300 mg) and triethylamine (0.34 mL) were added to the reaction mixture at room temperature, and the mixture was stirred at 60°C for 3 hours. The reaction mixture was cooled to room temperature, and then ethyl acetate and water were added thereto. The organic layer was separated, washed with a saturated aqueous sodium chloride solution, and then dried over anhydrous sodium sulfate, and the solvent was distilled away under reduced pressure. The obtained residue was purified by silica gel column chromatography (methanol-ethyl acetate) and silica gel column chromatography (ethyl acetate-hexane, NH silica gel), thereby obtaining 2-hexyloctyl 3-ethyl-6-(2-(octanoyloxy)ethyl)-11-octyl-10-oxo-9-oxa-3,6,11-triazahenicosan-21-oate (117 mg) as a colorless oily substance.

$^1$H-NMR ($CDCl_3$) δ: 4.16-4.07 (4H, m), 3.97 (2H, d, J = 6.0 Hz), 3.24-3.09 (4H, m), 2.84-2.75 (4H, m), 2.71-2.61 (2H, m), 2.58-2.46 (6H, m), 2.34-2.23 (4H, m), 1.68-1.42 (5H, m), 1.36-1.18 (52H, m), 1.02 (6H, t, J = 7.5 Hz), 0.93-0.83 (12H, m).
MS m/z (M + H): 853.

[Example 135]

(1)

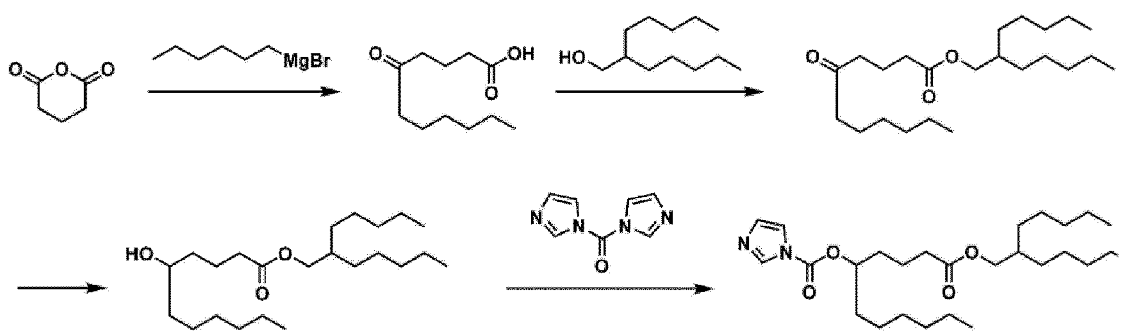

A 1 mol/L hexyl magnesium bromide-tetrahydrofuran solution (200 mL) was added dropwise to a tetrahydrofuran (273 mL) solution of glutaric anhydride (27.3 g) under ice cooling, and the mixture was stirred at the same temperature for 1 hour. A 2 mol/L aqueous hydrochloric acid solution (240 mL) was added to the reaction mixture under ice cooling, then ethyl acetate (270 mL) was added thereto, the organic layer was separated, washed with water (80 mL) and a saturated aqueous sodium chloride solution (80 mL), and dried over anhydrous magnesium sulfate, and then the solvent was distilled away under reduced pressure. The obtained residue was purified by silica gel column chromatography (ethyl acetate-hexane), then hexane (10 mL) was added thereto, and solids were collected by filtration, washed with hexane (10 mL), and then dried under reduced pressure, thereby obtaining 5-oxoundecanoic acid (16.0 g) as white solids.
$^1$H-NMR ($CDCl_3$) δ: 2.50 (2H, t, J = 7.2 Hz), 2.40 (4H, t, J = 7.2 Hz), 2.02-1.80 (2H, m), 1.63-1.48 (2H, m), 1.37-1.20 (6H, m), 0.88 (3H, t, J = 6.6 Hz).
p-Toluenesulfonic acid monohydrate (0.13 g) was added to a mixture of 5-oxoundecanoic acid (2.8 g), 2-pentylheptane-1-ol (2.3 g), and toluene (5.6 mL), and the mixture was stirred at 110°C for 1.5 hours. The reaction mixture was cooled to room temperature and then purified by silica gel column chromatography (ethyl acetate-hexane), thereby obtaining 2-pentylheptyl 5-oxoundecanoate (3.8 g) as a colorless oily substance.
$^1$H-NMR ($CDCl_3$) δ: 3.97 (2H, d, J = 5.1 Hz), 2.47 (2H, t, J = 7.2 Hz), 2.39 (2H, t, J = 7.2 Hz), 2.33 (2H, t, J = 7.2 Hz), 1.95-1.83 (2H, m), 1.66-1.49 (3H, m), 1.36-1.20 (22H, m), 0.92-0.82 (9H, m).
Sodium borohydride (0.47 g) was added to a mixture of 2-pentylheptyl 5-oxoundecanoate (3.8 g), methanol (15 mL), and toluene (15 mL) under ice cooling, and the mixture was stirred at the same temperature for 30 minutes. Water (6 mL) was added to the reaction mixture at the same temperature, a 1.0 mol/L aqueous hydrochloric acid solution (6 mL) was added thereto, the organic layer was separated and dried over anhydrous sodium sulfate, and then the solvent was distilled away under reduced pressure. The obtained residue was purified by silica gel column chromatography (ethyl acetate-hexane), thereby obtaining 2-pentylheptyl 5-hydroxyundecanoate (3.5 g) as a light yellow oily substance.
$^1$H-NMR ($CDCl_3$) δ: 3.97 (2H, d, J = 5.7 Hz), 3.65-3.53 (1H, m), 2.35 (2H, t, J = 7.2 Hz), 1.87-1.20 (32H, m), 0.92-0.84 (9H, m).

1,1'-Carbonyldiimidazole (0.33 g) was added to a tetrahydrofuran (5.0 mL) solution of 2-pentylheptyl 5-hydroxyundecanoate (0.50 g), and the mixture was stirred at room temperature for 30 hours. Water (10 mL) and hexane (20 mL) were added to the reaction mixture, the organic layer was separated, then washed with water and a saturated aqueous sodium chloride solution, and dried over anhydrous sodium sulfate, and then the solvent was distilled away under reduced pressure, thereby obtaining 1-oxo-1-((2-pentylheptyl)oxy)undecane-5-yl 1H-imidazole-1-carboxylate (0.64 g) as a light yellow oily substance.

$^1$H-NMR ($CDCl_3$) δ: 4.73-4.59 (1H, m), 4.23-4.01 (6H, m), 2.90-2.76 (4H, m), 2.72-2.62 (2H, m), 2.58-2.45 (6H, m), 2.35-2.22 (4H, m), 1.69-1.47 (10H, m), 1.44-1.18 (45H, m), 1.02 (6H, t, J = 7.5 Hz), 0.96-0.80 (12H, m).

(2)

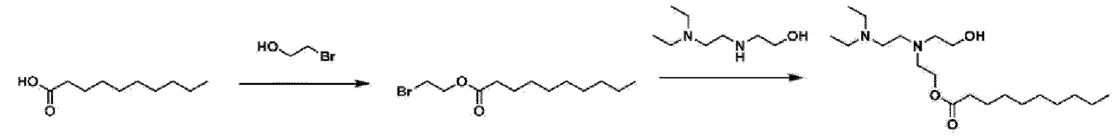

p-Toluenesulfonic acid monohydrate (0.41 g) was added to a mixture of decanoic acid (15.8 g), 2-bromoethanol (10.0 g), and toluene (30 mL), and the mixture was stirred at 130°C for 2 hours. The reaction mixture was cooled to room temperature, then water (100 mL) was added thereto, and the organic layer was separated. The organic layer was washed with a 10% aqueous sodium hydrogen carbonate solution (55 mL) and a saturated aqueous sodium chloride solution (50 mL), and dried over anhydrous sodium sulfate, then the solvent was distilled away under reduced pressure, thereby obtaining 2-bromoethyl decanoate (22.2 g) as light yellow oily substance.

$^1$H-NMR ($CDCl_3$) δ: 4.38 (2H, t, J = 6.4 Hz), 3.51 (2H, t, J = 6.4 Hz), 2.35 (2H, t, J = 7.6 Hz), 1.68-1.60 (2H, m), 1.35-1.20 (12H, m), 0.88 (3H, t, J = 7.2 Hz).

2-Bromoethyl decanoate (9.6 g) was added to a mixture of 2-((2-(diethylamino)ethyl)amino)ethane-1-ol (5.0 g), potassium carbonate (8.6 g) and acetonitrile (25 mL), and the mixture was stirred at 80°C for 1.5 hours. The reaction mixture was cooled to room temperature, then water (25 mL) and ethyl acetate (50 mL) were added thereto, the organic layer was separated, and the solvent was distilled away under reduced pressure. A 30% aqueous hydrochloric acid solution (12.5 mL), water (12.5 mL), and ethyl acetate (50 mL) were added to the obtained residue, and the aqueous layer was separated. A 10% aqueous sodium hydroxide solution (55 mL) was added to the obtained aqueous layer to adjust the pH to 10, then ethyl acetate was added thereto, the organic layer was separated and dried over anhydrous sodium sulfate. The solvent was distilled away under reduced pressure, then the obtained residue was purified by silica gel column chromatography (ethyl acetate-hexane, NH silica gel), thereby obtaining ethyl 2-((2-(diethylamino)ethyl)(2-hydroxyethyl)amino)decanoate (2.54 g) as a colorless oily substance.

$^1$H-NMR ($CDCl_3$) δ: 4.15 (2H, t, J = 6.0 Hz), 3.55-3.53 (2H, m), 2.84 (2H, t, J = 6.0 Hz), 2.71-2.64 (4H, m), 2.58-2.45 (6H, m), 2.30 (2H, t, J = 7.2 Hz), 1.64-1.57 (2H, m), 1.35-1.22 (12H, m), 1.03 (6H, t, J = 7.2 Hz), 0.88 (3H, t, J = 7.2 Hz).

(3)

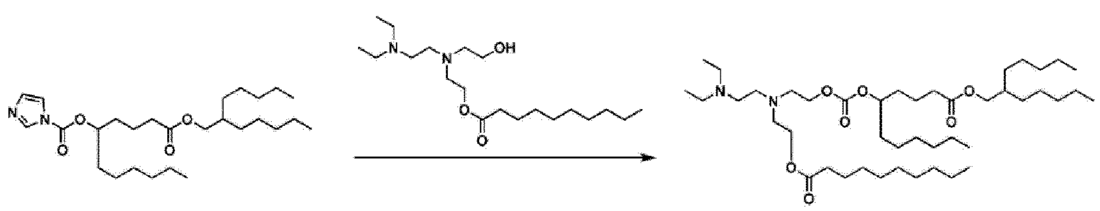

Potassium carbonate (0.20 g) was added to a mixture of 1-oxo-1-((2-pentylheptyl)oxy)undecane-5-yl 1H-imidazole-1-carboxylate (0.64 g), ethyl 2-((2-(diethylamino)ethyl)(2-hydroxyethyl)amino)decanoate (0.56 g) and acetonitrile (2.5 mL), and the mixture was stirred at 80°C for 2 hours. The reaction mixture was cooled to room temperature, then ethyl acetate (5 mL) was added thereto, the insoluble matters were filtered off, and the solvent was distilled away under reduced pressure. Ethyl acetate (20 mL) was added to the obtained residue, then the mixture was washed with water (10 mL) and a saturated aqueous sodium chloride solution, dried with anhydrous sodium sulfate, the solvent was distilled away under reduced pressure, and the obtained residue was purified by a silica gel column chromatography (methanol-ethyl acetate-hexane) thereby obtaining 2-pentylheptyl 6-(2-(decanoyloxy)ethyl)-3-ethyl-12-hexyl-10-oxo-9,11-dioxa-3,6-diazahexadecane-16-oate (0.68 g) as a colorless oily substance.

$^1$H-NMR ($CDCl_3$) δ: 4.72-4.65 (1H, m), 4.22-4.09 (4H, m), 3.97 (2H, d, J = 5.6 Hz), 2.86-2.77 (4H, m), 2.70-2.64 (2H, m), 2.55-2.48 (6H, m), 2.35-2.27 (4H, m), 1.71-1.55 (9H, m), 1.36-1.20 (36H, m), 1.02 (6H, t, J = 7.2 Hz), 0.94-0.82 (12H, m).
MS m/z (M + H): 755.

Test Example 1: Preparation of nucleic acid lipid particles and measurement of reporter protein knockdown rate in mice.

<Preparation of nucleic acid lipid particles>

The Compounds 24, 30, 31, 50, 56, 69, 88, 89, 103, 112, 118, 119, and 134 produced in the above Examples were used as a first lipid.

For comparison, a lipid having the following structure (Comparative Compound C) synthesized by the method described in WO2010/144740A was used.

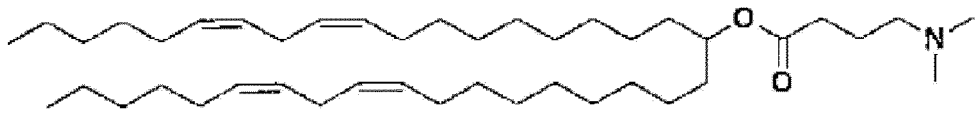

Furthermore, for comparison, a lipid having the following structure (Comparative Compound D) synthesized by the method described in WO2015/095340A was used.

Structure of the compound of Example 13 of WO2015/095340A.

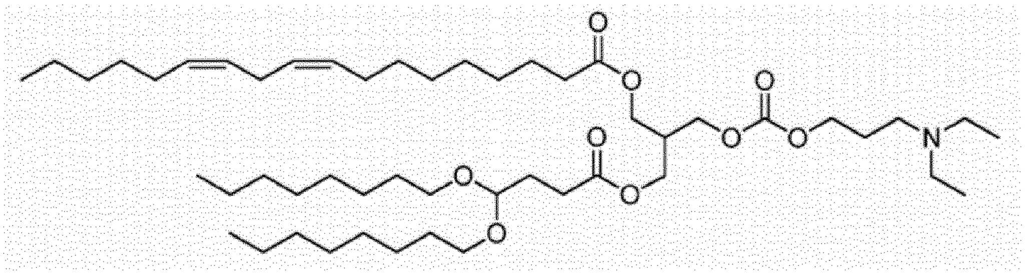

The above first lipid, 1,2-distearoyl-sn-glycero-3-phosphocholine (DSPC, trade name: COATSOME MC-8080; NOF corporation), cholesterol (trade name: Cholesterol HP; NIPPON FINE CHEMICAL CO., LTD.), DMG-PEG2000 (trade name: SUNBRIGHT(R) GM-020; NOF corporation) were dissolved in ethanol at the molar ratio shown in Table 1 such that the total lipid concentration was 20 mmol/L, thereby obtaining an oil phase.

5 mg of siFVII having the sequence on p. 878 of Molecular Therapy (2009) 17 was dissolved in 1 mL of sterile water, and diluted with 10 mmol/L acetate buffer having a pH 4 such that the nucleic acid concentration was 19.7 μmol/L, thereby obtaining a water phase. Then the water phase and the oil phase were mixed together with a micromixer (see JP5288254B) using a syringe pump such that the volume ratio of water phase:oil phase was 3:1, and the mixed solution was two-fold diluted with a phosphate buffered saline (PBS), thereby obtaining a nucleic acid lipid particle dispersion.

The ratio of the number of moles of the first lipid in the lipid composition to the number of moles of sterols in the lipid composition is shown in Table 1.

Table 1 also shows the weight ratio of the nucleic acid to the total lipids at the time of mixing.

[Table 1]

| | First lipid | Compositional ratio of lipid (mol%) | | | | Ratio of number of moles of first lipid to number of moles of sterols | weight ratio of nucleic acid to total lipids |
|---|---|---|---|---|---|---|---|
| | | First lipid | Phospholipid | Cholesterol | PEG lipid | | |
| | | | DSPC | | DME-PEG2000 | | |
| Comparative Examples 201 | Comparative Compound C | 50 | 10 | 38.5 | 1.5 | 1.299 | 6.7% |
| Comparative Examples 201 | Compound 24 | 50 | 10 | 38.5 | 1.5 | 1.299 | 6.3% |
| Comparative Examples 203 | Compound 30 | 50 | 10 | 38.5 | 1.5 | 1.299 | 6.2% |
| Comparative Examples 204 | Compound 31 | 50 | 10 | 38.5 | 1.5 | 1.299 | 6.3% |
| Examples 201 | Compound 24 | 50 | 0 | 48.5 | 1.5 | 1.031 | 7.2% |
| Examples 202 | Compound 30 | 50 | 0 | 48.5 | 1.5 | 1.031 | 7.1% |
| Examples 203 | Compound 31 | 50 | 0 | 48.5 | 1.5 | 1.031 | 7.1% |
| | | | | | | | |
| Comparative Examples 205 | Comparative Compound C | 50 | 10 | 38.5 | 1.5 | 1.299 | 6.7% |
| Comparative Examples 206 | Compound 50 | 50 | 10 | 38.5 | 1.5 | 1.299 | 6.3% |
| Examples 204 | Compound 50 | 50 | 0 | 48.5 | 1.5 | 1.031 | 6.8% |
| | | | | | | | |
| Examples 205 | Compound 56 | 50 | 0 | 48.5 | 1.5 | 1.031 | 5.7% |
| Examples 206 | Compound 88 | 50 | 0 | 48.5 | 1.5 | 1.031 | 6.3% |
| Examples 207 | Compound 89 | 50 | 0 | 48.5 | 1.5 | 1.031 | 6.7% |
| Examples 208 | Compound 103 | 50 | 0 | 48.5 | 1.5 | 1.031 | 6.2% |
| Examples 209 | Compound 112 | 50 | 0 | 48.5 | 1.5 | 1.031 | 6.8% |
| Examples 210 | Compound 118 | 50 | 0 | 48.5 | 1.5 | 1.031 | 5.5% |

(continued)

| | First lipid | Compositional ratio of lipid (mol%) | | | | Ratio of number of moles of first lipid to number of moles of sterols | weight ratio of nucleic acid to total lipids |
|---|---|---|---|---|---|---|---|
| | | First lipid | Phospholipid | Cholesterol | PEG lipid | | |
| | | | DSPC | | DME-PEG2000 | | |
| Examples 211 | Compound 119 | 50 | 0 | 48.5 | 1.5 | 1.031 | 6.0% |
| Examples 212 | Compound 69 | 50 | 0 | 48.5 | 1.5 | 1.031 | 6.7% |
| | | | | | | | |
| Examples 213 | Compound 88 | 40 | 0 | 58.5 | 1.5 | 0.684 | 6.8% |
| Examples 214 | Compound 88 | 50 | 0 | 48.5 | 1.5 | 1.031 | 6.3% |
| | | | | | | | |
| Examples 215 | Compound 88 | 40 | 0 | 59.5 | 0.5 | 0.672 | 7.0% |
| Examples 216 | Compound 88 | 50 | 0 | 48 | 2 | 1.042 | 6.2% |
| Examples 217 | Compound 89 | 45 | 0 | 53.5 | 1.5 | 0.841 | 6.9% |
| Examples 218 | Compound 56 | 50 | 0 | 48.5 | 1.5 | 1.031 | 5.4% |
| Examples 219 | Compound 88 | 50 | 0 | 48.5 | 1.5 | 1.031 | 6.1% |

<Measurement of particle size>

By using the dispersion liquid of lipid particle as it was, the particle size of the lipid particles was measured.
The measurement results are shown in Table 2.

<Evaluation of encapsulation rate of siRNA>

(Quantification of total nucleic acid concentration)

30 μL of 3 mol/L aqueous sodium acetate solution and 9 μL of glycogen were added to 60 μL of the lipid particles retaining nucleic acids, and then 1.5 mL of ethanol was added thereto, so that only the nucleic acids were precipitated while the lipid was dissolved. Then, the supernatant was removed by centrifugation. The precipitates were air-dried for 15 minutes or longer, then water was added thereto to redissolve the precipitates, and the concentration thereof was measured using Nanodrop NF1000 (Thermo Fisher Scientific), thereby quantifying the total nucleic acid concentration.

(Quantification of nucleic acid concentration in outer water phase)

The nucleic acid concentration was quantified using a Quant-iT RiboGreen RNA Assay Kit (Thermo Fisher Scientific) according to the protocol. First, a 20× TE buffer included in the above kit was diluted with water, thereby obtaining a 1× TE buffer. TE represents Tris/EDTA (ethylenediaminetetraacetic acid). In order to quantify only the nucleic acid in the outer water phase, the dispersion liquid of lipid particles retaining nucleic acids was 10,000-fold diluted with the 1× TE buffer.

100 μL of the 10,000-fold diluted dispersion liquid of lipid particles was put in a 96-well plate, then 100 μL of a RiboGreen reagent (reagent included in the Quanti-iT Ribogreen RNA Assay Kit described above) 2,000-fold diluted with the 1× TE buffer was added to a sample, and fluorescence (excitation wavelength: 485 nm, fluorescence wavelength: 535 nm) was measured using a plate reader Infinite F200 (TECAN), thereby quantifying the nucleic acid concentration in the outer water phase.

(Calculation of encapsulation rate)

By using the quantification results of the total nucleic acid concentration and the nucleic acid concentration in the outer water phase that were obtained through the above steps, the nucleic acid encapsulation rate of the nucleic acid lipid particles was calculated according to the following Equation.

$$\text{Nucleic acid encapsulation rate (\%)} = (\text{total nucleic acid concentration} - \text{nucleic acid concentration in outer water phase})/\text{total nucleic acid concentration} \times 100$$

The calculation results are shown in Table 2.

<Measurement of Factor VII (FVII) protein>

The Factor VII (FVII) protein was measured according to the method described in Nature Biotechnology (2010) 28, 172-176. C57BL6/J mice were randomly grouped (n = 3). The dispersion liquid of nucleic acid lipid particles prepared in <Preparation of nucleic acid lipid particles> was administered to the caudal vein of the mice at a dosage of 0.1 mg/kg. For comparison, the same volume of PBS was administered to the caudal vein of the mice. 24 hours after the administration, blood was collected from the caudal vena cava, thereby obtaining plasma. The amount of FVII protein was quantified by using the obtained plasma and Biophen FVII assay kit (ANIARA).

The FVII amount in the plasma sample of each individual in the PBS administration group was regarded as 100%, and the relative ratio of the FVII amount in the plasma sample of each individual was regarded as a measurement value. The results are shown in Table 2.

[Table 2]

| | Particle size (nm) | Encapsulation rate (%) | Relative amount of FVII protein (%) |
|---|---|---|---|
| Comparative Examples 201 | 65.9 | 74 | 30 |
| Comparative Examples 201 | 68.4 | 83 | 44 |
| Comparative Examples 203 | 65.9 | 82 | 35 |
| Comparative Examples 204 | 73.7 | 82 | 23 |
| Examples 201 | 79.1 | 81 | 18 |
| Examples 202 | 76.8 | 80 | 13 |
| Examples 203 | 76.8 | 74 | 10 |
| | | | |
| Comparative Examples 205 | 66.7 | 86 | 34 |
| Comparative Examples 206 | 66.2 | 82 | 23 |
| Examples 204 | 73.3 | 81 | 16 |
| | | | |
| Examples 205 | 81.9 | 64 | 1 |
| Examples 206 | 77.4 | 53 | 3 |
| Examples 207 | 74.5 | 64 | 5 |
| Examples 208 | 73.2 | 56 | 3 |
| Examples 209 | 74.6 | 74 | 4 |

(continued)

| | Particle size (nm) | Encapsulation rate (%) | Relative amount of FVII protein (%) |
|---|---|---|---|
| Examples 210 | 81.7 | 62 | 14 |
| Examples 211 | 72.5 | 65 | 4 |
| Examples 212 | 75.8 | 71 | 3 |
| | | | |
| Examples 213 | 70.7 | 93 | 5 |
| Examples 214 | 72.8 | 90 | 2 |
| | | | |
| Examples 215 | 151.1 | 47 | 16 |
| Examples 216 | 69.7 | 89 | 3 |
| Examples 217 | 74.3 | 93 | 9 |
| Examples 218 | 86.2 | 93 | 1 |
| Examples 219 | 79.5 | 86 | 4 |

In comparison with the nucleic acid lipid composition in Comparative Examples, the nucleic acid lipid composition according to the embodiment of the present invention showed a strong FVII inhibitory activity and an excellent nucleic acid delivery effect.

Test Example 2: Measurement of pharmacokinetic (PK) data (accumulation in liver).

<Preparation of nucleic acid lipid particles>

Compounds 56, 88, and 89 produced in the above Examples were used as the first lipid. The nucleic acid lipid particle dispersions shown in Table 3 was obtained in the same manner as in <Preparation of nucleic acid lipid particles> of Test Example 1.

The ratio of the number of moles of the first lipid in the lipid composition to the number of moles of sterols in the lipid composition is shown in Table 3.

Table 3 also show the weight ratio of the nucleic acid to the total lipids at the time of mixing.

[Table 3]

| | First lipid | Compositional ratio of lipid (mol%) | | | | Ratio of number of moles of first lipid to number of moles of sterols | weight ratio of nucleic acid to total lipids |
|---|---|---|---|---|---|---|---|
| | | First lipid | Phospholipid | Cholesterol | PEG lipid | | |
| | | | DSPC | | DME-PEG2000 | | |
| Comparative Examples 301 | Compound 56 | 50 | 10 | 38.5 | 1.5 | 1.299 | 5.4% |
| Examples 301 | Compound 56 | 50 | 0 | 48.5 | 1.5 | 1.031 | 5.7% |
| Comparative Examples 302 | Compound 88 | 50 | 10 | 38.5 | 1.5 | 1.299 | 5.9% |
| Examples 302 | Compound 88 | 50 | 0 | 48.5 | 1.5 | 1.031 | 6.3% |

(continued)

| | First lipid | Compositional ratio of lipid (mol%) | | | | Ratio of number of moles of first lipid to number of moles of sterols | weight ratio of nucleic acid to total lipids |
|---|---|---|---|---|---|---|---|
| | | First lipid | Phospholipid | Cholesterol | PEG lipid | | |
| | | | DSPC | | DME-PEG2000 | | |
| Comparative Examples 303 | Compound 89 | 50 | 10 | 38.5 | 1.5 | 1.299 | 6.3% |
| Examples 303 | Compound 89 | 50 | 0 | 48.5 | 1.5 | 1.031 | 6.7% |

<Measurement of particle size>

The particle size of the lipid particles was measured in the same manner as in Test Example 1.

The measurement results are shown in Table 4.

<Evaluation of encapsulation rate of siRNA>

The evaluation of the encapsulation rate of siRNA was performed in the same manner as in Test Example 1.

The calculation results are shown in Table 4.

<Measurement of accumulation in liver>

The dispersion liquid of nucleic acid lipid particles prepared in <Preparation of nucleic acid lipid particles> was administered to the caudal vein of the C57BL6/J mice at a dosage of 0.1 mg/kg. The liver was extracted 24 hours after administration, rapidly frozen with liquid nitrogen, and then freeze-pulverized with a multi-beads shocker. The extracted liver was thawed on ice, then 0.25% Triton-PBS was added thereto, thereby obtaining a liver homogenate. Reverse transcription was performed by using the obtained liver homogenate, Taqman MicroRNA Reverse Transcription kit (Applied Biosystems, 4366597), and Factor VII reverse transcription primer (Applied Biosystems). The reverse-transcribed sample was quantified by Real-time PCR using Taqman MGB gene expression kit (Applied Biosystems, 4324036), Taqman Universal PCR Master Mix No AmpErase UNG (Applied Biosystems, 4364341).

The measurement results are shown in Table 4.

[Table 4]

| | Particle size (nm) | Encapsulation rate (%) | Accumulation in liver |
|---|---|---|---|
| Comparative Examples 301 | 72.0 | 65 | 5.2 |
| Examples 301 | 81.9 | 64 | 6.3 |
| Comparative Examples 302 | 77.6 | 44 | 5.7 |
| Examples 302 | 77.4 | 53 | 9.0 |
| Comparative Examples 303 | 74.5 | 61 | 3.5 |
| Examples 303 | 74.5 | 64 | 3.9 |

In comparison with the nucleic acid lipid composition in Comparative Examples, the nucleic acid lipid composition according to the embodiment of the present invention showed a strong accumulation in the liver.

Test Example 3: Preparation of mRNA-encapsulating lipid particles and measurement of reporter protein expression rate in mice.

<Preparation of EPO mRNA-encapsulating lipid particles>

The compounds shown in Table 5, 1,2-distearoyl-sn-glycero-3-phosphocholine (trade name: COATSOME(R) MC-8080; NOF corporation), L-α-dioleoyl phosphatidylethanolamine (trade name: COATSOME(R) MC-8181; NOF corporation), cholesterol (trade name: Cholesterol HP; NIPPON FINE CHEMICAL CO., LTD.) and 1,2-dimyristoyl-rac-glycero-3-(methylpolyoxyethylene 2000) (hereinafter, referred to DMG-PEG2000) (trade name: SUNBRIGHT(R) GM-020; NOF corporation) were dissolved in ethanol at a molar ratio shown in Table 5 such that the total lipid concentration was 20 mmol/L, thereby obtaining an oil phase.

EPO mRNA (trade name: CleanCap EPO mRNA (5moU); TriLink) was diluted with 50 mmol/L citrate buffer having a pH 4 such that the weight ratio of total lipid concentration to mRNA concentration is about 16:1 to 64:1, obtaining a water phase. Then, the water phase and the oil phase were mixed together using NanoAssemblr (Precision NanoSystems) such that the volume ratio of water phase:oil phase was 3:1, and the mixed solution was 1.5-fold diluted with phosphate buffered saline (PBS), thereby obtaining a dispersion liquid of mRNA lipid particles. The dispersion liquid was dialyzed with a 10% aqueous sucrose solution using a dialysis cassette (Slide-A-Lyzer G2, MWCO: 10 kD, Thermo Fisher Scientific) to remove ethanol, thereby obtaining EPO mRNA-encapsulating lipid particles.

<Preparation of FLuc mRNA-encapsulating lipid particles>

The compounds shown in Table 6, 1,2-distearoyl-sn-glycero-3-phosphocholine (trade name: COATSOME(R) MC-8080; NOF corporation), L-α-dioleoyl phosphatidylethanolamine (trade name: COATSOME(R) MC-8181; NOF corporation), cholesterol (trade name: Cholesterol HP; NIPPON FINE CHEMICAL CO., LTD.) and 1,2-dimyristoyl-rac-glycero-3-(methylpolyoxyethylene 2000) (hereinafter, referred to DMG-PEG2000) (trade name: SUNBRIGHT(R) GM-020; NOF corporation) were dissolved in ethanol at a molar ratio shown in Table 6 such that the total lipid concentration was 20 mmol/L, thereby obtaining an oil phase.

FLuc mRNA (trade name: CleanCap FLuc mRNA (5 moU); TriLink) was diluted with 50 mmol/L citrate buffer having a pH 4 such that the weight ratio of total lipid concentration to mRNA concentration is about 19:1 to 64:1, obtaining a water phase. Then, the water phase and the oil phase were mixed together using NanoAssemblr (Precision NanoSystems) such that the volume ratio of water phase:oil phase was 3:1, and the mixed solution was 1.5-fold diluted with phosphate buffered saline (PBS), thereby obtaining a dispersion liquid of mRNA lipid particles. The dispersion liquid was dialyzed with a 10% aqueous sucrose solution using a dialysis cassette (Slide-A-Lyzer G2, MWCO: 10 kD, Thermo Fisher Scientific) to remove ethanol, thereby obtaining FLuc mRNA-encapsulating lipid particles.

[Table 5]

| | First lipid | Compositional ratio of lipid (mol%) | | | | | Ratio of number of moles of first lipid to number of moles of sterols | Mass ratio of nucleic acid to total lipids |
|---|---|---|---|---|---|---|---|---|
| | | First lipid | Phospholipid | | Cholesterol | PEG lipid | | |
| | | | DSPC | DOPE | | DME-PEG2000 | | |
| Comparative Examples 401 | Comparative Compound D | 45 | 9 | | 44 | 2 | 1.023 | 5.3% |
| Examples 401 | Compound 88 | 35 | | 30 | 34.5 | 0.5 | 1.014 | 1.6% |
| Examples 402 | Compound 56 | 35 | | 30 | 34.5 | 0.5 | 1.014 | 1.6% |
| | | | | | | | | |
| Comparative Examples 402 | Compound 88 | 50 | 45 | | 3.5 | 1.5 | 14.286 | 3.1% |

(continued)

| | First lipid | Compositional ratio of lipid (mol%) | | | | | Ratio of number of moles of first lipid to number of moles of sterols | Mass ratio of nucleic acid to total lipids |
|---|---|---|---|---|---|---|---|---|
| | | First lipid | Phospholipid | | Cholesterol | PEG lipid | | |
| | | | DSPC | DOPE | | DME-PEG2000 | | |
| Examples 403 | Compound 88 | 35 | | 30 | 33.5 | 1.5 | 1.045 | 1.6% |
| Examples 404 | Compound 88 | 25 | | 10 | 63.5 | 1.5 | 0.394 | 3.1% |
| Comparative Examples 403 | Compound 88 | 50 | | 45 | 3.5 | 1.5 | 14.286 | 6.3% |
| | | | | | | | | |
| Comparative Examples 404 | Compound 88 | 50 | 10 | | 38.5 | 1.5 | 1.299 | 1.6% |
| Examples 405 | Compound 88 | 35 | | 30 | 33.5 | 1.5 | 1.045 | 1.6% |
| Examples 406 | Compound 88 | 35 | | 10 | 53.5 | 1.5 | 0.654 | 3.1% |
| Examples 407 | Compound 88 | 35 | 0 | | 63.5 | 1.5 | 0.551 | 3.1% |
| Examples 408 | Compound 56 | 35 | | 30 | 33.5 | 1.5 | 1.045 | 1.6% |
| Examples 409 | Compound 56 | 35 | | 10 | 53.5 | 1.5 | 0.654 | 3.1% |
| Examples 410 | Compound 91 | 35 | | 10 | 53.5 | 1.5 | 0.654 | 3.1% |
| Examples 411 | Compound 93 | 35 | | 10 | 53.5 | 1.5 | 0.654 | 3.1% |
| | | | | | | | | |
| Examples 412 | Compound 134 | 35 | | 10 | 53.5 | 1.5 | 0.654 | 3.1% |
| Examples 413 | Compound 134 | 35 | 0 | | 63.5 | 1.5 | 0.551 | 3.1% |
| Comparative Examples 405 | Comparative Compound D | 45 | 9 | | 44 | 2 | 1.023 | 5.3% |
| Examples 414 | Compound 88 | 35 | 0 | | 63.5 | 1.5 | 0.551 | 3.1% |
| Examples 415 | Compound 88 | 35 | | 5 | 58.5 | 1.5 | 0.598 | 3.1% |

[Table 6]

| | First lipid | Compositional ratio of lipid (mol%) | | | | | Ratio of number of moles of first lipid to number of moles of sterols | Mass ratio of nucleic acid to total lipids |
|---|---|---|---|---|---|---|---|---|
| | | First lipid | Phospholipid | | Cholesterol | PEG lipid | | |
| | | | DSPC | DOPE | | DME-PEG2000 | | |
| Examples 501 | Compound 56 | 35 | | 30 | 34.5 | 0.5 | 1.014 | 1.6% |
| Examples 502 | Compound 88 | 35 | | 30 | 34.5 | 0.5 | 1.014 | 1.6% |
| Comparative Examples 501 | Comparative Compound D | 45 | 9 | | 44 | 2 | 1.023 | 5.3% |
| | | | | | | | | |
| Examples 503 | Compound 56 | 35 | | 30 | 34.5 | 0.5 | 1.014 | 1.6% |
| Examples 504 | Compound 56 | 35 | | 30 | 33.5 | 1.5 | 1.045 | 1.6% |
| Examples 505 | Compound 56 | 35 | | 30 | 32.5 | 2.5 | 1.077 | 1.6% |
| Examples 506 | Compound 56 | 35 | | 30 | 31.5 | 3.5 | 1.111 | 1.6% |
| Examples 507 | Compound 88 | 35 | | 30 | 34.5 | 0.5 | 1.014 | 1.6% |
| Examples 508 | Compound 88 | 35 | | 30 | 33.5 | 1.5 | 1.045 | 1.6% |
| Comparative Examples 502 | Comparative Compound D | 45 | 9 | | 44 | 2 | 1.023 | 5.3% |

<Measurement of particle size>

The particle size of the mRNA-encapsulating lipid particles was measured by 10-fold diluting the dispersion liquid of lipid particle with phosphate buffered saline (PBS) using Zeta-potential & Particle size Analyzer ELS-Z2 (Otsuka Electronics Co., Ltd.). The results are shown in Tables 7 and 8.

<Evaluation of encapsulation rate of mRNA>

(Quantification of total mRNA concentration)

15 to 30 μL of a 3 mol/L aqueous sodium acetate solution and 4.5 to 9 μL of glycogen were added to 30 to 60 μL of the lipid particles retaining mRNA, and 0.75 to 1.5 mL of ethanol was added thereto, so that only the nucleic acids were precipitated while the lipid was dissolved. Then, the supernatant was removed by centrifugation. The precipitates were air-dried for 15 minutes or longer, then water was added thereto to redissolve the precipitates, and the concentration thereof was measured using Nanodrop NF1000 (Thermo Fisher Scientific), thereby quantifying the total mRNA concentration.

(Quantification of mRNA concentration in outer water phase)

The mRNA concentration was quantified using a Quant-iT RiboGreen RNA Assay Kit (Thermo Fisher Scientific)

according to the protocol. First, a 20× TE buffer included in the above kit was diluted with water, thereby obtaining a 1× TE buffer. TE represents Tris/EDTA (ethylenediaminetetraacetic acid). In order to quantify only mRNA in the outer water phase, the dispersion liquid of lipid particles retaining mRNA was 10,000-fold diluted with the 1× TE buffer. 100 μL of the 10,000-fold diluted dispersion liquid of lipid particles was put in a 96-well plate, then 100 μL of a RiboGreen reagent (reagent included in the Quanti-iT Ribogreen RNA Assay Kit described above) 2,000-fold diluted with the 1× TE buffer was added to a sample, and fluorescence (excitation wavelength: 485 nm, fluorescence wavelength: 535 nm) was measured using a plate reader Infinite F200 (TECAN), thereby quantifying the mRNA concentration in the outer water phase.

(Calculation of encapsulation rate)

By using the quantification results of the total mRNA concentration and the mRNA concentration in the outer water phase that were obtained through the above steps, the mRNA encapsulation rate of the mRNA lipid particles was calculated according to the following Equation. The results are shown in Tables 7 and 8.

$$\text{mRNA encapsulation rate (\%)} = (\text{total mRNA concentration} - \text{mRNA concentration in outer water phase})/\text{total mRNA concentration} \times 100$$

<Measurement of EPO enzyme activity>

The dispersion liquid of mRNA lipid particles prepared in above <Preparation of EPO mRNA-encapsulating lipid particles> was intravenously administered to C57BL/6J mice at a mRNA dosage of 0.1 mg/kg. 20 to 24 hours after the administration, blood was collected from the caudal vena cava, thereby obtaining plasma. The human EPO enzyme activity was quantified by using the obtained plasma and ab119522 Erythropoietin (EPO) Human Elisa Kit (Abcam.plc).

The results are shown in Table 7.

<Luciferase luminescence measurement>

The dispersion liquid of mRNA lipid particles prepared in above <Preparation of FLuc mRNA-encapsulating lipid particles> was administered to the caudal vein of C57BL/6J mice at a dosage of 0.1 mg/kg. 5 hours after administration, D-luciferin potassium (Fujifilm Wako Pure Chemical Corporation) was intraperitoneally administered at a dosage of 150 mg/kg, euthanasia treatment by exsanguination was performed, and then the liver was collected. The luminescence intensity was quantified using IVIS Imaging System (PerkinElmer, Inc.).

The results are shown in Table 8. Luciferase [P/S] in Table 8 indicates photons/sec (light intensity).

[Table 7]

| | Particle size (nm) | Encapsulation rate (%) | EPO [mU/mL] |
|---|---|---|---|
| Comparative Examples 401 | 65.0 | 86 | 1100 |
| Examples 401 | 126.0 | 91 | 62717 |
| Examples 402 | 108.0 | 93 | 39146 |
| | | | |
| Comparative Examples 402 | 132.5 | 86 | - |
| Examples 403 | 69.0 | 90 | 3812 |
| Examples 404 | 85.9 | 91 | 11202 |
| Comparative Examples 403 | 111.3 | 90 | - |
| | | | |
| Comparative Examples 404 | 186.7 | 100 | 369 |
| Examples 405 | 70.9 | 100 | 6222 |
| Examples 406 | 78.0 | 100 | 29879 |

(continued)

| | Particle size (nm) | Encapsulation rate (%) | EPO [mU/mL] |
|---|---|---|---|
| Examples 407 | 134.0 | 100 | 64968 |
| Examples 408 | 67.7 | 93 | 31451 |
| Examples 409 | 59.9 | 91 | 25468 |
| Examples 410 | 64.4 | 93 | 4455 |
| Examples 411 | 84.1 | 95 | 2378 |
| | | | |
| Examples 412 | 79.1 | 91 | 3863 |
| Examples 413 | 86.8 | 98 | 21632 |
| Comparative Examples 405 | 60.3 | 77 | 852 |
| Examples 414 | 98.8 | 97 | 40213 |
| Examples 415 | 101.5 | 96 | 19728 |

[Table 8]

| | Particle size (nm) | Encapsulation rate (%) | Luciferase [P/S] |
|---|---|---|---|
| Examples 501 | 111.9 | 57 | 3.8.E+09 |
| Examples 502 | 139.3 | 73 | 1.3.E+09 |
| Comparative Examples 501 | 56.6 | 91 | 1.0.E+08 |
| | | | |
| Examples 503 | 116.7 | 82 | 2.9.E+09 |
| Examples 504 | 74.8 | 85 | 4.4.E+09 |
| Examples 505 | 59.3 | 81 | 9.0.E+08 |
| Examples 506 | 53.3 | 75 | 9.1.E+08 |
| Examples 507 | 134.4 | 91 | 2.9.E+09 |
| Examples 508 | 82.9 | 89 | 1.4.E+09 |
| Comparative Examples 502 | 139.2 | 76 | 6.9.E+08 |

In comparison with the nucleic acid lipid composition in Comparative Examples, the nucleic acid lipid composition according to the embodiment of the present invention showed a strong inhibitory activity on the reporter protein expression rate.

Test Example 4: Preparation of mRNA-encapsulating lipid particles and measurement of reporter protein expression rate in mice.

<Preparation of nucleic acid lipid particles>

Compounds 107, 129 and 135 produced in the above Examples were used as the first lipid.

<Preparation of EPO mRNA-encapsulating lipid particles>

The compounds shown in Table 9, 1,2-distearoyl-sn-glycero-3-phosphocholine (trade name: COATSOME(R) MC-8080; NOF corporation), 1,2-dioleoyl phosphatidylethanolamine (trade name: COATSOME(R) ME-8181; NOF corporation), cholesterol (trade name: Cholesterol HP; NIPPON FINE CHEMICAL CO., LTD.) and 1,2-dimyristoyl-rac-glycero-3-(methylpolyoxyethylene 2000) (hereinafter, referred to DMG-PEG2000) (trade name: SUNBRIGHT(R) GM-020; NOF corporation) were dissolved in ethanol at a molar ratio shown in Table 9 such that the total lipid concentration was 12.5 mmol/L, thereby obtaining an oil phase.

EPO mRNA (trade name: CleanCap EPO mRNA (5moU); TriLink) was mixed with 50 mmol/L citrate buffer having a pH 4 such that the weight ratio of total lipids to mRNA was about 16:1 to 64:1 after mixing with the oil phase, to dissolve mRNA with CA buffer, thereby obtaining a water phase. Then, the water phase and the oil phase were mixed together using NanoAssemblr (Precision NanoSystems) such that the volume ratio of water phase:oil phase was 3:1, and the mixed solution was 1.5-fold diluted with phosphate buffered saline (PBS), thereby obtaining a dispersion liquid of mRNA lipid particles. The dispersion liquid was dialyzed with 20 mM Tris buffer solution containing 8% sucrose using a dialysis cassette (Slide-A-Lyzer G2, MWCO: 10 kD, Thermo Fisher Scientific) to remove ethanol, thereby obtaining EPO mRNA-encapsulating lipid particles.

[Table 9]

| | First lipid | Compositional ratio of lipid (mol%) | | | | | Ratio of number of moles of first lipid to number of moles of sterols | Mass ratio of nucleic acid to total lipids |
|---|---|---|---|---|---|---|---|---|
| | | First lipid | Phospholipid | | Cholesterol | PEG lipid | | |
| | | | DSPC | DOPE | | DME-PEG2000 | | |
| Comparative Examples 601 | Comparative Compound D | 45 | 9 | | 44 | 2 | 1.023 | 5.3% |
| Examples 601 | Compound 107 | 45 | | 10 | 43.5 | 1.5 | 1.034 | 3.1% |
| Examples 602 | Compound 129 | 45 | | 10 | 43.5 | 1.5 | 1.034 | 3.1% |
| Examples 603 | Compound 135 | 45 | | 10 | 43.5 | 1.5 | 1.034 | 3.1% |

<Preparation of FLuc mRNA-encapsulating lipid particles>

The compounds shown in Table 10, 1,2-dioleoyl phosphatidylethanolamine (trade name: COATSOME(R) ME-8181; NOF corporation), 1,2-dioleoyl-sn-glycero-3-phosphocholine (hereinafter, referred to DOPC) (trade name: COATSOME(R) MC-8181; NOF corporation), cholesterol (trade name: Cholesterol HP; NIPPON FINE CHEMICAL CO., LTD.), and 1,2-dimyristoyl-rac-glycero-3-(methylpolyoxyethylene 2000) (hereinafter, called DMG-PEG2000) (trade name: SUNBRIGHT(R) GM-020; NOF corporation) were dissolved in ethanol at a molar ratio shown in Table 10 such that the total lipid concentration was 12.5 mmol/L, thereby obtaining an oil phase.

FLuc mRNA (trade name: CleanCap FLuc mRNA; TriLink) was mixed with 50 mmol/L citrate buffer having a pH 4 such that the weight ratio of total lipids to mRNA was about 16:1 to 64:1 after mixing with the oil phase, to dissolve mRNA with CA buffer, thereby obtaining a water phase. Then, the water phase and the oil phase were mixed together using NanoAssemblr (Precision NanoSystems) such that the volume ratio of water phase:oil phase was 3:1, and the mixed solution was 1.5-fold diluted with phosphate buffered saline (PBS), thereby obtaining a dispersion liquid of mRNA lipid particles. The dispersion liquid was dialyzed with 20 mM Tris buffer soluiton containing 8% sucrose using a dialysis cassette (Slide-A-Lyzer G2, MWCO: 10 kD, Thermo Fisher Scientific) to remove ethanol, thereby obtaining FLuc mRNA-encapsulating lipid particles.

[Table 10]

| | First lipid | Compositional ratio of lipid (mol%) | | | | | Ratio of number of moles of first lipid to number of moles of sterols | Mass ratio of nucleic acid to total lipids |
|---|---|---|---|---|---|---|---|---|
| | | First lipid | Phospholipid | | Cholesterol | PEG lipid | | |
| | | | DOPE | DOPC | | DME-PEG2000 | | |
| Examples 604 | Compound 107 | 45 | 10 | | 43.5 | 1.5 | 1.034 | 1.6% |
| Examples 605 | Compound 107 | 45 | 10 | | 43.5 | 1.5 | 1.034 | 5.0% |
| Examples 606 | Compound 135 | 45 | | 10 | 43.5 | 1.5 | 1.034 | 3.1% |
| Examples 607 | Compound 135 | 45 | | | 53.5 | 1.5 | 0.928 | 3.1% |
| Examples 608 | Compound 88 | 45 | 10 | | 43.5 | 1.5 | 1.034 | 1.6% |
| Examples 609 | Compound 88 | 40 | 10 | | 48.5 | 1.5 | 0.825 | 1.6% |
| Examples 610 | Compound 107 | 45 | 10 | | 43.5 | 1.5 | 1.034 | 3.1% |
| Examples 611 | Compound 107 | 45 | | 10 | 43.5 | 1.5 | 1.034 | 3.1% |
| Examples 612 | Compound 107 | 45 | | | 53.5 | 1.5 | 0.841 | 3.1% |
| Examples 613 | Compound 135 | 45 | 10 | | 43.5 | 1.5 | 1.034 | 3.1% |
| Examples 614 | Compound 135 | 40 | 10 | | 48.5 | 1.5 | 1.034 | 3.1% |
| Examples 615 | Compound 135 | 45 | | 10 | 43.5 | 1.5 | 1.034 | 3.1% |
| Examples 616 | Compound 135 | 45 | 10 | | 43.5 | 1.5 | 1.034 | 5.0% |
| Examples 617 | Compound 135 | 40 | 10 | | 48.5 | 1.5 | 1.034 | 5.0% |
| Examples 618 | Compound 135 | 45 | | 10 | 43.5 | 1.5 | 1.034 | 5.0% |

<Measurement of particle size>

The particle size of the mRNA-encapsulating lipid particles was measured by 5-fold diluting the dispersion liquid of lipid particle with phosphate buffered saline (PBS) using Zeta-potential & Particle size Analyzer ELS-Z2 (Otsuka Electronics Co., Ltd.). The results are shown in Tables 11 and 12.

<Evaluation of encapsulation rate of mRNA>

(Quantification of total mRNA concentration)

900 μL of methanol was added to 100 μL of mRNA and lipid particles encapsulating mRNA to dissolve the

lipid, and the absorbance at 260 nm was measured using an absorbance meter (Thermo Fisher Scientific), thereby quantifying the total mRNA concentration.

(Quantification of mRNA concentration in outer water phase)

The mRNA concentration was quantified using a Quant-iT RiboGreen RNA Assay Kit (Thermo Fisher Scientific) according to the protocol. First, a 20× TE buffer included in the above kit was diluted with water, thereby obtaining a 1× TE buffer. TE represents Tris/EDTA (ethylenediaminetetraacetic acid). In order to quantify only mRNA in the outer water phase, the dispersion liquid of lipid particles retaining mRNA was 50-fold diluted with the 1× TE buffer. 100 μL of the 50-fold diluted dispersion liquid of lipid particles was put in a 96-well plate, then 100 μL of a RiboGreen reagent (reagent included in the Quanti-iT Ribogreen RNA Assay Kit described above) 2,000-fold diluted with the 1× TE buffer was added to a sample, and fluorescence (excitation wavelength: 485 nm, fluorescence wavelength: 535 nm) was measured using a plate reader Infinit F200 (TECAN), thereby quantifying the mRNA concentration in the outer water phase.

(Calculation of encapsulation rate)

By using the quantification results of the total mRNA concentration and the mRNA concentration in the outer water phase that were obtained through the above steps, the mRNA encapsulation rate of the mRNA lipid particles was calculated according to the following Equation. The results are shown in Tables 11 and 12.

$$\text{mRNA encapsulation rate (\%)} = (\text{total mRNA concentration-mRNA concentration in outer water phase})/\text{total mRNA concentration} \times 100$$

<Measurement of EPO enzyme activity>

The dispersion liquid of mRNA lipid particles prepared in above <Preparation of EPO mRNA-encapsulating lipid particles> was intravenously administered to ICR mice at a mRNA dosage of 0.1 mg/kg. 6 hours after the administration, blood was collected from the caudal vena cava, thereby obtaining plasma. The human EPO enzyme activity was quantified by using the obtained plasma and ab119522 Erythropoietin (EPO) Human Elisa Kit (Abcam.plc).

The results are shown in Table 11.

[Table 11]

| | Particle size (nm) | Encapsulation rate (%) | EPO [mU/mL] |
|---|---|---|---|
| Comparative Examples 601 | 97.6 | 93.2 | 1.02.E+05 |
| Examples 601 | 88.9 | 98.1 | 2.69.E+05 |
| Examples 602 | 90.2 | 99.5 | 2.53.E+05 |
| Examples 603 | 82.5 | 99.6 | 6.49.E+05 |
| Examples 604 | 109.6 | 99.7 | 6.10.E+05 |
| Examples 605 | 90.8 | 99.6 | 3.90.E+05 |
| Examples 606 | 118.3 | 99.3 | 4.17.E+05 |
| Examples 607 | 106.2 | 97.5 | 5.30.E+05 |

In comparison with the nucleic acid lipid composition in Comparative Examples, the nucleic acid lipid composition according to the embodiment of the present invention showed a higher reporter protein expression rate.

<Luciferase luminescence measurement>

The dispersion liquid of mRNA lipid particles prepared in above <Preparation of FLuc mRNA-encapsulating lipid particles> was singly administered to the rectus femoris from the dorsal side of ICR mice at a mRNA dosage of 1 μg. 5 hours and 50 minutes after administration, D-luciferin potassium (Fujifilm Wako Pure Chemical Corporation) was

intraperitoneally administered at a dosage of 150 mg/kg, and 6 hours after administration, luminescence was measured using IVIS Imaging System (PerkinElmer, Inc.) in the prone position under isoflurane gas anesthesia. The ROI was set such that the entire lower limbs on the administered side were included, and the amount of luminescence (Photons/Sec) was quantified using Living Image Software (PerkinElmer, Inc.).

The results are shown in Table 12.

[Table 12]

| | Particle size (nm) | Encapsulation rate (%) | Total flux (p/s) |
|---|---|---|---|
| Examples 608 | 83.9 | 100 | 3.79E+08 |
| Examples 609 | 83.5 | 100 | 4.60E+08 |
| Examples 610 | 82.9 | 100 | 1.69E+09 |
| Examples 611 | 126.4 | 98.8 | 1.08E+09 |
| Examples 612 | 114.8 | 97.5 | 1.50E+09 |
| Examples 613 | 85.9 | 99.7 | 7.74.E+08 |
| Examples 614 | 83.7 | 99.4 | 8.62.E+08 |
| Examples 615 | 148.6 | 98.5 | 7.03.E+08 |
| Examples 616 | 93.6 | 98.8 | 1.21.E+09 |
| Examples 617 | 86.6 | 98.4 | 1.08.E+09 |
| Examples 618 | 148.3 | 97.6 | 3.96.E+08 |

In comparison with the nucleic acid lipid composition in Comparative Examples, the nucleic acid lipid composition according to the embodiment of the present invention showed better luminescence.

## Claims

**1.** A lipid composition comprising:

a first lipid which is a lipid represented by Formula (1) or a salt thereof;
sterols; and
nucleic acid,
wherein a ratio of the number of moles of the first lipid in the lipid composition to the number of moles of the sterols in the lipid composition is 0.300 or more and less than 1.299,

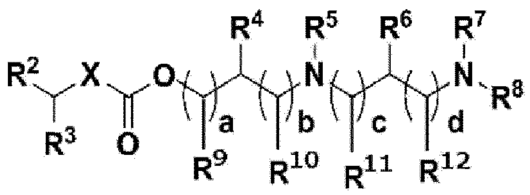

(1)

in the formula, X represents $-NR^1-$ or -O-,
$R^1$ represents a hydrogen atom, a hydrocarbon group having 6 to 24 carbon atoms, or a group represented by $R^{21}-L^1-R^{22}-$, where $R^{21}$ represents a hydrocarbon group having 1 to 24 carbon atoms, $L^1$ represents -O(CO)O-, -O(CO)-, -(CO)O-, -O-, or a group represented by the following formula,

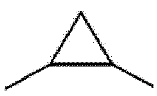

, and $R^{22}$ represents a divalent hydrocarbon linking group having 1 to 18 carbon atoms,

$R^2$ and $R^3$ each independently represent a hydrogen atom, a hydrocarbon group having 3 to 24 carbon atoms, or a group represented by $R^{31}$-$L^2$-$R^{32}$-, where $R^{31}$ represents a hydrocarbon group having 1 to 24 carbon atoms, $L^2$ represents -O(CO)O-, -O(CO)-, -(CO)O-, -O-, or a group represented by the following formula,

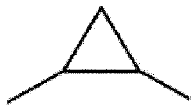

, and $R^{32}$ represents a divalent hydrocarbon linking group having 1 to 18 carbon atoms,
$R^4$, $R^5$, $R^6$, $R^7$, $R^8$, $R^9$, $R^{10}$, $R^{11}$, and $R^{12}$ each independently represent a hydrogen atom or an alkyl group having 1 to 18 carbon atoms which may be substituted,
groups in any one or more pairs among $R^4$ and $R^5$, $R^{10}$ and $R^5$, $R^5$ and $R^{12}$, $R^4$ and $R^6$, $R^5$ and $R^6$, $R^6$ and $R^7$, $R^6$ and $R^{10}$, $R^{12}$ and $R^7$, and $R^7$ and $R^8$ may be linked to each other to form a 4- to 7-membered ring which may contain an O atom,
a substituent on the alkyl group having 1 to 18 carbon atoms which may be substituted is a hydroxyl group, a carboxyl group, an amino group represented by $-NR^{45}R^{46}$, a substituted or unsubstituted aryl group, a substituted or unsubstituted heteroaryl group, or a group represented by -O(CO)O-$R^{41}$, -O(CO)-$R^{42}$, -(CO)O-$R^{43}$, or -O-$R^{44}$, where $R^{41}$, $R^{42}$, $R^{43}$, $R^{44}$, $R^{45}$, and $R^{46}$ each independently represent a hydrocarbon group having 1 to 18 carbon atoms,
a substituent on the substituted or unsubstituted aryl group and on the substituted or unsubstituted heteroaryl group is an alkyl group having 1 to 18 carbon atoms, a hydroxyl group, a carboxyl group, an amino group represented by $-NR^{45}R^{46}$, or a group represented by -O(CO)O-$R^{41}$, -O(CO)-$R^{42}$, -(CO)O-$R^{43}$, or -O-$R^{44}$, where $R^{41}$, $R^{42}$, $R^{43}$, $R^{44}$, $R^{45}$, and $R^{46}$ each independently represent a hydrocarbon group having 1 to 18 carbon atoms, and
a, b, c, and d each independently represent an integer of 0 to 3, a + b is 1 or more, and c + d is 1 or more.

**2.** The lipid composition according to claim 1,
wherein the sterols are cholesterol or a derivative thereof.

**3.** The lipid composition according to claim 1 or 2,
further comprising a lipid having a nonionic hydrophilic polymer structure.

**4.** The lipid composition according to claim 3,
wherein the lipid having a nonionic hydrophilic polymer structure is a lipid having a polyethylene glycol structure.

**5.** The lipid composition according to claim 4,
wherein the lipid having a polyethylene glycol structure is a lipid having a diacylglycerol structure and a polyethylene glycol structure.

**6.** The lipid composition according to any one of claims 3 to 5,
wherein a content of the lipid having a nonionic hydrophilic polymer structure with respect to a total lipids is 0.2 to 10 mol%.

**7.** The lipid composition according to any one of claims 1 to 6,
wherein a content of the first lipid with respect to a total lipids is 20 to 55 mol%.

**8.** The lipid composition according to any one of claims 1 to 7,
wherein a content of the sterols with respect to a total lipids is 20 to 70 mol%.

**9.** The lipid composition according to any one of claims 1 to 8,
wherein a content of the nucleic acid with respect to a total lipids is 1% to 25% by mass.

**10.** The lipid composition according to any one of claims 1 to 9,

wherein the compound represented by Formula (1) is a compound represented by Formula (2)

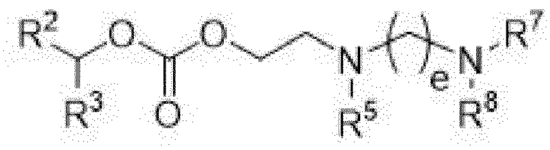 (2)

in the formula, $R^2$ and $R^3$ each independently represent a hydrogen atom or a hydrocarbon group having 3 to 24 carbon atoms, or a group represented by $-R^{31}-L^2-R^{32}-$, where $R^{31}$ represents a hydrocarbon group having 1 to 24 carbon atoms, $L^2$ represents -O(CO)O-, -O(CO)-, -(CO)O-, -O-, or a group represented by the following formula,

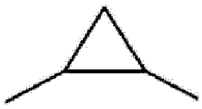

, and $R^{32}$ represents a divalent hydrocarbon linking group having 1 to 18 carbon atoms,
$R^5$ represents a hydrogen atom or an alkyl group having 1 to 18 carbon atoms which may be substituted,
$R^7$ and $R^8$ each independently represent a hydrogen atom or an alkyl group having 1 to 18 carbon atoms which may be substituted,
a substituent on the alkyl group having 1 to 18 carbon atoms which may be substituted is a hydroxyl group, a carboxyl group, an amino group represented by $-NR^{45}R^{46}$, a substituted or unsubstituted aryl group, a substituted or unsubstituted heteroaryl group, or a group represented by $-O(CO)O-R^{41}$, $-O(CO)-R^{42}$, $-(CO)O-R^{43}$, or $-O-R^{44}$, where $R^{41}$, $R^{42}$, $R^{43}$, $R^{44}$, $R^{45}$, and $R^{46}$ each independently represent a hydrocarbon group having 1 to 18 carbon atoms,
a substituent on the substituted or unsubstituted aryl group and on the substituted or unsubstituted heteroaryl group is an alkyl group having 1 to 18 carbon atoms, a hydroxyl group, a carboxyl group, an amino group represented by $-NR^{45}R^{46}$, or a group represented by $-O(CO)O-R^{41}$, $-O(CO)-R^{42}$, $-(CO)O-R^{43}$, or $-O-R^{44}$, where $R^{41}$, $R^{42}$, $R^{43}$, $R^{44}$, $R^{45}$, and $R^{46}$ each independently represent a hydrocarbon group having 1 to 18 carbon atoms, and
e represents 2 or 3.

**11.** The lipid composition according to claim 10,

wherein in Formula (2),
at least one of $R^2$ or $R^3$ represents a hydrocarbon group with 3 to 24 carbon atoms containing one or more unsaturated bonds, $R^2$ and $R^3$ each independently represent a group represented by $R^{31}-L^2-R^{32}-$, or one of $R^2$ and $R^3$ represents a group represented by $R^{31}-L^2-R^{32}-$ and the other represents a hydrocarbon group having 3 to 24 carbon atoms;
$R^5$ represents an unsubstituted alkyl group having 1 to 18 carbon atoms or an alkyl group having 1 to 18 carbon atoms substituted with $-O(CO)-R^{42}$ or $-(CO)O-R^{43}$;
$R^7$ and $R^8$ each independently represent an alkyl group having 1 to 4 carbon atoms; and
$R^{31}$, $L^2$, $R^{32}$, $R^{42}$, and $R^{43}$ have the same definitions as $R^{31}$, $L^2$, $R^{32}$, $R^{42}$, and $R^{43}$ in claim 10.

**12.** The lipid composition according to any one of claims 1 to 11, further comprising a pharmaceutically acceptable carrier.

**13.** The lipid composition according to any one of claims 1 to 12,
wherein the lipid composition is a composition for introducing nucleic acids into cells.

**14.** The lipid composition according to any one of claims 1 to 12,
wherein the lipid composition is a composition for in vivo delivery of nucleic acids.

## Patentansprüche

**1.** Lipidzusammensetzung umfassend:

ein erstes Lipid, das ein durch Formel (1) dargestelltes Lipid oder ein Salz davon ist;

Sterole; und
Nukleinsäure,
wobei ein Verhältnis der Molanzahl des ersten Lipids in der Lipidzusammensetzung zu der Molanzahl der Sterole in der Lipidzusammensetzung 0,300 oder mehr und weniger als 1,299 ist,

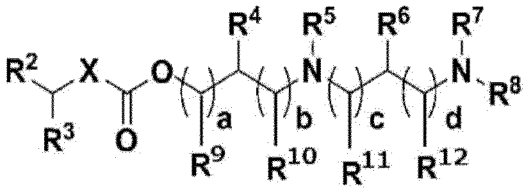 (1)

wobei in der Formel X $-NR^1-$ oder -O- darstellt,
$R^1$ ein Wasserstoffatom, eine Kohlenwasserstoffgruppe mit 6 bis 24 Kohlenstoffatomen oder eine durch $R^{21}-L^1-R^{22}-$ dargestellte Gruppe darstellt, wobei $R^{21}$ eine Kohlenwasserstoffgruppe mit 1 bis 24 Kohlenstoffatomen darstellt, $L^1$ -O(CO)O-, -O(CO)-, -(CO)O-, -O- oder eine durch die folgende Formel dargestellte Gruppe darstellt,

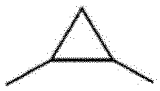

und $R^{22}$ eine divalente Kohlenwasserstoffverbindungsgruppe mit 1 bis 18 Kohlenstoffatomen darstellt,
$R^2$ und $R^3$ jeweils unabhängig ein Wasserstoffatom, eine Kohlenwasserstoffgruppe mit 3 bis 24 Kohlenstoffatomen oder eine durch $R^{31}-L^2-R^{32}-$ dargestellte Gruppe darstellen, wobei $R^{31}$ eine Kohlenwasserstoffgruppe mit 1 bis 24 Kohlenstoffatomen darstellt, $L^2$ -O(CO)O-, -O(CO)-, -(CO)O-, -O- oder eine durch die folgende Formel dargestellte Gruppe darstellt,

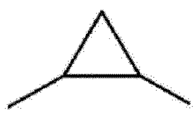

und $R^{32}$ eine divalente Kohlenwasserstoffverbindungsgruppe mit 1 bis 18 Kohlenstoffatomen darstellt,
$R^4$, $R^5$, $R^6$, $R^7$, $R^8$, $R^9$, $R^{10}$, $R^{11}$ und $R^{12}$ jeweils unabhängig ein Wasserstoffatom oder eine Alkylgruppe mit 1 bis 18 Kohlenstoffatomen darstellen, die substituiert sein können,
wobei Gruppen in jedem oder mehreren Paaren aus $R^4$ und $R^5$, $R^{10}$ und $R^5$, $R^5$ und $R^{12}$, $R^4$ und $R^6$, $R^5$ und $R^6$, $R^6$ und $R^7$, $R^6$ und $R^{10}$, $R^{12}$ und $R^7$ und $R^7$ und $R^8$ miteinander verbunden sein können, um einen 4- bis 7-gliedrigen Ring zu bilden, der ein O-Atom enthalten kann,
ein Substituent in der Alkylgruppe mit 1 bis 18 Kohlenstoffatomen, die substituiert sein kann, eine Hydroxygruppe, eine, eine Carboxygruppe, eine durch $NR^{45}R^{46}$ dargestellte Amingruppe, eine substituierte oder unsubstituierte Arylgruppe, eine substituierte oder unsubstituierte Heteroarylgruppe oder eine durch $-O(CO)O-R^{41}$, $-O(CO)-R^{42}$, $-(CO)O-R^{43}$ oder $-O-R^{44}$ dargestellte Gruppe ist, wobei $R^{41}$, $R^{42}$, $R^{43}$, $R^{44}$, $R^{45}$ und $R^{46}$ jeweils unabhängig eine Kohlenwasserstoffgruppe mit 1 bis 18 Kohlenstoffatomen darstellen,
ein Substituent in der substituierten oder unsubstituierten Arylgruppe und in der substituierten oder unsubstituierten Heteroarylgruppe eine Alkylgruppe mit 1 bis 18 Kohlenstoffatomen, eine Hydroxygruppe, eine Carboxygruppe, eine durch $-NR^{45}R^{46}$ dargestellte Amingruppe oder eine durch $-O(CO)O-R^{41}$, $-O(CO)-R^{42}$, $-(CO)O-R^{43}$ oder $-O-R^{44}$ dargestellte Gruppe ist, wobei $R^{41}$, $R^{42}$, $R^{43}$, $R^{44}$, $R^{45}$ und $R^{46}$ jeweils unabhängig eine Kohlenwasserstoffgruppe mit 1 bis 18 Kohlenstoffatomen darstellen und
a, b, c und d jeweils unabhängig eine ganze Zahl von 0 bis 3 sind, a + b 1 oder mehr ist und c + d 1 oder mehr ist.

2. Lipidzusammensetzung nach Anspruch 1,
wobei die Sterole Cholesterol oder ein Derivat davon sind.

3. Lipidzusammensetzung nach Anspruch 1 oder 2,
ferner umfassend ein Lipid mit einer nichtionischen hydrophilen Polymerstruktur.

**4.** Lipidzusammensetzung nach Anspruch 3, wobei das Lipid mit einer nichtionischen hydrophilen Polymerstruktur ein Lipid mit einer Polyethylenglykolstruktur ist.

**5.** Lipidzusammensetzung nach Anspruch 4, wobei das Lipid mit einer Polyethylenglykolstruktur ein Lipid mit einer Diacylglycerinstruktur und einer Polyethylenglykolstruktur ist.

**6.** Lipidzusammensetzung nach einem der Ansprüche 3 bis 5, wobei ein Gehalt an dem Lipid mit einer nichtionischen hydrophilen Polymerstruktur, bezogen auf die gesamten Lipide 0,2 bis 10 Mol% beträgt.

**7.** Lipidzusammensetzung nach einem der Ansprüche 1 bis 6, wobei ein Gehalt an dem ersten Lipid, bezogen auf die gesamten Lipide 20 bis 55 Mol% beträgt.

**8.** Lipidzusammensetzung nach einem der Ansprüche 1 bis 7, wobei ein Gehalt an den Sterolen, bezogen auf die gesamten Lipide 20 bis 70 Mol% beträgt.

**9.** Lipidzusammensetzung nach einem der Ansprüche 1 bis 8, wobei ein Gehalt an der Nukleinsäure, bezogen auf die gesamten Lipide 1 Masse% bis 25 Masse% beträgt.

**10.** Lipidzusammensetzung nach einem der Ansprüche 1 bis 9,

wobei die durch Formel (1) dargestellte Verbindung eine durch Formel (2) dargestellte Verbindung ist

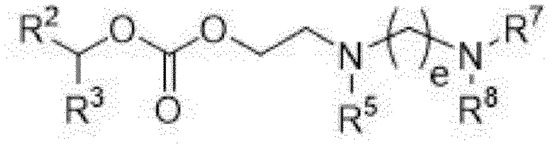

(2)

wobei in der Formel $R^2$ und $R^3$ jeweils unabhängig ein Wasserstoffatom oder eine Kohlenwasserstoffgruppe mit 3 bis 24 Kohlenstoffatomen oder eine durch $-R^{31}-L^2-R^{32}$-dargestellte Gruppe darstellen,
wobei $R^{31}$ eine Kohlenwasserstoffgruppe mit 1 bis 24 Kohlenstoffatomen darstellt,
$L^2$ -O(CO)O-, -O(CO)-, -(CO)O-, -O- oder eine durch die folgende Formel dargestellte Gruppe darstellt,

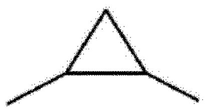

und $R^{32}$ eine divalente Kohlenwasserstoffverbindungsgruppe mit 1 bis 18 Kohlenstoffatomen darstellt,
$R^5$ ein Wasserstoffatom oder eine Alkylgruppe mit 1 bis 18 Kohlenstoffatomen darstellt, die substituiert sein kann,
$R^7$ und $R^8$ jeweils unabhängig ein Wasserstoffatom oder eine Alkylgruppe mit 1 bis 18 Kohlenstoffatomen darstellen, die substituiert sein kann,
ein Substituent in der Alkylgruppe mit 1 bis 18 Kohlenstoffatomen, die substituiert sein kann, eine Hydroxygruppe, eine Carboxygruppe, eine durch $-NR^{45}R^{46}$ dargestellte Amingruppe, eine substituierte oder unsubstituierte Arylgruppe, eine substituierte oder unsubstituierte Heteroarylgruppe oder eine durch $-O(CO)O-R^{41}$, $-O(CO)-R^{42}$, $-(CO)O-R^{43}$ oder $-O-R^{44}$ dargestellte Gruppe ist, wobei $R^{41}$, $R^{42}$, $R^{43}$, $R^{44}$, $R^{45}$ und $R^{46}$ jeweils unabhängig eine Kohlenwasserstoffgruppe mit 1 bis 18 Kohlenstoffatomen darstellen,
ein Substituent in der substituierten oder unsubstituierten Arylgruppe und in der substituierten oder unsubstituierten Heteroarylgruppe eine Alkylgruppe mit 1 bis 18 Kohlenstoffatomen, eine Hydroxygruppe, eine Carboxygruppe, eine durch $-NR^{45}R^{46}$ dargestellte Amingruppe oder eine durch $-O(CO)O-R^{41}$, $-O(CO)-R^{42}$, $-(CO)O-R^{43}$ oder $-O-R^{44}$ dargestellte Gruppe ist, wobei $R^{41}$, $R^{42}$, $R^{43}$, $R^{44}$, $R^{45}$ und $R^{46}$ jeweils unabhängig eine Kohlenwasserstoffgruppe mit 1 bis 18 Kohlenstoffatomen darstellen und
e 2 oder 3 darstellt.

**11.** Lipidzusammensetzung nach Anspruch 10,

wobei in Formel (2),
mindestens eines aus $R^2$ und $R^3$ eine Kohlenwasserstoffgruppe mit 3 bis 24 Kohlenstoffatomen, die eine oder mehrere ungesättigte Bindungen enthält, ist, $R^2$ und $R^3$ jeweils unabhängig eine durch $R^{31}$-$L^2$-$R^{32}$- dargestellte Gruppe darstellen oder eines aus $R^2$ und $R^3$ eine durch $R^{31}$-$L^2$-$R^{32}$- dargestellte Gruppe darstellt und das andere eine Kohlenwasserstoffgruppe mit 3 bis 24 Kohlenstoffatomen darstellt;
$R^5$ eine unsubstituierte Alkylgruppe mit 1 bis 18 Kohlenstoffatomen oder eine mit -O(CO)-$R^{42}$ oder -(CO)O-$R^{43}$ substituierte Alkylgruppe mit 1 bis 18 Kohlenstoffatomen ist;
$R^7$ und $R^8$ jeweils unabhängig eine Alkylgruppe mit 1 bis 4 Kohlenstoffatomen darstellen; und
$R^{31}$, $L^2$, $R^{32}$, $R^{42}$ und $R^{43}$ dieselben Definitionen haben wie $R^{31}$, $L^2$, $R^{32}$, $R^{42}$ und $R^{43}$ in Anspruch 10.

**12.** Lipidzusammensetzung nach einem der Ansprüche 1 bis 11, ferner umfassend einen pharmazeutisch annehmbaren Träger.

**13.** Lipidzusammensetzung nach einem der Ansprüche 1 bis 12,
wobei die Lipidzusammensetzung eine Zusammensetzung zum Einbringen von Nukleinsäuren in Zellen ist.

**14.** Lipidzusammensetzung nach einem der Ansprüche 1 bis 12,
wobei die Lipidzusammensetzung eine Zusammensetzung zum in vivo Transport von Nukleinsäuren ist.

**Revendications**

**1.** Composition lipidique, comprenant :

un premier lipide, lequel est un lipide représenté par la Formule (1) ou un sel de celui-ci ;
des stérols, et
un acide nucléique,
dans laquelle un rapport entre le nombre de moles du premier lipide dans la composition lipidique et le nombre de moles dans les stérols dans la composition lipidique est supérieur ou égal à 0,300 et inférieur à 1299,

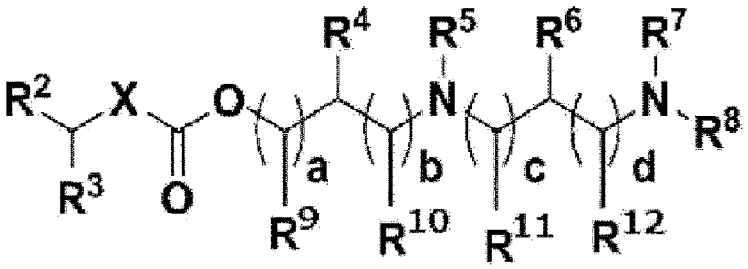

(1)

dans la formule, X représente -N$R^1$- ou -O- ;
$R^1$ représente un atome d'hydrogène, un groupe hydrocarboné présentant de 6 à 24 atomes de carbone, ou un groupe représenté par $R^{21}$-$L^1$-$R^{22}$- ; où $R^{21}$ représente un groupe hydrocarboné présentant de 1 à 24 atomes de carbone ; $L^1$ représente -O(CO)O-, -O(CO)-,-(CO)O-, -O-, ou un groupe représenté par la formule suivante,

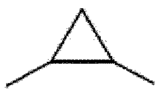

et
$R^{22}$ représente un groupe de liaison hydrocarboné divalent présentant de 1 à 18 atomes de carbone ;
$R^2$ et $R^3$ représentent chacun indépendamment un atome d'hydrogène, un groupe hydrocarboné présentant de 3 à 24 atomes de carbone, ou un groupe représenté par $R^{31}$-$L^2$-$R^{32}$- ; où $R^{31}$ représente un groupe hydrocarboné présentant de 1 à 24 atomes de carbone ; $L^2$ représente -O(CO)O-, -O(CO)-, -(CO)O-, -O-, ou un groupe représenté par la formule suivante,

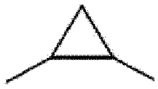

, et

$R^{32}$ représente un groupe de liaison hydrocarboné divalent présentant de 1 à 18 atomes de carbone ; $R^4$, $R^5$, $R^6$, $R^7$, $R^8$, $R^9$, $R^{10}$, $R^{11}$ et $R^{12}$ représentent chacun indépendamment un atome d'hydrogène, ou un groupe alkyle présentant de 1 à 18 atomes de carbone pouvant être substitués ;

des groupes dans l'une ou plusieurs quelconques paires parmi $R^4$ et $R^5$, $R^{10}$ et $R^5$, $R^5$ et $R^{12}$, $R^4$ et $R^6$, $R^5$ et $R^6$, $R^6$ et $R^7$, $R^6$ et $R^{10}$, $R^{12}$ et $R^7$, et $R^7$ et $R^8$ peuvent être liés entre eux pour former un cycle de 4 à 7 chaînons, lequel peut contenir un atome O ;

un substituant sur le groupe alkyle présentant de 1 à 18 atomes de carbone, lesquels peuvent être substitués, est un groupe hydroxyle, un groupe carboxyle, un groupe amino représenté par $-NR^{45}R^{46}$, un groupe aryle substitué ou non substitué, un groupe hétéroaryle substitué ou non substitué, ou un groupe représenté par $-O(CO)O-R^{41}$, $-O(CO)-R^{42}$, $-(CO)O-R^{43}$, ou $-O-R^{44}$, où $R^{41}$, $R^{42}$, $R^{43}$, $R^{44}$, $R^{45}$, et $R^{46}$ représentent chacun indépendamment un groupe hydrocarboné représentant de 1 à 18 atomes de carbone ;

un substituant sur le groupe aryle substitué ou non substitué et sur le groupe hétéroaryle substitué ou non substitué est un groupe alkyle présentant de 1 à 18 atomes de carbone, un groupe hydroxyle, un groupe carboxyle, un groupe amino représenté par$-NR^{45}R^{46}$, ou un groupe représenté par $-O(CO)O-R^{41}$, $-O(CO)-R^{42}$, $-(CO)O-R^{43}$, ou $-O-R^{44}$, où $R^{41}$, $R^{42}$, $R^{43}$, $R^{44}$, $R^{45}$, et $R^{46}$ représentent chacun indépendamment un groupe hydrocarboné représentant de 1 à 18 atomes de carbone, et

a, b, c, et d représentent chacun indépendamment un entier allant de 0 à 3, a+b est supérieur ou égal à 1, et c+d est supérieur ou égal à 1.

**2.** Composition lipidique selon la revendication 1, dans laquelle les stérols sont du cholestérol ou un dérivé de celui-ci.

**3.** Composition lipidique selon la revendication 1 ou 2, comprenant en outre un lipide présentant une structure polymère hydrophile non ionique.

**4.** Composition lipidique selon la revendication 3, dans laquelle le lipide présentant une structure polymère hydrophile non ionique est un lipide présentant une structure de polyéthylène glycol.

**5.** Composition lipidique selon la revendication 4, dans laquelle le lipide présentant une structure de polyéthylène glycol est un lipide présentant une structure de diacylglycérol et une structure de polyéthylène glycol.

**6.** Composition lipidique selon l'une quelconque des revendications 3 à 5, dans laquelle une teneur du lipide présentant une structure polymère hydrophile non ionique par rapport aux lipides totaux s'étend de 0,2 à 10 % en moles.

**7.** Composition lipidique selon l'une quelconque des revendications 1 à 6, dans laquelle une teneur du premier lipide par rapport aux lipides totaux s'étend de 20 à 55 % en moles.

**8.** Composition lipidique selon l'une quelconque des revendications 1 à 7, dans laquelle une teneur des stérols par rapport aux lipides totaux s'étend de 20 à 70 % en moles.

**9.** Composition lipidique selon l'une quelconque des revendications 1 à 8, dans laquelle une teneur de l'acide nucléique par rapport aux lipides totaux s'étend de 1 % à 25 % en masse.

**10.** Composition lipidique selon l'une quelconque des revendications 1 à 9,

dans laquelle le composé représenté par la Formule (1) est un composé représenté par la Formule (2),

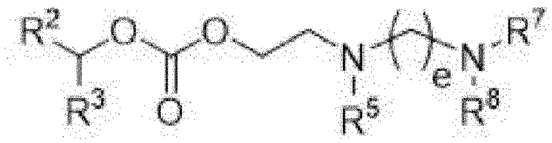 (2)

dans la Formule, $R^2$ et $R^3$ représentent indépendamment un atome d'hydrogène, ou un groupe hydrocarboné présentant de 3 à 24 atomes de carbone, ou un groupe représenté par $R^{31}$-$L^2$-$R^{32}$- ;
où $R^{31}$ représente un groupe hydrocarboné présentant de 1 à 24 atomes de carbone ;
$L^2$ représente -O(CO)O-, -O(CO)-, -(CO)O-, -O-, ou un groupe représenté par la formule suivante,

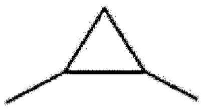

et
$R^{32}$ représente un groupe de liaison hydrocarboné divalent présentant de 1 à 18 atomes de carbone ;
$R^5$ représente un atome d'hydrogène, ou un groupe alkyle présentant de 1 à 18 atomes de carbone, lequel peut être substitué ;
$R^7$ et $R^8$ représentent chacun indépendamment un atome d'hydrogène ou un groupe alkyle représentant de 1 à 18 atomes de carbone, lequel peut être substitué ;
un substituant sur le groupe alkyle présentant de 1 à 18 atomes de carbone, lesquels peuvent être substitués, est un groupe hydroxyle, un groupe carboxyle, un groupe amino représenté par -$NR^{45}R^{46}$, un groupe aryle substitué ou non substitué, un groupe hétéroaryle substitué ou non substitué, ou un groupe représenté par -O(CO)O-$R^{41}$, -O(CO)-$R^{42}$, -(CO)O$R^{43}$, ou-O-$R^{44}$, où $R^{41}$, $R^{42}$, $R^{43}$, $R^{44}$, $R^{45}$, et $R^{46}$ représentent chacun indépendamment un groupe hydrocarboné représentant de 1 à 18 atomes de carbone ;
un substituant sur le groupe aryle substitué ou non substitué et sur le groupe hétéroaryle substitué ou non substitué est un groupe alkyle présentant de 1 à 18 atomes de carbone, un groupe hydroxyle, un groupe carboxyle, un groupe amino représenté par - $NR^{45}R^{46}$, ou un groupe représenté par -O(CO)O-$R^{41}$, -O(CO)-$R^{42}$, -(CO)O-$R^{43}$, ou -O-$R^{44}$, où $R^{41}$, $R^{42}$, $R^{43}$, $R^{44}$, $R^{45}$, et $R^{46}$ représentent chacun indépendamment un groupe hydrocarboné représentant de 1 à 18 atomes de carbone, et
e représente 2 ou 3.

**11.** Composition lipidique selon la revendication 10,

dans laquelle dans la Formule (2),
au moins un élément parmi $R^2$ ou $R^3$ représente un groupe hydrocarboné présentant de 3 à 24 atomes de carbone contenant une ou plusieurs liaisons insaturées ; $R^2$ et $R^3$ représentent chacun indépendamment un groupe représenté par $R^{31}$-$L^2$-$R^{32}$-, ou un élément parmi $R^2$ et $R^3$ représente un groupe représenté par $R^{31}$-$L^2$-$R^{32}$-, et l'autre représente un groupe hydrocarboné présentant de 3 à 24 atomes de carbone,
$R^5$ représente un groupe alkyle non substitué présentant de 1 à 18 atomes de carbone, ou un groupe alkyle représentant de 1 à 18 atomes de carbone substitués avec -O(CO)-$R^{42}$ ou -(CO)O-$R^{43}$ ;
$R^7$ et $R^8$ représentent chacun indépendamment un groupe alkyle représentant de 1 à 4 atomes de carbone, et $R^{31}$, $L^2$, $R^{32}$, $R^{42}$, et $R^{43}$ présentent les mêmes définitions que $R^{31}$, $L^2$, $R^{32}$, $R^{42}$, et $R^{43}$ selon la revendication 10.

**12.** Composition lipidique selon l'une quelconque des revendications 1 à 11, comprenant en outre un support acceptable sur le plan pharmaceutique.

**13.** Composition lipidique selon l'une quelconque des revendications 1 à 12, dans laquelle la composition lipidique est une composition pour introduire des acides nucléiques dans des cellules.

**14.** Composition lipidique selon l'une quelconque des revendications 1 à 12, dans laquelle la composition lipidique est une composition pour une administration in vivo d'acides nucléiques.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2010054401 A
- WO 2010054405 A
- WO 2015095340 A
- US 20130245107 A
- WO 2009127060 A
- WO 2010144740 A
- WO 2016081029 A1
- WO 2016081029 A
- WO 2015005253 A
- JP 5288254 B


### Non-patent literature cited in the description


- **T. W. GREENE et al.** Protective Groups in Organic Synthesis. John Wiley & Sons, INC, 2007, 696-926
- *European Journal of Medicinal Chemistry,* 2016, vol. 109, 134-145
- *Molecular Therapy,* 2009, vol. 17, 878
- *Nature Biotechnology,* 2010, vol. 28, 172-176